(19) Europäisches Patentamt
European Patent Office
Office européen des brevets

(11) EP 2 254 885 B1

(12) **EUROPÄISCHE PATENTSCHRIFT**

(45) Veröffentlichungstag und Bekanntmachung des Hinweises auf die Patenterteilung:
**04.12.2013 Patentblatt 2013/49**

(21) Anmeldenummer: **09721416.7**

(22) Anmeldetag: **10.03.2009**

(51) Int Cl.:
***C07D 403/06*** $^{(2006.01)}$ ***C07D 403/14*** $^{(2006.01)}$
***A61K 31/4166*** $^{(2006.01)}$ ***A61P 35/00*** $^{(2006.01)}$

(86) Internationale Anmeldenummer:
**PCT/EP2009/001714**

(87) Internationale Veröffentlichungsnummer:
**WO 2009/115213 (24.09.2009 Gazette 2009/39)**

(54) **(PYRAZOLYL-CARBONYL) IMIDAZOLIDINONE-DERIVATE ZUR BEHANDLUNG RETROVIRALER ERKRANKUNGEN**

(PYRAZOLYL CARBONYL)IMIDAZOLIDINONE DERIVATIVES FOR THE TREATMENT OF RETROVIRAL DISEASES

DÉRIVÉS DE (PYRAZOLYL-CARBONYL)IMIDAZOLIDINONE POUR LE TRAITEMENT DE MALADIES RÉTROVIRALES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**BA RS**

(30) Priorität: **17.03.2008 DE 102008015033**

(43) Veröffentlichungstag der Anmeldung:
**01.12.2010 Patentblatt 2010/48**

(73) Patentinhaber: **AiCuris GmbH & Co. KG**
**42117 Wuppertal (DE)**

(72) Erfinder:
- **THEDE, Kai**
  **10405 Berlin (DE)**
- **GRESCHAT, Susanne**
  **40237 Düsseldorf (DE)**
- **WILDUM, Steffen**
  **58332 Schwelm (DE)**
- **PAULSEN, Daniela**
  **42105 Wuppertal (DE)**

(74) Vertreter: **Kilger, Ute et al**
**Boehmert & Boehmert**
**Pettenkoferstrasse 20-22**
**80336 München (DE)**

(56) Entgegenhaltungen:
**WO-A-02/100853**
**WO-A-2004/024147**
**WO-A-2004/031178**
**US-A1- 2005 054 707**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue substituierte (Pyrazolylcarbo-nyl)imidazolidinone, Verfahren zu ihrer Herstellung, diese Verbindungen zur Verwendung in der Behandlung und/oder Prophylaxe von Krankheiten sowie ihre Verwendung zur Herstellung von Arzneimitteln zur Behandlung und/oder Prophylaxe von Krankheiten, insbesondere von retroviralen Erkrankungen, bei Menschen und/oder Tieren.

HIV (Virus der humanen Immundefizienz) verursacht eine chronisch-persistente, progrediente Infektion. Die Erkrankung verläuft über verschiedene Stadien von der asymptomatischen Infektion bis zum Krankheitsbild AIDS (*Acquired Immunodeficiency Syndrom*). AIDS ist das finale Stadium der durch Infektion hervorgerufenen Erkrankung. Charakteristisch für die HIV/AIDS-Erkrankung ist die lange klinische Latenzzeit mit persistierender Virämie, die im Endstadium zum Versagen der Immunabwehr führt.

Durch die Einführung der anti-HIV Kombinationstherapie gelang es in den 90-er Jahren, die Krankheitsprogression nachhaltig zu verlangsamen und damit die Lebenserwartung HIV-infizierter Patienten substantiell zu verlängern (Palella et al., N. Engl. J. Med. 1998, 238, 853-860).

Die derzeit auf dem Markt befindlichen anti-HIV-Substanzen hemmen die Replikation des HI-Virus durch Inhibition der essentiellen viralen Enzyme Reverse Transkriptase (RT), Protease oder Integrase oder des Eintritts von HIV in die Zielzelle (Übersicht in Flexner, Nature Reviews Drug Discovery 2007, 6, 959-966). Es existieren zwei Klassen von RT-Inhibitoren: Nukleosidische und nukleotidische RT-Inhibitoren (NRTI) wirken durch kompetitive Inhibition oder Kettenabbruch bei der DNA-Polymerisation. Nicht-nukleosidische RT-Inhibitoren (NNRTI) binden allosterisch an einer hydrophoben Tasche in der Nähe des aktiven Zentrums der RT und vermitteln eine Konformationsänderung des Enzyms. Die derzeit verfügbaren Proteaseinhibitoren (PI) blockieren das aktive Zentrum der viralen Protease und verhindern somit die Reifung neuentstehender Partikel zu infektiösen Virionen. Der einizige derzeit zugelassene Integrase-Inhibitor Raltegravir bindet in das aktive Zentrum der HIV-Integrase und verhindert die Integration der proviralen DNA in das Wirtszellgenom. "Entry-Inhibitoren" (Fusions-Inhibitoren und Korezeptor-Antagonisten) verhindern die HIV-Infektion von Zellen durch Interaktion mit dem HIV-Hüllprotein oder durch Blockierung der zellulären Korezeptoren CCRS oder CXCR4.

Da die Monotherapie mit den momentan verfügbaren anti-HIV-Medikamenten innerhalb sehr kurzer Zeit zum Therapieversagen durch Selektion resistenter Viren führt, erfolgt üblicherweise eine Kombinationstherapie mit mehreren anti-HIV-Substanzen aus verschiedenen Klassen (highly active antiretroviral therapy = HAART; Carpenter et al., J. Am. Med. Assoc. 2000, 283, 381-390).

Trotz der Fortschritte in der antiretroviralen Chemotherapie zeigen neuere Untersuchungen, dass mit den zur Verfügung stehenden Medikamenten eine Eradikation von HIV und damit verbunden eine Heilung der HIV-Infektion nicht zu erwarten ist. Das latente Virus verbleibt in ruhenden Lymphozyten und stellt ein Reservoir für eine Reaktivierung und damit für eine erneute Virusausbreitung dar (Finzi et al., Nature Med. 1999, 5, 512-517; Ramratnam et al., Nature Med. 2000, 6, 82-85). HIVinfizierte Patienten sind daher zeitlebens auf eine effiziente antivirale Therapie angewiesen. Trotz Kombinationstherapie kommt es nach einiger Zeit zur Selektion resistenter Viren. Da für jede therapeutische Klasse charakteristische Resistenzmutationen akkumulieren, bedeutet das Versagen einer Therapie oft einen Wirkverlust der kompletten Substanzklasse. Diese Kreuzresistenzproblematik ist bei der Klasse der NNRTIs am ausgeprägtesten, da hier oft schon eine einzelne Punktmutation in der RT ausreichen kann, um einen Wirkverlust aller NNRTIs zu bewirken (Übersicht in Kavlick & Mitsuya, Antiretroviral Chemotherapy (Hrsg. De Clercq E.), 2001, ASM Press, 279-312).

Begünstigt wird die Entstehung von Resistenzen meist durch die schlechte Compliance der Patienten, die durch ein ungünstiges Nebenwirkungsprofil und kompliziertes Dosierungsschema der anti-HIV-Medikamente hervorgerufen wird.

Somit besteht ein dringender Bedarf nach neuen therapeutischen Optionen zur Bekämpfung der HIV-Infektion. Dazu ist es ein vordringliches Ziel der Therapieforschung zu HIV, neue chemische Leitstrukturen zu identifizieren, die entweder ein neues Target in der Vermehrung des HIV adressieren und/oder gegen die wachsende Zahl resistenter klinischer HIV-Isolate wirksam sind.

US 5,624,941 und EP 576357 beschreiben Pyrazole als Cannabinoid-Rezeptor-Antagonisten, EP 418845, EP 554829 und WO 04/050632 unter anderem zur Behandlung von inflammatorischen und thrombotischen Erkrankungen, WO 03/037274 als Natriumionen-Kanal-Inhibitoren zur Behandlung von Schmerz, WO 06/015860 als Adenosin-Rezeptor-Liganden zur Behandlung von inflammatorischen und obstruktiven Atemwegserkrankungen, EP 1762568 und EP 1591443 als Inhibitoren der Plättchenaggregation, WO 07/002559 als Modulatoren der Aktivität von Nuklearrezeptoren, WO 07/020388 und WO 05/080343 als Cannabinoid-Rezeptor-Modulatoren unter anderem zur Behandlung von Fettsucht und psychiatrischen und neurologischen Störungen, WO 07/009701 und EP 1743637 zur Behandlung kardiovaskulärer Risikofaktoren, WO 2005/002576 als Inhibitoren verschiedener Kinasen und DE 10 2004 054 666 zur Bekämpfung von Schadpflanzen oder zur Wachstumsregulierung von Pflanzen.

US 2005/054707 A1, WO 2004/024147 A, WO 02/100853 A und WO 2004/031178 A beschreiben verschiedene Pyrazolderivate zur Behandlung von HIV-Infektionen. Eine Aufgabe der vorliegenden Erfindung ist es daher, neue

Verbindungen mit gleicher öder verbesserter antiviraler Wirkung zur Behandlung von viralen Infektionskrankheiten bei Menschen und Tieren zur Verfügung zu stellen, die die zuvor beschriebenen Nachteile nicht aufweisen.

Überraschenderweise wurde gefunden, dass die in der vorliegenden Erfindung beschriebenen substituierten (Pyrazolyl-carbonyl)imidazolidinone antiviral wirksam sind.

Gegenstand der Erfindung sind Verbindungen der Formel

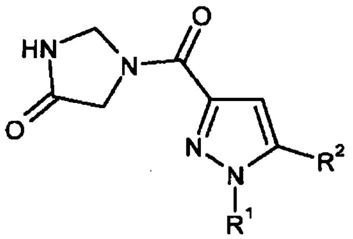

(I),

in welcher

$R^1$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, ($C_1$-$C_4$)-Alkyl und ($C_1$-$C_4$)-Alkoxy,
worin
($C_1$-$C_4$)-Alkyl und ($C_1$-$C_4$)-Alkoxy ihrerseits ein- bis dreifach, gleich oder verschieden, mit Resten ausgewählt aus der Reihe Halogen, Cyano, Hydroxy, ($C_1$-$C_4$)-Alkoxy, Amino, Mono-($C_1$-$C_4$)-alkylamino, Di-($C_1$-$C_4$)-alkylamino, ($C_3$-$C_7$)-Cycloalkyl und 4- bis 7-gliedriges Heterocyclyl substituiert sein können,
wobei die letztgenannten Cycloalkyl- und Heterocyclyl-Reste ihrerseits jeweils bis zu dreifach, gleich oder verschieden, mit Halogen, Cyano, ($C_1$-$C_4$)-Alkyl, Trifluormethyl, Hydroxy, ($C_1$-$C_4$)-Alkoxy, Trifluormethoxy, Oxo, Amino, Mono-($C_1$-$C_4$)-alkylamino und Di-($C_1$-$C_4$)-alkylamino substituiert sein können,

$R^2$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, ($C_1$-$C_4$)-Alkyl und ($C_1$-$C_4$)-Alkoxy,
worin
($C_1$-$C_4$)-Alkyl und ($C_1$-$C_4$)-Alkoxy ihrerseits ein- bis dreifach, gleich oder verschieden, mit Resten ausgewählt aus der Reihe Halogen, Cyano, Hydroxy, ($C_1$-$C_4$)-Alkoxy, Amino, Mono-($C_1$-$C_4$)-alkylamino, Di-($C_1$-$C_4$)-alkylamino, ($C_3$-$C_7$)-Cycloalkyl und 4- bis 7-gliedriges Heterocyclyl substituiert sein können,
wobei die letztgenannten Cycloalkyl- und Heterocyclyl-Reste ihrerseits jeweils bis zu dreifach, gleich oder verschieden, mit Halogen, Cyano, ($C_1$-$C_4$)-Alkyl; Trifluormethyl, Hydroxy, ($C_1$-$C_4$)-Alkoxy, Trifluormethoxy, Oxo, Amino, Mono-($C_1$-$C_4$)-alkylamino und Di-($C_1$-$C_4$)-alkylamino substituiert sein können,

und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb auch die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als <u>Salze</u> sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind, aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von

Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl sowie die Alkylteile in Alkoxy und Alkoxycarbonyl stehen für geradkettiges oder verzweigtes Alkyl und umfassen, wenn nicht anders angegeben, $(C_1-C_6)$-Alkyl, insbesondere $(C_1-C_4)$-Alkyl, wie z.B. Methyl, Ethyl, Propyl, Isopropyl, Butyl, Isobutyl, *t*-Butyl.

Alkoxy steht im Rahmen der Erfindung vorzugsweise für einen geradkettigen oder verzweigten Alkoxyrest insbesondere mit 1 bis 6, 1 bis 4 bzw. 1 bis 3 Kohlenstoffatomen. Bevorzugt ist ein geradkettiger oder verzweigter Alkoxyrest mit 1 bis 3 Kohlenstoffatomen. Beispielhaft und vorzugsweise seien genannt: Methoxy, Ethoxy, n-Propoxy, Isopropoxy, *t*-Butoxy, *n*-Pentoxy und *n*-Hexoxy.

Alkoxycarbonyl steht beispielhaft und vorzugsweise für Methoxycarbonyl, Ethoxycarbonyl, *n*-Propoxycarbonyl, Isopropoxycarbonyl, *t*-Butoxycarbonyl, *n*-Pentoxycarbonyl und *n*-Hexoxycarbonyl.

Heterocyclyl steht für einen monocyclischen, heterocyclischen Rest mit 4 bis 7, vorzugsweise 5 bis 6 Ringatomen und bis zu 3, vorzugsweise bis zu 2 Heteroatomen und/oder Heterogruppen aus der Reihe N, O, S, SO, $SO_2$, wobei ein Stickstoffatom auch ein N-Oxid bilden kann. Der Heterocyclus kann gesättigt oder teilweise ungesättigt sein. Bevorzugt sind 5- bis 7-gliedrige, monocyclische gesättigte Heterocyclen mit bis zu zwei Heteroatomen aus der Reihe O, N und S, beispielhaft und vorzugsweise 1,4-Oxazepanyl, Pyrrolidin-1-yl, Pyrrolidin-2-yl, Pyrrolidin-3-yl, Tetrahydrofuranyl, Tetrahydrothienyl, Pyranyl, 1,3-Thiazolidinyl, Piperidin-1-yl, Piperidin-2-yl, Piperidin-3-yl, Piperidin-4-yl, Thiopyranyl, Morpholin-2-yl, Morpholin-3-yl, Morpholin-4-yl, Thiomorpholin-2-yl, Thiomorpholin-3-yl, Thiomorpholin-4-yl, Perhydroazepinyl, Piperazin-1-yl, Piperazin-2-yl.

Halogen steht für Fluor, Chlor, Brom oder Jod, wobei Fluor und Chlor bevorzugt sind, wenn nichts anderes angegeben ist.

Mono-$(C_1-C_4)$-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, *n*-Propylamino, Isopropylamino, *n*-Butylamino, *tert*.-Butylamino, *n*-Pentylamino und *n*-Hexylamino.

Di-$(C_1-C_4)$-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N*-Dimethylamino, *N,N*-Diethylamino, *N*-Ethyl-*N*-methylamino, *N*-Methyl-*N*-*n*-propylamino, *N*-Isopropyl-*N*-*n*-propylamino, *N*,*N*-Diisopropylamino, *N*-*n*-Butyl-*N*-methylamino, *N*-*tert*.-Butyl-*N*-methylamino, *N*-Methyl-*N*-*n*-pentylamino und *N*-*n*-Hexyl-*N*-methylamino.

$(C_3-C_7)$-Cycloalkyl steht im Rahmen der Erfindung für einen monocyclischen, gesättigten Carbocyclus mit 3 bis 7 bzw. 3 bis 6 Ring-Kohlenstoffatomen. Beispielhaft und vorzugsweise seien genannt: Cyclopropyl, Cyclobutyl, Cyclopentyl, Cyclohexyl und Cycloheptyl.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen angegebenen Restedefinitionen werden unabhängig von den jeweilig angegebenen Kombinationen der Reste beliebig auch durch Restedefinitionen anderer Kombinationen ersetzt.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und $(C_1-C_4)$-Alkoxy,

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethoxy, Trifluormethylthio, $(C_1-C_4)$-Alkyl und$(C_1-C_4)$-Alkoxy,
worin
$(C_1-C_4)$-Alkoxy seinerseits ein- bis dreifach, gleich oder verschieden, mit Resten ausgewählt aus der Reihe Halogen, Cyano, Hydroxy, $(C_1-C_4)$-Alkoxy, Amino, Mono-$(C_1-C_4)$-alkylamino, Di-$(C_1-C_4)$-alkylamino, $(C_3-C_7)$-Cycloalkyl und 4- bis 7-gliedriges Heterocyclyl substituiert sein kann,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkylamino und Di-$(C_1-C_4)$-alkylamino substituiert sein können,

und ihre Salze, ihre Solvate und die Solvate ihrer Salze.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl und Methoxy,

$R^2$ für Phenyl steht,
wobei Phehyl substituiert ist mit 1 bis 2 Substituenten, wobei die Substituenten unabhängig voneinander ausgewählt werden aus der Gruppe bestehend aus Halogen, Cyano, Trifluormethoxy, Methyl und $(C_1-C_3)$-Alkoxy,
worin
$(C_1-C_3)$-Alkoxy seinerseits ein- bis dreifach, gleich oder verschieden, mit Resten ausgewählt aus der Reihe Hydroxy, $(C_1-C_4)$-Alkoxy, Amino, Mono-$(C_1-C_4)$-alkylamino, Di-$(C_1-C_4)$-alkylamino und 4- bis 7-gliedriges Heterocyclyl substituiert sein kann,
wobei die letztgenannten Heterocyclyl-Reste ihrerseits jeweils mit $(C_1-C_4)$-Alkyl substituiert sein können,

und ihre Salze, ihre Solvate und die Solvate ihrer Salze.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyanö, Trifluormethyl und Methoxy,

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethoxy, Methyl und Methoxy,

und ihre Salze, ihre Solvate und die Solvate ihrer Salze.

Gegenstand der Erfindung sind auch Verbindungen der Formel (I), in welcher

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano,

$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano

und ihre Salze, ihre Solvate und die Solvate ihrer Salze.

Gegenstand der Erfindung sind auch Verbindungen der Formel

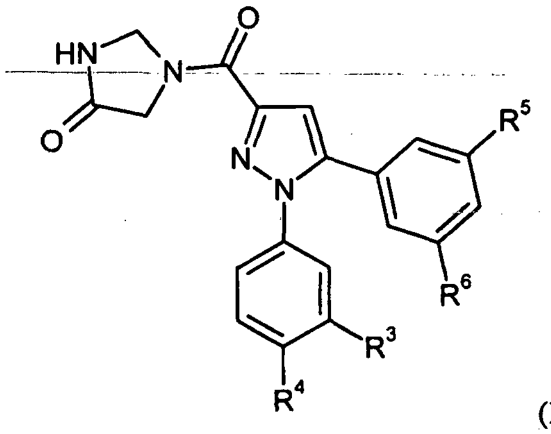

(Ia),

in welcher

$R^3$ für Wasserstoff, Halogen, Hydroxy, Cyano, Nitro, Trifluormethyl, Trifluormethoxy, Trifluormethylthio, $(C_1-C_4)$-Alkyl oder $(C_1-C_4)$-Alkoxy steht,

$R^4$ für Wasserstoff oder Halogen steht,

$R^5$ für Halogen, Hydroxy, Cyano, Nitro, Trifluormethyl, Trifluormethoxy, Trifluormethylthio, $(C_1-C_4)$-Alkyl oder $(C_1-C_4)$-Alkoxy steht,
worin
$(C_1-C_4)$-Alkoxy seinerseits ein- bis dreifach, gleich oder verschieden, mit Resten ausgewählt aus der Reihe Halogen, Cyano, Hydroxy, $(C_1-C_4)$-Alkoxy, Amino, Mono-$(C_1-C_4)$-alkylamino, Di-$(C_1-C_4)$-alkylamino, $(C_3-C_7)$-Cycloalkyl und 4- bis 7-gliedriges Heterocyclyl substituiert sein kann,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkylamino und Di-$(C_1-C_4)$-alkylamino substituiert sein können,

$R^6$ für Wasserstoff oder Halogen steht,

und ihre Salze, ihre Solvate und die Solvate ihrer Salze.

Gegenstand der Erfindung sind auch Verbindungen der Formel (Ia) in welchen

$R^3$ für Halogen, Hydroxy, Cyano, Nitro, Trifluormethyl, Trifluormethoxy, Trifluormethylthio, $(C_1-C_4)$-Alkyl oder $(C_1-C_4)$-Alkoxy steht,

$R^4$ für Wasserstoff oder Halogen steht,

$R^5$ für Halogen, Hydroxy, Cyano, Nitro, Trifluormethoxy, Trifluormethylthio, $(C_1-C_4)$-Alkyl oder $(C_1-C_4)$-Alkoxy steht,
worin
$(C_1-C_4)$-Alkoxy seinerseits ein- bis dreifach, gleich oder verschieden, mit Resten ausgewählt aus der Reihe Halogen, Cyano, Hydroxy, $(C_1-C_4)$-Alkoxy, Amino, Mono-$(C_1-C_4)$-alkylamino, Di-$(C_1-C_4)$-alkylamino, $(C_3-C_7)$-Cycloalkyl und 4- bis 7-gliedriges Heterocyclyl substituiert sein kann,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkylamino und Di-$(C_1-C_4)$-alkylamino substituiert sein können,

$R^6$ für Wasserstoff oder Halogen steht,

und ihre Salze, ihre Solvate und die Solvate ihrer Salze.

Gegenstand der Erfindung sind auch Verbindungen der Formel (Ia), in welcher

$R^3$ für Halogen, Cyano, Trifluormethyl oder Methoxy steht,

$R^4$ für Wasserstoff oder Halogen steht,

$R^5$ für Halogen, Cyano, Trifluormethoxy, Methyl oder ($C_1$-$C_3$)-Alkoxy steht,

worin ($C_1$-$C_3$)-Alkoxy seinerseits ein- bis dreifach, gleich oder verschieden, mit Resten ausgewählt aus der Reihe Hydroxy, ($C_1$-$C_4$)-Alkoxy, Amino, Mono-($C_1$-$C_4$)-alkylamino, Di-($C_1$-$C_4$)-alkylamino und 4- bis 7-gliedriges Heterocyclyl substituiert sein kann,
wobei die letztgenannten Heterocyclyl-Reste ihrerseits jeweils mit ($C_1$-$C_4$)-Alkyl substituiert sein können,

$R^6$ für Wasserstoff oder Halogen steht,

und ihre Salze, ihre Solvate und die Solvate ihrer Salze.

Gegenstand der Erfindung sind auch Verbindungen der Formel (Ia), in welcher

$R^3$ für Halogen, Cyano, Trifluormethyl oder Methoxy steht,
$R^4$ für Wasserstoff, Chlor oder Fluor steht,
$R^5$ für Halogen, Cyano, Trifluormethoxy, Methyl oder Methoxy steht,
$R^6$ für Wasserstoff, Chlor oder Fluor steht,

und ihre Salze, ihre Solvate und die Solvate ihrer Salze.

Gegenstand der Erfindung sind auch Verbindungen der Formel (Ia), in welcher

$R^3$ für Halogen oder Cyano steht,
$R^4$ für Wasserstoff, oder Fluor steht,
$R^5$ für Halogen oder Cyano steht,
$R^6$ für Wasserstoff oder Fluor steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,
$R^4$ für Fluor steht,
$R^5$ für Chlor oder Cyano steht,
$R^6$ für Fluor steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,
$R^4$ für Fluor steht,
$R^5$ für Chlor oder Cyano steht,
$R^6$ für Wasserstoff steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,
$R^4$ für Wasserstoff steht,
$R^5$ für Chlor oder Cyano steht,
$R^6$ für Wasserstoff steht,

und ihre Salze, ihre Solvate und die Solvate ihrer Salze.

Gegenstand der Erfindung sind auch Verbindungen der Formel (Ia), in welcher

$R^3$ für Chlor oder Cyano steht,
$R^4$ für Wasserstoff steht,
$R^5$ für Chlor oder Cyano steht,
$R^6$ für Fluor steht,

und ihre Salze, ihre Solvate und die Solvate ihrer Salze.

Gegenstand der Erfindung sind auch Verbindungen der Formel (Ia), in welcher

$R^3$ für Halogen, Cyano, Trifluormethyl oder Methoxy steht,

$R^4$ für Wasserstoff oder Halogen steht,

$R^5$ für Trifluormethyl steht,

$R^6$ für Fluor steht,

und ihre Salze, ihre Solvate und die Solvate ihrer Salze.

Gegenstand der Erfindung sind auch Verbindungen der Formel (Ia), in welcher

$R^3$ für Wasserstoff steht,

$R^4$ für Fluor oder Chlor steht,

$R^5$ für Halogen, Cyano, Trifluormethoxy, Methyl oder Methoxy steht,

$R^6$ für Wasserstoff oder Halogen steht,

und ihre Salze, ihre Solvate und die Solvate ihrer Salze.

Gegenstand der Erfindung ist weiterhin ein Verfahren zur Herstellung der Verbindungen der Formel (I) und (Ia), wobei Verbindungen der Formel

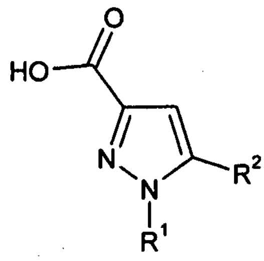

(II),

in welcher
$R^1$ und $R^2$ die oben angegebene Bedeutung aufweisen,
mit Imidazolidin-4-on oder einem Salz von Imidazolidin-4-on umgesetzt werden.

Die Umsetzung erfolgt im Allgemeinen in inerten Lösungsmitteln, in Gegenwart eines Dehydratisierungsreagenzes, gegebenenfalls in Gegenwart einer Base, bevorzugt in einem Temperaturbereich von -30 °C bis 50 °C bei Normaldruck.

Inerte Lösungsmittel sind beispielsweise Halogenkohlenwasserstoffe wie Dichlormethan oder Trichlormethan, Kohlenwasserstoffe wie Benzol oder Toluol, Nitromethan, Tetrahydrofuran, 1,4-Dioxan, Dimethylformamid oder Acetonitril. Ebenso ist es möglich, Gemische der Lösemittel einzusetzen. Besonders bevorzugt sind Dichlormethan, Dimethylformamid, Tetrahydrofuran oder Toluol.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Als Dehydratisierungsreagenzien eignen sich hierbei beispielsweise Carbodiimide wie z.B. *N,N'*-Diethyl-, *N,N,'*-Dipropyl-, *N,N'*-Diisopropyl-, *N,N,'*-Dicyclohexylcarbodiimid, *N*-(3-Dimethylaminoisopropyl)-*N'*-ethylcarbodiimid-hydrochlorid (EDC), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid, oder *O*-(Benzotriazol-1-yl)-*N,N,N',N'*-tetramethyl-uroniumhexafluorophosphat (HBTU), 2-(2-Oxo-1-(2H)-pyridyl)-1,1,3,3-tetramethyl-uroniumtetrafluoroborat (TPTU) oder *O*-(7-Azabenzotriazol-1-yl)-*N,N,N'N'*-tetramethyluroniumhexafluorophosphat (HATU), oder 1-Hydroxybenzotriazol (HOBt), oder Benzotriazol-1-yloxytris(dimethylamino)-phosphoniumhexafluorophosphat (BOP), oder Benzotriazol-1-yloxytris(pyrrolidino)-phosphoniumhexafluorophosphat (PyBOP), oder *N*-Hydroxysuccinimid, oder Mischungen aus diesen, mit Basen.

Vorzugsweise wird die Kondensation mit PyBOP, TBTU oder mit EDC in Gegenwart von HOBt durchgeführt.

In einem alternativen Verfahren können die Verbindungen der Formel (II) zunächst mit Thionylchlorid und in der zweiten Stufe mit Imidazolidin-4-on oder einem Salz von Imidazolidin-4-on in Gegenwart einer Base, wie z. B. Triethylamin, umgesetzt werden.

Die nach den oben angegebenen Verfahren hergestellten Verbindungen der Formel (I) und (Ia) tragen gegebenenfalls Schutzgruppen, die nach dem Fachmann bekannten Bedingungen abgespalten werden können, um weitere Verbindungen der Formel (I) und (Ia) zu erhalten.

Die Verbindungen der Formel (II) sind bekannt oder können hergestellt werden, indem in Verbindungen der Formel

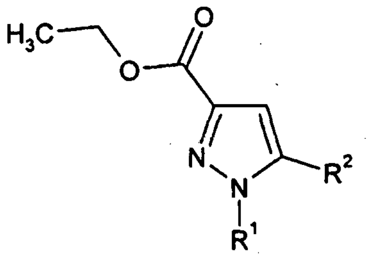

(III),

in welcher

$R^1$ und $R^2$ die oben angegebene Bedeutung aufweisen,

der Ester mit einer Base verseift wird.

Die Verseifung des Esters mit einer Base erfolgt im Allgemeinen in inerten Lösungsmitteln, bevorzugt in einem Temperaturbereich von Raumtemperatur bis zum Rückfluss des Lösungsmittels bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt sind Lithium-, Kalium- oder Natriumhydroxid.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-*tert*-butylether, 1,2-Dimethoxyethan, 1,4-Dioxan, Tetrahydrofuran, Glykoldimethylether oder Diethylenglykoldimethylether, Alkohole wie Methanol, Ethanol, n-Propanol, iso-Propanol, n-Butanol oder *tert*-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Dimethylsulfoxid, Acetonitril oder Pyridin, oder Wasser, oder Gemische von Lösungsmitteln. Als Lösungsmittel sind bevorzugt 1,4-Dioxan, Tetrahydrofuran und/oder Methanol. Bevorzugt ist Lithiumhydroxid in Tetrahydrofuran- oder 1,4-Dioxan-Wasser-Gemischen oder Kaliumhydroxid in Methanol.

Die Verbindungen der Formel (III) sind bekannt oder können hergestellt werden, indem in der ersten Stufe Verbindungen der Formel

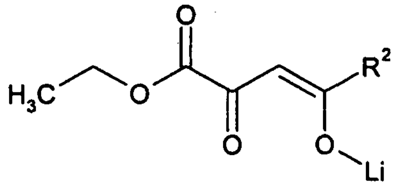

(IV),

in welcher

$R^2$ die oben angegebene Bedeutung aufweist,

mit Verbindungen der Formel

$R^1$-NH-$NH_2$ (V),

oder einem Salz der Verbindungen der Formel (V),
in welcher
$R^1$ die oben angegebene Bedeutung aufweist,
umgesetzt werden und in der zweiten Stufe in Essigsäure erhitzt wird.

Die Umsetzung der ersten Stufe erfolgt im Allgemeinen in inerten Lösungsmitteln, bevorzugt in einem Temperaturbereich von Raumtemperatur bis zum Rückfluss des Lösungsmittels bei Normaldruck.

Inerte Lösungsmittel sind beispielsweise Alkohole wie Methanol, Ethanol, n-Propanol, iso-Propanol, n-Butanol, *tert*-Butanol oder 2-Methoxyethanol, bevorzugt ist Ethanol.

Die Umsetzung der zweiten Stufe in Essigsäure erfolgt im Allgemeinen in einem Temperaturbereich von Raumtemperatur bis zum Rückfluss der Essigsäure bei Normaldruck. Die Reaktion kann auch in Methanol, Ethanol oder Dioxan in einem Temperaturbereich von Raumtemperatur bis zum Rückfluss der Lösungsmittel durchgeführt werden. Geeignet sind Mischungen von Methanol, Ethanol oder Dioxan mit Essigsäure im Volumenverhältnis von 0.5/99.5 bis 99.5/0.5. Auch können Mischungen von Methanol, Ethanol, Dioxan oder Essigsäure mit anderen Säuren wie z.B. Salzsäure, Methansulfonsäure, p-Toluolsulfonsäure, Camphersulfonsäure oder Trifluoressigsäure unter den genannten Bedingungen eingesetzt werden. Bevorzugt wird die Umsetzung in Essigsäure unter Rückfluss durchgeführt.

Alternativ können die Verbindungen der Formel (III) hergestellt werden, indem Verbindungen der Formel

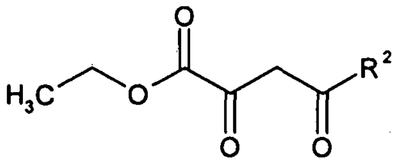

(VI),

in welcher $R^2$ die oben angegebene Bedeutung aufweist mit Verbindungen der Formel (V) umgesetzt werden.

Die Umsetzung erfolgt im Allgemeinen in inerten Lösungsmitteln, bevorzugt in einem Temperaturbereich von Raumtemperatur bis zum Rückfluss des Lösungsmittels bei Normaldruck, ggf. in Gegenwart einer Säure.

Inerte Lösungsmittel sind bspw. Alkohole wie Methanol, Ethanol, n-Propanol, Isopropanol, n-Butanol, *tert*-Butanol oder 2-Methoxyethanol, oder andere Lösungsmittel wie *N,N*-Dimethylformamid, *N,N*-Dimethylacetamid oder Dimethylsulfoxid.

Erfolgt die Reaktion in Gegenwart einer Säure, kann diese von Anfang an der Reaktionslösung zugesetzt werden oder nach einer Zeit von 1 bis 4 Stunden zugegeben werden, wobei bei der späteren Zugabe der Säure die Reaktionslösung ggf. auf eine Temperatur bis zum Rückfluss des Lösemittels erwärmt wird.

Die Säure ist bspw. eine konzentrierte Mineralsäure oder eine konzentrierte Carbonsäure wie bspw. konzentrierte Salzsäure, konzentrierte Salpetersäure, konzentrierte Schwefelsäure oder konzentrierte Essigsäure.

Die Verbindungen der Formeln (IV), (V) und (VI) sind bekannt oder lassen sich nach bekannten Verfahren aus den entsprechenden Edukten synthetisieren.

Die Herstellung der erfindungsgemäßen Verbindungen kann durch das folgende Syntheseschema beispielhaft verdeutlicht werden.

<u>Syntheseschema:</u>

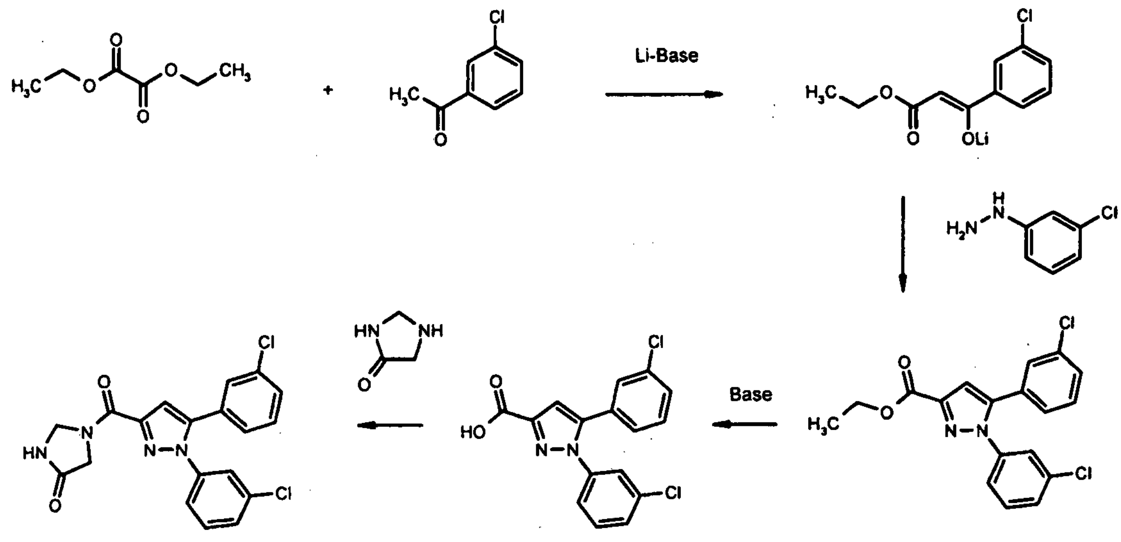


Die erfindungsgemäßen Verbindungen zeigen ein nicht vorhersehbares, wertvolles pharmakologisches Wirkspektrum.

Sie eignen sich daher zur Verwendung als Arzneimittel zur Behandlung und/oder Prophylaxe von Krankheiten bei Menschen und Tieren.

Die Verbindungen der vorliegenden Erfindung zeichnen sich insbesondere durch ein vorteilhaftes anti-retrovirales Wirkspektrum aus.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer therapeutisch wirksamen Menge der erfindungsgemäßen Verbindungen.

Als Indikationsgebiete in der Humanmedizin können beispielsweise genannt werden:

1.) Die Behandlung und Prophylaxe von menschlichen Retrovirusinfektionen

2.) Die Behandlung und Prophylaxe von durch HIV-1 (Virus der humanen Immundefizienz; früher HTLV III / LAV genannt) und HIV-2 verursachten Infektionen und Erkrankungen (AIDS) und den damit assoziierten Stadien wie ARC (AIDS related complex) und LAS (Lymphadenopathie-Syndrom) sowie der durch dieses Virus verursachten Immunschwäche und Enzephalopathie.

3.) Die Behandlung von HIV-Infektionen hervorgerufen durch einfach-, mehrfach- oder multi-resistente HI-Viren.

Der Ausdruck resistente HI-Viren bedeutet z.B. Viren mit Resistenzen gegen nukleosidische Inhibitoren (NRTI), nicht-nukleosidische Inhibitoren. (NNRTI) oder Protease-inhibitoren (PI) oder Viren mit Resistenzen gegen andere Wirkprinzipien, z.B. T20 (Fusionsinhibitoren).

4.) Die Behandlung oder die Prophylaxe des AIDS-carrier Zustandes (AIDS-Überträger-Zustand).

5.) Die Behandlung oder die Prophylaxe einer HTLV-I oder HTLV-II Infektion.

Als Indikationen in der Tiermedizin können beispielsweise angeführt werden:

Infektionen mit

a) Maedivisna (bei Schafen und Ziegen)

b) progressivem Pneumonievirus (PPV) (bei Schafen und Ziegen)

c) caprine arthritis encephalitis Virus (bei Schafen und Ziegen)

d) Zwoegerziekte Virus (bei Schafen)

e) infektiösem Virus der Anämie (des Pferdes)

f) Infektionen verursacht durch das Katzenleukämievirus

g) Infektionen verursacht durch das Virus der Katzen-Immundefizienz (FIV)

h) Infektionen verursacht durch das Virus der Affen-Immundefizienz (SIV)

Bevorzugt werden aus dem Indikationsgebiet in der Humanmedizin die oben aufgeführten Punkte. 2, 3 und 4.

Insbesondere geeignet sind die Substanzen zur Bekämpfung von HI-Viren, die Resistenzen gegen bekannte nicht-nukleosidische Inhibitoren der reversen Transkripatse, wie z.B. Efavirenz oder Nevirapin, zeigen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.

Die erfindungsgemäßen Verbindungen können, insbesondere in den oben aufgeführten Punkten 2, 3 und 4, auch vorteilhaft als Bestandteile einer Kombinationstherapie mit einer oder mehreren anderen, in diesen Anwendungsbereichen aktiven Verbindungen eingesetzt werden. Beispielhaft können diese Verbindungen in Kombination mit wirksamen Dosen von antiviral wirksamen Substanzen, die auf den unten aufgeführten Wirkprinzipien beruhen, eingesetzt werden:

Inhibitoren der HIV Protease; beispielhaft seien genannt: Saquinavir, Indinavir, Ritonavir, Nelfinavir, Amprenavir, Lopinavir, Atazanavir, Fosamprenavir, Tipranavir, Darunavir;

Nukleosidische, nukleotidische und nicht-nukleosidische Inhibitoren der HIV Reversen Transkriptase; beispielhaft seien genannt: Zidovudin, Lamivudin, Didanosin, Zalcitabin, Stavudin, Lamivudin, Abacavir, Tenofovir, Adefovir, Emtricitabin, Amdoxovir, Apricitabin, Racivir, Nevirapin, Delavirdin, Efavirenz, Etravirin, Rilpivirin, UK-453,061;

Inhibitoren der HIV Integrase, beispielhaft seien genannt: Raltegravir, Elvitegravir;

Inhibitoren der HIV Fusion; beispielhaft sei genannt: Enfuvirtid;

Inhibitioren der CXCR4/CCR5/gp120 Wechselwirkung; beispielhaft seien genannt: Maraviroc, Vicriviroc, INCB009471, AMD-070;

Inhibitioren der Polyproteinreifung; beispielhaft sei genannt: Bevirimat.

Diese Auswahl soll zur Verdeutlichung der Kombinationsmöglichkeiten, nicht jedoch zur Einschränkung auf die hier aufgeführten Beispiele dienen. Prinzipiell ist jede Kombination der erfindungsgemäßen Verbindungen mit antiviral wirksamen Substanzen als im Rahmen der Erfindung zu betrachten.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie be beispie-sweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidanzien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich sowohl in der Human als auch in der Veterinärmedizin als vorteilhaft erwiesen, den oder die erfindungsgemäßen Wirkstoffe in Gesamtmengen von 0.1 bis 200 mg/kg, vorzugsweise 1 bis 100 mg/kg Körpergewicht je 24 Stunden, gegebenenfalls in Form mehrerer Einzelgaben, zur Erzielung des gewünschten Ergebnisses zu verabreichen. Eine Einzelgabe enthält den oder die Wirkstoffe vorzugsweise in Mengen von 1 bis 80 mg/kg, insbesondere 1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10 % w/v": 100 ml Lösung oder Suspension enthalten 10 g Substanz.

## A) <u>Beispiele</u>

**<u>Abkürzungen</u>:**

| | |
|---|---|
| aq. | wässrig, wässrige Lösung |
| DCI | direkte chemische Ionisation (bei MS) |
| DMA | *N,N*-Dimethylacetamid |
| DMF | *N,N*-Dimethylformamid |
| DMSO | Dimethylsulfoxid |
| d. Th. | der Theorie (bei Ausbeute) |
| EDC | *N'*-(3-Dimethylaminopropyl)-N-ethylcarbodiimid x HCl |
| eq. | Äquivalent(e) |
| ESI | Elektrospray-Ionisation (bei MS) |
| h | Stunde(n) |
| HATU | O-(7-Azabenzotriazol-1-yl)-*N,N,N',N'*-tetramethyluronium-Hexafluorophosphat |
| HPLC | Hochdruck-, Hochleistungsflüssigchromatographie |
| i.Vak. | im Vakuum |
| konz. | Konzentriert |
| LC-MS | Flüssigchromatographie-gekoppelte Massenspektroskopie |
| min | Minute(n) |
| MS | Massenspektroskopie |
| NMR | Kernresonanzspektroskopie |
| PyBOP | Benzotriazol-1-yloxy-tris(pyrrolidino)phosphonium-Hexafluorophosphat |
| $R_t$ | Retentionszeit (bei HPLC) |
| RT | Raumtemperatur |

TBTU *O*-(Benzotriazol-1-yl)-*N,N,N',N'*-tetramethyluronium-Tetrafluoroborat
TFA Trifluoressigsäure
THF Tetrahydrofuran
TMOF Trimethylorthoformiat

**LC-MS/GC-MS-Methoden:**

Methode 1:

Gerätetyp MS: Micromass ZQ; Gerätetyp HPLC: HP 1100 Series; UV DAD; Säule: Phenomenex Gemini 3μ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

Methode 2:

Instrument: Micromass Quattro LCZ mit HPLC Agilent Serie 1100; Säule: Phenomenex Synergi 2μ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

Methode 3:

Gerätetyp MS: Micromass ZQ; Gerätetyp HPLC: Waters Alliance 2795; Säule: Phenomenex Synergi 2μ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

Methode 4:

Instrument: Micromass Quattro LCZ mit HPLC Agilent Serie 1100; Säule: Phenomenex Onyx Monolithic C18, 100 mm x 3 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 208-400 nm.

Methode 5:

Instrument: Micromass QuattroPremier mit Waters UPLC Acquity; Säule: Thermo Hypersil GOLD 1.9μ 50 mm x 1mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 1.5 min 10%A → 2.2 min 10%A; Ofen: 50°C; Fluss: 0.33 ml/min; UV-Detektion: 210 nm.

Methode 6:

Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 210 nm.

Methode 7:

Gerätetyp MS: Micromass ZQ; Gerätetyp HPLC: Waters Alliance 2795; Säule: Phenomenex Synergi 2.5 μ MAX-RP 100A Mercury 20 mm x 4mm; Eluent A: 11 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 3.0 min 5%A → 4.0 min 5%A → 4.01 min 90%A; Fluss: 2 ml/min; Ofen: 50°C; UV-Detektion: 210 nm.

Methode 8:

Instrument: Micromass Quattro LCZ mit HPLC Agilent Serie 1100; Säule: Phenomenex Gemini 3μ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208-400 nm.

Methode 9:

Instrument: Micromass Quattro LCZ mit HPLC Agilent Serie 1100; Säule: Phenomenex Synergi 2.5 μ MAX-RP 100A Mercury 20 mm x 4 mm; Eluent A: 1 1 Wasser + 0.5 ml 50%ige Ameisensäure, Eluent B: 1-1 Acetonitril + 0.5 ml 50%ige Ameisensäure; Gradient: 0.0 min 90%A → 0.1 min 90%A 3.0 min 5%A → 4.0 min 5%A → 4.1 min 90%A; Fluss: 2 ml/min; Ofen: 50°C; UV-Detektion: 208-400 nm.

Methode 10:

Gerätetyp MS: Waters (Micromass) Quattro Micro; Gerätetyp HPLC: Agilent 1100 Serie; Säule: Thermo Hypersil GOLD 3μ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100%A → 3.0 min 10%A → 4.0 min 10%A → 4.01 min 100%A (Fluss 2.5ml/min) → 5.00 min 100%A; Ofen: 50°C; Fluss: 2 ml/min; UV-Detektion: 210 nm.

Methode 11:

Instrument: Micromass GCT, GC6890; Säule: Restek RTX-35, 15 m x 200 μm x 0.33 μm; konstanter Fluss mit Helium: 0.88 ml/min; Ofen: 70°C; Inlet: 250°C; Gradient: 70°C, 30°C/min → 310°C (3 min halten).

Methode 12:

Instrument: Waters ACQUITY SQD UPLC System; Säule: Waters Acquity UPLC HSS T3 1.8 μ 50 x 1 mm; Eluent A: 1 1 Wasser + 0.25 ml 99%ige Ameisensäure , Eluent B: 1 1 Acetonitril + 0.25 ml 99%ige Ameisensäure; Gradient: 0.0 min 90% → 1.2 min 5% A → 2.0 min 5% A; Ofen: 50 °C; Fluss: 0.40 ml/min; UV-Detektion: 210 - 400 nm.

**Ausgangsverbindungen und Intermediate:**

**Beispiel 1A**

Lithium-1-(3-chlor-5-fluorphenyl)-4-ethoxy-3,4-dioxobut-1-en-1-olat

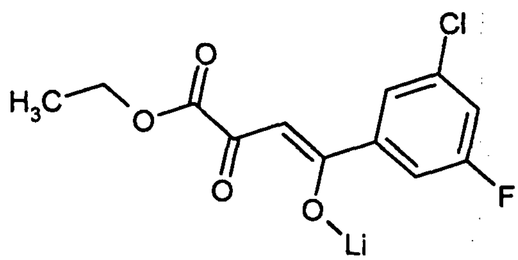

Eine Lösung aus 78 ml (78 mmol) Lithiumhexamethyldisilazid (1N Lösung in Tetrahydrofuran) in 60 ml Diethylether wird bei -78 °C unter Argon vorgelegt und mit einer Lösung aus 12.5 g (72.4 mmol) 1-(3-Chlor-5-fluorphenyl) ethanon in 190 ml Diethylether versetzt. Nach 45 Minuten bei -78 °C werden 11.6 g (79.7 mmol) Oxalsäurediethylester zugetropft und über Nacht bei Raumtemperatur gerührt. Man engt das Reaktionsgemisch ein und erhält 28.6 g mit 71%iger Reinheit (100% d. Th.) der Titelverbindung, die ohne weitere Aufreinigung umgesetzt werden.

$^1$H-NMR (400 MHz, DMSO-$d_6$): δ = 7.63 (t, 1H), 7.55-7.50 (m, 2H), 6.33 (s, 1H), 4.14 (q, 2H), 1.24 (t, 3H).

LC-MS (Methode 1): $R_t$ = 2.65 min; MS (ESIpos): m/z = 273 $[M-Li+2H]^+$.

**Beispiel 2A**

Lithium-1-(3,5-difluorphenyl)-4-ethoxy-3,4-dioxobut-1-en-1-olat

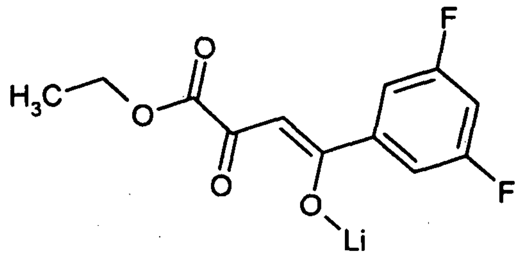

Die Herstellung der Titelverbindung erfolgt ausgehend von 1-(3,5-Difluorphenyl)ethanon und Oxalsäurediethylester analog zur Synthese der Verbindung aus Beispiel 1A. Man erhält 5.43 g (65% d. Th.) der Titelverbindung.

$^1$H-NMR (400 MHz, DMSO-$d_6$): $\delta$ = 7.45 (d, 2H), 7.35 (t, 1H), 6.38 (s, 1H), 4.17 (q, 2H), 1.26 (t, 3H).

LC-MS (Methode 3): $R_t$ = 2.43 min; MS -(ESIpos): m/z = 257 $[M-Li+2H]^+$.

**Beispiel 3A**

Lithium-1-(3-chlorphenyl)-4-ethoxy-3,4-dioxobut-1-en-1-olat

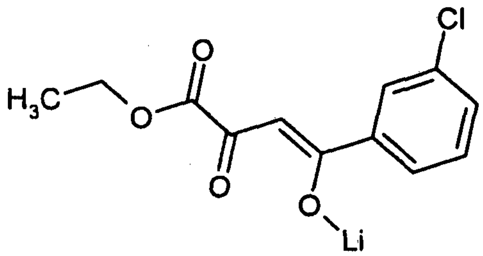

Die Herstellung der Titelverbindung erfolgt ausgehend von 1-(3-Chlorphenyl)ethanon und Oxalsäurediethylester analog zur Synthese der Verbindung aus Bespiel 1A. Man erhält 40.4 g (92% d. Th.) der Titelverbindung.

LC-MS (Methode 4): $R_t$ = 3.91 min; MS (ESIpos): m/z = 255 $[M-Li+2H]^+$.

**Beispiel 4A**

Lithium-1-(3-cyanphenyl)-4-ethoxy-3,4-dioxobut-1-en-1-olat

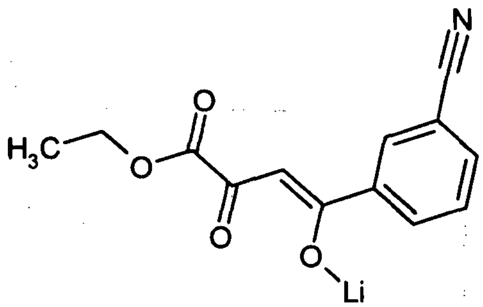

Die Herstellung der Titelverbindung erfolgt ausgehend von 3-Acetylbenzolcarbonitril und Oxalsäurediethylester analog zur Synthese der Verbindung aus Beispiel 1A. Man erhält 0.53 g mit 62%iger Reinheit (76% d. Th.) der Titelverbindung.

LC-MS (Methode 5): $R_t$ = 1.11 min; MS (ESIpos): m/z = 246 $[M-Li+2H]^+$.

**Beispiel 5A**

Lithium-1-(3-chlor-4-fluorphenyl)-4-ethoxy-3,4-dioxobut-1-en-1-olat

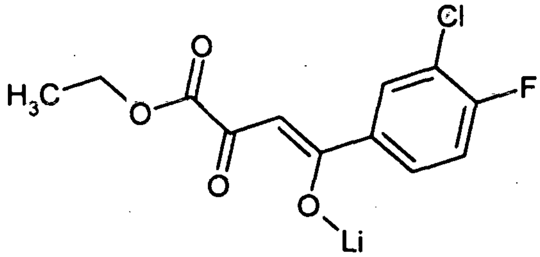

Die Herstellung der Titelverbindung erfolgt ausgehend von 1-(3-Chlor-4-fluorphenyl)ethanon und Oxalsäurediethylester analog zur Synthese der Verbindung aus Beispiel 1A. Man erhält 9.7 g mit 66%iger Reinheit (79% d. Th.) der Titelverbindung.

LC-MS (Methode 1): $R_t$ = 2.69 min; MS (ESIpos): m/z = 273 $[M-Li+2H]^+$.

**Beispiel 6A**

Lithium-4-ethoxy-3,4-dioxo-1-[3-(trifluormethoxy)phenyl]but-1-en-1-olat

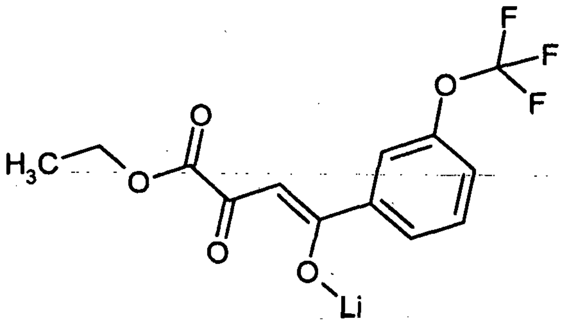

Die Herstellung der Titelverbindung erfolgt ausgehend von 1-[3-(Trifluormethoxy)phenyl]ethanon und Oxalsäurediethylester analog zur Synthese der Verbindung aus Beispiel 1A. Man erhält 7.7 g mit 65%iger Reinheit (80% d. Th.) der Titelverbindung.

$^1$H-NMR (400 MHz, DMSO-$d_6$): $\delta$ = 7.84 (d, 1H), 7.70 (s, 1H), 7.56 (t, 1H), 7.45 (d, 1H), 6.41 (s, 1H), 4.15 (q, 2H), 1.25 (t, 3H).

LC-MS (Methode 5): $R_t$ = 1.39 min; MS (ESIpos):m/z = 305 $[M-Li+2H]^+$.

**Beispiel 7A**

Lithium-4-ethoxy-1-[4-fluor-3-(trifluormethyl)phenyl]-3,4-dioxobut-1-en-1-olat

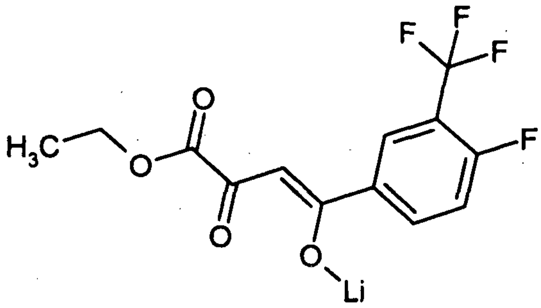

Die Herstellung der Titelverbindung erfolgt ausgehend von 1-[4-Fluor-3-(trifluormethyl)phenyl]ethanon und Oxalsäurediethylester analog zur Synthese der Verbindung aus Beispiel 1A. Man erhält 8.7 g mit 78%iger Reinheit (90% d. Th.) der Titelverbindung.

$^1$H-NMR (400 MHz, DMSO-$d_6$): $\delta$ = 7.91 (s, 1H), 7.89 (d, 1H), 7.79 (d, 1H), 6.40 (s, 1H), 4.16(q, 2H), 1.25(t, 3H).

LC-MS (Methode 3): $R_t$ = 2.64 min; MS (ESIpos): m/z = 307 $[M-Li+2H]^+$.

**Beispiel 8A**

Lithium-4-ethoxy-1-(3-fluorphenyl)-3,4-dioxobut-1-en-1-olat

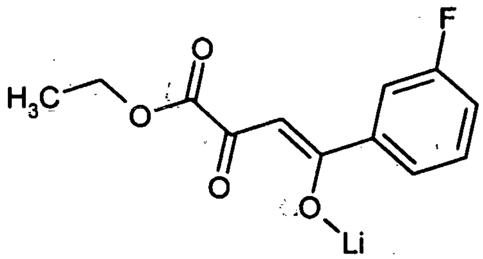

Die Herstellung der Titelverbindung erfolgt ausgehend von 1-(3-Fluorphenyl)ethanon und Oxalsäurediethylester analog zur Synthese der Verbindung aus Beispiel 1A. Man erhält 9.4 g (98% d. Th.) der Titelverbindung.

LC-MS (Methode 5): $R_t$ = 1.22 min; MS (ESIpos): m/z = 239 $[M-Li+2H]^+$.

**Beispiel 9A**

Lithium-4-ethoxy-3,4-dioxo-1-[3-(trifluormethyl)phenyl]but-1-en-1-olat

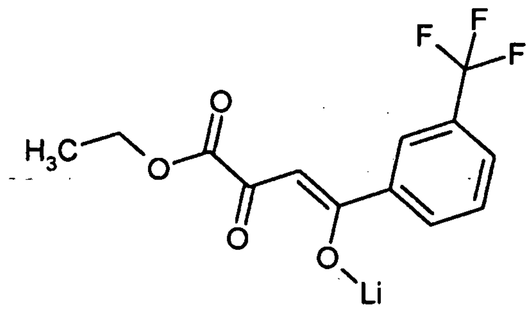

Die Herstellung der Titelverbindung erfolgt ausgehend von 1-[3-(Trifluormethyl)phenyl]ethanon und Oxalsäurediethylester analog zur Synthese der Verbindung aus Beispiel 1A. Man erhält 44.2 g mit 62%iger Reinheit (70% d. Th.) der Titelverbindung.

$^1$H-NMR (400 MHz, DMSO-$d_6$): $\delta$ = 8.12 (d, 1H), 8.07 (s, 1H), 7.83 (d, 1H), 7.67 (t, 1H), 6.45 (s, 1H), 4.16 (q,

2H), 1.25 (t, 3H).
LC-MS (Methode 3): $R_t$ = 2.54 min; MS (ESIpos): m/z = 289 [M-Li+2H]$^+$.

**Beispiel 10A**

Lithium-4-ethoxy-1-(3-methoxyphenyl)-3,4-dioxobut-1-en-1-olat

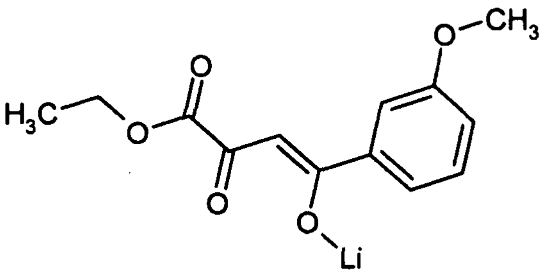

Die Herstellung der Titelverbindung erfolgt ausgehend von 1-(3-Methoxy-phenyl)ethanon und Oxalsäurediethylester analog zur Synthese der Verbindung aus Beispiel 1A. Man erhält 0.62 g mit 64%iger Reinheit (93% d. Th.) der Titelverbindung.
LC-MS (Methode 5): $R_t$ = 1.22 min; MS (ESIpos): m/z = 251 [M-Li+2H]$^+$.

**Beispiel 11A**

Lithium-1-(3,4-difluorphenyl)-4-ethoxy-3,4-dioxobut-1-en-1-olat

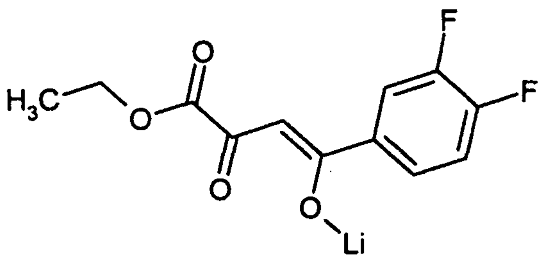

Die Herstellung der Titelverbindung erfolgt ausgehend von 1-(3,4-Difluorphenyl)ethanon und Oxalsäurediethylester analog zur Synthese der Verbindung aus Beispiel 1A. Man erhält 8.47 g (100% d. Th.) der Titelverbindung.
$^1$H-NMR (400 MHz, DMSO-$d_6$): $\delta$ = 7.79 (ddd, 1H), 7.74-7.68 (m, 1H), 7.47 (q, 1H), 6.40 (s, 1H), 4.16 (q, 2H), 1.26 (t, 3H).
LC-MS (Methode 3): $R_t$ = 2.40 min; MS (ESIpos): m/z = 257 [M-Li+2H]$^+$.

**Beispiel 12A**

Lithium-1-(3-brom-4-fluorphenyl)-4-ethoxy-3,4-dioxobut-1-en-1-olat

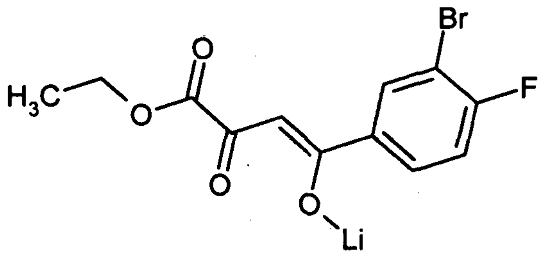

Die Herstellung der Titelverbindung erfolgt ausgehend von 1-(3-Brom-4-fluorphenyl)ethanon und Oxalsäurediethylester analog zur Synthese der Verbindung aus Beispiel 1A. Man erhält 7.8 g mit 75%iger Reinheit (79% d. Th.) der Titelverbindung.

$^1$H-NMR (400 MHz, DMSO-$d_6$): $\delta$ = 8.05 (dd, 1H), 7.86 (ddd, 1H), 7.40 (t, 1H), 6.36 (s, 1H), 4.15 (q, 2H), 1.25 (t, 3H).

LC-MS (Methode 3): $R_t$ = 2.59 min; MS (ESIpos): m/z = 318 $[M-Li+2H]^+$.

Beispiel 13A

Lithium-4-ethoxy-1-(2-fluorphenyl)-3,4-dioxobut-1-en-1-olat

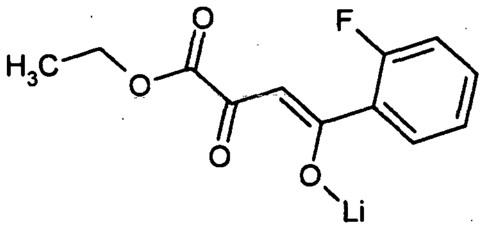

Die Herstellung der Titelverbindung erfolgt ausgehend von 1-(2-Fluorphenyl)ethanon und Oxalsäurediethylester analog zur Synthese der Verbindung aus Beispiel 1A. Man erhält 4.5 g (82% d. Th.) der Titelverbindung.

$^1$H-NMR (400 MHz, DMSO-$d_6$): $\delta$ = 7.67 (dt, 1H), 7.49-7.42 (m, 1H), 7.27-7.17 (m, 2H), 6.24 (s, 1H), 4.14 (q, 2H), 1.24 (t, 3H).

LC-MS (Methode 3): $R_t$ = 2.36 min; MS (ESIpos): m/z = 239 $[M-Li+2H]^+$.

Beispiel 14A

Lithium-1-(2,3-difluorphenyl)-4-ethoxy-3,4-dioxobut-1-en-1-olat

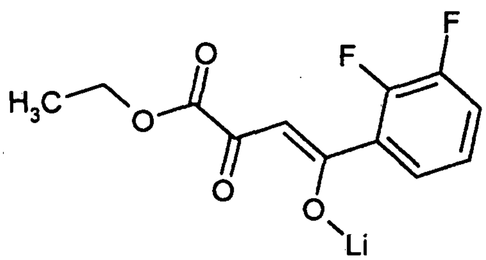

Die Herstellung der Titelverbindung erfolgt ausgehend von 1-(2,3-Difluorphenyl)ethanon und Oxalsäurediethylester analog zur Synthese der Verbindung aus Beispiel 1A. Man erhält 1.7 g mit 26%iger Reinheit (48% d. Th.) der Titelverbindung.

LC-MS (Methode 3): $R_t$ = 2.33 min; MS (ESIpos): m/z = 257 $[M-Li+2H]^+$.

**Beispiel 15A**

Lithium-4-ethoxy-1-(3-fluor-5-methoxyphenyl)-3,4-dioxobut-1-en-1-olat

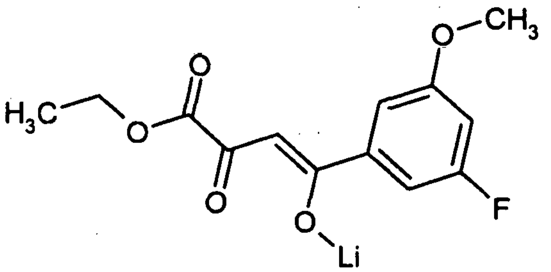

5.00 g (29.4 mmol) 3-Fluor-5-methoxybenzoesäure werden in 51 ml Toluol vorgelegt, mit 6.4 ml (88.2 mmol) Thionylchlorid versetzt und 2 h unter Rückfluss erhitzt. Anschließend engt man die Reaktionsmischung ein, nimmt den Rückstand in Dichlormethan auf, versetzt mit 3.73 g (38.2 mmol) *N,O*-Dimethylhydroxylaminhydrochlorid und bei 0 °C mit 10.7 ml (76.4 mmol) Triethylamin und rührt über Nacht bei Raumtemperatur. Man setzt Wasser hinzu, trennt die Phasen, extrahiert mit Dichlormethan, trocknet die vereinigten organischen Phasen über Natriumsulfat, filtriert und engt ein. Der Rückstand wird in 105 ml Diethylether gelöst, unter Argon bei Raumtemperatur zu 9.6 ml (28.6 mmol) einer 3M Lösung von Methylmagnesiumbromid in Diethylether gegeben und 2 h unter Rückfluss erhitzt. Anschließend gibt man gesättigte wässrige Ammoniumchlorid-Lösung hinzu, extrahiert mit Dichlormethan, trocknet die vereinigten organischen Phasen über Natriumsulfat, filtriert und engt ein. Das Rohprodukt 1-(3-Fluor-5-methoxyphenyl)ethanon wird mit Oxalsäurediethylester analog zur Synthese der Verbindung aus Beispiel 1A umgesetzt. Man erhält 4.02 g mit 61%iger Reinheit (30% d. Th.) der Titelverbindung.

LC-MS (Methode 5): $R_t$ = 1.26 min; MS (ESIpos): m/z = 269 $[M-Li+2H]^+$.

**Beispiel 16A**

Lithium-1-[3-(benzyloxy)phenyl]-4-ethoxy-3,4-dioxobut-1-en-1-olat

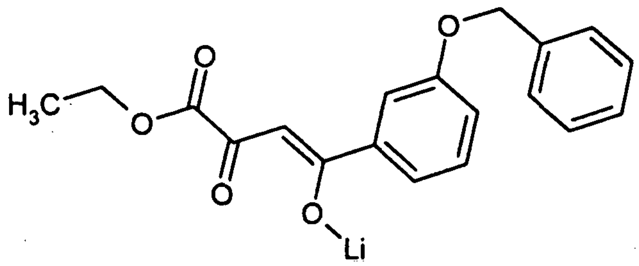

Die Herstellung der Titelverbindung erfolgt ausgehend von 1-[3-(Benzyloxy)phenyl]ethanon und Oxalsäurediethylester analog zur Synthese der Verbindung aus Beispiel 1A. Man erhält 8.1 g mit 74%iger Reinheit (82% d. Th.) der Titelverbindung.

$^1$H-NMR (400 MHz, DMSO-$d_6$): $\delta$ = 7.49-7.44 (m, 2H), 7.43-7.36 (m, 4H), 7.36-7.30 (m, 2H), 7.14-7.08 (m, 1H), 6.39 (s, 1H), 5.15 (s, 2H), 4.14 (q, 2H), 1.25 (t, 3H).

LC-MS (Methode 2): $R_t$ = 2.96 min; MS (ESIpos): m/z = 327 $[M-Li+2H]^+$.

**Beispiel 17A**

Lithium-4-ethoxy-1-(3-fluor-5-trifluormethylphenyl)-3,4-dioxobut-1-en-1-olat

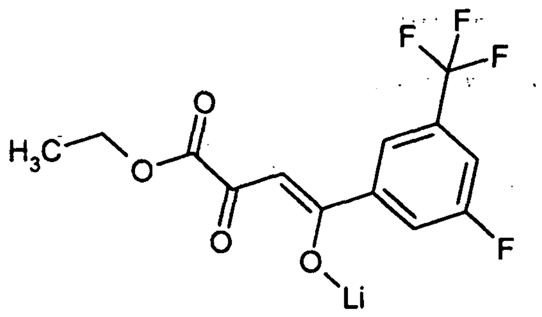

Die Herstellung der Titelverbindung erfolgt ausgehend von 1-[3-Fluor-5-(trifluormethyl)phenyl]ethanon und Oxalsäurediethylester analog zur Synthese der Verbindung aus Beispiel 1A. Man erhält 1.5 g mit 62%iger Reinheit (62% d. Th.) der Titelverbindung.
LC-MS (Methode 5): $R_t$ = 1.37 min; MS (ESIpos): m/z = 307 $[M-Li+2H]^+$.

**Beispiel 18A**

Lithium-1-(3-brom-5-fluorphenyl)-4-ethoxy-3,4-dioxobut-1-en-1-olat

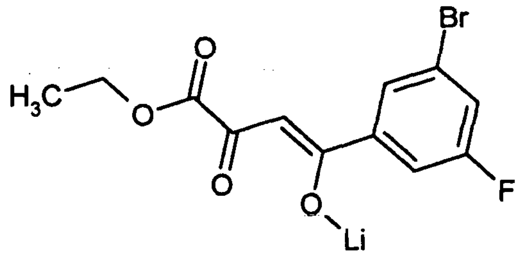

Die Herstellung der Titelverbindung erfolgt ausgehend von 1-(3-Brom-5-fluorphenyl)ethanon (beschrieben in US2003/229096A1) und Oxalsäurediethylester analog zur Synthese der Verbindung aus Beispiel 1A. Man erhält 4.7 g mit 62%iger Reinheit (74% d. Th.) der Titelverbindung.
LC-MS (Methode 7): $R_t$ = 2.29 min; MS (ESIpos): m/z = 317 $[M-Li+2H]^+$.

**Beispiel 19A**

Lithium-1-[3,5-bis(trifluormethyl)phenyl]-4-ethoxy-3,4-dioxobut-1-en-1-olat

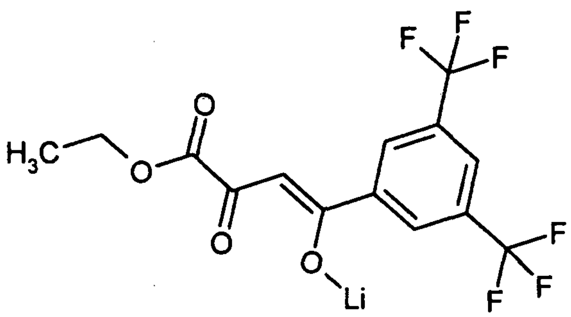

Die Herstellung der Titelverbindung erfolgt ausgehend von 1-[3,5-Bis(trifluormethyl)phenyl]ethanon und Oxalsäurediethylester analog zur Synthese der Verbindung aus Beispiel 1A. Man erhält 6.1 g (86% d. Th.) der Titelverbindung.
$^1$H-NMR (400 MHz, DMSO-$d_6$): $\delta$ = 8.36 (s, 2H), 8.23 (s, 1H), 6.48 (s, 1H), 4.18 (q, 2H), 1.27 (t, 3H).
LC-MS (Methode 3): $R_t$ = 2.88 min; MS (ESIneg): m/z = 355 $[M-Li]^-$.

**Beispiel 20A**

Lithium-4-ethoxy-1-(3-fluor-5-methylphenyl)-3,4-dioxobut-1-en-1-olat

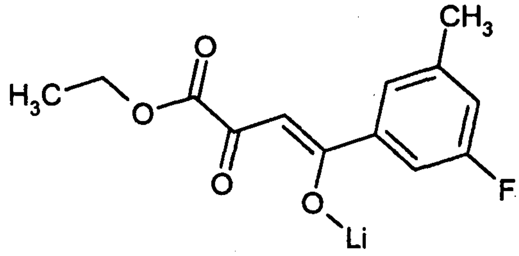

Die Herstellung der Titelverbindung erfolgt ausgehend von 1-(3-Fluor-5-methylphenyl)ethanon und Oxalsäurediethylester analog zur Synthese der Verbindung aus Beispiel 1A. Man erhält 8.88 g mit 66%iger Reinheit (70% d. Th.) der Titelverbindung.
LC-MS (Methode 1): $R_t$ = 2.67 min; MS (ESIpos): m/z = 253 $[M-Li+2H]^+$.

**Beispiel 21A**

1-(3-Chlor-4-fluorphenyl)-5-(3-chlor-5-fluorphenyl)-1*H*-pyrazol-3-carbosäureethyl-ester

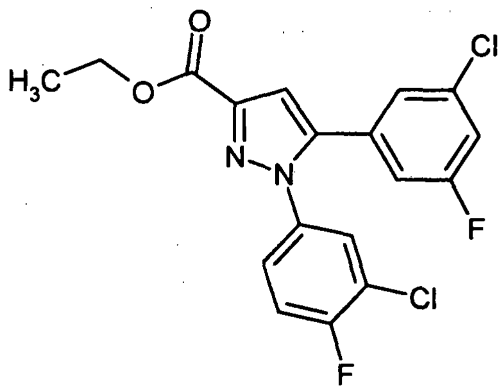

28.6 g mit 71%iger Reinheit (72.9 mmol) der Verbindung aus Beispiel 1A werden in 350 ml Ethanol vorgelegt, mit 15.8 g (80.2 mmol) 3-Chlor-4-fluorphenylhydrazinhydrochlorid versetzt und über Nacht bei Raumtemperatur gerührt. Das Reaktionsgemisch wird eingeengt und der Rückstand in 350 ml konzentrierter Essigsäure aufgenommen und 2 h unter Rückfluss erhitzt. Das Reaktionsgemisch wird auf Essigsäureethylester gegeben und mit Wasser und gesättigter wässriger Natriumhydrogencarbonat-Lösung gewaschen. Die organische Phase wird eingeengt und mittels Flash-Chromatographie (Laufmittel: Cyclohexan/Essigsäureethylester 20:1) gereinigt. Man erhält 22.6 g (76% d. Th.) der Titelverbindung.
$^1$H-NMR (400 MHz, DMSO-$d_6$): $\delta$ = 7.80 (dd, 1H), 7.58-7.50 (m, 2H), 7.38 (ddd, 1H), 7.31 (s, 1H), 7.27 (s, 1H), 7.19 (dt, 1H), 4.34 (q, 2H), 1.32 (t, 3H).
LC-MS (Methode 5): $R_t$ = 1.52 min; MS (ESIpos): m/z = 397 $[M+H]^+$.

**Beispiel 22A**

5-(3-Chlor-5-fluorphenyl)-1-(3-chlorphenyl)-1*H*-pyrazol-3-carbonsäureethylester

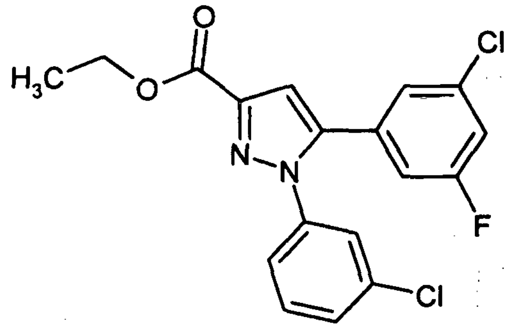

Die Herstellung der Titelverbindung erfolgt ausgehend von der Verbindung aus Beispiel 1A und 3-Chlorphenylhydrazinhydrochlorid analog zur Synthese der Verbindung aus Beispiel 21A. Man erhält 5.82 g der Titelverbindung.
$^1$H-NMR (400 MHz, DMSO-$d_6$): $\delta$ = 7.61-7.56 (m, 2H), 7.54-7.47 (m, 2H), 7.32 (s, 1H), 7.30 (d, 1H), 7.25 (d, 1H), 7.18 (dt, 1H), 4.35 (q, 2H), 1.32 (t, 3H).
LC-MS (Methode 5): $R_t$ = 1.51 min; MS (ESIpos): m/z = 379 $[M+H]^+$.

**Beispiel 23A**

1-(3-Chlorphenyl)-5-(3,5-difluorphenyl)-1*H*-pyrazol-3-carbonsäureethylester

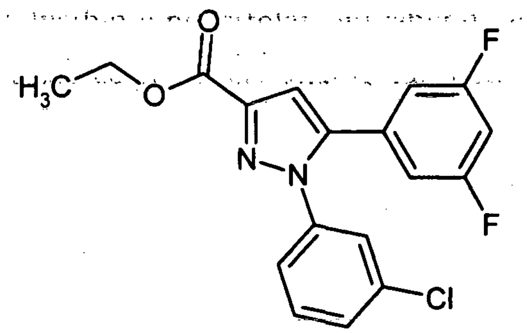

Die Herstellung der Titelverbindung erfolgt ausgehend von der Verbindung aus Beispiel 2A und 3-Chlorphenylhydrazinhydrochlorid analog zur Synthese der Verbindung aus Beispiel 21A. Nach Reinigung mittels Flash-Chromatographie (Laufmittel: Cyclohexan/Essigsäureethylester 3:1) erhält man 2.58 g (68% d. Th.) der Titelverbindung.
$^1$H-NMR (400 MHz, DMSO-$d_6$): $\delta$ = 7.60-7.56 (m, 2H), 7.53-7.47 (m, 1H), 7.37-7.27 (m, 3H), 7.10-7.03 (m, 2H), 4.35 (q, 2H), 1.32 (t, 3H).
LC-MS (Methode 3): $R_t$ = 2.74 min; MS (ESIpos): m/z = 363 $[M+H]^+$.

**Beispiel 24A**

1,5-Bis(3-chlorphenyl)-1H-pyrazol-3-carbonsäureethylester

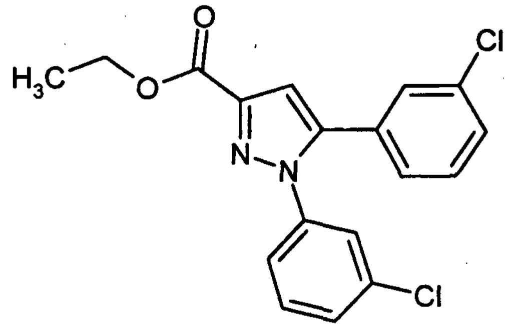

112 g (413 mmol) der Verbindung aus Beispiel 3A werden in 1120 ml Ethanol vorgelegt, mit 101 g (561 mmol) 3-Chlorphenylhydrazinhydrochlorid versetzt und über Nacht bei Raumtemperatur gerührt. Das Reaktionsgemisch wird eingeengt und der Rückstand in 1120 ml konzentrierter Essigsäure aufgenommen und 2 h unter Rückfluss erhitzt. Man lässt über Nacht bei Raumtemperatur stehen, saugt den entstandenen Niederschlag ab, rührt diesen mit Cyclohexan, saugt den Niederschlag ab, wäscht mit Cyclohexan und trocknet im Hochvakuum. Man erhält 126 g (80% d. Th.) der Titelverbindung.

$^1$H-NMR (400 MHz, DMSO-$d_6$): $\delta$ = 7.58-7.54 (m, 2H), 7.51-7.43 (m, 3H), 7.40 (t, 1H), 7.30-7.26 (m, 1H), 7.25 (s, 1H), 7.19 (d, 1H), 4.34 (q, 2H), 1.32 (t, 3H).

LC-MS (Methode 2): $R_t$ = 3.00 min; MS (ESIpos): m/z = 361 $[M+H]^+$.

**Beispiel 25A**

1,5-Bis(3-chlor-4-fluorphenyl)-1*H*-pyrazol-3-carbonsäureethylester

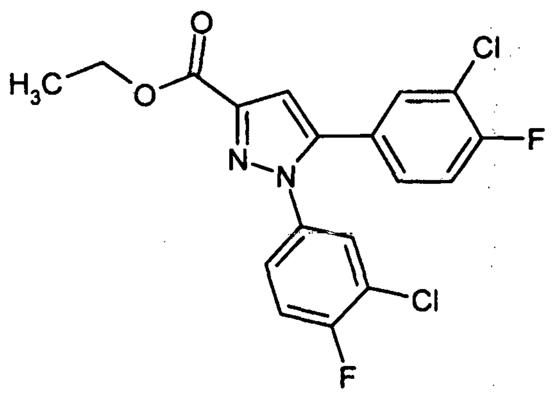

Die Herstellung der Titelverbindung erfolgt ausgehend von der Verbindung aus Beispiel 5A analog zur Synthese der Verbindung aus Beispiel 21A. Man erhält 5.78 g der Titelverbindung.

$^1$H-NMR (400 MHz, DMSO-$d_6$): $\delta$ = 7.78 (dd, 1H), 7.67 (dd, 1H), 7.53 (t, 1H), 7.44 (t, 1H), 7.35 (ddd, 1H), 7.25 (s, 1H), 7.22 (ddd, 1H), 4.34 (q, 2H), 1.32 (t, 3H).

LC-MS (Methode 5): $R_t$ = 1.51 min; MS (ESIpos): m/z = 397 $[M+H]^+$.

**Beispiel 26A**

1-(3-Chlorphenyl)-5-[3-(trifluormethoxy)phenyl]-1*H*-pyrazol-3-carbonsäureethylester

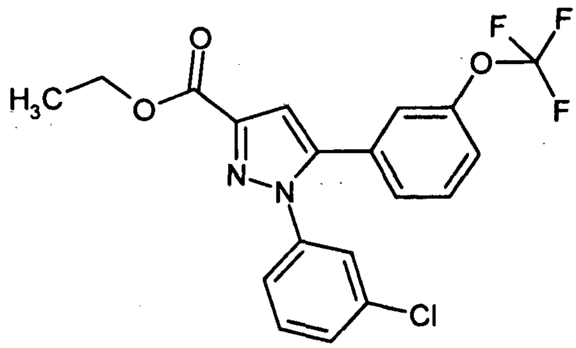

Die Herstellung der Titelverbindung erfolgt ausgehend von der Verbindung aus Beispiel 6A und 3-Chlorphenylhydrazinhydrochlorid analog zur Synthese der Verbindung aus Beispiel 21A. Man erhält 4.56 g (95% d. Th.) der Titelverbindung.

$^1$H-NMR (400 MHz, DMSO-$d_6$): $\delta$ = 7.59-7.46 (m, 4H), 7.46-7.38 (m, 2H), 7.32-7.27 (m, 2H), 7.22-7.19 (m, 1H), 4.35 (q, 2H), 1.32 (t, 3H).

LC-MS (Methode 3): $R_t$ = 2.92 min; MS (ESIpos): m/z = 411 $[M+H]^+$.

**Beispiel 27A**

1-(3-Chlorphenyl)-5-[4-fluor-3-(trifluormethyl)phenyl]-1*H*-pyrazol-3-carbonsäure-ethylester

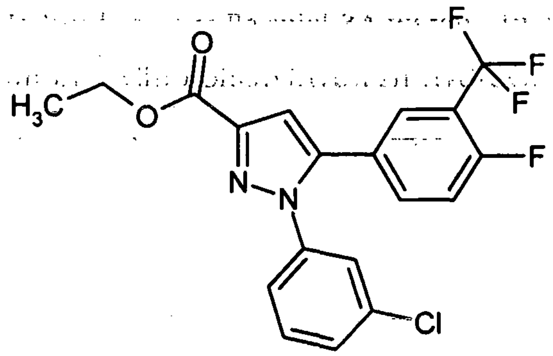

Die Herstellung der Titelverbindung erfolgt ausgehend von der Verbindung aus Beispiel 7A und 3-Chlorphenylhydrazinhydrochlorid analog zur Synthese der Verbindung aus Beispiel 21A. Man erhält 3.60 g der Titelverbindung.

$^1$H-NMR (400 MHz, DMSO-$d_6$): $\delta$ = 7.76 (d, 1H), 7.61-7.56 (m, 3H), 7.50 (t, 1H), 7.43 (s, 1H), 7.42 (s, 1H), 7.34-7.30 (m, 1H), 4.35 (q, 2H), 1.33 (t, 3H).

LC-MS (Methode 1): $R_t$ = 3.05 min; MS (ESIpos): m/z = 413 $[M+H]^+$.

**Beispiel 28A**

1-(3-Chlor-4-fluorphenyl)-5-[3-(trifluormethoxy)phenyl]-1*H*-pyrazol-3-carbonsäure-ethylester

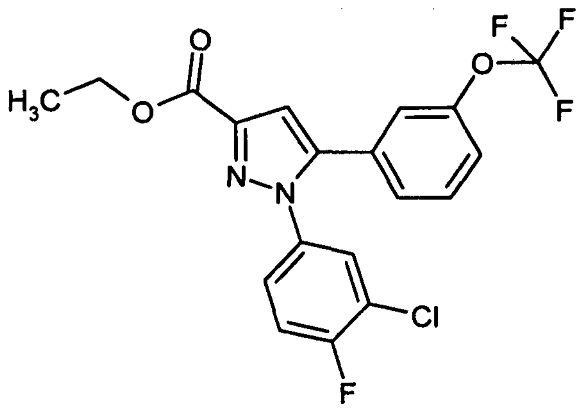

Die Herstellung der Titelverbindung erfolgt ausgehend von der-Verbindung aus Beispiel 6A analog zur Synthese der Verbindung aus Beispiel 21A. Man erhält 3.50 g (66% d. Th.) der Titelverbindung.

$^1$H-NMR (400 MHz, DMSO-$d_6$): δ = 7.74 (dd, 1H), 7.59-7.51 (m, 2H), 7.44-7.35 (m, 3H), 7.28 (s, 1H), 7.21 (s, 1H), 4.35 (q, 2H), 1.32 (t, 3H).

LC-MS (Methode 1): $R_t$ = 3.04 min; MS (ESIpos): m/z = 429 $[M+H]^+$.

**Beispiel 29A**

5-(3-Chlor-4-fluorphenyl)-1-(3-chlorphenyl)-1*H*-pyrazol-3-carbonsäureethylester

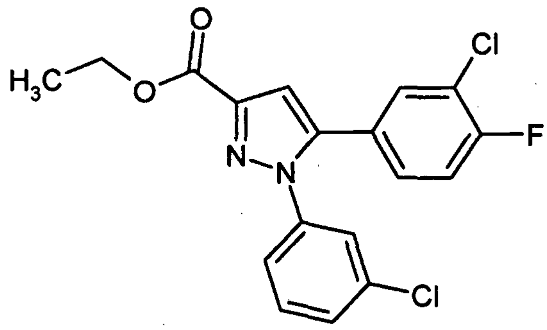

Die Herstellung der Titelverbindung erfolgt ausgehend von der Verbindung aus Beispiel 5A und 3-Chlorphenylhydrazinhydrochlorid analog zur Synthese der Verbindung aus Beispiel 21A. Man erhält 5.67 g der Titelverbindung.

$^1$H-NMR (400 MHz, DMSO-$d_6$): δ = 7.64 (dd, 1H), 7.58-7.54 (m, 2H), 7.51-7.42 (m, 2H), 7.29-7.21 (m, 3H), 4.34 (q, 2H), 1.32 (t, 3H).

LC-MS (Methode 5): $R_t$ = 1.50 min; MS (ESIpos): m/z = 379 $[M+H]^+$.

**Beispiel 30A**

5-(3-Fluorphenyl)-1-(3-methoxyphenyl)-1*H*-pyrazol-3-carbonsäureethylester

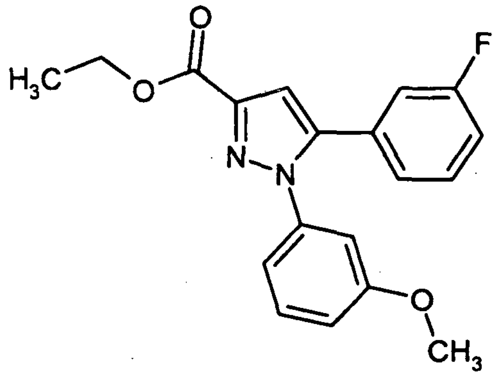

Die Herstellung der Titelverbindung erfolgt ausgehend von der Verbindung aus Beispiel 8A und 3-Methoxyphenylhydrazinhydrochlorid analog zur Synthese der Verbindung aus Beispiel 21A. Man erhält 30.4 g (62% d. Th.) der Titelverbindung.

$^1$H-NMR (400 MHz, DMSO-$d_6$): $\delta$ = 7.44-7.33 (m, 2H), 7.24 (dd, 1H), 7.21 (s, 1H), 7.17 (dt, 1H), 7.08 (d, 1H), 7.04 (dd, 1H), 6.95 (t, 1H), 6.86 (dd, 1H), 4.34 (q, 2H), 3.73 (s, 3H), 1.32 (t, 3H).

LC-MS (Methode 1): $R_t$ = 2.78 min; MS (ESIpos): m/z = 341 $[M+H]^+$.

**Beispiel 31A**

1-(3-Chlorphenyl)-5-(3-methoxyphenyl)-1*H*-pyrazol-3-carbonsäureethylester

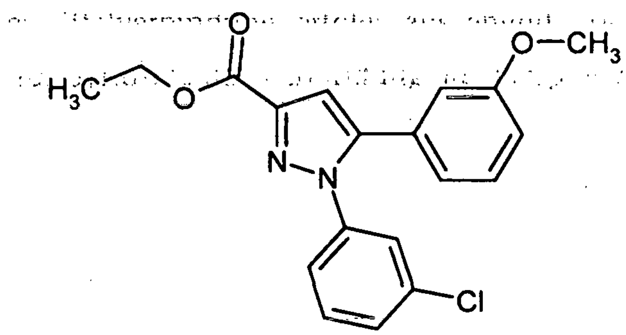

Die Herstellung der Titelverbindung erfolgt ausgehend von der Verbindung aus Beispiel 10A und 3-Chlorphenylhydrazinhydrochlorid unter Reinigung über präparative HPLC (RP18-Säule; Laufmittel: Acetonitril/Wasser-Gradient) analog zur Synthese der Verbindung aus Beispiel 21A. Man erhält 1.60 g der Titelverbindung.

$^1$H-NMR (400 MHz, DMSO-$d_6$): $\delta$ = 7.57-7.51 (m, 2H), 7.47 (t, 1H), 7.31-7.25 (m, 2H), 7.17 (s, 1H), 6.96 (dd, 1H), 6.89 (s, 1H), 6.80 (d, 1H), 4.34 (q, 2H), 3.69 (s, 3H), 1.32 (t, 3H).

LC-MS (Methode 3): $R_t$ = 2.68 min; MS (ESIpos): m/z = 357 $[M+H]^+$.

**Beispiel 32A**

5-(3-Chlorphenyl)-1-[3-(trifluormethyl)phenyl]-1*H*-pyrazol-3-carbonsäureethylester

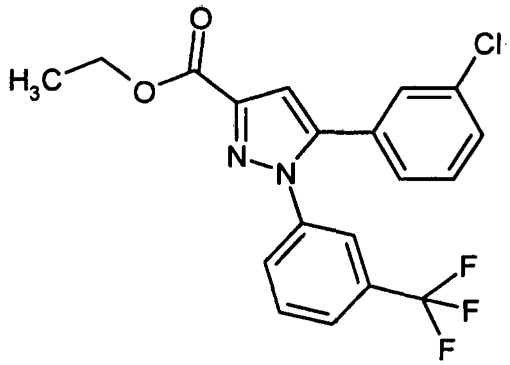

Die Herstellung der Titelverbindung erfolgt ausgehend von der Verbindung aus Beispiel 3A und 3-(Trifluormethyl)phenylhydrazinhydrochlorid unter Reinigung über präparative HPLC (RP18-Säule; Laufmittel: Acetonitril/Wasser-Gradient) analog zur Synthese der Verbindung aus Beispiel 21A. Man erhält 2.00 g (42% d. Th.) der Titelverbindung.
$^1$H-NMR (400 MHz, DMSO-$d_6$): $\delta$ = 7.85 (d, 1H), 7.75 (s, 1H), 7.71 (t, 1H), 7.64 (d, 1H), 7.50-7.43 (m, 2H), 7.40 (t, 1H), 7.27 (s, 1H), 7.20 (d, 1H), 4.35 (q, 2H), 1.33 (t, 3H).
LC-MS (Methode 1): $R_t$ = 3.09 min; MS (ESIpos): m/z = 395 $[M+H]^+$.

**Beispiel 33A**

1-(3-Chlorphenyl)-5-(3,4-difluorphenyl)-1*H*-pyrazol-3-carbonsäureethylester

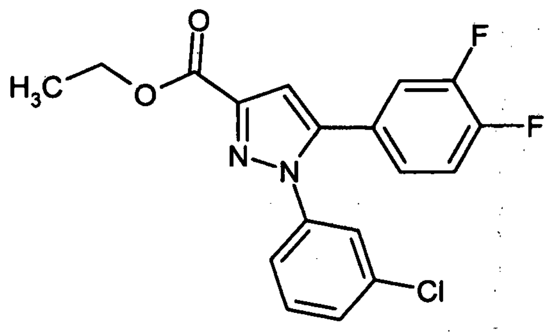

Die Herstellung der Titelverbindung erfolgt ausgehend von der Verbindung aus Beispiel 11A und 3-Chlorphenylhydrazinhydrochlorid analog zur Synthese der Verbindung aus Beispiel 21A. Man erhält 2.83 g (49% d. Th.) der Titelverbindung.
$^1$H-NMR (400 MHz, DMSO-$d_6$): $\delta$ = 7.58-7.43 (m, 5H), 7.28-7.24 (m, 1H), 7.22 (s, 1H), 7.11-7.06 (m, 1H), 4.34 (q, 2H), 1.32 (t, 3H).
LC-MS (Methode 3): $R_t$ = 2.72 min; MS (ESIpos): m/z = 363 $[M+H]^+$.

**Beispiel 34A**

5-(3-Brom-4-fluorphenyl)-1-(3-chlorphenyl)-1*H*-pyrazol-3-carbonsäureethylester

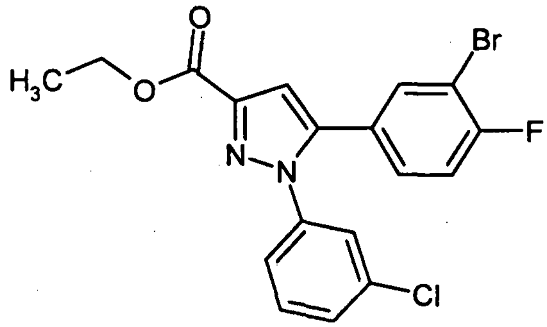

Die Herstellung der Titelverbindung erfolgt ausgehend von der Verbindung aus Beispiel 12A und 3-Chlorphenylhydrazinhydrochlorid analog zur Synthese der Verbindung aus Beispiel 21A. Man erhält 3.68 g der Titelverbindung.
$^1$H-NMR (400 MHz, DMSO-$d_6$): $\delta$ = 7.74 (dd, 1H), 7.58-7.54 (m, 2H), 7.48 (t, 1H), 7.41 (t, 1H), 7.30-7.23 (m, 3H), 4.34 (q, 2H), 1.32 (t, 3H).
LC-MS (Methode 1): $R_t$ = 3.03 min; MS (ESIpos): m/z = 423 $[M+H]^+$.

**Beispiel 35A**

5-(3-Chlorphenyl)-1-(4-fluorphenyl)-1*H*-pyrazol-3-carbonsäureethylester

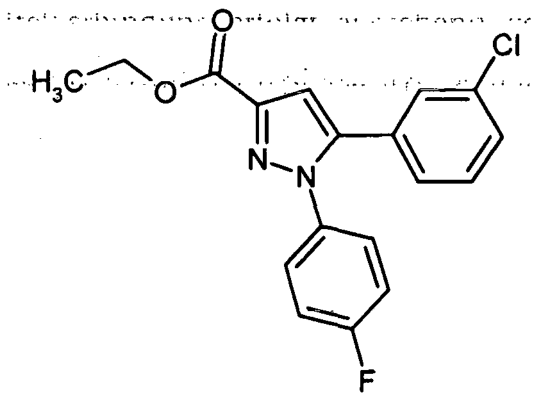

45.0 g (155 mmol) der Verbindung aus Beispiel 3A werden in 400 ml Ethanol vorgelegt, mit 34.4 g (211 mmol) 4-Fluorphenylhydrazinhydrochlorid versetzt und über Nacht bei Raumtemperatur gerührt. Das Reaktionsgemisch wird eingeengt und der Rückstand in 400 ml konzentrierter Essigsäure aufgenommen und 2 Stunden unter Rückfluss erhitzt. Das Reaktionsgemisch wird eingeengt, der Rückstand in Dichlormethan aufgenommen, mit gesättigter wässriger Natriumhydrogencarbonat-Lösung und Wasser gewaschen, über Natriumsulfat getrocknet und eingeengt. Der Rückstand wird in Diethylether unter Rückfluss gerührt und der Niederschlag abgesaugt. Die Mutterlauge wird eingeengt, der Rückstand mit Cyclohexan gerührt und der Niederschlag abgesaugt. Man erhält insgesamt 23 g (43% d. Th.) der Titelverbindung.
$^1$H-NMR (400 MHz, DMSO-$d_6$): $\delta$ = 7.47-7.39 (m, 4H), 7.39-7.30 (m, 3H), 7.23 (s, 1H), 7.16 (dt, 1H), 4.34 (q, 2H), 1.32 (t, 3H).
LC-MS (Methode 1): $R_t$ = 2.89 min; MS (ESIpos): m/z = 345 $[M+H]^+$.

**Beispiel 36A**

1-(3-Chlorphenyl)-5-(2-fluorphenyl)-1*H*-pyrazol-3-carbonsäureethylester

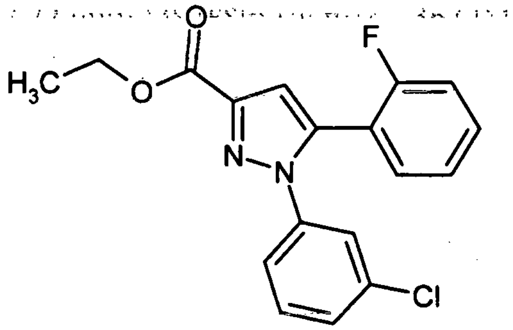

Die Herstellung der Titelverbindung erfolgt ausgehend von der Verbindung aus Beispiel 13A und 3-Chlorphenylhydrazinhydrochlorid analog zur Synthese der Verbindung aus Beispiel 21A. Man erhält 2.34 g (73% d. Th.) der Titelverbindung.
$^1$H-NMR (400 MHz, DMSO-$d_6$): $\delta$ = 7.56-7.41 (m, 5H), 7.33-7.21 (m, 3H), 7.16 (s, 1H), 4.35 (q, 2H), 1.33 (t, 3H).
LC-MS (Methode 1): $R_t$ = 2.85 min; MS (ESIpos): m/z = 345 $[M+H]^+$.

**Beispiel 37A**

1-(4-Fluorphenyl)-5-[3-(trifluormethyl)phenyl]-1*H*-pyrazol-3-carbonsäureethylester

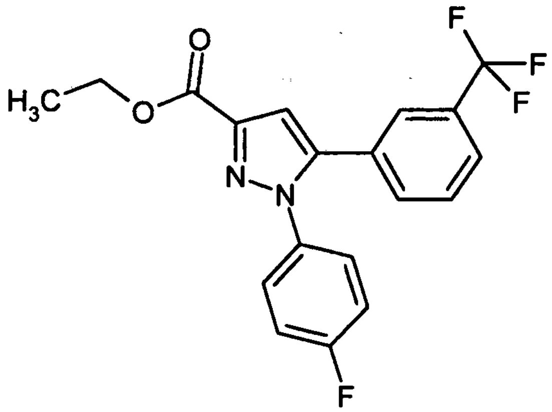

Die Herstellung der Titelverbindung erfolgt ausgehend von der Verbindung aus Beispiel 9A und 4-Fluorphenylhydrazinhydrochlorid analog zur Synthese der Verbindung aus Beispiel 21A. Man erhält 4.63 g (99% d. Th.) der Titelverbindung.
$^1$H-NMR (400 MHz, DMSO-$d_6$): $\delta$ = 7.74 (d, 1H), 7.64-7.54 (m, 3H), 7.47-7.41 (m, 2H), 7.37-7.30 (m, 3H), 4.34 (q, 2H), 1.32 (t, 3H).
LC-MS (Methode 4): $R_t$ = 4.10 min; MS (ESIpos): m/z = 379 $[M+H]^+$.

**Beispiel 38A**

1-(3-Chlorphenyl)-5-(2,3-difluorphenyl)-1*H*-pyrazol-3-carbonsäureethylester

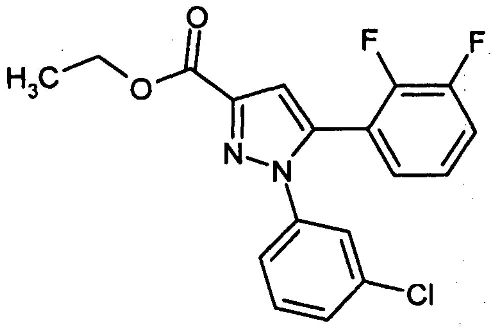

Die Herstellung der Titelverbindung erfolgt ausgehend von der Verbindung aus Beispiel 14A und 3-Chlorphenylhydrazinhydrochlorid analog zur Synthese der Verbindung aus Beispiel 21A. Man erhält 240 mg (37% d. Th.) der Titelverbindung.

$^1$H-NMR (400 MHz, $CDCl_3$): $\delta$ = 7.46 (t, 1H), 7.36-7.31 (m, 1H), 7.28-7.18 (m, 2H), 7.14-7.06 (m, 3H), 7.00-6.94 (m, 1H), 4.47 (q, 2H), 1.43 (t, 3H).

LC-MS (Methode 1): $R_t$ = 2.91 min; MS (ESIpos): m/z = 363 $[M+H]^+$.

**Beispiel 39A**

5-(3-Chlorphenyl)-1-(4-chlorphenyl)-1*H*-pyrazol-3-carbonsäureethylester

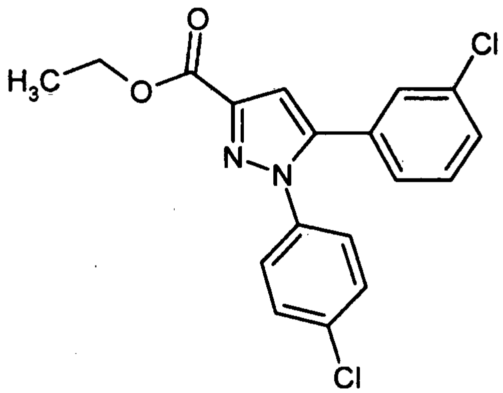

Die Herstellung der Titelverbindung erfolgt ausgehend von der Verbindung aus Beispiel 3A und 4-Chlorphenylhydrazinhydrochlorid analog zur Synthese der Verbindung aus Beispiel 21A. Nach zusätzlicher Reinigung durch Rühren in Methanol, Absaugen des Niederschlags und Trocknung am Hochvakuum erhält man 26.8 g (57% d. Th.) der Titelverbindung.

$^1$H-NMR (400 MHz, DMSO-$d_6$): $\delta$ = 7.58-7.53 (m, 2H), 7.49-7.43 (m, 2H), 7.42-7.36 (m, 3H), 7.24 (s, 1H), 7.19-7.14 (m, 1H), 4.34 (q, 2H), 1.32 (t, 3H).

LC-MS (Methode 1): $R_t$ = 3.07 min; MS (ESIpos): m/z = 361 $[M+H]^+$.

**Beispiel 40A**

5-(3-Fluorphenyl)-1-(4-fluorphenyl)-1*H*-pyrazol-3-carbonsäureethylester

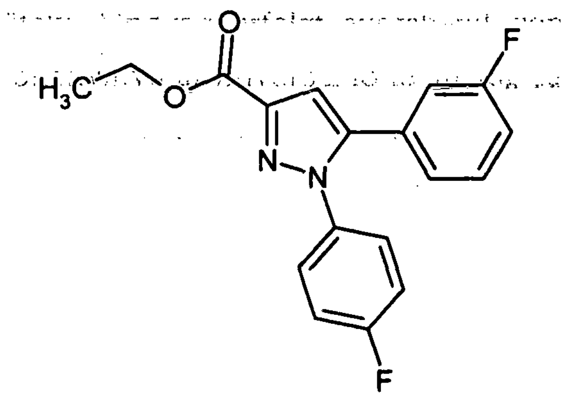

Die Herstellung der Titelverbindung erfolgt ausgehend von der Verbindung aus Beispiel 8A und 4-Fluorphenylhydrazinhydrochlorid analog zur Synthese der Verbindung aus Beispiel 21A. Man erhält 23.0 g (44% d. Th.) der Titelverbindung.

$^1$H-NMR (400 MHz, DMSO-$d_6$): δ = 7.46-7.37 (m, 3H), 7.37-7.29 (m, 2H), 7.26-7.16 (m, 3H), 7.05 (d, 1H), 4.34 (q, 2H), 1.32 (t, 3H).

LC-MS (Methode 1): $R_t$ = 2.76 min; MS (ESIpos): m/z = 329 $[M+H]^+$.

**Beispiel 41A**

1-(3-Chlorphenyl)-5-(3-fluor-5-methoxyphenyl)-1*H*-pyrazol-3-carbonsäureethylester

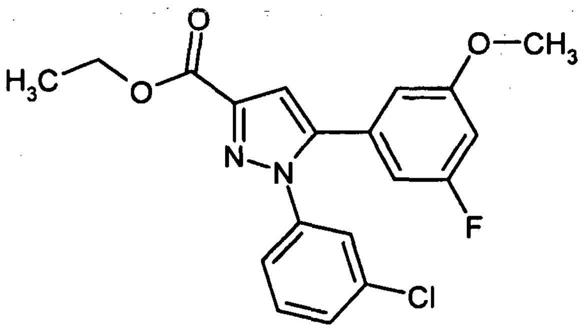

Die Herstellung der Titelverbindung erfolgt ausgehend von der Verbindung aus Beispiel 15A und 3-Chlorphenylhydrazinhydrochlorid analog zur Synthese der Verbindung aus Beispiel 21A. Man,erhält 2.20 g mit 68%iger Reinheit der Titelverbindung.

$^1$H-NMR (400 MHz, DMSO-$d_6$): δ = 7.59-7.55 (m, 2H), 7.50 (t, 1H), 7.31-7.27 (m, 1H), 7.24 (s, 1H), 6.89 (dt, 1H), 6.74-6.69 (m, 2H), 4.34 (q, 2H), 3.70 (s, 3H), 1.32 (t, 3H).

LC-MS (Methode 1): $R_t$ = 2.80 min; MS (ESIpos): m/z = 375 $[M+H]^+$.

**Beispiel 42A**

1-(3-Chlor-4-fluorphenyl)-5-(3-fluor-5-methoxyphenyl)-1*H*-pyrazol-3-carbonsäureethyl-ester

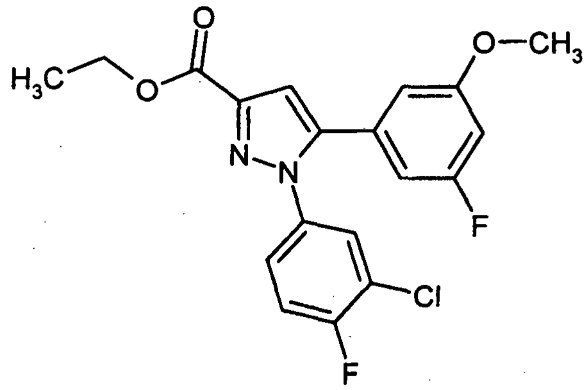

Die Herstellung der Titelverbindung erfolgt ausgehend von der Verbindung aus Beispiel 15A und 3-Chlor-4-fluorphenylhydrazinhydrochlorid analog zur Synthese der Verbindung aus Beispiel 21A. Man erhält 2.36 g mit 82%iger Reinheit der Titelverbindung.

$^1$H-NMR (400 MHz, DMSO-$d_6$): $\delta$ = 7.77 (dd, 1H), 7.54 (t, 1H), 7.36 (ddd, 1H), 7.24 (s, 1H), 6.89 (dt, 1H), 6.75-6.70 (m, 2H), 4.34 (q, 2H), 3.71 (s, 3H), 1.32 (t, 3H).

LC-MS (Methode 1): $R_t$ = 2.82 min; MS (ESIpos): m/z = 393 $[M+H]^+$.

**Beispiel 43A**

5-[3-(Benzyloxy)phenyl]-1-(3-chlorphenyl)-1*H*-pyrazol-3-carbonsäureethylester

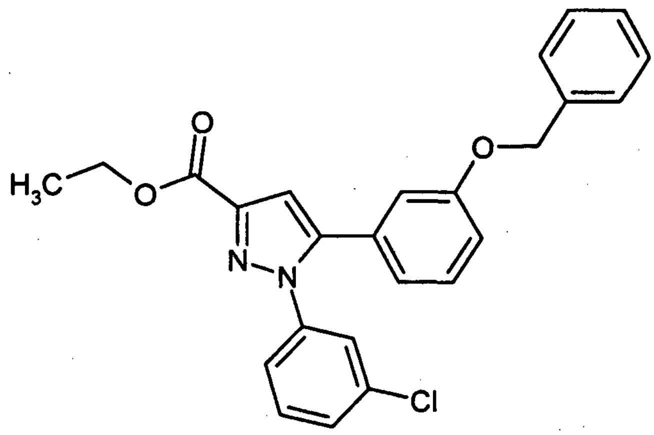

Die Herstellung der Titelverbindung erfolgt ausgehend von der Verbindung aus Beispiel 16A und 3-Chlorphenylhydrazinhydrochlorid analog zur Synthese der Verbindung aus Beispiel 21A. Man erhält 14.1 g der Titelverbindung.

$^1$H-NMR (400 MHz, DMSO-$d_6$): $\delta$ = 7.56-7.44 (m, 3H), 7.40-7.24 (m, 7H), 7.16 (s, 1H), 7.04 (dd, 1H), 7.01-6.98 (m, 1H), 6.80 (d, 1H), 5.05 (s, 2H), 4.34 (q, 2H), 1.32 (t, 3H).

LC-MS (Methode 3): $R_t$ = 3.03 min; MS (ESIpos): m/z = 433 $[M+H]^+$.

**Beispiel 44A**

1-(3-Chlorphenyl)-5-(3-hydroxyphenyl)-1*H*-pyrazol-3-carbonsäureethylester

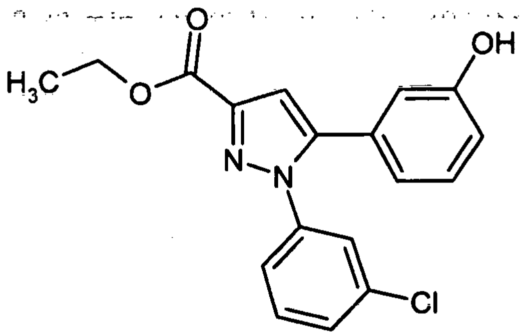

19.1 g (44.1 mmol) der Verbindung aus Beispiel 43A werden in 367 ml konz. Essigsäure vorgelegt, mit 23.5 g (11.0 mmol) Palladium auf Aktivkohle (5%ig) versetzt und bei Raumtemperatur unter Wasserstoffatmosphäre über Nacht gerührt. Anschließend wird das Reaktionsgemisch filtriert, das Filtrat eingeengt, der Rückstand in Dichlormethan aufgenommen, mit wässriger Natriumhydrogencarbonat-Lösung gewaschen, die organische Phase über Natriumsulfat getrocknet und eingeengt. Man erhält 12.7 g mit 63%iger Reinheit (53% d. Th.) der Titelverbindung.
LC-MS (Methode 1): $R_t$ = 2.41 min; MS (ESIpos): m/z = 343 $[M+H]^+$.

**Beispiel 45A**

1-(3-Chlor-4-fluorphenyl)-5-(3-cyanphenyl)-1*H*-pyrazol-3-carbonsäureethylester

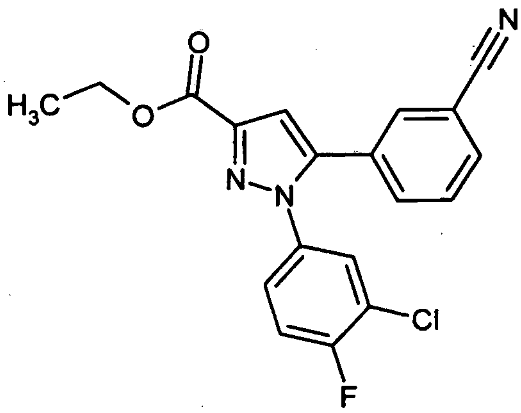

Die Herstellung der Titelverbindung erfolgt ausgehend von der Verbindung aus Beispiel 4A analog zur Synthese der Verbindung aus Beispiel 21A. Man erhält 0.50 g (80% d. Th.) der Titelverbindung.
$^1$H-NMR (400 MHz, DMSO-$d_6$): $\delta$ = 7.94-7.91 (m, 1H), 7.87 (dt, 1H), 7.77 (dd, 1H), 7.61-7.49 (m, 3H), 7.35 (ddd, 1H), 7.31 (s, 1H), 4.35 (q, 2H), 1.32 (t, 3H).
LC-MS (Methode 1): $R_t$ = 2.61 min; MS (ESIpos): m/z = 370 $[M+H]^+$.

**Beispiel 46A**

1-(3-Chlor-4-fluorphenyl)-5-(3-fluorphenyl)-1*H*-pyrazol-3-carbonsäureethylester

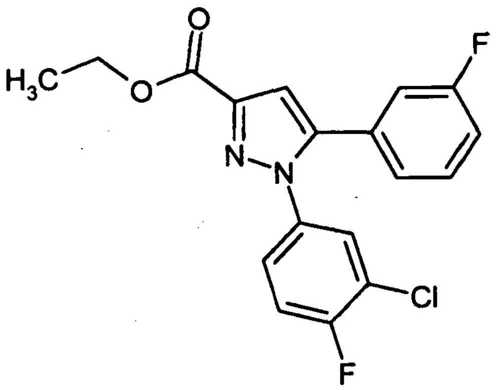

Die Herstellung der Titelverbindung erfolgt ausgehend von der Verbindung aus Beispiel 8A analog zur Synthese der Verbindung aus Beispiel 21A. Man erhält 0.60 g (93% d. Th.) der Titelverbindung.
$^1$H-NMR (400 MHz, DMSO-$d_6$): $\delta$ = 7.75 (dd, 1H), 7.53 (t, 1H), 7.47-7.39 (m, 1H), 7.35 (ddd, 1H), 7.29-7.22 (m, 3H), 7.09-7.05 (m, 1H), 4.34 (q, 2H), 1.32 (t, 3H).
LC-MS (Methode 1): $R_t$ = 2.77 min; MS (ESIpos): m/z = 363 $[M+H]^+$.

**Beispiel 47A**

1-(3-Chlor-4-fluorphenyl)5-[3-(trifluormethyl)phenyl]-1*H*-pyrazol-3-carbonsäureethylester

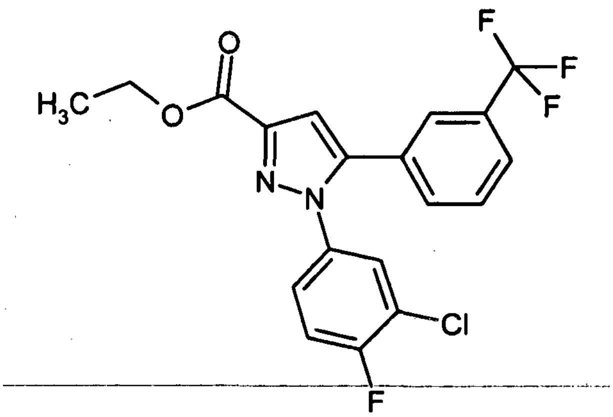

Die Herstellung der Titelverbindung erfolgt ausgehend von der Verbindung aus Beispiel 9A analog zur Synthese der Verbindung aus Beispiel 21A. Man erhält 5.10 g (100% d. Th.) der Titelverbindung.
$^1$H-NMR (400 MHz, DMSO-$d_6$): $\delta$ = 7.80-7.74 (m, 2H), 7.72-7.50 (m, 4H), 7.37 (ddd, 1H), 7.33 (s, 1H), 4.35 (q, 2H), 1.33 (t, 3H).
LC-MS (Methode 3): $R_t$ = 2.87 min; MS (ESIpos): m/z = 413 $[M+H]^+$.

**Beispiel 48A**

1-(3-Chlor-4-fluorphenyl)-5-[3-fluor-5-(trifluormethyl)phenyl]-1*H*-pyrazol-3-carbonsäureethylester

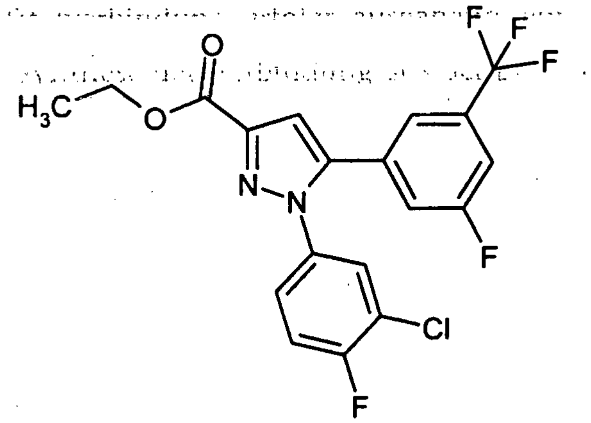

Die Herstellung der Titelverbindung erfolgt ausgehend von der Verbindung aus Beispiel 17A analog zur Synthese der Verbindung aus Beispiel 21A. Man erhält 2.43 g mit 36%iger Reinheit der Titelverbindung.

LC-MS (Methode 1): $R_t$ = 2.95 min; MS (ESIpos): m/z = 431 $[M+H]^+$.

**Beispiel 49A**

1-(3-Chlorphenyl)-5-[3-fluor-5-(trifluormethyl)phenyl]-1*H*-pyrazol-3-carbonsäureethylester

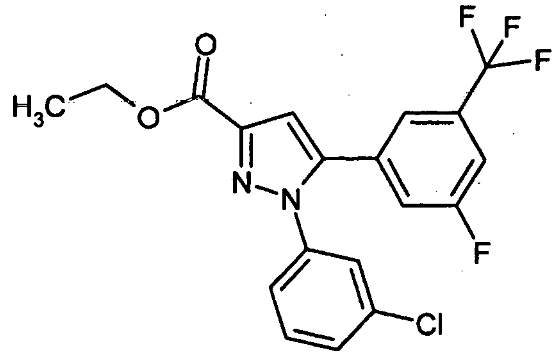

Die Herstellung der Titelverbindung erfolgt ausgehend von der Verbindung aus Beispiel 17A und 3-Chlorphenylhydrazinhydrochlorid analog zur Synthese der Verbindung aus Beispiel 21A. Man erhält 2.08 g der Titelverbindung.

LC-MS (Methode 5): $R_t$ = 1.52 min; MS (ESIpos): m/z = 413 $[M+H]^+$.

**Beispiel 50A**

1-(3-Bromphenyl)-5-(3-chlorphenyl)-1*H*-pyrazol-3-carbonsäureethylester

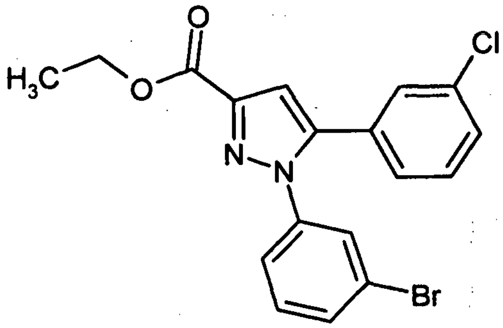

Die Herstellung der Titelverbindung erfolgt ausgehend von der Verbindung aus Beispiel 3A und 3-Bromphenylhydrazinhydrochlorid analog zur Synthese der Verbindung aus Beispiel 21A. Man erhält 2.50 g mit 70%iger Reinheit (67% d. Th.) der Titelverbindung.
LC-MS (Methode 1): $R_t$ = 2.93 min; MS (ESIpos): m/z = 405 $[M+H]^+$.

__Beispiel 51A__

1-(3-Bromphenyl)-5-(3-chlor-4-fluorphenyl)-1*H*-pyrazol-3-carbonsäureethylester

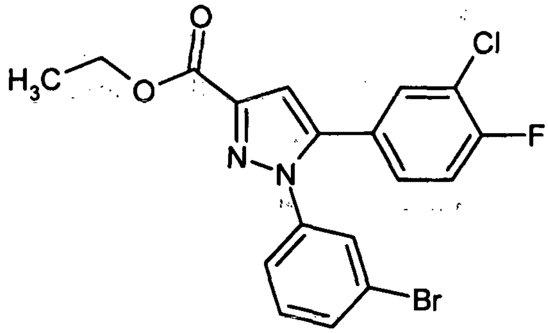

Die Herstellung der Titelverbindung erfolgt ausgehend von der Verbindung aus Beispiel 1A und 3-Bromphenylhydrazinhydrochlorid analog zur Synthese der Verbindung aus Beispiel 21A. Man erhält 689 mg mit 76%iger Reinheit (77% d. Th.) der Titelverbindung.
$^1$H-NMR (400 MHz, DMSO-$d_6$): $\delta$ = 7.73-7.69 (m, 2H), 7.52 (dt, 1H), 7.43 (t, 1H), 7.35-7.30 (m, 2H), 7.25 (s, 1H), 7.18 (dt, 1H), 4.35 (q, 2H), 1.32 (t, 3H).
LC-MS (Methode 7): $R_t$ = 2.55 min; MS (ESIpos): m/z = 423 $[M+H]^+$.

__Beispiel 52A__

5-(3-Brom-5-fluorphenyl)-1-(3-chlor-4-fluorphenyl)-1*H*-pyrazol-3-carbonsäureethylester

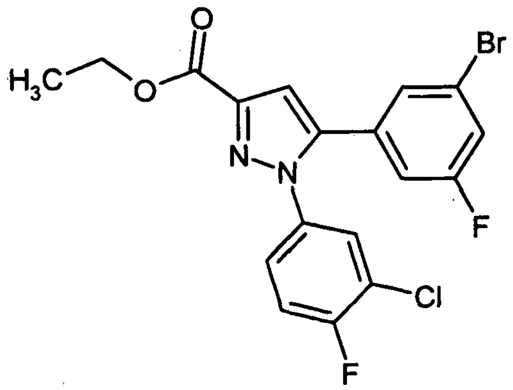

Die Herstellung der Titelverbindung erfolgt ausgehend von der Verbindung aus Beispiel 18A analog zur Synthese der Verbindung aus Beispiel 21A. Man erhält 3.87 g (89% d. Th.) der Titelverbindung.
$^{1}$H-NMR (400 MHz, DMSO-$d_6$): $\delta$ = 7.80 (dd, 1H), 7.63 (dt, 1H), 7.55 (t, 1H), 7.41-7.35 (m, 2H), 7.31 (s, 1H), 7.24-7.20 (m, 1H), 4.34 (q, 2H), 1.32 (t, 3H).
LC-MS (Methode 1): $R_t$ = 2.98 min; MS (ESIpos): m/z = 441 $[M+H]^+$.

**Beispiel 53A**

1-(3-Chlor-4-fluorphenyl)-5-(3-methoxyphenyl)-1*H*-pyrazol-3-carbonsäureethylester

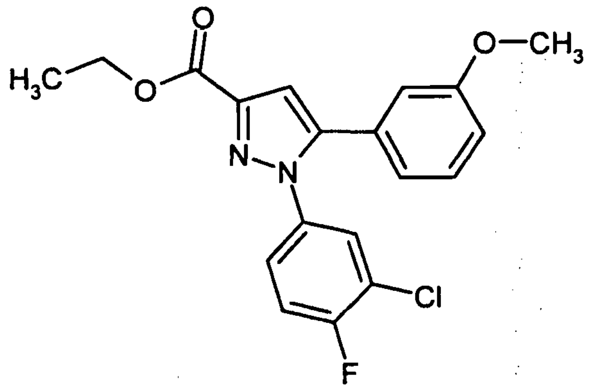

Die Herstellung der Titelverbindung erfolgt ausgehend von der Verbindung aus Beispiel 10A analog zur Synthese der Verbindung aus Beispiel 21A. Man erhält 0.60 g (97% d. Th.) der Titelverbindung.
$^{1}$H-NMR (400 MHz, DMSO-$d_6$): $\delta$ = 7.74 (dd, 1H), 7.52 (t, 1H), 7.34 (ddd, 1H), 7.29 (t, 1H), 7.17 (s, 1H), 6.96 (dd, 1H), 6.91 (t, 1H), 6.79 (d, 1H), 4.34 (q, 2H), 3.71 (s, 3H), 1.32 (t, 3H).
LC-MS (Methode 1): $R_t$ = 2.77 min; MS (ESIpos): m/z = 375 $[M+H]^+$.

**Beispiel 54A**

5-[3,5-Bis(trifluormethyl)phenyl]-1-(3-chlorphenyl)-1*H*-pyrazol-3-carbonsäureethyl-ester

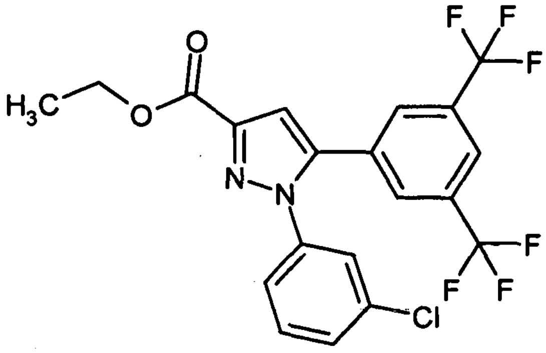

Die Herstellung der Titelverbindung erfolgt ausgehend von der Verbindung aus Beispiel 19A und 3-Chlorphenylhydrazinhydrochlorid analog zur Synthese der Verbindung aus Beispiel 21A. Man erhält 2.42 g (62% d. Th.) der Titelverbindung.
$^1$H-NMR (400 MHz, DMSO-d$_6$): $\delta$ = 8.15 (s, 1H), 7.96 (s, 2H), 7.63-7.57 (m, 2H), 7.55 (s, 1H), 7.50 (t, 1H), 7.34 (d, 1H), 4.36 (q, 2H), 1.33 (t, 3H).
LC-MS (Methode 1): $R_t$ = 3.16 min; MS (ESIpos): m/z = 463 $[M+H]^+$.

**Beispiel 55A**

1-(4-Chlorphenyl)-5-[3-(trifluormethyl)phenyl]-1*H*-pyrazol-3-carbonsäureethylester

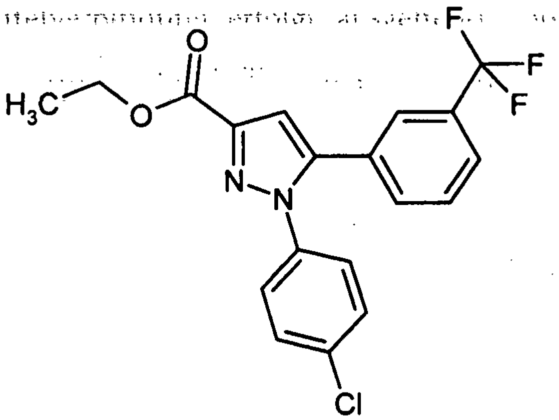

Die Herstellung der Titelverbindung erfolgt ausgehend von der Verbindung aus Beispiel 9A und 4-Chlorphenylhydrazinhydrochlorid analog zur Synthese der Verbindung aus Beispiel 21A. Man erhält 4.78 g (98% d. Th.) der Titelverbindung.
$^1$H-NMR (400 MHz, DMSO-d$_6$): $\delta$ = 7.76 (d, 1H), 7.67-7.59 (m, 2H), 7.58-7.53 (m, 3H), 7.43-7.38 (m, 2H), 7.33 (s, 1H), 4.35 (q, 2H), 1.33 (t, 3H).
LC-MS (Methode 6): $R_t$ = 4.11 min; MS (ESIpos): m/z = 395 $[M+H]^+$.

**Beispiel 56A**

1-(4-Chlorphenyl)-5-(3-fluorphenyl)-1*H*-pyrazol-3-carbonsäureethylester

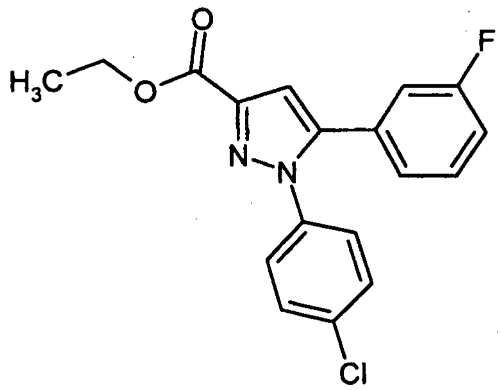

Die Herstellung der Titelverbindung erfolgt ausgehend von der Verbindung aus Beispiel 8A und 4-Chlorphenylhydrazinhydrochlorid analog zur Synthese der Verbindung aus Beispiel 21A. Man erhält 43.4 g (87% d. Th.) der Titelverbindung.

$^1$H-NMR (400 MHz, DMSO-$d_6$): $\delta$ = 7.58-7.53 (m, 2H), 7.46-7.36 (m, 3H), 7.28-7.19 (m, 3H), 7.06 (d, 1H), 4.34 (q, 2H), 1.32 (t, 3H).

LC-MS (Methode 6): $R_t$ = 3.93 min; MS (ESIpos): m/z = 345 $[M+H]^+$.

**Beispiel 57A**

1-(3-Chlor-4-fluorphenyl)-5-(3-fluor-5-methylphenyl)-1*H*-pyrazol-3-carbonsäureethylester

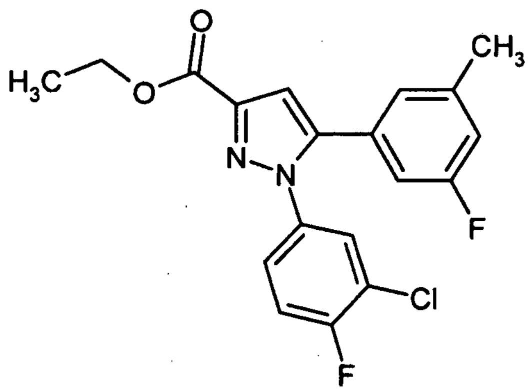

Die Herstellung der Titelverbindung erfolgt ausgehend von der Verbindung aus Beispiel 20A analog zur Synthese der Verbindung aus Beispiel 21A. Man erhält 2.79 g mit 78%iger Reinheit der Titelverbindung.

$^1$H-NMR (400 MHz, DMSO-$d_6$): $\delta$ = 7.75 (dd, 1H), 7.53 (t, 1H), 7.34 (ddd, 1H), 7.19 (s, 1H), 7.10 (d, 1H), 7.01 (s, 1H), 6.91 (s, 1H), 4.34 (q, 2H), 2.27 (s, 3H), 1.32 (t, 3H).

LC-MS (Methode 1): $R_t$ = 2.98 min; MS (ESIpos): m/z = 377 $[M+H]^+$.

**Beispiel 58A**

1-(3-Chlorphenyl)-5-(3-fluor-5-methylphenyl)-1*H*-pyrazol-3-carbonsäureethylester

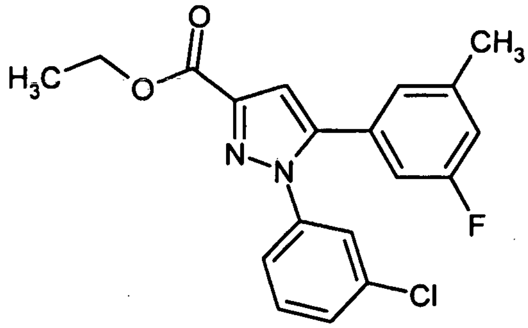

Die Herstellung der Titelverbindung erfolgt ausgehend von der Verbindung aus Beispiel 20A und 3-Chlorphenylhydrazinhydrochlorid analog zur Synthese der Verbindung aus Beispiel 21A. Man erhält 1.75 g der Titelverbindung.
$^1$H-NMR (400 MHz, DMSO-$d_6$): $\delta$ = 7.58-7.53 (m, 2H), 7.48 (t, 1H), 7.29-7.25 (m, 1H), 7.19 (s, 1H), 7.12-7.07 (m, 1H), 7.01 (s, 1H), 6.91-6.86 (m, 1H), 4.34 (q, 2H), 2.27 (s, 3H), 1.32 (t, 3H).
LC-MS (Methode 1): $R_t$ = 2.97 min; MS (ESIpos): m/z = 359 $[M+H]^+$.

**Beispiel 59A**

5-(3-Chlor-4-fluorphenyl)-1-(3-cyanphenyl)-1*H*-pyrazol-3-carbonsäureethylester

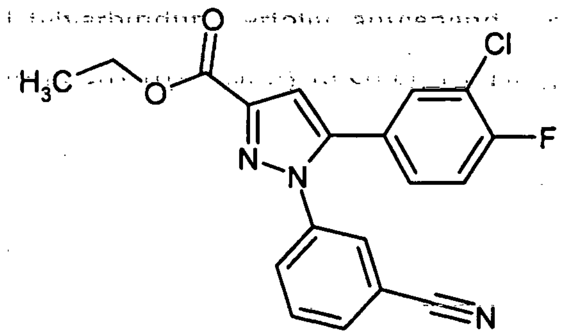

100 mg (0.236 mmol) der Verbindung aus Beispiel 51A werden in 1 ml 1-Methyl-2-pyrrolidon vorgelegt, mit 55.4 mg (0.472 mmol) Zinkcyanid und 27.3 mg (0.024 mmol) Tetrakis(triphenylphosphin)palladium(0) versetzt und 30 min bei 200 °C unter Mikrowellenbestrahlung in einem geschlossenen Glasgefäß erhitzt. Anschließend wird das Reaktionsgemisch über Kieselgur filtriert, mit Methanol eluiert, das Filtrat mit 1N wässriger Hydrogenchlorid-Lösung schwach sauer gestellt, eingeengt und der Rückstand über die präparative HPLC (RP18-Säule; Laufmittel: Acetonitril/Wasser-Gradient) gereinigt. Man erhält 33 mg (34% d. Th.) der Titelverbindung.
LC-MS (Methode 1): $R_t$ = 2.72 min; MS (ESIpos): m/z = 370 $[M+H]^+$.

**Beispiel 60A**

5-(3-Chlorphenyl)-1-(3-cyan-4-fluorphenyl)-1*H*-pyrazol-3-carbonsäureethylester

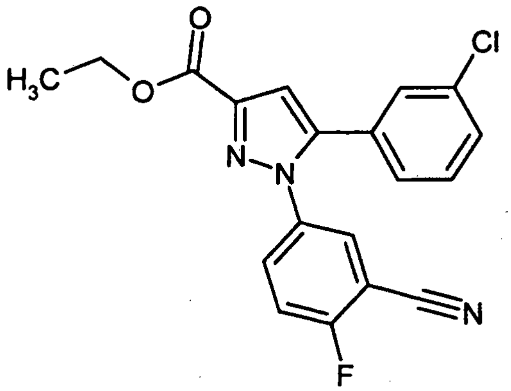

Die Herstellung der Titelverbindung erfolgt ausgehend von der Verbindung aus Beispiel 3A und 3-Cyano-4-fluorphenylhydrazinhydrochlorid analog zur Synthese der Verbindung aus Beispiel 21A. Man erhält 0.76 g mit 46%iger Reinheit (23% d. Th.) der Titelverbindung.

LC-MS (Methode 7): $R_t$ = 2.29 min; MS (ESIpos): m/z = 370 $[M+H]^+$.

## Beispiel 61A

5-(3-Chlorphenyl)-1-(3-cyanphenyl)-1*H*-pyrazol-3-carbonsäureethylester

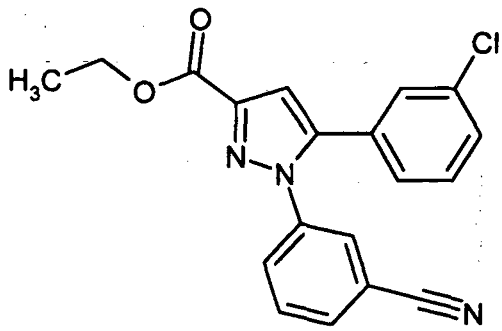

Die Herstellung der Titelverbindung erfolgt ausgehend von der Verbindung aus Beispiel 3A und 3-Cyanophenylhydrazinhydrochlorid analog zur Synthese der Verbindung aus Beispiel 21A. Man erhält 540 mg mit 69%iger Reinheit (25% d. Th.) der Titelverbindung.

LC-MS (Methode 7): $R_t$ = 2.24 min; MS (ESIpos): m/z = 352 $[M+H]^+$.

## Beispiel 62A

1,5-Bis(3-cyanphenyl)-1*H*-pyrazol-3-carbonsäureethylester

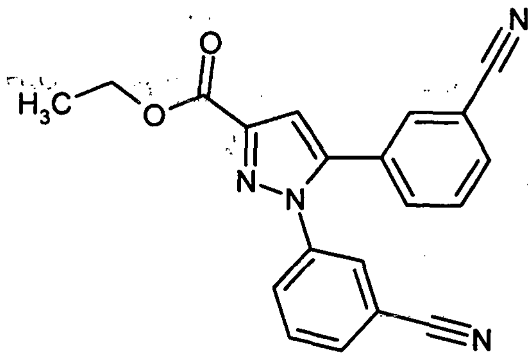

Die Herstellung der Titelverbindung erfolgt ausgehend von der Verbindung aus Beispiel 4A und 3-Cyanophenylhydrazinhydrochlorid analog zur Synthese der Verbindung aus Beispiel 21A. Man erhält 440 mg mit 26%iger Reinheit der Titelverbindung.

LC-MS (Methode 7): $R_t$ = 1.95 min; MS (ESIpos): m/z = 343 $[M+H]^+$.

**Beispiel 63A**

1-(3-Cyan-4-fluorphenyl)-5-(3-cyanphenyl)-1*H*-pyrazol-3-carbonsäureethylester

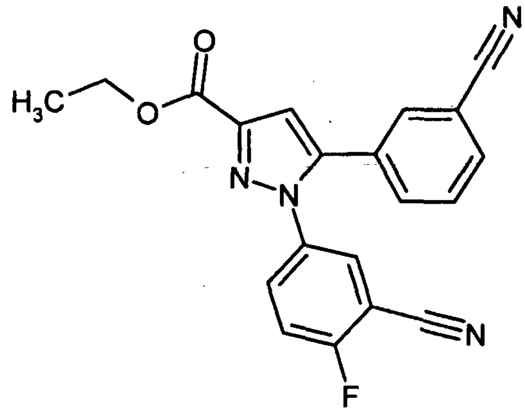

Die Herstellung der Titelverbindung erfolgt ausgehend von der Verbindung aus Beispiel 4A und 3-Cyano-4-fluorphenylhydrazinhydrochlorid analog zur Synthese der Verbindung aus Beispiel 21A. Man erhält 510 mg mit 27%iger Reinheit der Titelver-bindung.

LC-MS (Methode 7): $R_t$ = 2.00 min; MS (ESIpos): m/z = 361 $[M+H]^+$.

**Beispiel 64A**

5-(3-Chlor-5-fluorphenyl)-1-(3-cyanphenyl)-1*H*-pyrazol-3-carbonsäureethylester

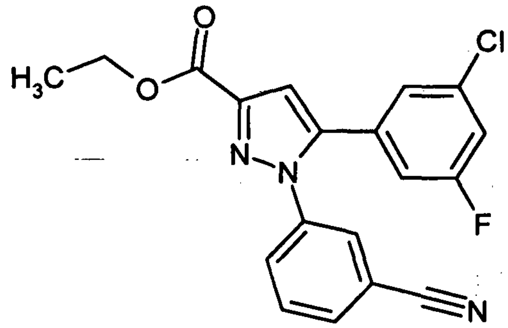

Die Herstellung der Titelverbindung erfolgt ausgehend von der Verbindung aus Beispiel 1A und 3-Cyanophenylhydrazinhydrochlorid analog zur Synthese der Verbindung aus Beispiel 21A. Man erhält 1.96 g mit 55%iger Reinheit (86% d. Th.) der Titelverbindung.
LC-MS (Methode 1): $R_t$ = 2.71 min; MS (ESIpos): m/z = 370 $[M+H]^+$.

__Beispiel 65A__

5-(3-Chlor-5-fluorphenyl)-1-(3-cyan-4-fluorphenyl)-1*H*-pyrazol-3-carbonsäureethylester

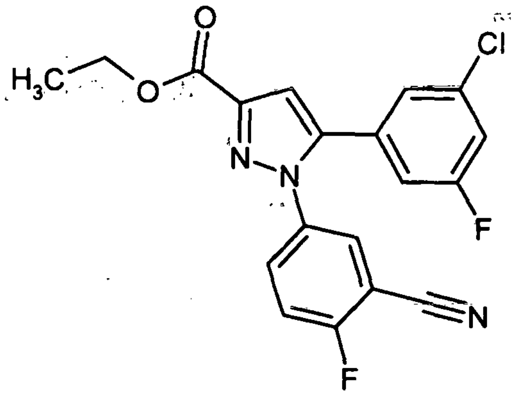

Die Herstellung der Titelverbindung erfolgt ausgehend von der Verbindung aus Beispiel 1A und 3-Cyano-4-fluorphenylhydrazinhydrochlorid analog zur Synthese der Verbindung aus Beispiel 21A. Man erhält 2.66 g mit 49%iger Reinheit (100% d. Th.) der Titelverbindung.
LC-MS (Methode 1): $R_t$ = 2.75 min; MS (ESIpos): m/z = 388 $[M+H]^+$.

__Beispiel 66A__

1-(3-Chlorphenyl)-5-[3-(2-hydroxyethoxy)phenyl]-1*H*-pyrazol-3-carbonsäureethylester

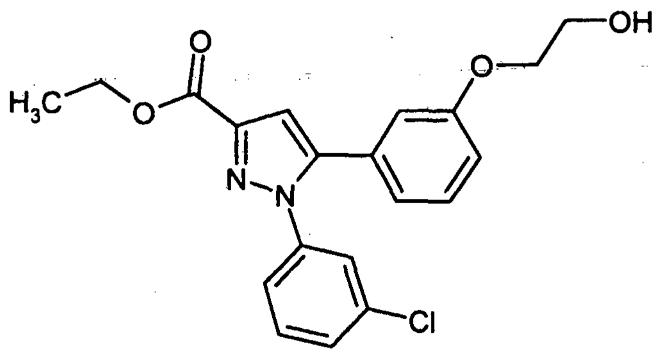

100 mg (0.29 mmol) der Verbindung aus Beispiel 44A werden in 2 ml trockenem Aceton vorgelegt, mit 44.4 mg (0.32 mmol) Kaliumcarbonat und 36.5 mg (0.29 mmol) 2-Bromethanol versetzt und über Nacht unter Rückfluss erhitzt. Man reinigt anschließend die Reaktionsmischung über die präparative HPLC (RP18-Säule; Laufmittel: Acetonitril/Wasser-Gradient unter Zusatz von 0.1% Ameisensäure) und erhält 13.1 mg der Titelrbindung.
$^1$H-NMR (400 MHz, DMSO-$d_6$): $\delta$ = 7.56-7.44 (m, 3H), 7.30-7.24 (m, 2H), 7.16 (s, 1H), 6.96 (dd, 1H), 6.91-6.89 (m, 1H), 6.78 (d, 1H), 4.85 (t, 1H), 4.34 (q, 2H), 3.91 (t, 2H), 3.66 (q, 2H), 1.32 (t, 3H).
LC-MS (Methode 1): $R_t$ = 2.35 min; MS (ESIpos): m/z = 387 $[M+H]^+$.

**Beispiel 67A**

S-{3-[3-(Acetyloxy)propoxy]phenyl}-1-(3-chlorphenyl)-1*H*-pyrazol-3-carbonsäureethylester

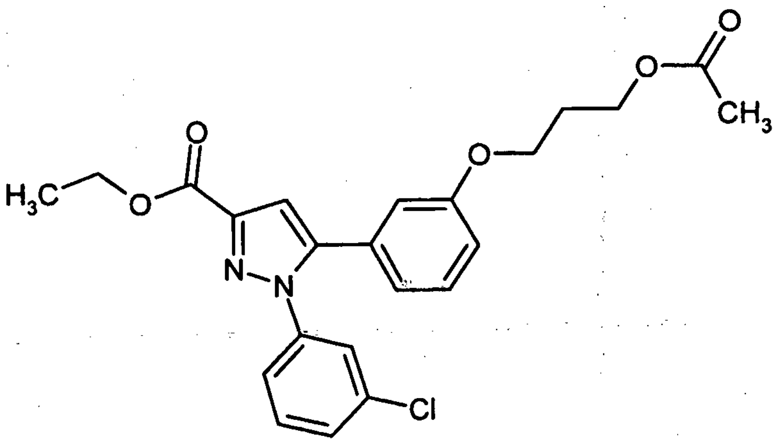

100 mg (0.292 mmol) der Verbindung aus Beispiel 44A werden in 1 ml trockenem DMF vorgelegt, mit 242 mg (1.75 mmol) Kaliumcarbonat und 158 mg (0.875 mmol) 1-Acetoxy-3-brompropan versetzt und 4 h bei 90°C gerührt. Man reinigt anschließend die Reaktionsmischung über die präparative HPLC (RP18-Säule; Laufmittel: Acetonitril/Wasser-Gradient unter Zusatz von 0.1% Ameisensäure) und erhält 109 mg (84% d. Th.) der Titelverbindung.
LC-MS (Methode 1): $R_t$ = 2.79 min; MS (ESIpos): m/z = 443 $[M+H]^+$.

**Beispiel 68A**

1-(3-Chlorphenyl)-5-[3-(3-chlorpropoxy)phenyl]-1*H*-pyrazol-3-carbonsäureethylester

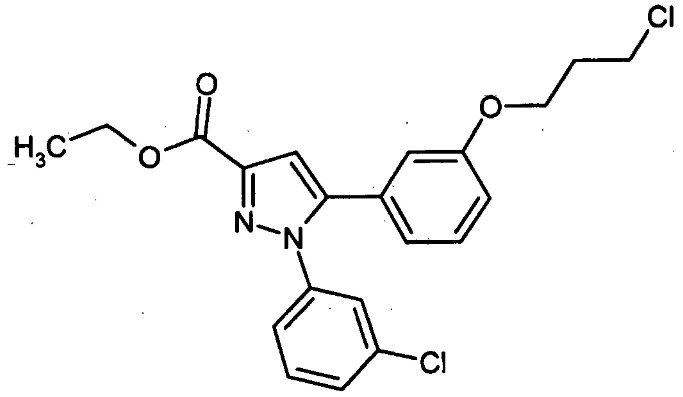

100 mg (0.292 mmol) der Verbindung aus Beispiel 44A werden in 3 ml trockenem DMF vorgelegt, mit 76.6 mg (0.554 mmol) Kaliumcarbonat und 133 mg (0.846 mmol) 1-Brom-3-chlorpropan versetzt und 4 h unter Rückfluss erhitzt. Man reinigt anschließend die Reaktionsmischung über die präparative HPLC (RP18-Säule; Laufmittel: Acetonitril/Wasser-Gradient unter Zusatz von 0.1% Ameisensäure) und erhält 42 mg mit 49%iger Reinheit (17% d. Th.) der Titelverbindung.

LC-MS (Methode 7): $R_t$ = 2.53 min; MS (ESIpos): m/z = 419 $[M+H]^+$.

__Beispiel 69A__

1-(3-Chlorphenyl)-5-[3-(2-methoxyethoxy)phenyl]-1*H*-pyrazol-3-carbonsäureethylester

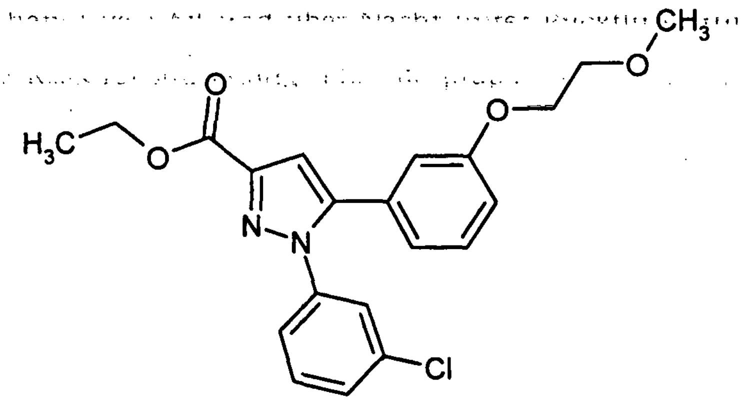

100 mg (0.292 mmol) der Verbindung aus Beispiel 44A werden in 2 ml trockenem Aceton vorgelegt, mit 44.4 mg (0.321 mmol) Kaliumcarbonat und 40.6 mg (0.292 mmol) 2-Bromethylmethylether versetzt und über Nacht unter Rückfluss erhitzt. Man reinigt anschließend die Reaktionsmischung über die präparative HPLC (RP18-Säule; Laufmittel: Acetonitril/Wasser-Gradient unter Zusatz von 0.1% Ameisensäure) und erhält 48 mg (41% d. Th.) der Titelverbindung.

$^1$H-NMR (400 MHz, DMSO-$d_6$): $\delta$ = 7.56-7.44 (m, 3H), 7.30-7.24 (m, 2H), 7.17 (s, 1H), 6.97 (dd, 1H), 6.93-6.89 (m, 1H), 6.79 (d, 1H), 4.34 (q, 2H), 4.05-4.00 (m, 2H), 3.61-3.57 (m, 2H), 3.28 (s, 3H), 1.32 (t, 3H).

LC-MS (Methode 1): $R_t$ = 2.68 min; MS (ESIpos): m/z = 401 $[M+H]^+$.

__Beispiel 70A__

1-(3-Chlorphenyl)-5-[3-(3-pyrrolidin-1-ylpropoxy)phenyl]-1*H*-pyrazol-3-carbonsäureethylester

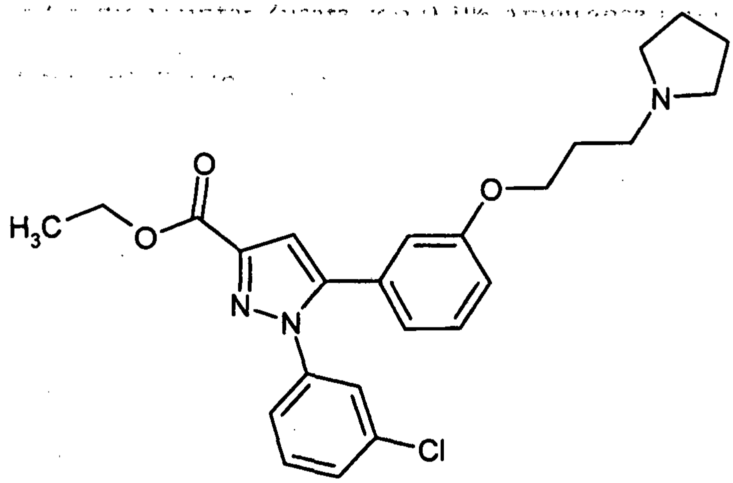

41.5 mg (0.10 mmol) der Verbindung aus Beispiel 68A und 83 µl (0.99 mmol) Pyrrolidin in 3 ml Ethanol werden über Nacht bei 80°C gerührt. Man reinigt anschließend die Reaktionsmischung über die präparative HPLC (RP18-Säule; Laufmittel: Acetonitril/Wasser-Gradient). Man erhält 18.3 mg der Titelverbindung.
LC-MS (Methode 7): $R_t$ = 1.51 min; MS (ESIpos): m/z = 454 $[M+H]^+$.

**Beispiel 71A**

1-(3-Chlor-4-fluorphenyl)-5-(3-chlor-5-fluorphenyl)-1*H*-pyrazol-3-carbonsäure

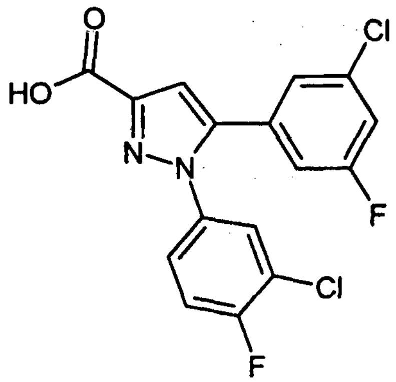

5.11 g (12.9 mmol) der Verbindung aus Beispiel 21A werden in 142 ml Tetrahydrofuran vorgelegt und bei Raumtemperatur mit 3.08 g (129 mmol) Lithiumhydroxid und 47 ml Wasser versetzt. Man rührt bei Raumtemperatur über Nacht, gibt anschließend 1N wässrige Hydrogenchlorid-Lösung bis zu einem sauren pH-Wert hinzu, extrahiert mit Essigsäureethylester, wäscht die organische Phase mit Wasser, trocknet über Natriumsulfat, filtriert und engt ein. Man erhält 4.51 g (90% d. Th.) der Titelverbindung.
$^1H$-NMR (400 MHz, DMSO-$d_6$): $\delta$ = 13.2 (s, 1H), 7.78 (dd, 1H), 7.58-7.49 (m, 2H), 7.36 (ddd, 1H), 7.28-7.25 (m, 1H), 7.24 (s, 1H), 7.21-7.16 (m, 1H).
LC-MS (Methode 1): $R_t$ = 2.52 min; MS (ESIpos): m/z = 369 $[M+H]^+$.

**Beispiel 72A**

5-(3-Chlor-5-fluorphenyl)-1-(3-chlorphenyl)-1*H*-pyrazol-3-carbonsäure

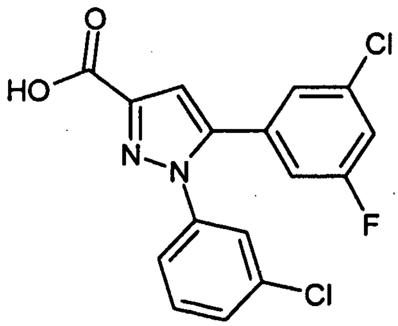

Die Herstellung der Titelverbindung erfolgt ausgehend von der Verbindung aus Beispiel 22A analog zur Synthese der Verbindung aus Beispiel 71A. Man erhält 4.18 g (78% d. Th.) der Titelverbindung.
$^1$H-NMR (400 MHz, DMSO-$d_6$): $\delta$ = 13.1 (s, 1H), 7.59-7.55 (m, 2H), 7.54-7.45 (m, 2H), 7.30-7.26 (m, 1H), 7.26-7.23 (m, 2H), 7.20-7.15 (m, 1H).
LC-MS (Methode 1): $R_t$ = 2.49 min; MS (ESIpos): m/z = 351 $[M+H]^+$.

**Beispiel 73A**

1-(3-Chlorphenyl)-5-(3,5-difluorphenyl)-1*H*-pyrazol-3-carbonsäure

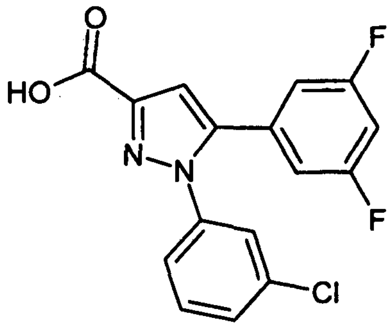

2.50 g (6.89 mmol) der Verbindung aus Beispiel 23A werden in 62 ml einer 2:1 Mischung aus 1,4-Dioxan und Wasser vorgelegt, mit 35 ml (70 mmol) einer 2N Lösung von Lithiumhydroxid in Wasser versetzt und bei Raumtemperatur über Nacht gerührt. Man engt ein, gibt anschließend zu dem Rückstand 2N wässrige Hydrogenchlorid-Lösung bis zu einem sauren pH-Wert hinzu, extrahiert mit Essigsäureethylester, trocknet die organische Phase über Natriumsulfat, filtriert und engt ein. Man erhält 2.28 g (99% d. Th.) der Titelverbindung.
$^1$H-NMR (400 MHz, DMSO-$d_6$): $\delta$ = 13.2 (s, 1H), 7.59-7.54 (m, 2H), 7.53-7.47 (m, 1H), 7.33 (tt, 1H), 7.30-7.26 (m, 1H), 7.24 (s, 1H), 7.10-7.02 (m, 2H).
LC-MS (Methode 3): $R_t$ = 2.21 min; MS (ESIpos): m/z = 335 $[M+H]^+$.

**Beispiel 74A**

1,5-Bis(3-chlorphenyl)-1*H*-pyrazol-3-carbonsäure

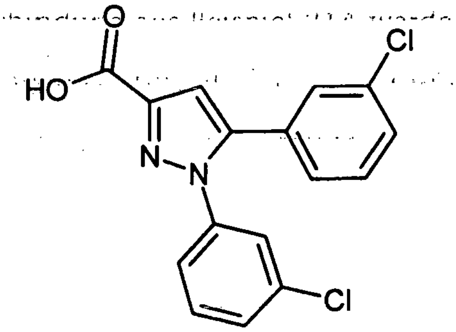

Die Herstellung der Titelverbindung erfolgt ausgehend von der Verbindung aus Beispiel 24A analog zur Synthese der Verbindung aus Beispiel 71A. Man erhält 5.97 g (94% d. Th.) der Titelverbindung.
$^1$H-NMR (400 MHz, DMSO-$d_6$): $\delta$ = 13.1 (s, 1H), 7.57-7.37 (m, 6H), 7.28-7.24 (m, 1H), 7.21-7.18 (m, 1H), 7.17 (s, 1H).
LC-MS (Methode 3): $R_t$ = 2.28 min; MS (ESIpos): m/z = 333 $[M+H]^+$.

**Beispiel 75A**

1,5-Bis(3-chlor-4-fluorphenyl)-1*H*-pyrazol-3-carbonsäure

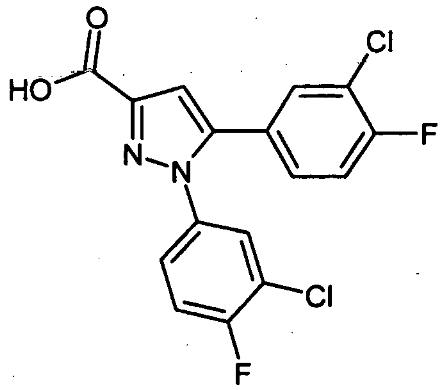

Die Herstellung der Titelverbindung erfolgt ausgehend von der Verbindung aus Beispiel 25A analog zur Synthese der Verbindung aus Beispiel 71A. Man erhält 5.15 g (96% d. Th.) der Titelverbindung.
$^1$H-NMR (400 MHz, DMSO-$d_6$): $\delta$ = 13.1 (s, 1H), 7.76 (dd, 1H), 7.65 (dd, 1H), 7.52 (t, 1H), 7.44 (t, 1H), 7.33 (ddd, 1H), 7.23 (ddd, 1H), 7.18 (s, 1H)
LC-MS (Methode 1): $R_t$ = 2.49 min; MS (ESIpos): m/z = 369 $[M+H]^+$.

**Beispiel 76A**

1-(3-Chlorphenyl)-5-[3-(trifluormethoxy)phenyl]-1*H*-pyrazol-3-carbonsäure

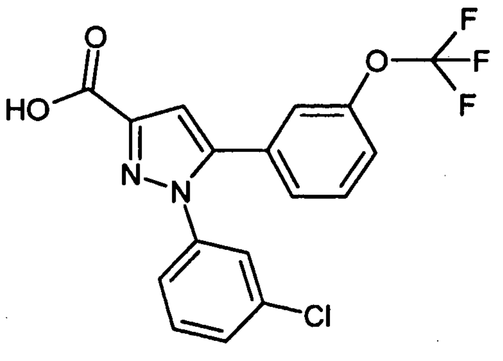

10.8 g (26.2 mmol) der Verbindung aus Beispiel 26A werden in 236 ml 1,4-Dioxan vorgelegt, mit 236 ml (472 mmol) einer 2N Lösung von Lithiumhydroxid in Wasser versetzt und 2 h bei 70°C gerührt. Man engt ein, gibt anschließend zu dem Rückstand 2N wässrige Hydrogenchlorid-Lösung bis zu einem sauren pH-Wert hinzu, extrahiert mit Dichlormethan, trocknet die organische Phase über Magnesiumsulfat, filtriert und engt ein. Man erhält 9.90 g (99% d. Th.) der Titelverbindung.

$^{1}$H-NMR (400 MHz, DMSO-$d_6$): $\delta$ = 13.1 (s, 1H), 7.59-7.45 (m, 4H), 7.44-7.38 (m, 2H), 7.31-7.26 (m, 1H), 7.22-7.18 (m, 2H).

LC-MS (Methode 1): $R_t$ = 2.77 min; MS (ESIpos): m/z = 383 $[M+H]^+$.

__Beispiel 77A__

1-(3-Chlorphenyl)-5-[4-fluor-3-(trifluormethyl)phenyl]-1*H*-pyrazol-3-carbonsäure

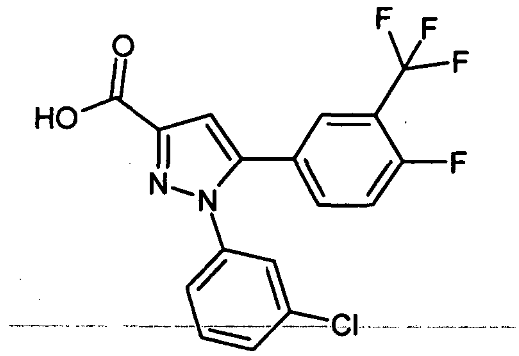

3.60 g (8.72 mmol) der Verbindung aus Beispiel 27A werden in 50 ml 1,4-Dioxan vorgelegt, mit 50 ml (50 mmol) einer 1N Lösung von Lithiumhydroxid in Wasser versetzt und 1 h unter Rückfluss erhitzt. Man engt ein, verdünnt den Rückstand mit Wasser, gibt anschließend konz. wässrige Hydrogenchlorid-Lösung bis zu einem sauren pH-Wert hinzu, extrahiert mit Essigsäureethylester, trocknet die organische Phase über Magnesiumsulfat, filtriert und engt ein. Man erhält 3.30 g (98% d. Th.) der Titelverbindung.

$^{1}$H-NMR (400 MHz, DMSO-$d_6$): $\delta$ = 7.70 (d, 1H), 7.55-7.44 (m, 3H), 7.40 (t, 1H), 7.30 (s, 1H), 7.18 (d, 1H), 6.99 (s, 1H).

LC-MS (Methode 3): $R_t$ = 2.41 min; MS (ESIpos): m/z = 385 $[M+H]^+$.

__Beispiel 78A__

1-(3-Chlor-4-fluorphenyl)-5-[3-(trifluormethoxy)phenyl]-1*H*-pyrazol-3-carbonsäure

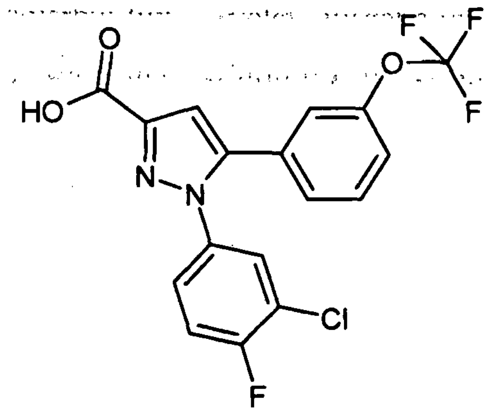

Die Herstellung der Titelverbindung erfolgt ausgehend von der Verbindung aus Beispiel 28A analog zur Synthese der Verbindung aus Beispiel 71A. Man erhält 3.14 g (96% d. Th.) der Titelverbindung.
$^1$H-NMR (400 MHz, DMSO-$d_6$): $\delta$ = 13.1 (s, 1H), 7.72 (dd, 1H), 7.59-7.50 (m, 2H), 7.44-7.34 (m, 3H), 7.23-7.18 (m, 2H).
LC-MS (Methode 1): $R_t$ = 2.56 min; MS (ESIpos): m/z = 401 $[M+H]^+$.

**Beispiel 79A**

5-(3-Chlor-4-fluorphenyl)-1-(3-chlorphenyl)-1*H*-pyrazol-3-carbonsäure

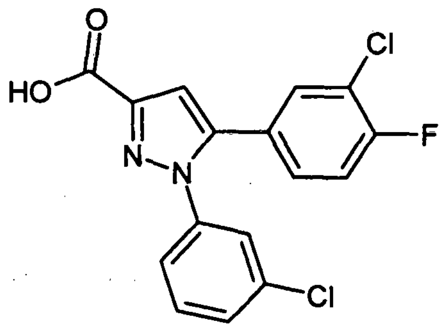

Die Herstellung der Titelverbindung erfolgt ausgehend von der Verbindung aus Beispiel 29A analog zur Synthese der Verbindung aus Beispiel 71A. Man erhält 5.30 g (100% d. Th.) der Titelverbindung.
$^1$H-NMR (400 MHz, DMSO-$d_6$): $\delta$ = 13.1 (s, 1H), 7.63 (dd, 1H), 7.57-7.52 (m, 2H), 7.51-7.42 (m, 2H), 7.28-7.21 (m, 2H), 7.17 (s, 1H).
LC-MS (Methode 1): $R_t$ = 2.46 min; MS (ESIpos): m/z = 351 $[M+H]^+$.

**Beispiel 80A**

5-(3-Fluorphenyl)-1-(3-methoxyphenyl)-1*H*-pyrazol-3-carbonsäure

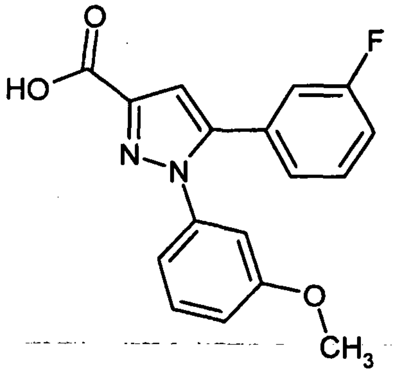

34.0 g (99.9 mmol) der Verbindung aus Beispiel 30A werden in 150 ml 1,4-Dioxan vorgelegt, mit 150 ml (300 mmol) einer 2N Lösung von Lithiumhydroxid in Wasser versetzt und 1 h bei 70 °C gerührt. Man engt ein, gibt anschließend zu dem Rückstand konz. wässrige Hydrogenchlorid-Lösung bis zu einem sauren pH-Wert hinzu, extrahiert mit Dichlormethan, trocknet die organische Phase über Magnesiumsulfat, filtriert und engt ein. Der Rückstand wird aus Diethylether umkristallisiert. Man erhält 26.1 g (84% d. Th.) der Titelverbindung.
$^1$H-NMR (400 MHz, DMSO-$d_6$): $\delta$ = 13.0 (s, 1H), 7.45-7.32 (m, 2H), 7.26-7.13 (m, 3H), 7.08 (d, 1H), 7.03 (dd, 1H), 6.95 (t, 1H), 6.85 (d, 1H), 3.72 (s, 3H).
LC-MS (Methode 1): $R_t$ = 2.33 min; MS (ESIpos): m/z = 313 [M+H]$^+$.

**Beispiel 81A**

1-(3-Chlorphenyl)-5-(3-methoxyphenyl)-1*H*-pyrazol-3-carbonsäure

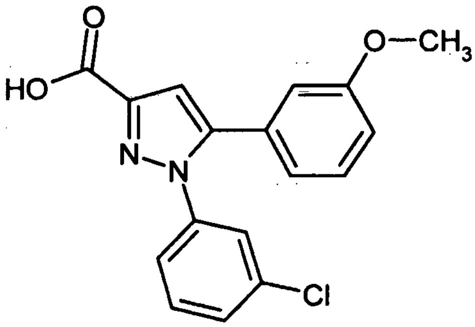

1.50 g (4.20 mmol) der Verbindung aus Beispiel 31A werden in 20 ml 1,4-Dioxan vorgelegt, mit 20 ml (20 mmol) einer 1N Lösung von Lithiumhydroxid in Wasser versetzt und 1 h bei 70°C gerührt. Man engt ein, gibt anschließend zu dem Rückstand 1N wässrige Hydrogenchlorid-Lösung bis zu einem sauren pH-Wert hinzu, extrahiert mit Essigsäureethylester, trocknet die vereinigten organischen Phasen über Magnesiumsulfat, filtriert und engt ein. Man erhält 1.32 g (92% d. Th.) der Titelverbindung.
$^1$H-NMR (400 MHz, DMSO-$d_6$): $\delta$ = 13.1 (s, 1H), 7.56-7.44 (m, 3H), 7.32-7.23 (m, 2H), 7.11 (s, 1H), 6.96 (dd, 1H), 6.88 (s, 1H), 6.80 (d, 1H), 3.69 (s, 3H).
LC-MS (Methode 3): $R_t$ = 2.14 min; MS (ESIpos): m/z = 329 [M+H]$^+$.

**Beispiel 82A**

5-(3-Chlorphenyl)-1-[3-(trifluormethyl)phenyl]-1*H*-pyrazol-3-carbonsäure

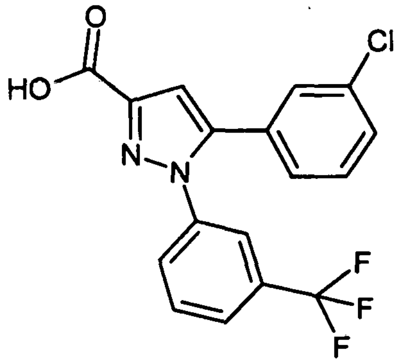

2.00 g (5.07 mmol) der Verbindung aus Beispiel 32A werden in 37 ml 1,4-Dioxan vorgelegt, mit einer Lösung aus 1.07 g (20.0 mmol) Lithiumhydroxid in 37 ml Wasser versetzt und über Nacht bei Raumtemperatur gerührt. Man engt ein, gibt anschliesend zu dem Rückstand 1N wässrige Hydrogenchlorid-Lösung bis zu einem sauren pH-Wert hinzu, extrahiert mit Essigsäureethylester, trocknet die vereinigten organischen Phasen über Natriumsulfat, filtriert und engt ein. Man erhält 0.74 g (40% d. Th.) der Titelverbindung.

$^1$H-NMR (500 MHz, DMSO-$d_6$): $\delta$ = 7.75 (d, 1H), 7.70-7.62 (m, 2H), 7.54 (d, 1H), 7.45 (d, 1H), 7.42-7.36 (m, 2H), 7.19 (d, 1H), 6.92 (s, 1H).

LC-MS (Methode 1): $R_t$ = 2.65 min; MS (ESIpos): m/z = 367 $[M+H]^+$.

**Beispiel 83A**

1-(3-Chlorphenyl)-5-(3,4-difluorphenyl)-1*H*pyrazol-3-carbonsäure

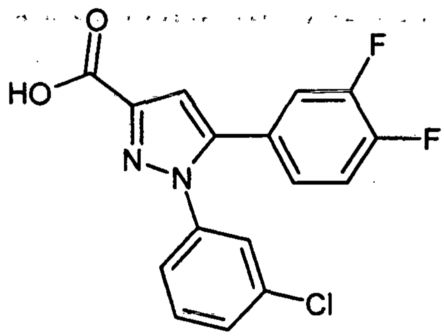

Die Herstellung der Titelverbindung erfolgt ausgehend von der Verbindung aus Beispiel 33A analog zur Synthese der Verbindung aus Beispiel 73A. Man erhält 2.49 g (96% d. Th.) der Titelverbindung.

$^1$H-NMR (400 MHz, DMSO-$d_6$): $\delta$ = 13.1 (s, 1H), 7.57-7.43 (m, 5H), 7.27-7.22 (m, 1H), 7.16 (s, 1H), 7.12-7.06 (m, 1H).

LC-MS (Methode 3): $R_t$ = 2.21 min; MS (ESIpos): m/z = 335 $[M+H]^+$.

**Beispiel 84A**

5-(3-Brom-4-fluorphenyl)-1-(3-chlorphenyl)-1*H*-pyrazol-3-carbonsäure

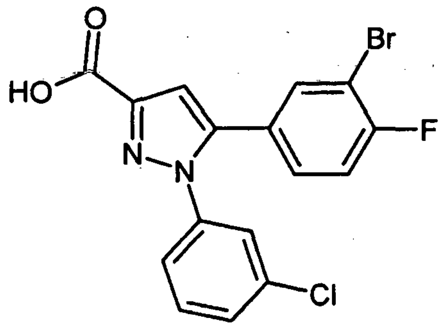

Die Herstellung der Titelverbindung erfolgt ausgehend von der Verbindung aus Beispiel 34A analog zur Synthese der Verbindung aus Beispiel 77A. Man erhält 3.30 g (98% d. Th.) der Titelverbindung.
$^1$H-NMR (400 MHz, DMSO-$d_6$): $\delta$ = 13.1 (s, 1H), 7.72 (dd, 1H), 7.58-7.52 (m, 2H), 7.48 (t, 1H), 7.4,1 (t, 1H), 7.31-7.22 (m, 2H), 7.17 (s, 1H).
LC-MS (Methode 3): $R_t$ = 2.34 min; MS (ESIpos): m/z = 395 $[M+H]^+$.

**Beispiel 85A**

5-(3-Chlorphenyl)-1-(4-fluorphenyl)-1*H*-pyrazol-3-carbonsäure

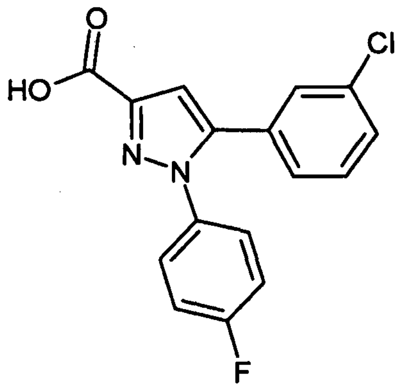

23.0 g (66.7 mmol) der Verbindung aus Beispiel 35A werden in 100 ml 1,4-Dioxan vorgelegt, mit 100 ml (200 mmol) einer 2N Lösung von Lithiumhydroxid in Wasser versetzt und 2 h bei 50 °C gerührt. Man engt ein, gibt anschließend konz. wässrige Hydrogenchlorid-Lösung bis zu einem sauren pH-Wert hinzu, extrahiert mit Dichlormethan, trocknet die organische Phase über Magnesiumsulfat, filtriert und engt ein. Der Rückstand wird in wenig Diethylether gerührt, abfiltriert und am Hochvakuum getrocknet. Man erhält 17.8 g (84% d. Th.) der Titelverbindung.
$^1$H-NMR (400 MHz, DMSO-$d_6$): $\delta$ = 13.1 (s, 1H), 7.46-7.29 (m, 7H), 7.19-7.14 (m, 2H).
LC-MS (Methode 4): $R_t$ = 3.46 min; MS (ESIpos): m/z = 317 $[M+H]^+$

**Beispiel 86A**

1-(3-Chlorphenyl)-5-(2-fluorphenyl)-1*H*-pyrazol-3-carbonsäure

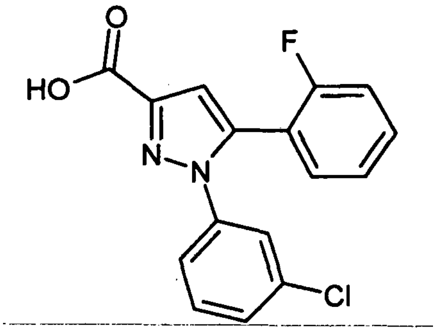

2.30 g (6.67 mmol) der Verbindung aus Beispiel 36A werden in 32 ml einer 2:1 Mischung aus 1,4-Dioxan und Wasser vorgelegt, mit 17 ml (33.4 mmol) einer 2N Lösung von Lithiumhydroxid in Wasser versetzt und 2 h bei 50 °C gerührt. Man engt ein, gibt anschließend zu dem Rückstand 2N wässrige Hydrogenchlorid-Lösung bis zu einem sauren pH-Wert hinzu, extrahiert mit Essigsäureethylester, trocknet die organische Phase über Natriumsulfat, filtriert und engt ein. Der Rückstand wird in wenig einer Mischung aus Petrolether und Diethylether gerührt, abfiltriert und am Hochvakuum getrocknet. Man erhält 1.58 g (75% d. Th.) der Titelverbindung.

1H-NMR (400 MHz, DMSO-d6): δ = 13.1 (s, 1H), 7.56-7.40 (m, 5H), 7.34-7.20 (m, 3H), 7.11 (s, 1H).

LC-MS (Methode 1): $R_t$ = 2.35 min; MS (ESIpos): m/z = 317 $[M+H]^+$.

__Beispiel 87A__

1-(4-Fluorphenyl)-5-[3-(trifluormethyl)phenyl]-1*H* pyrazol-3-carbonsäure

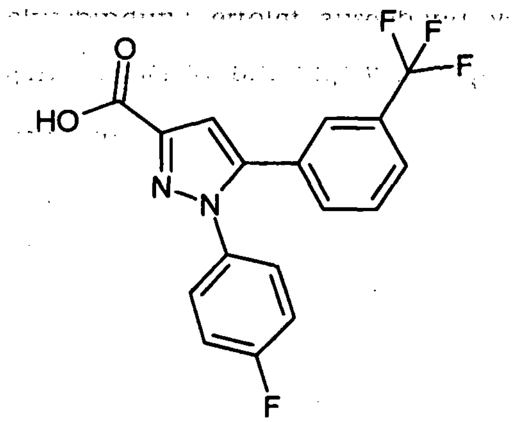

4.50 g (11.9 mmol) der Verbindung aus Beispiel 37A werden in 107 ml 1,4-Dioxan vorgelegt, mit 107 ml (214 mmol) einer 2N Lösung von Lithiumhydroxid in Wasser versetzt und 2 h bei 70 °C gerührt. Man engt ein, gibt anschließend zu dem Rückstand 2N wässrige Hydrogenchlorid-Lösung bis zu einem sauren pH-Wert hinzu, extrahiert mit Dichlormethan, trocknet die organische Phase über Magnesiumsulfat, filtriert und engt ein. Man erhält 4.11 g (99% d. Th.) der Titelverbindung.

$^1$H-NMR (400 MHz, DMSO-$d_6$): δ = 13.1 (s, 1H), 7.73 (d, 1H), 7.64-7.54 (m, 3H), 7.46-7.40 (m, 2H), 7.37-7.29 (m, 2H), 7.25 (s, 1H).

LC-MS (Methode 4): $R_t$ = 3.53 min; MS (ESIpos): m/z = 351 $[M+H]^+$.

__Beispiel 88A__

1-(3-Chlorphenyl)-5-(2,3-difluorphenyl)-1*H*-pyrazol-3-carbonsäure

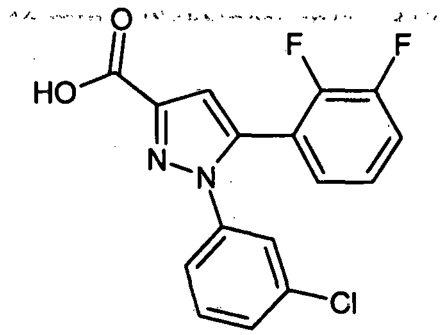

0.21 g (0.526 mmol) der Verbindung aus Beispiel 38A werden in 3.5 ml Methanol vorgelegt und bei Raumtemperatur mit 295 mg (5.26 mmol) Kaliumhydroxid versetzt. Man erhitzt 5 min unter Rückfluss, gibt anschließend 1N wässrige Hydrogenchlorid-Lösung bis zu einem pH-Wert von 5 hinzu, saugt den entstandenen Niederschlag ab, wäscht diesen mit Diethylether und trocknet am Hochvakuum. Man erhält 145 mg (82% d. Th.) der Titelverbindung.
$^1$H-NMR (400 MHz, DMSO-$d_6$): $\delta$ = 13.2 (s, 1H), 7.60-7.43 (m, 4H), 7.33-7.19 (m, 3H), 7.18 (s, 1H).
LC-MS (Methode 2): $R_t$ = 2.32 min; MS (ESIpos): m/z = 335 $[M+H]^+$.

**Beispiel 89A**

5-(3-Chlorphenyl)-1-(4-chlorphenyl)-1*H*-pyrazol-3-carbonsäure

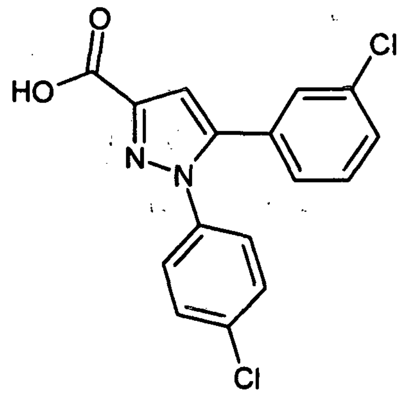

26.8 g (74.2 mmol) der Verbindung aus Beispiel 39A werden in 300 ml 1,4-Dioxan vorgelegt, mit 56 ml (111 mmol) einer 2N Lösung von Lithiumhydroxid in Wasser versetzt und 2 h bei 70 °C gerührt. Man engt ein, gibt anschließend zu dem Rückstand 1N wässrige Hydrogenchlorid-Lösung bis zu einem sauren pH-Wert hinzu, extrahiert mit Dichlormethan, trocknet die vereinigten organischen Phasen über Magnesiumsulfat, filtriert und engt ein. Man erhält 24.5 g (99% d. Th.) der Titelverbindung.
$^1$H-NMR (400 MHz, DMSO-$d_6$): $\delta$ = 13.1 (s, 1H), 7.58-7.52 (m, 2H), 7.48-7.35 (m, 5H), 7.19-7.14 (m, 2H).
LC-MS (Methode 6): $R_t$ = 3.51 min; MS (ESIpos): m/z = 333 $[M+H]^+$.

**Beispiel 90A**

5-(3-Fluorphenyl)-1-(4-fluorphenyl)-1*H* pyrazol-3-carbonsäure

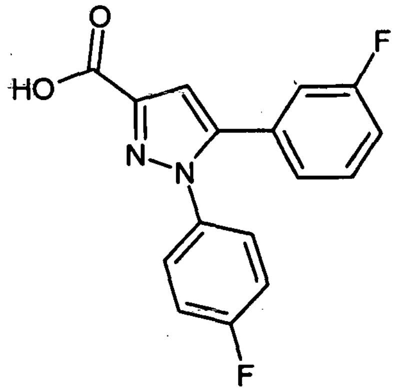

23.9 g (72.8 mmol) der Verbindung aus Beispiel 40A werden in 100 ml 1,4-Dioxan vorgelegt, mit 100 ml (200 mmol) einer 2N Lösung von Lithiumhydroxid in Wasser versetzt und 1 h bei 50 °C gerührt. Man engt ein, verdünnt den Rückstand mit Wasser, gibt anschließend konz. wässrige Hydrogenchlorid-Lösung bis zu einem sauren pH-Wert hinzu, extrahiert mit Dichlormethan, trocknet die organische Phase über Magnesiumsulfat, filtriert und engt ein. Der Rückstand wird in wenig Diethylether gerührt, abfiltriert und am Hochvakuum getrocknet. Man erhält 22.0 g (100% d. Th.) der Titelverbindung.

$^{1}$H-NMR (400 MHz, DMSO-$d_6$): $\delta$ = 7.44-7.37 (m, 3H), 7.36-7.28 (m, 2H), 7.22 (dt, 1H), 7.16 (dt, 1H), 7.12 (s, 1H), 7.05 (d, 1H).

LC-MS (Methode 1): $R_t$ = 2.32 min; MS (ESIpos): m/z = 301 $[M+H]^+$.

**Beispiel 91A**

1-(3-Chlorphenyl)-5-(3-fluor-5-methoxyphenyl)-1*H*-pyrazol-3-carbonsäure

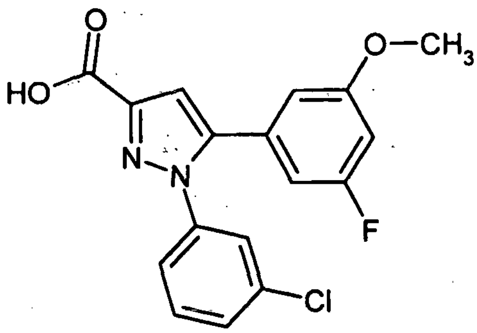

Die Herstellung der Titelverbindung erfolgt ausgehend von der Verbindung aus Beispiel 41A analog zur Synthese der Verbindung aus Beispiel 71A. Man erhält 1.31 g der Titelverbindung.

$^{1}$H-NMR (400 MHz, DMSO-$d_6$): $\delta$ = 13.1-(s, 1H), 7.58-7.53 (m, 2H), 7.52-7.46 (m, 1H), 7.30-7.25 (m, 1H), 7.18 (s, 1H), 6.88 (dt, 1H) 6.73-6.69 (m, 2H), 3.70 (s, 3H).

LC-MS (Methode 1): $R_t$ = 2.37 min; MS (ESIpos): m/z = 347 $[M+H]^+$.

**Beispiel 92A**

1-(3-Chlor-4-fluorphenyl)-5-(3-fluor-5-methoxyphenyl)-1*H*-pyrazol-3-carbonsäure

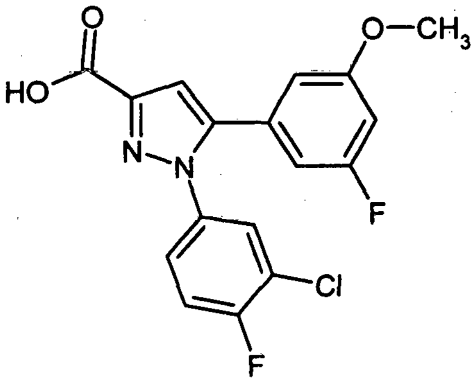

Die Herstellung der Titelverbindung erfolgt ausgehend von der Verbindung aus Beispiel 42A analog zur Synthese der Verbindung aus Beispiel 71A jedoch unter 6-stündigem Rühren. Man erhält 1.38 g der Titelverbindung.

$^{1}$H-NMR (400 MHz, DMSO-$d_6$): $\delta$ = 13.1 (s, 1H), 7.76 (dd, 1H), 7.54 (t, 1H), 7.35 (ddd, 1H), 7.17 (s, 1H), 6.89 (dt, 1H), 6.74-6.69 (m, 2H), 3.71 (s, 3H).

LC-MS (Methode 7): $R_t$ = 1.95 min; MS (ESIpos): m/z = 365 $[M+H]^+$.

**Beispiel 93A**

1-(3-Chlor-4-fluorphenyl)-5-(3-cyanphenyl)-1*H*-pyrazol-3-carbonsäure

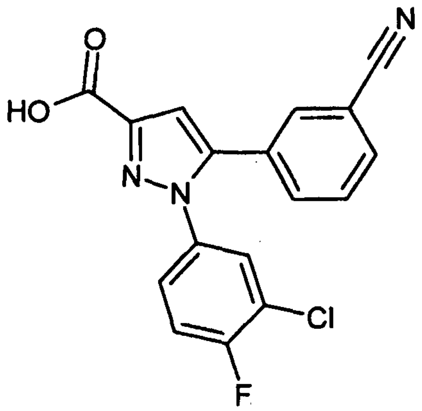

Die Herstellung der Titelverbindung erfolgt ausgehend von der Verbindung aus Beispiel 45A analog zur Synthese der Verbindung aus Beispiel 71A. Man erhält 348 mg (68% d. Th.) der Titelverbindung.

$^{1}$H-NMR (400 MHz, DMSO-$d_6$): $\delta$ = 13.1 (s, 1H), 7.91 (s, 1H), 7.87 (dt, 1H), 7.75 (dd, 1H), 7.61-7.49 (m, 3H), 7.33 (ddd, 1H), 7.24 (s, 1H).

LC-MS (Methode 1): $R_t$ = 2.21 min; MS (ESIpos): m/z = 342 $[M+H]^+$.

**Beispiel 94A**

1-(3-Chlor-4-fluorphenyl)-5-(3-fluörphenyl)-1*H*-pyrazol-3-carbonsäure

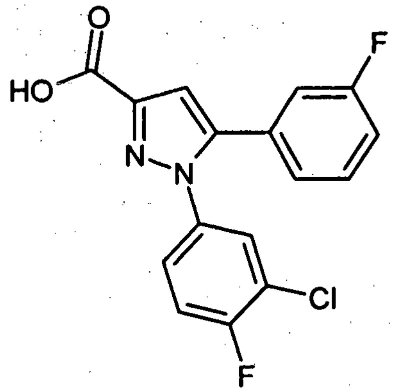

Die Herstellung der Titelverbindung erfolgt ausgehend von der Verbindung aus Beispiel 46A analog zur Synthese der Verbindung aus Beispiel 71A. Man erhält 550 mg (99% d. Th.) der Titelverbindung.
$^1$H-NMR (400 MHz, DMSO-$d_6$): $\delta$ = 13.1 (s, 1H), 7.73 (dd, 1H), 7.52 (t, 1H), 7.46-7.39 (m, 1H), 7.34 (ddd, 1H), 7.28-7.21 (m, 2H), 7.16 (s, 1H), 7.07 (dt, 1H).
LC-MS (Methode 1): $R_t$ = 2.36 min; MS (ESIpos): m/z = 335 $[M+H]^+$.

**Beispiel 95A**

1-(3-Chlor-4-fluorphenyl)-5-[3-(trifluormethyl)phenyl]-1*H*-pyrazol-3-carbonsäure

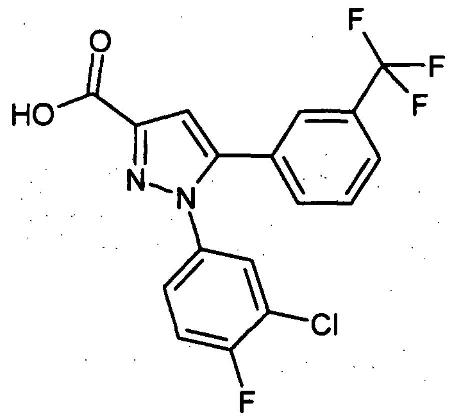

Die Herstellung der Titelverbindung erfolgt ausgehend von der Verbindung aus Beispiel 47A analog zur Synthese der Verbindung aus Beispiel 71A. Man erhält 344 mg (97% d. Th.) der Titelverbindung.
$^1$H-NMR (400 MHz, DMSO-$d_6$): $\delta$ = 13.1 (s, 1H), 7.82-7.74 (m, 2H), 7.67-7.50 (m, 4H), 7.36 (ddd, 1H), 7.26 (s, 1H).
LC-MS (Methode 1): $R_t$ = 2.53 min; MS (ESIpos): m/z = 385 $[M+H]^+$

**Beispiel 96A**

1-(3-Chlor-4-fluorphenyl)-5-[3-fluor-5-(trifluormethyl)phenyl]-1*H*-pyrazol-3-carbonsäure

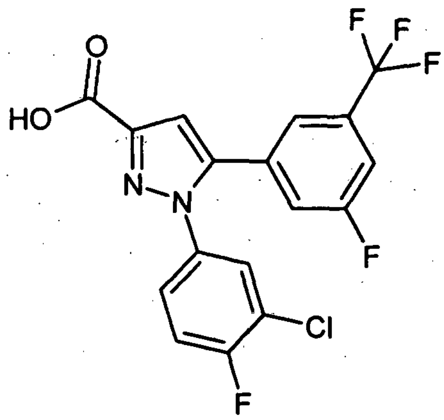

Die Herstellung der Titelverbindung erfolgt ausgehend von der Verbindung aus Beispiel 48A analog zur Synthese der Verbindung aus Beispiel 71A. Man erhält 851 mg mit 51%iger Reinheit der Titelverbindung.
LC-MS (Methode 1): $R_t$ = 2.58 min; MS (ESIpos): m/z = 403 $[M+H]^+$.

**Beispiel 97A**

1-(3-Chlorpheiiyl)-5-[3-fluor-5-(trifluormethyl)phenyl]-1*H*-pyrazol-3-carbonsäure

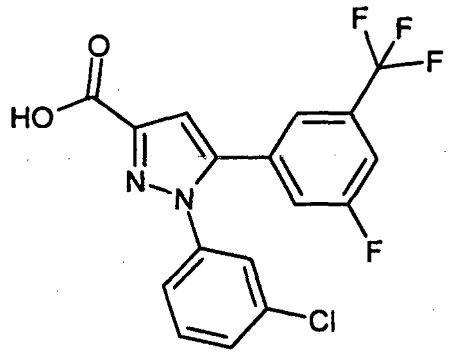

Die Herstellung der Titelverbindung erfolgt ausgehend von der Verbindung aus Beispiel 49A analog zur Synthese der Verbindung aus Beispiel 71A. Man erhält 1.82 g (85% d. Th.) der Titelverbindung.
LC-MS (Methode 1): $R_t$ = 2.55 min; MS (ESIpos): m/z = 385 $[M+H]^+$.

**Beispiel 98A**

1-(3-Bromphenyl)-5-(3-chlorphenyl)-1*H*-pyrazol-3-carbonsäure

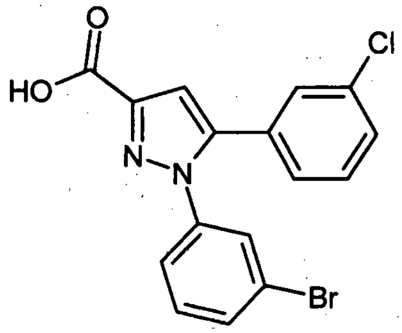

Die Herstellung der Titelverbindung erfolgt ausgehend von der Verbindung aus Beispiel 51A analog zur Synthese der Verbindung aus Beispiel 71A. Man erhält 1.74 g der Titelverbindung.

LC-MS (Methode 1): $R_t$ = 2.47 min; MS (ESIpos): m/z = 377 $[M+H]^+$.

**Beispiel 99A**

1-(3-Bromphenyl)-5-(3-chlor-4-fluorphenyl)-1*H*-pyrazol-3-carbonsäure

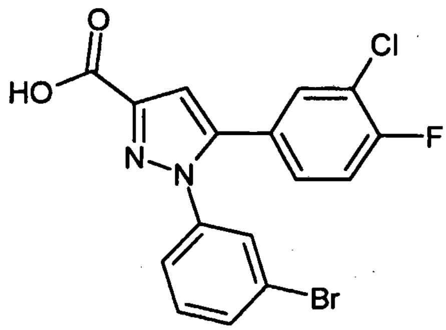

688 mg mit 76%iger Reinheit (1.23 mmol) der Verbindung aus Beispiel 51A werden in 29 ml Tetrahydrofuran vorgelegt und bei Raumtemperatur mit 389 mg (16.2 mmol) Lithiumhydroxid und 10 ml Wasser versetzt. Man rührt 5 h bei Raumtemperatur, gibt anschließend 1N wässrige Hydrogenchlorid-Lösung bis zu einem sauren pH-Wert hinzu, extrahiert mit Essigsäureethylester, trocknet über Natriumsulfat, filtriert und engt ein. Der Rückstand wird in wenig Cyclohexan gerührt und der Niederschlag abgesaugt. Man erhält 413 mg (83% d. Th.) der Titelverbindung.

$^1$H-NMR (400 MHz, DMSO-$d_6$): $\delta$ = 13.1 (s, 1H), 7.72-7.68 (m, 2H), 7.51 (dt, 1H), 7.42 (t, 1H), 7.33-7.29 (m, 1H), 7.26-7.23 (m, 2H), 7.18 (dt, 1H).

LC-MS (Methode 1): $R_t$ = 2.53 min; MS (ESIpos): m/z = 395 $[M+H]^+$.

**Beispiel 100A**

5-[3-(Benzyloxy)phenyl]-1-(3-chlorphenyl)-1*H*-pyrazol-3-carbonsäure

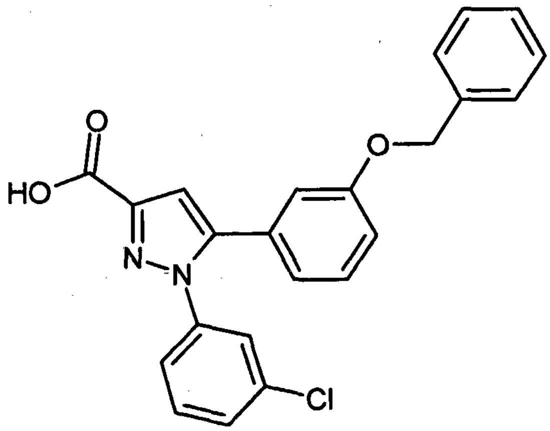

Die Herstellung der Titelverbindung erfolgt ausgehend von der Verbindung aus Beispiel 43A analog zur Synthese der Verbindung aus Beispiel 71A. Man erhält 60.0 g (85% d. Th.) der Titelverbindung.

$^1$H-NMR (400 MHz, DMSO-$d_6$): $\delta$ = 13.1 (s, 1H), 7.55-7.43 (m, 3H), 7.40-7.22 (m, 7H), 7.10 (s, 1H), 7.04 (dd, 1H), 7.00-6.97 (m, 1H), 6.80 (d, 1H), 5.04 (s, 2H).

LC-MS (Methode 9): $R_t$ = 2.40 min; MS (ESIpos): m/z = 405 $[M+H]^+$.

**Beispiel 101A**

1-(3-Chlor-4-fluorphenyl)-5-(3-fluor-5-hydroxyphenyl)-1H-pyrazol-3-carbonsäure

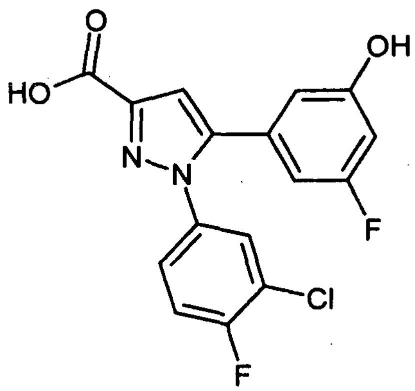

200 mg (0.453 mmol) der Verbindung aus Beispiel 52A werden in einer Mischung aus 1.5 ml 1,4-Dioxan und 0.9 ml entgastem Wasser unter Argon vorgelegt, mit 152 mg (2.72 mmol) Kaliumhydroxid, 23.1 mg (0.054 mmol) 2-Ditert-butylphosphino-2',4',6'-triisopropylbiphenyl und 16.6 mg (0.018 mmol) Tris(dibenzylidenaceton)dipalladium(0) versetzt und über Nacht bei 80 °C erhitzt. Anschließend wird 1N wässrige Hydrogenchlorid-Lösung bis zu einem sauren pH-Wert hinzugegeben und die Reaktionsmischung über die präparative HPLC (RP18-Säule; Laufmittel: Acetonitril/Wasser-Gradient) gereinigt. Man erhält 100 mg (63% d. Th.) der Titelverbindung.

$^1$H-NMR (400 MHz, DMSO-$d_6$): $\delta$ = 13.1 (s, 1H), 10.2 (s, 1H), 7.75 (dd, 1H), 7.54 (t, 1H), 7.33 (ddd, 1H), 7.09 (s, 1H), 6.63 (dt, 1H), 6.59 (dt, 1H), 6.44 (t, 1H).

LC-MS (Methode 1): $R_t$ = 2.14 min; MS (ESIpos): m/z = 351 $[M+H]^+$.

**Beispiel 102A**

1-(3-Chlor-4-fluorphenyl)-5-(3-methoxyphenyl)-1*H*-pyrazol-3-carbonsäure

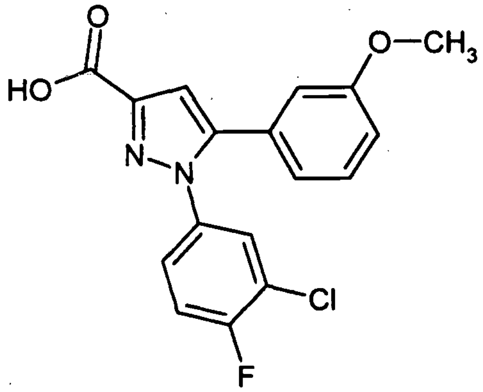

Die Herstellung der Titelverbindung erfolgt ausgehend von der Verbindung aus Beispiel 53A analog zur Synthese der Verbindung aus Beispiel 71A. Man erhält 515 mg (93% d. Th.) der Titelverbindung.

$^1$H-NMR (400 MHz, DMSO-$d_6$): $\delta$ = 13.1 (s, 1H), 7.72 (dd, 1H), 7.52 (t, 1H), 7.35-7.26 (m, 2H), 7.11 (s, 1H), 6.96 (dd, 1H), 6.90 (t, 1H), 6.79 (d, 1H), 3.70 (s, 3H).

LC-MS (Methode 1): $R_t$ = 2.34 min; MS (ESIpos): m/z = 347 $[M+H]^+$.

**Beispiel 103A**

5-[3,5-Bis(trifluormethyl)phenyl]-1-(3-chlorphenyl)-1*H*-pyrazol-3-carbonsäure

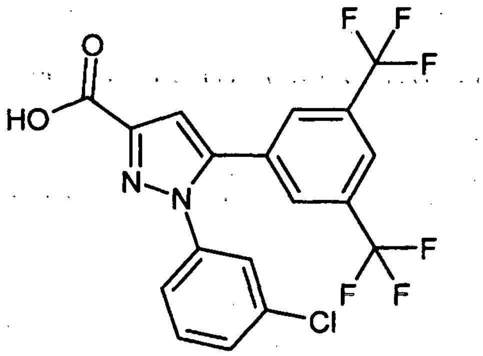

Die Herstellung der Titelverbindung erfolgt ausgehend von der Verbindung aus Beispiel 54A analog zur Synthese der Verbindung aus Beispiel 86A. Man erhält 1.78 g (79% d. Th.) der Titelverbindung.

$^1$H-NMR (400 MHz, DMSO-$d_6$): $\delta$ = 13.1 (s, 1H), 8.15 (s, 1H), 7.96 (s, 2H), 7.62-7.56 (m, 2H), 7.52-7.45 (m, 2H), 7.35-7.30 (m, 1H).

LC-MS (Methode 1): $R_t$ = 2.80 min; MS (ESIpos): m/z = 435 $[M+H]^+$.

**Beispiel 104A**

1-(4-Chlorphenyl)-5-[3-(trifluormethyl)phenyl]-1*H*-pyrazol-3-carbonsäure

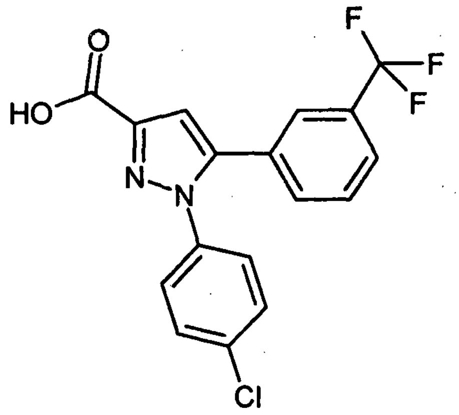

4.75 g (12.0 mmol) der Verbindung aus Beispiel 55A werden in 108 ml 1,4-Dioxan vorgelegt, mit 108 ml (216 mmol) einer 2N Lösung von Lithiumhydroxid in Wasser versetzt und 2 h bei 70 °C gerührt. Man engt ein, gibt anschließend zu dem Rückstand konz. wässrige Hydrogenchlorid-Lösung bis zu einem sauren pH-Wert hinzu, extrahiert mit Dichlormethan, trocknet die organische Phase über Magnesiumsulfat, filtriert und engt ein. Man erhält 4.40 g (100% d. Th.) der Titelverbindung.
$^1$H-NMR (400 MHz, DMSO-$d_6$): $\delta$ = 13.1 (s, 1H), 7.75 (d, 1H), 7.65-7.52 (m, 5H), 7.42-7.36 (m, 2H), 7.25 (s, 1H).
LC-MS (Methode 4): $R_t$ = 3.71 min; MS (ESIpos): m/z = 367 $[M+H]^+$.

**Beispiel 105A**

1-(4-Chlorphenyl)-5-(3-fluorphenyl)-1*H*pyrazol-3-carbonsäure

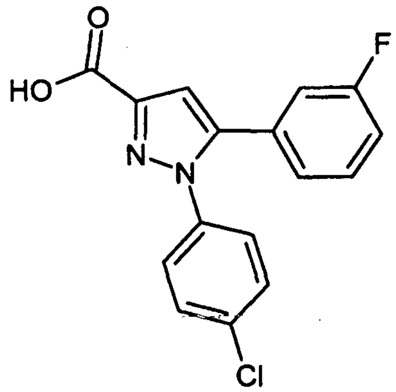

43.0 g (125 mmol) der Verbindung aus Beispiel 56A werden in 504 ml 1,4-Dioxan vorgelegt, mit 94 ml (188 mmol) einer 2N Lösung von Lithiumhydroxid in Wasser versetzt und 2 h bei 70 °C gerührt. Man engt ein, gibt anschließend zu dem Rückstand konz. wässrige Hydrogenchlorid-Lösung bis zu einem sauren pH-Wert hinzu, extrahiert mit Dichlormethan, trocknet die organische Phase über Magnesiumsulfat, filtriert und engt ein. Man erhält 39.5 g (100% d. Th.) der Titelverbindung.
$^1$H-NMR (400 MHz, DMSO-$d_6$): $\delta$ = 7.56-7.51 (m, 2H), 7.46-7.34 (m, 3H), 7.27-7.16 (m, 2H), 7.10 (s, 1H) 7.06 (d, 1H).
LC-MS (Methode 1): $R_t$ = 2.49 min; MS (ESIpos): m/z = 317 $[M+H]^+$.

**Beispiel 106A**

1-(3-Chlor-4-fluorphenyl)-5-(3-fluor-5-methylphenyl)-1*H*-pyrazol-3-carbonsäure

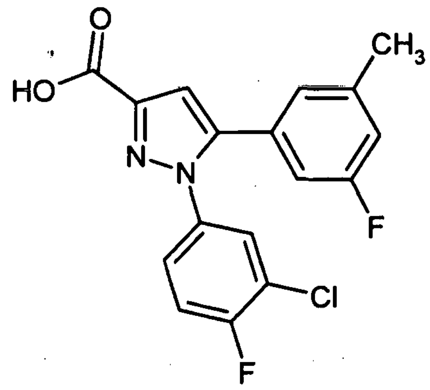

Die Herstellung der Titelverbindung erfolgt ausgehend von der Verbindung aus Beispiel 57A analog zur Synthese der Verbindung aus Beispiel 71A jedoch unter 6-stündigem Rühren. Man erhält 2.34 g mit 79%iger Reinheit der Titelverbindung.

$^1$H-NMR (400 MHz, DMSO-$d_6$): $\delta$ = 13.1 (s, 1H), 7.73 (dd, 1H), 7.52 (t, 1H), 7.32 (ddd, 1H), 7.13 (s, 1H), 7.09 (d, 1H), 7.00 (s, 1H) 6.94-6.89 (m, 1H), 2.27 (s, 3H).

LC-MS (Methode 1): $R_t$ = 2.52 min; MS (ESIpos): m/z = 349 $[M+H]^+$.

**Beispiel 107A**

1-(3-Chlorphenyl)-5-(3-fluor-5-methylphenyl)-1*H*-pyrazol-3-carbonsäure

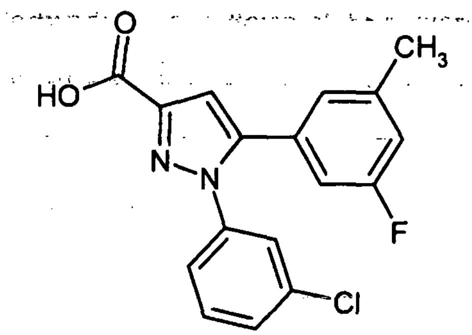

Die Herstellung der Titelverbindung erfolgt ausgehend von der Verbindung aus Beispiel 58A analog zur Synthese der Verbindung aus Beispiel 71A jedoch unter 6-stündigem Rühren. Man erhält 1.64 g (100% d. Th.) der Titelverbindung.

$^1$H-NMR (400 MHz, DMSO-$d_6$): $\delta$ = 13.1 (s, 1H), 7.57-7.52 (m, 2H), 7.48 (t, 1H), 7.25 (ddd, 1H), 7.13 (s, 1H), 7.09 (d, 1H), 7.01 (s, 1H), 6.89 (d, 1H), 2.27 (s, 3H).

LC-MS (Methode 1): $R_t$ = 2.49 min; MS (ESIpos): m/z = 331 $[M+H]^+$.

**Beispiel 108A**

5-(3-Chlor-4-fluorphenyl)-1-(3-cyanphenyl)-1*H*-pyrazol-3-carbonsäure

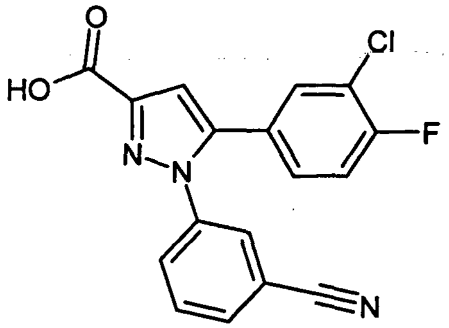

Die Herstellung der Titelverbindung erfolgt ausgehend von der Verbindung aus Beispiel 59A analog zur Synthese der Verbindung aus Beispiel 71A. Man erhält 21 mg mit 83%iger Reinheit (64% d. Th.) der Titelverbindung.
$^1$H-NMR (400 MHz, DMSO-$d_6$): $\delta$ = 13.2 (s, 1H), 7.99-7.95 (m, 2H), 7.70-7.66 (m, 2H), 7.52 (dt, 1H), 7.27 (s, 1H), 7.26-7.23 (m, 1H), 7.20-7.15 (m, 1H).
LC-MS (Methode 7): $R_t$ = 1.78 min; MS (ESIpos): m/z = 342 $[M+H]^+$.

**Beispiel 109A**

5-(3-Chlorphenyl)-1-(3-cyan-4-fluorphenyl)-1*H*-pyrazol-3-carbonsäure

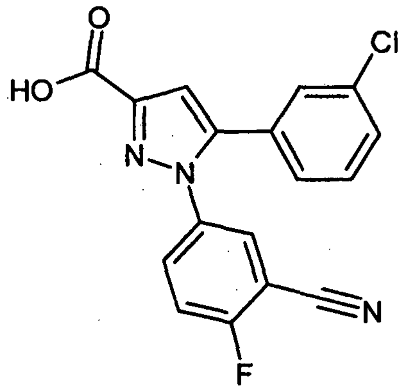

Die Herstellung der Titelverbindung erfolgt ausgehend von der Verbindung aus Beispiel 60A analog zur Synthese der Verbindung aus Beispiel 71A jedoch unter 6-stündigem Rühren. Man erhält 672 mg mit 61%iger Reinheit der Titelverbindung.
LC-MS (Methode 7): $R_t$ = 1.86 min; MS (ESIpos): m/z = 342 $[M+H]^+$.

**Beispiel 110A**

5-(3-Chlorphenyl)-1-(3-cyanphenyl)-1*H*-pyrazol-3-carbonsäure

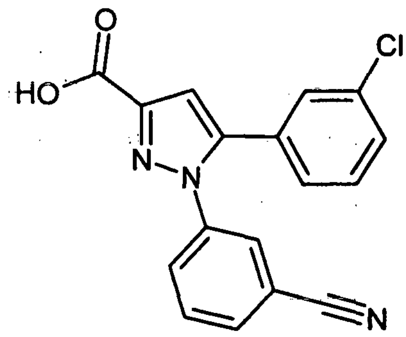

Die Herstellung der Titelverbindung erfolgt ausgehend von der Verbindung aus Beispiel 61A analog zur Synthese der Verbindung aus Beispiel 71A jedoch unter 6-stündigem Rühren. Man erhält 470 mg mit 77%iger Reinheit der Titelverbindung.
LC-MS (Methode 7): $R_t$ = 1.76 min; MS (ESIpos): m/z = 324 $[M+H]^+$.

**Beispiel 111A**

1,5-Bis(3-cyan phenyl)-1*H*-pyrazol-3-carbonsäure

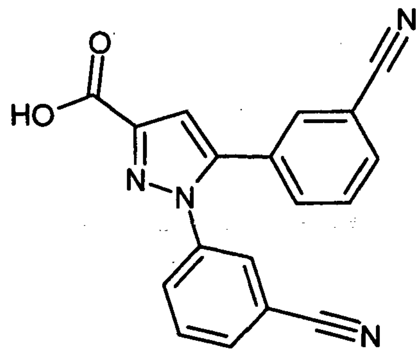

Die Herstellung der Titelverbindung erfolgt ausgehend von der Verbindung aus Beispiel 62A analog zur Synthese der Verbindung aus Beispiel 71A jedoch unter 6-stündigem Rühren. Man erhält 397 mg mit 36%iger Reinheit der Titelverbindung.
LC-MS (Methode 7): $R_t$ = 1.48 min; MS (ESIpos): m/z = 315 $[M+H]^+$.

**Beispiel 112A**

1-(3-Cyan-4-fluorphenyl)-5-(3-cyanphenyl)-1*H*-pyrazol-3-carbonsäure

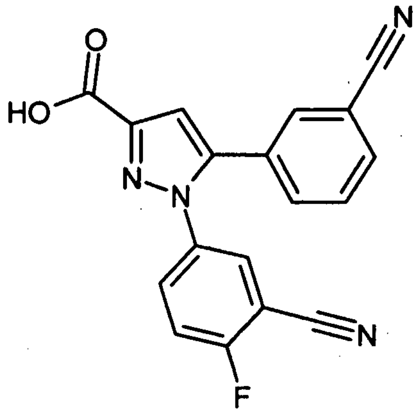

Die Herstellung der Titelverbindung erfolgt ausgehend von der Verbindung aus Beispiel 63A analog zur Synthese der Verbindung aus Beispiel 71A jedoch unter 6-stündigem Rühren. Man erhält 430 mg mit 43%iger Reinheit der Titelverbindung.

LC-MS (Methode 7): $R_t$ = 1.58 min; MS (ESIpos): m/z = 333 $[M+H]^+$.

__Beispiel 113A__

5-(3-Chlor-5-fluorphenyl)-1-(3-cyanphenyl)-1*H*-pyrazol-3-carbonsäure

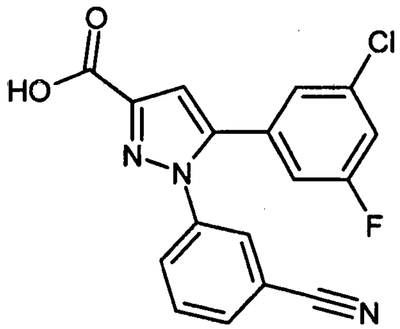

Die Herstellung der Titelverbindung erfolgt ausgehend von der Verbindung aus Beispiel 64A analog zur Synthese der Verbindung aus Beispiel 71A jedoch unter 6-stündigem Rühren. Man erhält 1.21 g mit 53%iger Reinheit der Titelverbindung.

LC-MS (Methode 7): $R_t$ = 1.83 min; MS (ESIpos): m/z = 342 $[M+H]^+$.

__Beispiel 114A__

5-(3-Chlor-5-fluorphenyl)-1-(3-cyan-4-fluorphenyl)-1*H*-pyrazol-3-carbonsäure

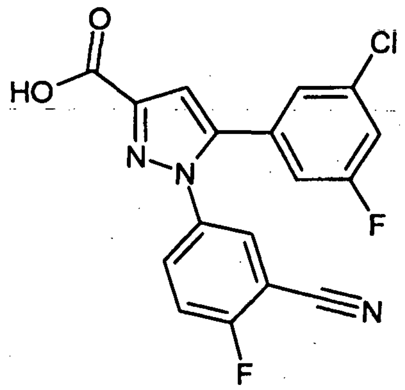

Die Herstellung der Titelverbindung erfolgt ausgehend von der Verbindung aus Beispiel 65A analog zur Synthese der Verbindung aus Beispiel 71A jedoch unter 6-stündigem Rühren. Man erhält 1.88 g mit 64%iger Reinheit der Titelverbindung.
LC-MS (Methode 7): $R_t$ = 1.91 min; MS (ESIpos): m/z = 360 $[M+H]^+$.

**Beispiel 115A**

1-(3-Chlorphenyl)-5-[3-(2-hydroxyethoxy)phenyl]-1*H*-pyrazol-3-carbonsäure

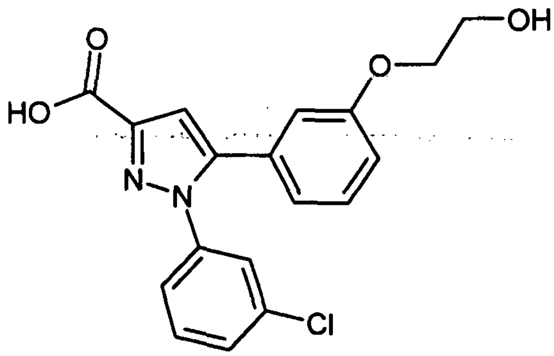

Die Herstellung der Titelverbindung erfolgt ausgehend von der Verbindung aus Beispiel 66A analog zur Synthese der Verbindung aus Beispiel 71A. Man erhält 11 mg (82% d. Th.) der Titelverbindung.
LC-MS (Methode 1): $R_t$ = 1.94 min; MS (ESIpos): m/z = 359 $[M+H]^+$.

**Beispiel 116A**

1-(3-Chlorphenyl)-5-[3-(3-hydroxypropoxy)phenyl]-1*H*-pyrazol-3-carbonsäure

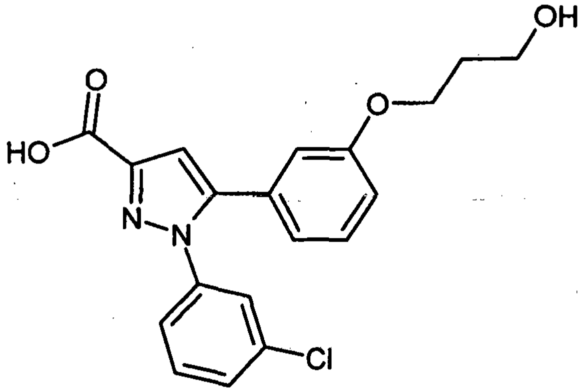

Die Herstellung der Titelverbindung erfolgt ausgehend von der Verbindung aus Beispiel 67A analog zur Synthese der Verbindung aus Beispiel 71A. Man erhält 99 mg mit 65%iger Reinheit (70% d. Th.) der Titelverbindung.
$^1$H-NMR (400 MHz, DMSO-$d_6$): $\delta$ = 13.0 (s, 1H), 7.55-7.43 (m, 3H), 7.30-7.23 (m, 2H), 7.10 (s, 1H), 6.94 (dd, 1H), 6.90-6.86 (m, 1H), 6.77 (d, 1H), 4.52 (t, 1H), 3.97 (t, 2H), 3.50 (q, 2H), 1.82-1.74 (m, 2H).
LC-MS (Methode 1): $R_t$ = 2.04 min; MS (ESIpos): m/z = 373 $[M+H]^+$.

**Beispiel 117A**

1-(3-Chlorphenyl)-5-[3-(2- methoxyethoxy)phenyl]-1*H*-pyrazol-3-carbonsäure

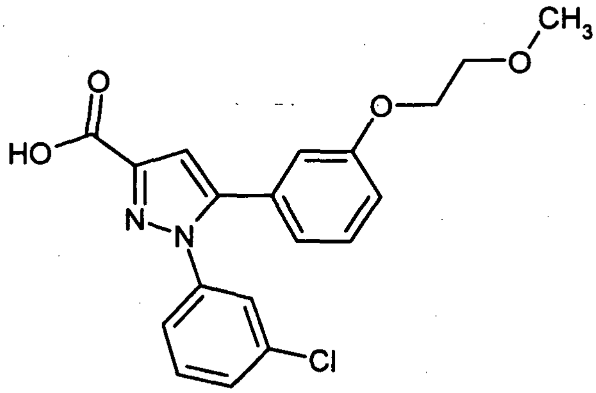

Die Herstellung der Titelverbindung erfolgt ausgehend von der Verbindung aus Beispiel 69A analog zur Synthese der Verbindung aus Beispiel 71A und unter Reinigung über die präparative HPLC (RP18-Säule; Laufmittel: Acetonitril/Wasser-Gradient unter Zusatz von 0.1% Ameisensäure). Man erhält 46 mg (100% d. Th.) der Titelverbindung.
$^1$H-NMR (400 MHz, DMSO-$d_6$): $\delta$ = 13.0 (s, 1H), 7.55-7.44 (m, 3H), 7.31-7.23 (m, 2H), 7.10 (s, 1H), 6.96 (dd, 1H), 6.91-6.88 (m, 1H), 6.80 (d, 1H), 4.05-4.00 (m, 2H), 3.61-3.54 (m, 2H), 3.28 (s, 3H).
LC-MS (Methode 1): $R_t$ = 2.23 min; MS (ESIpos): m/z = 373 $[M+H]^+$.

**Beispiel 118A**

1-(3-Chlorphenyl)-5-[3-(3-pyrrolidin-1-ylpropoxy)phenyl]-1*H*-pyrazol-3-carbonsäure

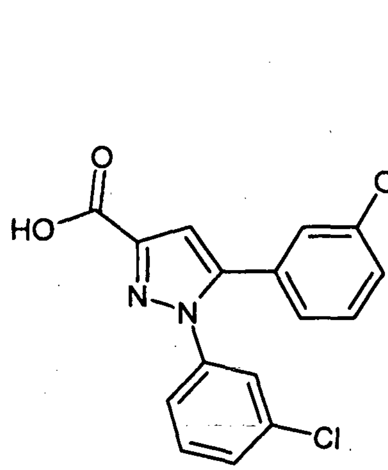

Die Herstellung der Titelverbindung erfolgt ausgehend von der Verbindung aus Beispiel 70A analog zur Synthese der Verbindung aus Beispiel 71A. Man erhält 10 mg mit 79%iger Reinheit (44% d. Th.) der Titelverbindung.
LC-MS (Methode 1): $R_t$ = 1.44 min; MS (ESIpos): m/z = 426 $[M+H]^+$.

**Beispiel 119A**

1-({5-[3-(2-Chlorethoxy)-5-fluorphenyl]-1-(3-chlor-4-fluorphenyl)-1*H*-pyrazol-3-yl}carbonyl)imidazolidin-4-on

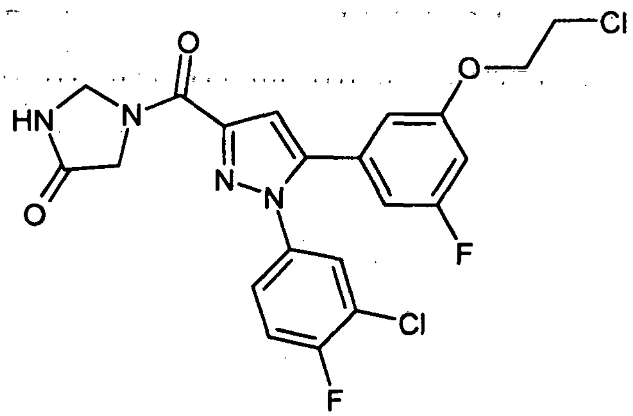

1.66 g (4.73 mmol) der Verbindung aus Beispiel 101A, 1.04 g (5.21 mmol) der Verbindung aus Beispiel 125A und 3.69 g (7.10 mmol) PyBOP werden in 80 ml Tetrahydrofuran vorgelegt und bei Raumtemperatur mit 1.73 ml (9.94 mmol) N,N-Diisopropylethylamin versetzt. Man rührt bei Raumtemperatur über Nacht, versetzt mit Wasser, entfernt das Tetrahydrofuran im Vakuum, extrahiert den Rückstand mit Essigsäurethylester, engt ein und reinigt den Rückstand per Flash-Chromatographie (Laufmittel: Essigsäureethylester/Methanol 200/1). Man erhält 1.24 g (63% d. Th.) 1-{[1-(3-Chlor-4-fluorphenyl)-5-(3-fluor-5-hydroxyphenyl)-1*H*pyrazol-3-yl]carbonyl}imi-dazolidin-4-on.
LC-MS (Methode 7): $R_t$ = 1.59 min; MS (ESIpos): m/z = 419 $[M+H]^+$.
200 mg (0.48 mmol) 1-{[1-(3-Chlor-4-fluorphenyl)-5-(3-fluor-5-hydroxyphenyl)-1*H*-pyrazol-3-yl]carbonyl}imidazolidin-4-on werden in 2.2 ml Tetrahydrofuran vorgelegt und bei Raumtemperatur mit 48 μl (0.72 mmol) 2-Chlorethanol, 188 mg (0.72 mmol) Triphenylphosphin und 145 mg (0.72 mmol) Azodicarbonsäurediisopropylester versetzt. Man rührt bei Raumtemperatur über Nacht und reinigt anschließend die Reaktionsmischung über die präparative HPLC (RP18-Säule; Laufmittel: Acetonitril/Wasser-Gradient). Man erhält 24 mg (10% d. Th.) der Titelverbindung.
$^1$H-NMR (400 MHz, DMSO-$d_6$): $\delta$ = 8.72 (s, 0.6H), 8.62 (s, 0.4H), 7.80 (dd, 1H), 7.54 (dt, 1H), 7.37 (ddd, 1H), 7.20 (d, 1H), 6.95 (dt, 1H), 6.79 (s, 1H), 6.77-6.71 (m, 1H), 5.32, (s, 0.8H), 4.90 (s, 1.2), 4.44 (s, 1.2H), 4.24 (t, 2H), 3.98 (s, 0.8H), 3.90 (t, 2H).
LC-MS (Methode 1): $R_t$ = 2.40 min; MS (ESIpos): m/z = 481 $[M+H]^+$.

**Beispiel 120A**

3-(3-{1-(3-Chlorphenyl)-3-[(4-oxoimidazolidin-1-yl)carbonyl]-1*H*-pyrazol-5-yl}pheno-xy)propyl methansulfonat

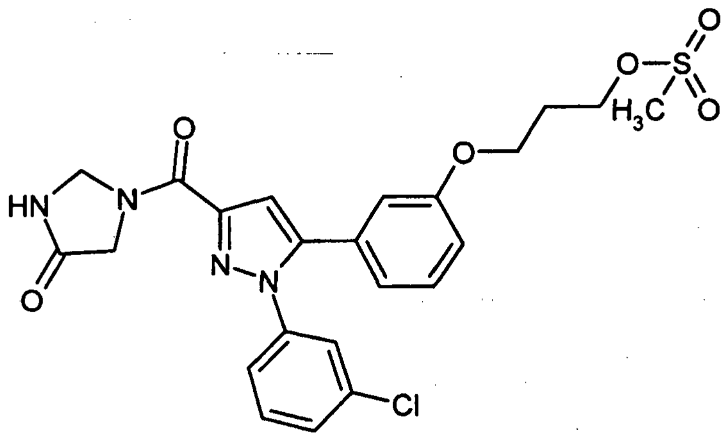

3.63 g (8.23 mmol) der Verbindung aus Beispiel 24 werden in 24 ml Dichlormethan vorgelegt und bei Raumtemperatur mit 2.2 ml (19.3 mmol) 2,6-Dimethylpyridin und 0.75 ml (9.63 mmol) Methansulfonsäurechlorid versetzt. Man rührt bei Raumtemperatur über Nacht, verdünnt mit Dichlormethan, wäscht mit Wasser und wässriger Natriumcarbonat-Lösung, trocknet über Natriumsulfat und engt ein. Man erhält 4.33 g mit 55%iger Reinheit (56% d. Th.) der Titelverbindung.
LC-MS (Methode 1): $R_t$ = 2.15 min; MS (ESIpos): m/z = 519 $[M+H]^+$.

**Beispiel 121A**

2-(3-{1-(3-Chlorphenyl)-3-[(4-oxoimidazolidin-1-yl)carbonyl]-1*H*-pyrazol-5-yl}phen-oxy)ethyl methansulfonat

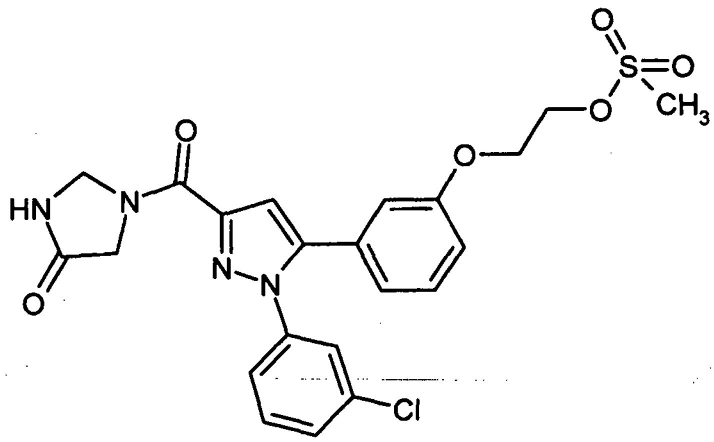

Die Herstellung der Titelverbindung erfolgt ausgehend von der Verbindung aus Beispiel 23 analog zur Synthese der Verbindung aus Beispiel 120A. Man erhält 6.14 g mit 51%iger Reinheit (57% d. Th.) der Titelverbindung.
LC-MS (Methode 1): $R_t$ = 2.08 min; MS (ESIpos): m/z = 505 $[M+H]^+$.

**Beispiel 122A**

$N^2$-Benzylglycinamid

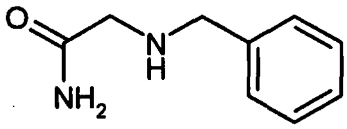

44.2 g (0.40 mol) Glycinamid-hydrochlorid werden in 2.2 1 Dichlormethan bei Raumtemperatur unter Argon vorgelegt, mit 112 ml (0.80 mol) Triethylamin versetzt und über Nacht bei Raumtemperatur gerührt. Dann werden 42.5 g (0.40 mol) Benzaldehyd zugegeben und über Nacht am Wasserabscheider unter Rückfluss erhitzt. Man engt ein, löst den Rückstand in 400 ml Tetrahydrofuran/Methanol (1:1), versetzt bei 0 °C portionsweise mit 16.7 g (0.44 mol) Natriumborhydrid und rührt zwei Tage bei Raumtemperatur. Die Suspension wird abgesaugt, das Filtrat eingeengt und im Hochvakuum getrocknet. Der Rückstand wird in Essigsäureethylester gerührt, der Niederschlag abfiltriert, das Filtrat eingeengt und der Rückstand in Toluol über Nacht gerührt. Nach Filtration des Feststoffs und anschließendem Trocknen im Hochvakuum erhält man 56.5 g (84% d. Th.) der Titelverbindung.

$^1$H-NMR (400 MHz, DMSO-$d_6$): $\delta$ = 7.36-7.28 (m, 4H), 7.27-7.19 (m, 1H), 3.68-3.64 (m, 2H), 3.03-3.00 (m, 2H).

LC-MS (Methode 10): $R_t$ = 0.40 min; MS (ESIpos): m/z = 165 $[M+H]^+$.

**Beispiel 123A**

1-Benzyl-3-(hydroxymethyl)imidazolidin-4-on

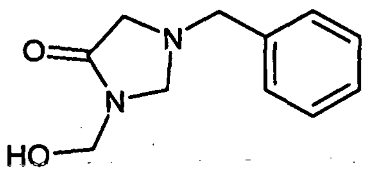

56.5 g (0.34 mol) der Verbindung aus Beispiel 122A werden mit 172 ml (6.20 mol) 37%iger Formaldehyd-Lösung versetzt und 30 Minuten unter Rückfluss erhitzt. Es wird mit Dichlormethan extrahiert, die vereinigten organischen Phasen über Natriumsulfat getrocknet, filtriert und eingeengt. Man erhält 74.5 g (100% d. Th.) der Titelverbindung.

LC-MS (Methode 5): $R_t$ = 0.51 min; MS (ESIpos): m/z = 207 $[M+H]^+$.

**Beispiel 124A**

1-Benzylimidazolidin-4-on-trifluoracetat

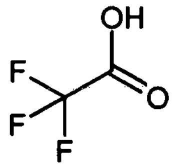

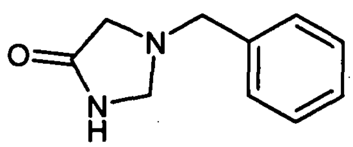

74.5 g (0.36 mol) der Verbindung aus Beispiel 123A werden für 6 h bei 150 °C im Hochvakuum unter Abdestillieren flüchtiger Reaktionsprodukte erhitzt. Die Aufreinigung des Rückstands erfolgt durch HPLC (Säule: Sunfire C18 5μ, 250 x 20 mm; Eluent: 0.2% Trifluoressigsäure/Wasser-Acetonitril-Gradient). Man erhält 28.4 g (27% d. Th.) der Titelverbindung.

$^1$H-NMR (400 MHz, DMSO-$d_6$): $\delta$ = 8.75 (s, 1H), 7.48-7.39 (m, 5H), 4.41 (s, 2H), 4.22 (s, 2H), 3.54 (s, 2H).

LC-MS (Methode 10): $R_t$ = 0.94 min; MS (ESIpos): m/z = 177 $[M-CF_3COOH+H]^+$.

**Beispiel 125A**

Imidazolidin-4-on-trifluoracetat

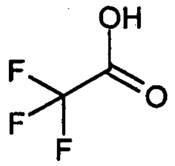 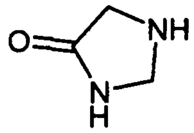

28.4 g (97.9 mmol) der Verbindung aus Beispiel 124A werden in 750 ml Ethanol gelöst und unter Argon mit 4.5 g (42.3 mmol) Palladium auf Aktivkohle (5%ig) versetzt. Es wird 24 h bei Raumtemperatur unter Wasserstoffatmosphäre gerührt. Die Suspension wird über Celite filtriert, eingeengt und am Hochvakuum getrocknet. Man erhält 19.2. g (98% d. Th.) der Titelverbindung.

$^1$H-NMR (400 MHz, DMSO-$d_6$): $\delta$ = 10.1 (s, 2H), 8.89 (s, 1H), 4.55 (s, 2H), 3.63 (s, 2H).

GC-MS (Methode 11): $R_t$ = 3.92 min MS (EIpos): m/z = 86 [M-$CF_3COOH$]$^+$.

**Beispiel 126A**

3-Acetyt-5-nuorbenzolcarbonitril

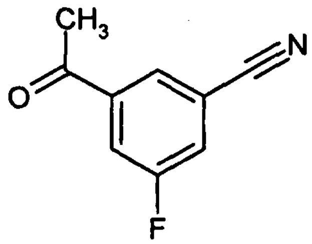

9.00 g (45.0 mmol) 3-Brom-5-fluorbenzolcarbonitril in Toluol (300 ml) werden unter einer Argonatmosphäre mit 824 mg (0.900 mmol) Tris(dibenzylidenaceton)dipal-ladium und 1.23 g (1.98 mmol) rac-1,1'-Binaphthalen-2,2'-diylbis (diphenylphos-phan) versetzt. Nach Zugabe von 19.5 g (54.0 mmol) (1-Ethoxyvinyl)-tributylstannan wird unter Rückfluss über Nacht gerührt. Anschließend wird die Reaktionsmischung eingeengt und der Rückstand in 300 ml THF aufgenommen. Nach Zugabe von 100 ml wässriger 2 N Hydrogenchlorid-Lösung wird 2 h bei Raumtemperatur gerührt. Die Reaktionsmischung wird anschließend mit gesättigter wässriger Natriumhydrogencarbonat-Lösung neutralisiert und mit Ethylacetat extrahiert. Die vereinigten organischen Phasen werden über Magnesiumsulfat getrocknet, filtriert und i. Vak. eingeengt. Das Rohprodukt wird mittels Flash-Chromatographie (Laufmittel: Cyclohe-xan/Essigsäureethylester 9:1) gereinigt. Man erhält 7.11 g (97% d. Th.) der Titelverbindung.

$^1$H-NMR (400 MHz, DMSO-$d_6$): $\delta$ = 8.28 (t, 1H), 8.18-8.14 (m, 1H), 8.08-8.04 (m, 1H), 2.65 (s, 3H).

GC-MS (Methode 11): $R_t$ = 3.97 min; MS (EIpos): m/z = 163 [M]$^+$.

**Beispiel 127A**

4-(3-Cyan-5-fluorphenyl)-2,4-dioxobuttersäureethylester

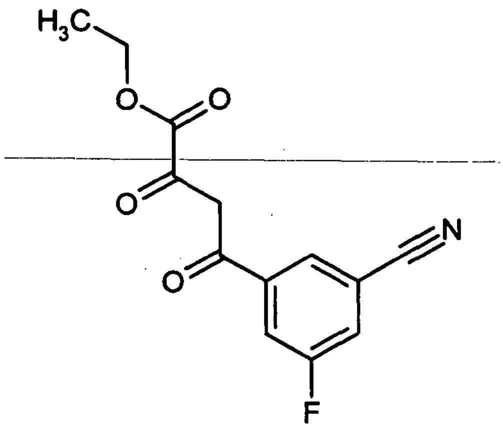

42.9 ml (42.9 mmol, 1 M in Hexan) Lithiumbis(trimethylsilyl)amid in 130 ml Diethylether werden bei -78 °C und unter einer Argonatmosphäre mit einer Lösung von 5.00 g (30.6 mmol) des Acetophenons aus Beispiel 126A in 50 ml Diethylether versetzt. Es wird 1 h bei -78 °C gerührt und anschließend werden 5.00 ml (36.8 mmol) Oxalsäurediethylester langsam zugetropft. Es wird langsam auf RT erwärmt und über Nacht gerührt. Die entstandene Suspension wird langsam mit 1 N wässriger Hydrogenchlorid-Lösung versetzt, mit Wasser verdünnt und mit Dichlormethan extrahiert. Die vereinigten organischen Phasen werden über Magnesiumsulfat getrocknet, filtriert und i. Vak. eingeengt. Das Rohprodukt wird in Diethylether suspendiert und 30 min gerührt. Nach Filtration und Trocknung am Hochvakuum erhält man 6.50 g (81% d. Th.) der Titelverbindung als ca. 9:1 Keto/Enol-Mischung.

LC-MS (Methode 12): $R_t$ = 0.73, 1.04 min; MS (ESIneg): m/z = 262 $[M-H]^-$.

**<u>Beispiel 128A</u>**

1-(3-Chlor-4-fluorphenyl)-5-(3-cyan-5-fluorphenyl)-1*H*-pyrazol-3-carbonsäureethyl-ester

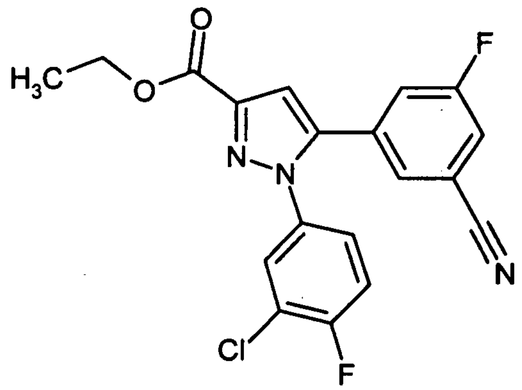

1.00 g (3.80 mmol) der 1,3-Diketo-Verbindung aus Beispiel 127A in 15 ml *N,N*-Dimethylacetamid wird mit 898 mg (4.56 mmol) 3-Chlor-4-fluorphenylhydrazin Hydrochlorid versetzt und anschließend werden zu der entstandenen Lösung 0.190 ml 10 N wässrige Hydrogenchlorid-Lösung gegeben. Es wird für 24 h bei RT gerührt und anschließend direkt über präparative HPLC (RP18-Säule; Laufmittel: Acetonitril/Wasser-Gradient) gereinigt. Man erhält 1.22 g (83% d. Th.) der Titelverbindung.

$^1$H-NMR (400 MHz, DMSO-$d_6$): $\delta$ = 7.93 (d, 1H), 7.80 (dd, 1H), 7.72 (s, 1H), 7.58-7.49 (m, 2H), 7.41-7.33 (m, 2H), 4.35 (q, 2H), 1.32 (t, 3H).

LC-MS (Methode 5): $R_t$ = 1.37 min; MS (ESIpos): m/z = 388 $[M+H]^+$.

**Beispiel 129A**

1-(3-Chlor-4-fluorphenyl)-5-(3-cyan-5-fluorphenyl)-1*H*-pyrazol-3-carbonsäure

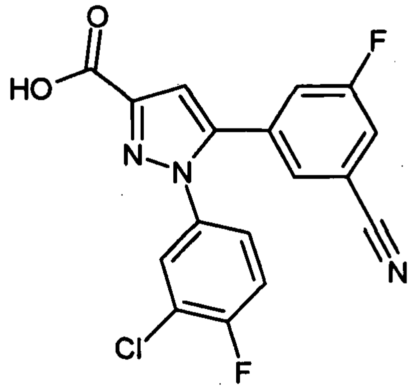

1.20 g (3.10 mmol) des Carbonsäureester aus Beispiel 128A in 36.0 ml THF werden mit 12.0 ml Wasser und 741 mg (30.1 mmol) Lithiumhydroxid versetzt. Es wird für 6 h bei RT gerührt und anschließend wird die Reaktionslösung mit 1 N wässriger Hydrogenchlorid-Lösung versetzt. Es wird mit Dichlormethan extrahiert und die vereinigten organischen Phasen werden über Magnesiumsulfat getrocknet, filtriert und i. Vak. eingeengt. Man erhält 1.10 g (95% d. Th.) der Titelverbindung.
$^1$H-NMR (400 MHz, DMSO-$d_6$): $\delta$ = 13.2 (s, 1H), 7.97-7.88 (m, 1H), 7.78 (dd, 1H), 7.71 (s, 1H), 7.59-7.46 (m, 2H), 7.35 (ddd, 1H), 7.29 (s, 1H).
LC-MS (Methode 5): $R_t$ = 1.15 min; MS (ESIpos): m/z = 360 $[M+H]^+$.

**Beispiel 130A**

1-(3-Chlorphenyl)-5-(3-cyan-5-fluorphen yl)-1*H*-pyrazol-3-carbonsäureethylester

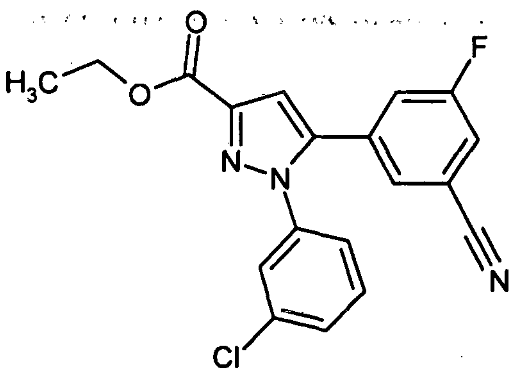

1.30 g (4.83 mmol) der 1,3-Diketo-Verbindung aus Beispiel 127A in 50 ml Ethanol werden mit 1.87 g (6.28 mmol) 3-Chlorphenylhydrazin Hydrochlorid mit 60%iger Reinheit versetzt und anschließend 2 h bei RT gerührt. Die Reaktionslösung wird nachfolgend mit 40 ml konz. Essigsäure versetzt und 2 h bei RT, dann 1 h bei 60 °C gerührt. Der Ansatz wird mit 1 N wässriger Hydrogenchlorid-Lösung versetzt und mit Dichlormethan extrahiert. Die vereinigten organischen Phasen werden über Magnesiumsulfat getrocknet, filtriert und i. Vak. eingeengt. Der Rückstand wird mittels Flash-Chromatographie (Laufmittel: Cyclohexan/Ethylacetat 20:1→1:1) gereinigt. Man erhält 1.16 g (65% d. Th.) der Titelverbindung.
$^1$H-NMR (400 MHz, DMSO-$d_6$): $\delta$ = 7.93 (d, 1H), 7.70 (s, 1H), 7.63-7.56 (m, 2H), 7.57-7.41 (m, 2H), 7.37 (s, 1H), 7.28 (d, 1H), 4.35 (q, 2H), 1.33 (t, 3H).
LC-MS (Methode 10): $R_t$ = 2.51 min; MS (ESIpos): m/z = 370 $[M+H]^+$.

**Beispiel 131A**

1-(3-Chlorphenyl)-5-(3-cyan-5-fluorphenyl)-1*H*-pyrazol-3-carbonsäure

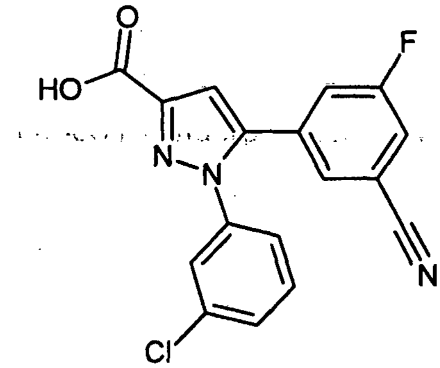

700 mg (1.89 mmol) des Carbonsäureesters aus Beispiel 130A in 60.0 ml THF werden mit 20.0 ml Wasser und 453 mg (18.9 mmol) Lithiumhydroxid versetzt. Es wird über Nacht bei RT gerührt und anschließend die Reaktionslösung mit 1 N wässriger Hydrogenchlorid-Lösung versetzt. Es wird mit Dichlormethan extrahiert und die vereinigten organischen Phasen werden über Magnesiumsulfat getrocknet, filtriert und i. Vak. eingeengt. Das Rohprodukt wird über präparative HPLC (RP18-Säule; Laufmittel: Acetonitril/Wasser-Gradient) gereinigt. Man erhält 712 mg (89% d. Th.) der Titelverbindung in 81%iger Reinheit.

LC-MS (Methode 10): $R_t$ = 2.11 min; MS (ESIpos): m/z = 342 $[M+H]^+$.

**Beispiel 132A**

1-(3-Cyan-4-fluorphenyl)-5-(3-cyan-5-fluorphenyl)-1*H*-pyrazol-3-carbonsäureethyl-ester

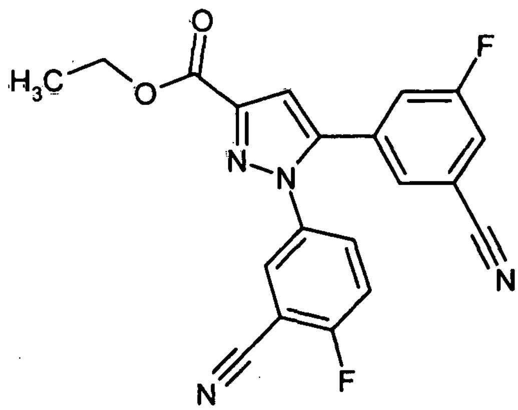

800 mg (3.04 mmol) der 1,3-Diketo-Verbindung aus Beispiel 127A in 12 ml *N*,*N*-Dimethylacetamid werden mit 1.96 g (3.65 mmol) 2-Fluor-S-hydrazinylbenzolcarbonitril mit 35%iger Reinheit versetzt und zu der entstandenen Lösung werden anschließend 0.152 ml 10 N wässrige Hydrogenchlorid-Lösung gegeben. Es wird für 20 h bei RT gerührt und anschließend direkt über präparative HPLC (RP18-Säule; Laufmittel: Acetonitril/Wasser-Gradient) gereinigt. Man erhält 931 mg (61% d. Th.) der Titelverbindung mit 75%iger Reinheit.

LC-MS (Methode 5): $R_t$ = 1.25 min; MS (ESIpos): m/z = 379 $[M+H]^+$.

**Beispiel 133A**

1-(3-Cyan-4-fluorphenyl)-5-(3-cyan-5-fluorphenyl)-1*H*-pyrazol-3-carbonsäure

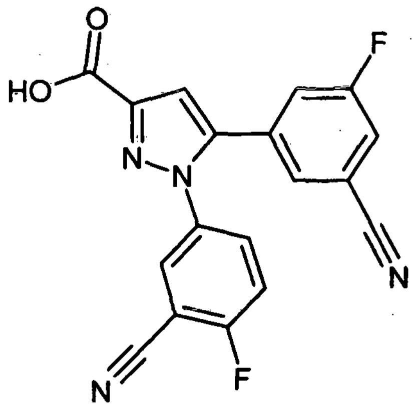

900 mg (1.78 mmol) mit 75%iger Reinheit des Carbonsäureesters aus Beispiel 132A in 30.0 ml THF werden mit 10.0 ml Wasser und 570 mg (23.8 mmol) Lithiumhydroxid versetzt. Es wird für 5 h bei RT gerührt und die Reaktionslösung wird anschließend mit 1 N wässriger Hydrogenchlorid-Lösung versetzt. Es wird mit Dichlormethan extrahiert und die vereinigten organischen Phasen werden über Magnesiumsulfat getrocknet, filtriert und i. Vak. eingeengt. Das Rohprodukt wird über präparative HPLC (RP18-Säule; Laufmittel: Acetonitril/Wasser-Gradient) gereinigt. Man verhält 527 mg (86% d. Th.) der Titelverbindung.

$^1$H-NMR (400 MHz, DMSO-$d_6$): $\delta$ = 8.09 (br. s., 1H), 7.93 (d, 1H), 7.81-7.69 (m, 2H), 7.69-7.60 (m, 1H), 7.55 (d, 1H), 7.28 (br. s., 1H).

LC-MS (Methode 5): $R_t$ = 1.03 min; MS (ESIpos): m/z = 351 $[M+H]^+$.

**Beispiel 134A**

5-(3-Cyan-5-fluorphenyl)-1-(3-cyanphenyl)-1*H*-pyrazol-3-carbonsäureethylester

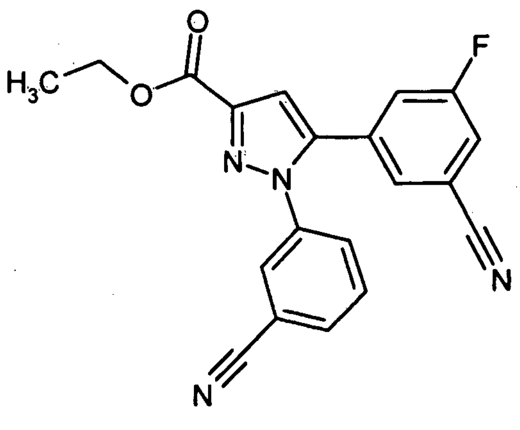

1.00 g (3.80 mmol) der 1,3-Diketo-Verbindung aus Beispiel 127A in 20 ml *N,N*-Dimethylacetamid werden mit 985 mg (4.56 mmol) 3-Hydrazinylbenzonitril mit 79%iger Reinheit versetzt und anschließend werden zu der entstandenen Lösung 0.190 ml 10 N wässrige Hydrogenchlorid-Lösung gegeben. Es wird für 20 h bei RT gerührt und anschließend direkt über präparative HPLC (RP18-Säule; Laufmittel: Acetonitril/Wasser-Gradient) gereinigt. Man erhält 1.22 g (80% d. Th.) der Titelverbindung mit 90%iger Reinheit.

LC-MS (Methode 7): $R_t$ = 2.04 min; MS (ESIpos): m/z = 361 $[M+H]^+$.

**Beispiel 135A**

5-(3-Cyan-5-fluorphenyl)-1-(3-cyanphenyl)-1*H*-pyrazol-3-carbonsäure

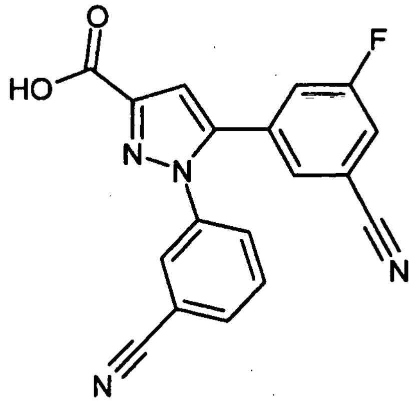

1.20 g (3.00 mmol) mit 90%iger Reinheit des Carbonsäureesters aus Beispiel 134A in 45.0 ml THF werden mit 15.0 ml Wasser und 797 mg Lithiumhydroxid (33.3 mmol) versetzt. Es wird für 5 h bei RT gerührt und die Reaktionslösung wird anschließend mit 1 N wässriger Hydrogenchlorid-Lösung versetzt. Es wird mit Dichlormethan extrahiert und die vereinigten organischen Phasen werden über Magnesiumsulfat getrocknet, filtriert und i. Vak. eingeengt. Das Rohprodukt wird über präparative HPLC (RP18-Säule; Laufmittel: Acetonitril/Wasser-Gradient) gereinigt. Man erhält 821 mg (82% d. Th.) der Titelverbindung.

$^1$H-NMR (400 MHz, DMSO-$d_6$): $\delta$ = 13.23 (br. s., 1H), 8.01-7.88 (m, 3H), 7.73-7.63 (m, 3H), 7.55 (d, 1H), 7.32 (s, 1H).

LC-MS (Methode 7): $R_t$ = 1.57 min; MS (ESIpos): m/z = 332 $[M+H]^+$.

**Ausführungsbeispiele:**

**Beispiel 1**

1-{1[1-(3-Chlor-4-fluorphenyl)-5-(3-chlor-5-fluorphenyl)-1*H*-pyrazol-3-yl]carbonyl}imi-dazolidin-4-on

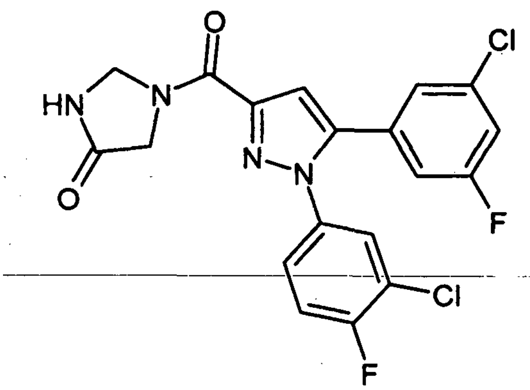

50.0 mg (0.14 mmol) der Verbindung aus Beispiel 71A, 12.8 mg (0.15 mmol) Imidazolidin-4-on und 106 mg (0.20 mmol) PyBOP werden in 2.5 ml Tetrahydrofuran vorgelegt und bei Raumtemperatur mit 50 µl (0.28 mmol) N,N-Diisopropylethylamin versetzt. Man rührt bei Raumtemperatur über Nacht und reinigt anschließend die Reaktionsmischung über die präparative HPLC (RP18-Säule; Laufmittel: Acetonitril/Wasser-Gradient). Man erhält 49.0 mg (83% d. Th.) der Titelverbindung.

$^1$H-NMR (400 MHz, DMSO-$d_6$): $\delta$ = 8.73 (s, 0.6H), 8.63 (s, 0.4H), 7.85-7.81 (m, 1H), 7.58-7.51 (m, 2H), 7.41-7.34

(m, 1H), 7.29-7.26 (m, 2H), 7.22-7.17 (m, 1H), 5.32 (s, 0.8H), 4.90 (s, 1.2H), 4.43 (s, 1.2H), 3.98 (s, 0.8H).

LC-MS (Methode 7): $R_t$ = 1.94 min; MS (ESIpos): m/z = 437 $[M+H]^+$.

**Beispiele 2**

1-{[5-(3-Chlor-5-fluorphenyl)-1-(3-chlorphenyl)-1*H*-pyrazol-3-yl]carbonyl}imidazo-lidin-4-on

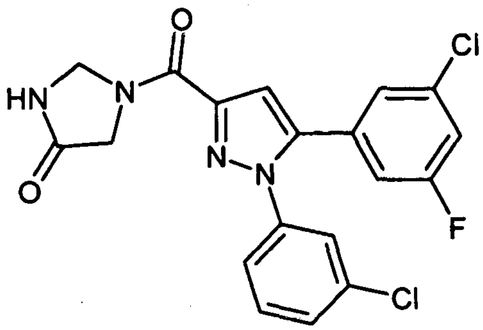

Die Herstellung der Titelverbindung erfolgt ausgehend von der Verbindung aus Beispiel 72A analog zur Synthese der Verbindung aus Beispiel 1. Man erhält 50 mg (84% d. Th.) der Titelverbindung.

$^1$H-NMR (400 MHz, DMSO-$d_6$): $\delta$ = 8.73 (s, 0.6H), 8.63 (s, 0.4H), 7.63-7.61 (m, 1H), 7.59-7.55 (m; 1H), 7.55-7.47 (m, 2H), 7.33-7.29 (m, 1H), 7.29-7.26 (m, 1H), 7.26-7.24 (m, 1H), 7.21-7.16 (m, 1H), 5.32 (s, 0.8H), 4.90 (s, 1.2H), 4.44 (s, 1.2H), 3.98 (s, 0.8H).

LC-MS (Methode 7): $R_t$ = 1.90 min; MS (ESIpos): m/z = 419 $[M+H]^+$.

**Beispiel 3**

1-{[1-(3-Chlorphenyl)-5-(3,5difluorphenyl)-1*H*-pyrazol-3-yl]carbonyl}imidazolidin-4-on

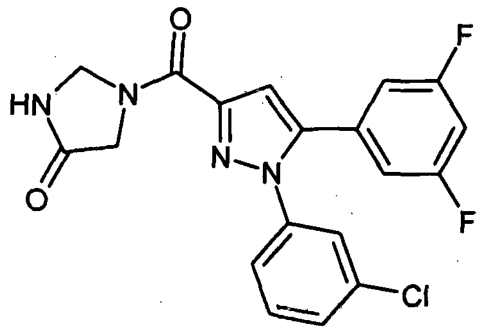

Die Herstellung der Titelverbindung erfolgt ausgehend von der Verbindung aus Beispiel 73A analog zur Synthese der Verbindung aus Beispiel 1. Man erhält 28 mg (73% d. Th.) der Titelverbindung.

$^1$H-NMR (400 MHz, DMSO-$d_6$): $\delta$ = 8.73 (s, 0.6H), 8.62 (s, 0.4H), 7.61 (t, 1H), 7.59-7.54 (m, 1H), 7.53-7.47 (m, 1H), 7.38-7.27 (m, 2H), 7.27-7.25 (m, 1H), 7.10-7.04 (m, 2H), 5.33 (s, 0.8H), 4.90 (s, 1.2H), 4.44 (s, 1.2H), 3.98 (s, 0.8H).

LC-MS (Methode 5): $R_t$ = 1.15 min; MS (ESIpos): m/z = 403 $[M+H]^+$.

**Beispiel 4**

1-{[1,5-Bis(3-chlorphenyl)-1*H*-pyrazol-3-yl]carbonyl}imidazolidin-4-on

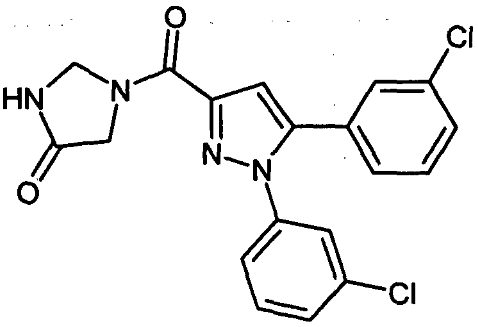

Die Herstellung der Titelverbindung erfolgt ausgehend von der Verbindung aus Beispiel 74A analog zur Synthese der Verbindung aus Beispiel 1. Man erhält 110 mg (91% d. Th.) der Titelverbindung.
$^1$H-NMR (400 MHz, DMSO-$d_6$): $\delta$ = 8.72 (s, 0.6H), 8.62 (s, 0.4H), 7.58 (t, 1H), 7.56-7.52 (m, 1H), 7.51-7.39 (m, 4H), 7.31-7.25 (m, 1H), 7.22-7.18 (m, 2H), 5.34 (s, 0.8H), 4.91 (s, 1.2H), 4.45 (s, 1.2H), 3.98 (s, 0.8H).
LC-MS (Methode 9): $R_t$ = 2.03 min; MS (ESIpos): m/z = 401 $[M+H]^+$.

**Beispiel 5**

1-{[1,5-Bis(3-chlor-4-fluorphenyl)-1H-pyrazol-3-yl]carbonyl}imidazolidin-4-on

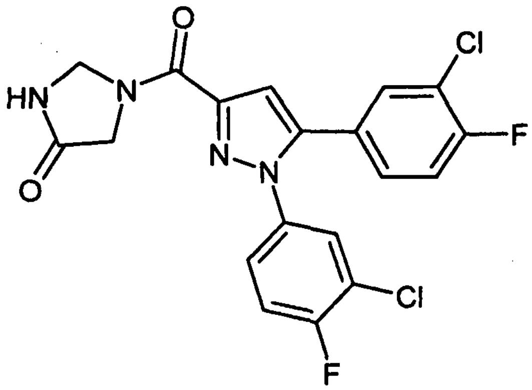

Die Herstellung der Titelverbindung erfolgt ausgehend von der Verbindung aus Beispiel 75A analog zur Synthese der Verbindung aus Beispiel 1. Man erhält 23 mg (39% d. Th.) der Titelverbindung.
$^1$H-NMR (400 MHz, DMSO-$d_6$): $\delta$ = 8.73 (s, 0.6H), 8.63 (s, 0.4H), 7.82 (dd, 1H), 7.68 (dt, 1H), 7.53 (dt, 1H), 7.45 (t, 1H), 7.38-7.32 (m, 1H), 7.25-7.19 (m, 2H), 5.33 (s, 0.8H), 4.90 (s, 1.2H), 4.44 (s, 1.2H), 3.98 (s, 0.8H).
LC-MS (Methode 7): $R_t$ = 1.93 min; MS (ESIpos): m/z = 437 $[M+H]^+$.

**Beispiel 6**

1-({1-(3-Chlorphenyl)-5-[3-(tritluormethoxy)phenyl]-1*H*-pyrazol-3-yl}carbonyl)imida-zolidin-4-on

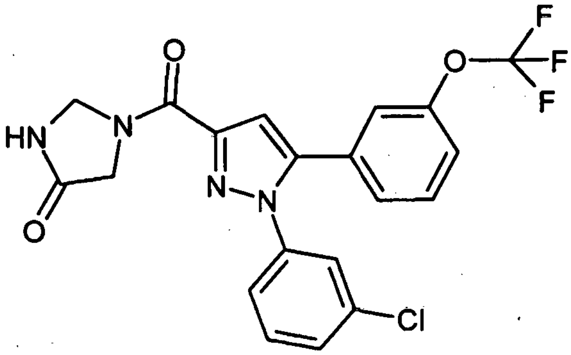

Die Herstellung der Titelverbindung erfolgt ausgehend von der Verbindung aus Beispiel 76A analog zur Synthese der Verbindung aus Beispiel 1. Man erhält 24 mg (64% d. Th.) der Titelverbindung.
$^1$H-NMR (400 MHz, DMSO-$d_6$): δ = 8.72 (s, 0.6H), 8.63 (s, 0.4H), 7.60-7.53 (m, 3H), 7.52-7.46 (m, 1H), 7.45-7.40 (m, 2H), 7.34-7.27 (m, 1H), 7.24-7.22 (m, 1H), 7.21-7.18 (m, 1H) 5.34 (s, 0.8H), 4.91 (s, 1.2H), 4.45 (s, 1.2H), 3.99 (s, 0.8H).
LC-MS (Methode 7): $R_t$ = 1.97 min; MS (ESIpos): m/z = 451 $[M+H]^+$.

**Beispiel 7**

1-({1-(3-Chlorphenyl)-5-[4-fluor-3-(trifluormethyl)phenyl]-1*H*-pyrazol-3-yl}carbonyl)imidazolidin-4-on

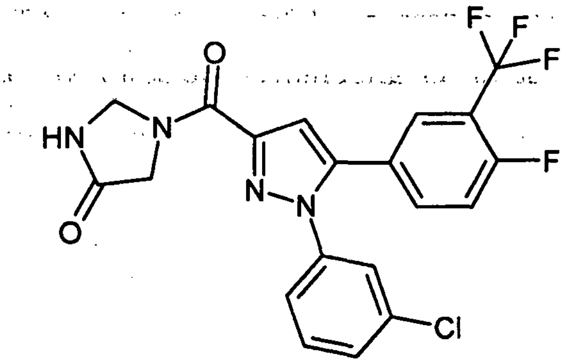

Die Herstellung der Titelverbindung erfolgt ausgehend von der Verbindung aus Beispiel 77A analog zur Synthese der Verbindung aus Beispiel 1. Man erhält 28 mg (74% d. Th.) der Titelverbindung.
$^1$H-NMR (400 MHz, DMSO-$d_6$): δ = 8.73 (s, 0.6H), 8.63 (s, 0.4H), 7.77 (ddd, 1H), 7.65-7.55 (m, 3H), 7.53-7.46 (m, 1H), 7.44-7.40 (m, 1H), 7.38-7.36 (m, 1H), 7.35-7.29 (m, 1H), 5.33 (s, 0.8H), 4.91 (s, 1.2H), 4.45 (s, 1.2H), 3.99 (s, 0.8H).
LC-MS (Methode 5): $R_t$ = 1.25 min; MS (ESIpos): m/z = 453 $[M+H]^+$.

**Beispiel 8**

1-({1-(3-Chlor-4-fluorphenyl)-5-[3-(trifluormethoxy)phenyl]-1*H*-pyrazol-3-yl}carbonyl)imidazolidin-4-on

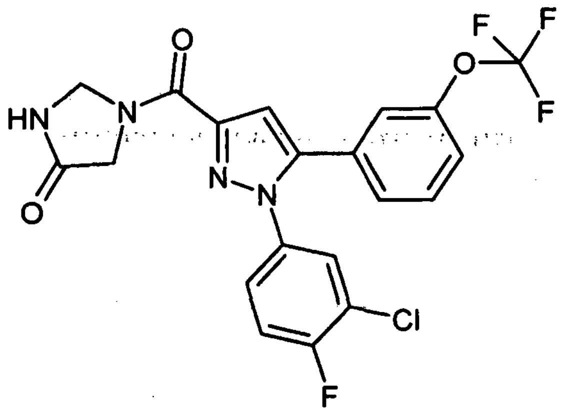

Die Herstellung der Titelverbindung erfolgt ausgehend von der Verbindung aus Beispiel 78A analog zur Synthese der Verbindung aus Beispiel 1. Man erhält 53 mg (91% d. Th.) der Titelverbindung.
$^1$H-NMR (400 MHz, DMSO-$d_6$): δ = 8.73 (s, 0.6H), 8.63 (s, 0.4H), 7.78-7.74 (m, 1H), 7.60-7.51 (m, 2H), 7.45-7.35 (m, 3H), 7.24-7.19 (m, 2H), 5.33 (s, 0.8H), 4.91 (s, 1.2H), 4.44 (s, 1.2H), 3.99 (s, 0.8H).
LC-MS (Methode 7): $R_t$ = 2.02 min; MS (ESIpos): m/z = 469 [M+H]+.

__Beispiele 9__

1-{[5-(3-Chlor-4-fluorphenyl)-1-(3-chlorphenyl)-1*H*-pyrazol-3-yl]carbonyl}imidazolidin-4-on

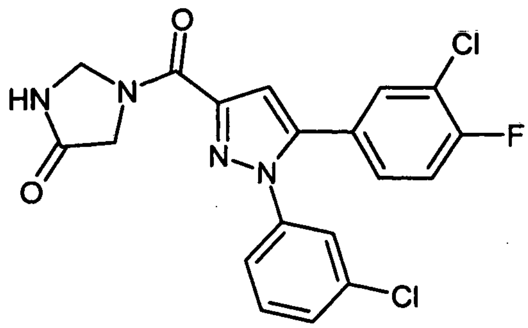

Die Herstellung der Titelverbindung erfolgt ausgehend von der Verbindung aus Beispiel 79A analog zur Synthese der Verbindung aus Beispiel 1. Man erhält 52 mg (87% d. Th.) der Titelverbindung.
$^1$H-NMR (400 MHz, DMSO-$d_6$): δ = 8.73 (s, 0.6H), 8.62 (s, 0.4H), 7.67-7.63 (m, 1H), 7.61 (t, 1H), 7.57-7.52 (m, 1H), 7.51-7.41 (m, 2H), 7.30-7.21 (m, 2H), 7.21-7.19 (m, 1H), 5.33 (s, 0.8H), 4.91 (s, 1.2H), 4.45 (s, 1.2H), 3.98 (s, 0.8H).
LC-MS (Methode 7): $R_t$ = 1.89 min; MS (ESIpos): m/z = 419 $[M+H]^+$.

__Beispiel 10__

1-{[5-(3-Fluorphenyl)-1-(3-methoxyphenyl)-1*H*-pyrazol-3-yl]carbonyl}imidazolidin-4-on

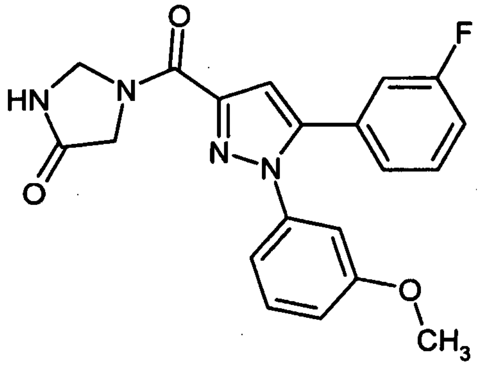

Die Herstellung der Titelverbindung erfolgt ausgehend von der Verbindung aus Beispiel 80A analog zur Synthese der Verbindung aus Beispiel 1. Man erhält 27 mg (70% d. Th.) der Titelverbindung.

$^1$H-NMR (400 MHz, DMSO-$d_6$): $\delta$ = 8.71 (s, 0.6H), 8.62 (s, 0.4H), 7.45-7.34 (m, 2H), 7.27-7.15 (m, 3H), 7.09 (d, 1H), 7.06-7.01 (m, 1H), 6.98-6.95 (m, 1H), 6.92-6.86 (m, 1H), 5.33 (s, 0.8H), 4.90 (s, 1.2H), 4.44 (s, 1.2H), 3.98 (s, 0.8H), 3.72 (s, 3H).

LC-MS (Methode 7): $R_t$ = 1.63 min; MS (ESIpos): m/z = 381 $[M+H]^+$.

**Beispiel 11**

1-{[1-(3-Chlorphenyl)-5-(3-methoxyphepenyl)-1*H*-pyrazol-3-yl]carbonyl}imidazolidin-4-on

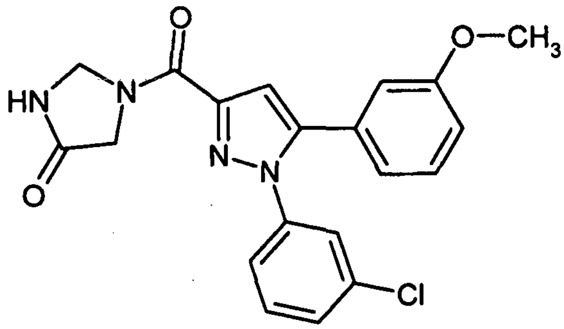

Die Herstellung der Titelverbindung erfolgt ausgehend von der Verbindung aus Beispiel 81A analog zur Synthese der Verbindung aus Beispiel 1. Man erhält 31 mg (84% d. Th.) der Titelverbindung.

$^1$H-NMR (400 MHz, DMSO-$d_6$): $\delta$ = 8.72 (s, 0.6H), 8.62 (s, 0.4H), 7.57-7.44 (m, 3H), 7.33-7.25 (m, 2H), 7.14-7.12 (m, 1H), 7.00-6.95 (m, 1H), 6.91-6.87 (m, 1H), 6.83-6.78 (m, 1H), 5.34 (s, 0.8H), 4.90 (s, 1.2H), 4.46 (s, 1.2H), 3.98 (s, 0.8H), 3.70 (s, 3H).

LC-MS (Methode 7): $R_t$ = 1.73 min; MS (ESIpos): m/z = 397 $[M+H]^+$.

**Beispiel 12**

1-({5-(3-Chlorphenyl)-1-[3-(trifluormethyl)phenyl]-1*H*-pyrazol-3-yl}carbonyl)imidazolidin-4-on

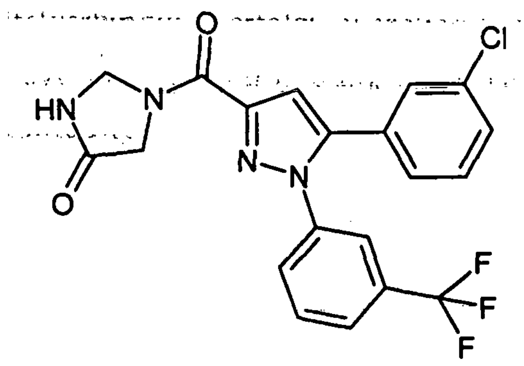

Die Herstellung der Titelverbindung erfolgt ausgehend von der Verbindung aus Beispiel 82A analog zur Synthese der Verbindung aus Beispiel 1. Man erhält 22 mg (58% d. Th.) der Titelverbindung.
$^1$H-NMR (400 MHz, DMSO-$d_6$): $\delta$ = 8.73 (s, 0.6H), 8.64 (s, 0.4H), 7.86-7.81 (m, 1H), 7.76-7.66 (m, 3H), 7.51-7.47 (m, 1H), 7.46-7.38 (m, 2H), 7.24-7.18 (m, 2H), 5.35 (s, 0.8H), 4.91 (s, 1.2H), 4.46 (s, 1.2H), 3.99 (s, 0.8H).
LC-MS (Methode 5): $R_t$ = 1.23 min; MS (ESIpos): m/z = 435 $[M+H]^+$.

## Beispiel 13

1-{[1-(3-Chlorphenyl)-5-(3,4-difluorphenyl)-1*H*-pyrazol-3-yl]carbonyl}imidazolidin-4-on

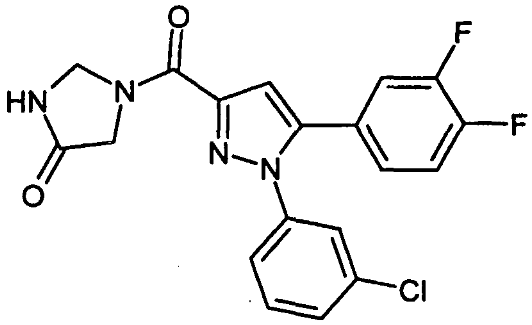

Die Herstellung der Titelverbindung erfolgt ausgehend von der Verbindung aus Beispiel 83A analog zur Synthese der Verbindung aus Beispiel 1. Man erhält 16 mg (41%, d. Th.) der Titelverbindung.
$^1$H-NMR (400 MHz, DMSO-$d_6$): $\delta$ = 8.72 (s, 0.6H), 8.62 (s, 0.4H), 7.60 (t, 1H), 7.56-7.44 (m, 4H), 7.30-7.24 (m, 1H), 7.19-7.17 (m, 1H), 7.12-7.06 (m, 1H), 5.34 (s, 0.8H), 4.90 (s, 1.2H), 4.45 (s, 1.2H), 3.98 (s, 0.8H).
LC-MS (Methode 5): $R_t$ = 1.16 min; MS (ESIpos): m/z = 403 $[M+H]^+$.

## Beispiel 14

1-{[5-(3-Brom-4-fluorphenyl)-1-(3-chlorphenyl)-1*H*-pyrazol-3-yl]carbonyl}imidazolidin-4-on

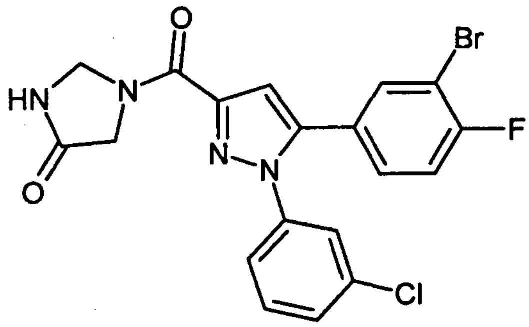

Die Herstellung der Titelverbindung erfolgt ausgehend von der Verbindung aus Beispiel 84A analog zur Synthese der Verbindung aus Beispiel 1. Man erhält 26 mg (70% d. Th.) der Titelverbindung.
$^1$H-NMR (400 MHz, DMSO-$d_6$): $\delta$ = 8.72 (s, 0.6H), 8.62 (s, 0.4H), 7.77-7.72 (m, 1H), 7.60 (t, 1H), 7.57-7.52 (m, 1H), 7.51-7.45 (m, 1H), 7.41 (t, 1H), 7.31-7.24 (m, 2H), 7.21-7.19 (m, 1H), 5.33 (s, 0.8H), 4.90 (s, 1.2H), 4.44 (s, 1.2H), 3.98 (s, 0.8H).
LC-MS (Methode 7): $R_t$ = 1.92 min; MS (ESIpos): m/z = 463 $[M+H]^+$.

**Beispiel 15**

1-{[5-(3-Chlorphenyl)-1-(4-fluorphenyl)-1*H*-pyrazol-3-yl]carbonyl}imidazolidin-4-on

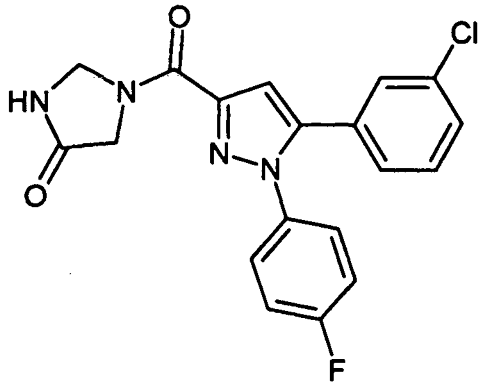

Die Herstellung der Titelverbindung erfolgt ausgehend von der Verbindung aus Beispiel 85A analog zur Synthese der Verbindung aus Beispiel 1. Man erhält 53 mg (42% d. Th.) der Titelverbindung.
$^1$H-NMR (400 MHz, DMSO-$d_6$): $\delta$ = 8.71 (s, 0.6H), 8.63 (s, 0.4H), 7.48-7.31 (m, 7H), 7.20-7.15 (m, 2H), 5.32 (s, 0.8H), 4.90 (s, 1.2H), 4.43 (s, 1.2H), 3.98 (s, 0.8H).
LC-MS (Methode 7): $R_t$ = 1.77 min; MS (ESIpos): m/z = 385 $[M+H]^+$.

**Beispiel 16**

1-{[1-(3-Chlorphenyl)-5-(2-fluorphenyl)-1*H*-pyrazol-3-yl]carbonyl}imidazolidin-4-on

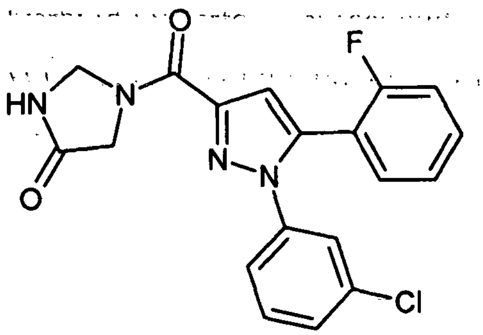

Die Herstellung der Titelverbindung erfolgt ausgehend von der Verbindung aus Beispiel 86A analog zur Synthese der Verbindung aus Beispiel 1. Man erhält 34 mg (88% d. Th.) der Titelverbindung.

$^1$H-NMR (400 MHz, DMSO-$d_6$): δ=8.73 (s, 0.6H), 8.64 (s, 0.4H), 7.57-7.40 (m, 5H), 7.35-7.21 (m, 3H), 7.14-7.11 (m, 1H), 5.38 (s, 0.8H), 4.91 (s, 1.2H), 4.49 (s, 1.2H), 3.99 (s, 0.8H).

LC-MS (Methode 5): $R_t$ = 1.10 min; MS (ESIpos): m/z = 385 $[M+H]^+$.

## Beispiel 17

1-({1-(4-Fluorphenyl)-5-[3-(trifluormethyl)phenyl]-1*H*-pyrazol-3-yl}carbonyl)imidazolidin-4-on

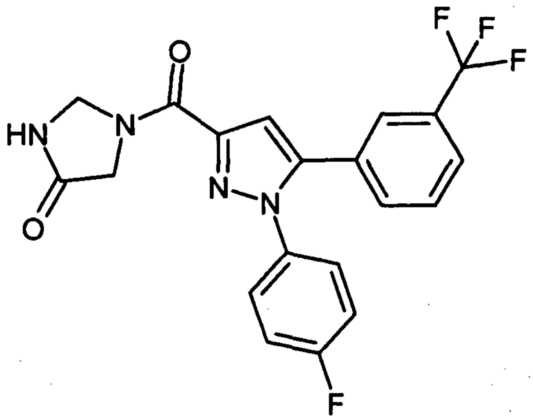

Die Herstellung der Titelverbindung erfolgt ausgehend von der Verbindung aus Beispiel 87A analog zur Synthese der Verbindung aus Beispiel 1. Man erhält 28 mg (74% d. Th.) der Titelverbindung.

$^1$H-NMR (400 MHz, DMSO-$d_6$): δ = 8.72 (s, 0.6H), 8.63 (s, 0.4H), 7.75 (d, 1H), 7.65-7.56 (m, 3H), 7.49-7.43 (m, 2H), 7.37-7.30 (m, 2H), 7.29-7.26 (m, 1H), 5.33 (s, 0.8H), 4.91 (s, 1.2H), 4.44 (s, 1.2H), 3.99 (s, 0.8H).

LC-MS (Methode 5): $R_t$ = 1.17 min; MS (ESIpos): m/z = 419 $[M+H]^+$.

## Beispiel 18

1-{[1-(3-Chlorphenyl)-5-(2,3-difluorphenyl)-1*H*-pyrazol-3-yl]carbonyl}imidazolidin-4-on

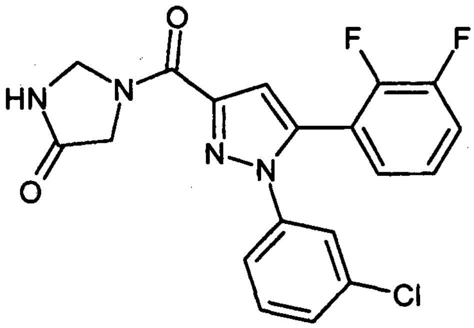

Die Herstellung der Titelverbindung erfolgt ausgehend von der Verbindung aus Beispiel 88A analog zur Synthese der Verbindung aus Beispiel 1. Man erhält 8 mg (100% d. Th.) der Titelverbindung.
$^1$H-NMR (400 MHz, DMSO-$d_6$): $\delta$ = 8.73 (s, 0.6H), 8.64 (s, 0.4H), 7.61-7.43 (m, 4H), 7.35-7.21 (m, 3H), 7.21-7.19 (m, 1H), 5.38 (s, 0.8H), 4.91 (s, 1.2H), 4.49 (s, 1.2H), 3.99 (s, 0.8H).
LC-MS (Methode 5): $R_t$ = 1.12 min; MS (ESIpos): m/z = 403 $[M+H]^+$.

**Beispiel 19**

1-{[5-(3-Chlorphenyl)-1-(4-chlorphenyl)-1*H*-pyrazol-3-yl]carbonyl}imidazolidin-4-on

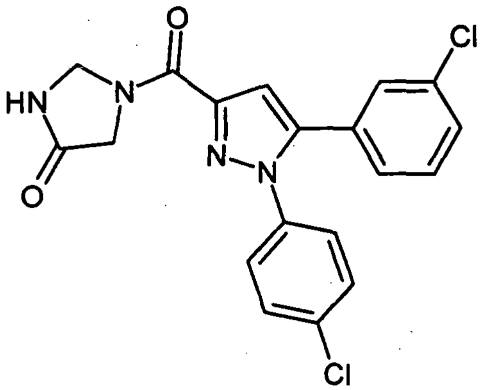

Die Herstellung der Titelverbindung erfolgt ausgehend von der Verbindung aus Beispiel 89A analog zur Synthese der Verbindung aus Beispiel 1. Man erhält 31 mg (80% d. Th.) der Titelverbindung.
$^1$H-NMR (400 MHz, DMSO-$d_6$): $\delta$ = 8.72 (s, 0.6H), 8.65 (s, 0.4H), 7.58-7.53 (m, 2H), 7.50-7.38 (m, 5H), 7.20-7.16 (m, 2H), 5.32 (s, 0.8H), 4.90 (s, 1.2H), 4.43 (s, 1.2H), 3.98 (s, 0.8H).
LC-MS (Methode 5): $R_t$ = 1.22 min; MS (ESIpos): m/z = 401 $[M+H]^+$.

**Beispiel 20**

1-{[5-(3-Fluorphenyl)-1-(4-fluorphenyl)-1*H*-pyrazol-3-yl]carbonyl}imidazolidin-4-on

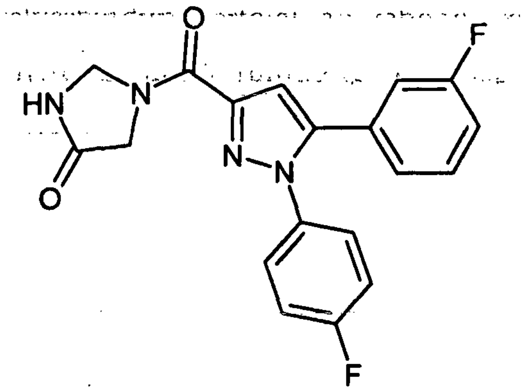

Die Herstellung der Titelverbindung erfolgt ausgehend von der Verbindung aus Beispiel 90A analog zur Synthese der Verbindung aus Beispiel 1. Man erhält 18 mg (47% d. Th.) der Titelverbindung.

$^{1}$H-NMR (400 MHz, DMSO-$d_6$): δ = 8.72 (s, 0.6H), 8.63 (s, 0.4H), 7.48-7.39 (m, 3H), 7.37-7.30 (m, 2H), 7.27-7.16 (m, 3H), 7.08-7.04 (m, 1H), 5.32 (s, 0.8H), 4.90 (s, 1.2H), 4.43 (s, 1.2H), 3.98 (s, 0.8H).

LC-MS (Methode 7): $R_t$ = 1.64 min; MS (ESIpos): m/z = 369 $[M+H]^+$.

## Beispiel 21

1-{[1-(3-Chlorphenyl)-5-(3-fluor-5-methoxyphenyl)-1*H*-pyrazol-3-yl]carbonyl}imidazolidin-4-on

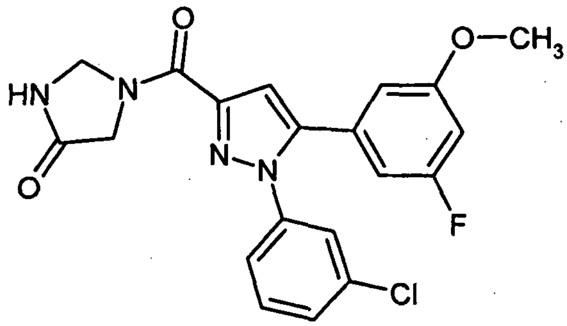

800 mg (2.31 mmol) der Verbindung aus Beispiel 91A werden in 19 ml trockenem Toluol vorgelegt, bei Raumtemperatur mit 0.48 ml (6.58 mmol) Thionylchlorid versetzt und 2.5 h unter Rückfluss erhitzt. Nach dem Abkühlen wird der Rückstand in 6 ml Dichlormethan aufgenommen, ein Achtel der Lösung bei 0 °C mit 0.13 ml (0.92 mmol) Triethylamin und 92.3 mg (0.46 mmol) der Verbindung aus Beispiel 125A versetzt und über Nacht bei RT gerührt. Man engt die Reaktionsmischung ein und reinigt den Rückstand über präparative HPLC (RP18-Säule; Laufmittel: Acetonitril/Wasser-Gradient) und per präparativer Dünnschichtchromatographie (Kieselgel; Laufmittel: Essigsäureethylester). Man erhält 11 mg (9% d. Th.) der Titelverbindung.

$^{1}$H-NMR (400 MHz, DMSO-$d_6$): δ = 8.72 (s, 0.6H), 8.61 (s, 0.4H), 7.59 (t, 1H), 7.57-7.53 (m, 1H), 7.53-7.46 (m, 1H), 7.33-7.26 (m, 1H), 7.21-7.19 (m, 1H), 6.90 (dt, 1H), 6.74-6.69 (m, 2H), 5.33 (s, 0.8H), 4.90 (s, 1.2H), 4.44 (s, 1.2H), 3.98 (s, 0.8H), 3.71 (s, 3H).

LC-MS (Methode 1): $R_t$ = 2.23 min; MS (ESIpos): m/z = 415 $[M+H]^+$.

## Beispiel 22

1-{[1-(3-Chlor-4-fluorphenyl)-5-(3-fluor-5-methoxyphenyl)-1*H*-pyrazol-3-yl]carbonyl}imidazolidin-4-on

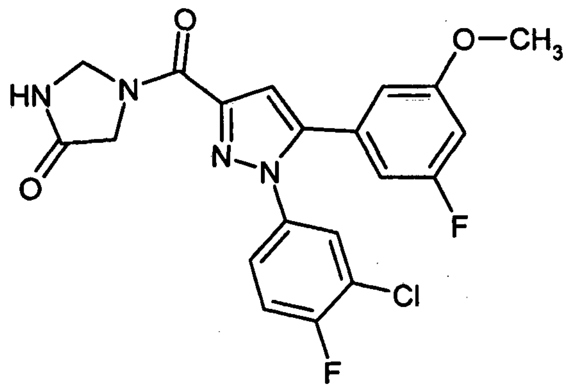

Die Herstellung der Titelverbindung erfolgt ausgehend von der Verbindung aus Beispiel 92A analog zur Synthese der Verbindung aus Beispiel 21. Man erhält 29 mg (24% d. Th.) der Titelverbindung.

$^1$H-NMR (400 MHz, DMSO-$d_6$): δ = 8.72 (s, 0.6H), 8.62 (s, 0.4H), 7.81 (dd, 1H), 7.54 (dt, 1H), 7.40-7.33 (m, 1H), 7.20-7.18 (m, 1H), 6.90 (dt, 1H), 6.75-6.70 (m, 2H), 5.32 (s, 0.8H), 4.90 (s, 1.2H), 4.44 (s, 1.2H), 3.98 (s, 0.8H), 3.72 (s, 3H).

LC-MS (Methode 1): $R_t$ = 2.27 min; MS (ESIpos): m/z = 433 $[M+H]^+$.

**Beispiel 23**

1-({1-(3-Chlorphenyl)-5-[3-(2-hydroxyethoxy)phenyl]-1*H*-pyrazol-3-yl}carbonyl)imidazolidin-4-on

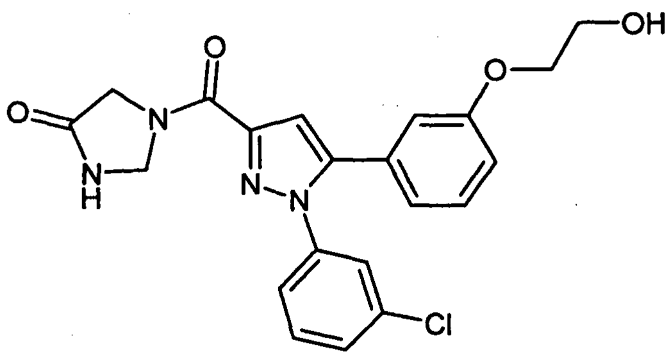

11.1 mg (0.031 mmol) der Verbindung aus Beispiel 115A, 6.8 mg (0.034 mmol) der Verbindung aus Beispiel 125A und 24.2 mg (0.046 mmol) PyBOP werden in 0.6 ml Tetrahydrofuran vorgelegt und bei Raumtemperatur mit 11 μl (0.065 mmol) N,N-Diisopropylethylamin versetzt. Man rührt bei Raumtemperatur über Nacht und reinigt anschließend die Reaktionsmischung über die präparative HPLC (RP18-Säule; Laufmittel: Acetonitril/Wasser-Gradient). Man erhält 3.5 mg (27% d. Th.) der Titelverbindung.

$^1$H-NMR (400 MHz, DMSO-$d_6$): δ=8.72 (s, 0.6H), 8.62 (s, 0.4H), 7.57-7.43 (m, 3H), 7.32-7.24 (m, 2H), 7.12-7.10 (m, 1H), 7.00-6.95 (m, 1H), 6.92-6.89 (m, 1H), 6.78 (d, 1H), 5.35 (s, 0.8H), 4.90 (s, 1.2H), 4.85 (t, 1H), 4.46 (s, 1.2H), 3.98 (s, 0.8H), 3.92 (t, 2H), 3.67 (q, 2H).

LC-MS (Methode 7): $R_t$ = 1.38 min; MS (ESIpos): m/z = 427 $[M+H]^+$.

**Beispiel 24**

1-({1-(3-Chlorphenyl)-5-[3-(3-hydroxypropoxy)phenyl]-1*H*-pyrazol-3-yl}carbonyl)imidazolidin-4-on

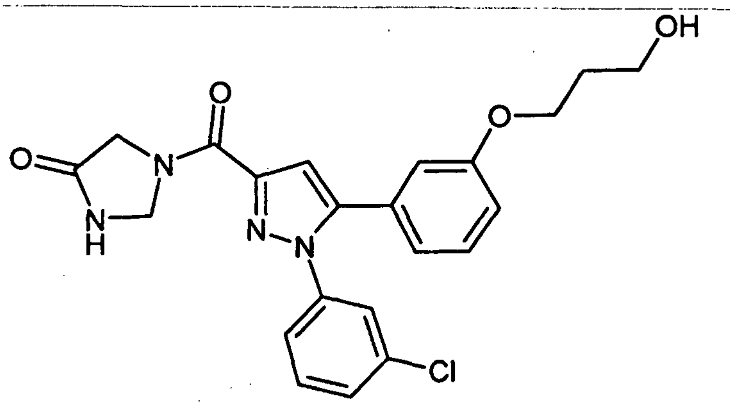

Die Herstellung der Titelverbindung erfolgt ausgehend von der Verbindung aus Beispiel 116A analog zur Synthese der Verbindung aus Beispiel 23. Man erhält 54 mg (69% d. Th.) der Titelverbindung.
$^1$H-NMR (400 MHz, DMSO-$d_6$): $\delta$ = 8.72 (s, 0.6H), 8.62 (s, 0.4H), 7.56-7.44 (m, 3H), 7.31-7.24 (m, 2H), 7.12-7.10 (m, 1H), 6.99-6.94 (m, 1H), 6.91-6.88 (m, 1H), 6.80-6.76 (m, 1H), 5.34 (s, 0.8H), 4.90 (s, 1.2H), 4.52 (t, 1H), 4.46 (s, 1.2H), 4.01-3.95 (m, 2.8H), 3.51 (q, 2H), 1.83-1.75 (m, 2H).
LC-MS (Methode 1): $R_t$ = 1.95 min; MS (ESIpos): m/z = 441 $[M+H]^+$.

## Beispiel 25

3{1-(3-Chlor-4-fluorphenyl)-3-[(4-oxoimidazolidin-1-yl)carbonyl]-1*H*-pyrazol-5-yl}benzolcarbonitril

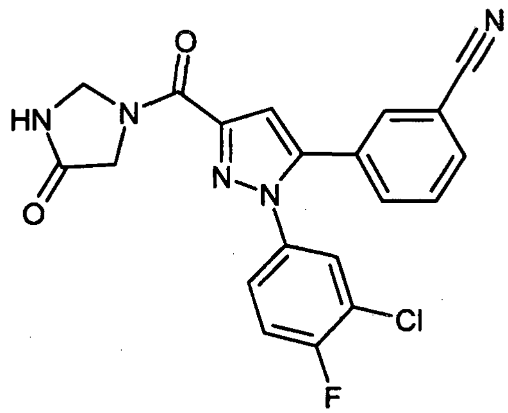

Die Herstellung der Titelverbindung erfolgt ausgehend von der Verbindung aus Beispiel 93A analog zur Synthese der Verbindung aus Beispiel 23. Man reinigt zusätzlich per präparativer Dünnschichtchromatographie (Kieselgel; Laufmittel: Essigsäureethylester) und erhält 8 mg (16% d. Th.) der Titelverbindung.
$^1$H-NMR (400 MHz, DMSO-$d_6$): $\delta$ = 8.73 (s, 0.6H), 8.63 (s, 0.4H), 7.95-7.92 (m, 1H), 7.89 (dt, 1H), 7.81 (dd, 1H), 7.59 (t, 1H), 7.56-7.49 (m, 2H), 7.38-7.31 (m, 1H), 7.27-7.25 (m, 1H), 5.34 (s, 0.8 H), 4.91 (s, 1.2H), 4.45 (s, 1.2H), 3.99 (s, 0.8H).
LC-MS (Methode 7): $R_t$ = 1.64 min; MS (ESIpos): m/z = 410 $[M+H]^+$.

## Beispiel 26

1-{[1-(3-Chlor-4-fluorphenyl)-5-(3-fluorphenyl)-1*H*-pyrazol-3-yl]carbonyl}imidazolidin-4-on

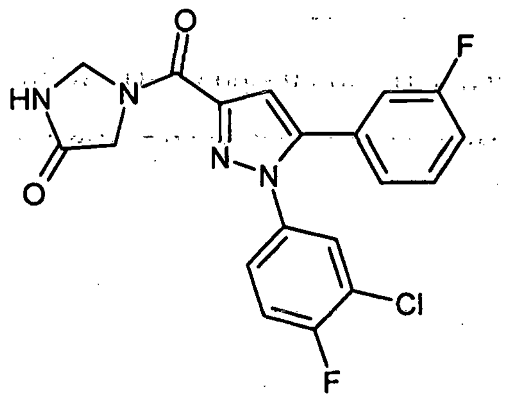

Die Herstellung der Titelverbindung erfolgt ausgehend von der Verbindung aus Beispiel 94A analog zur Synthese der Verbindung aus Beispiel 23. Man reinigt zusätzlich per präparativer Dünnschichtchromatographie (Kieselgel; Laufmittel: Essigsäureethylester) und erhält 10 mg (16% d. Th.) der Titelverbindung.
$^1$H-NMR (400 MHz, DMSO-$d_6$): $\delta$ = 8.72 (s, 0.6H), 8.63 (s, 0.4H), 7.79 (dd, 1H), 7.55 (dt, 1H), 7.48-7.40 (m, 1H), 7.39-7.32 (m, 1H), 7.30-7.23 (m, 2H), 7.19-7.17 (m, 1H), 7.10-7.05 (m, 1H), 5.33 (s, 0.8H), 4.90 (s, 1.2H), 4.45 (s, 1.2H), 3.98 (s, 0.8H).
LC-MS (Methode 7): $R_t$ = 1.78 min; MS (ESIpos): m/z = 403 $[M+H]^+$.

__Beispiel 27__

1-({1-(3-Chlor-4-fluorphenyl)-5-[3-(trifluormethyl)phenyl]-1*H*-pyrazol-3-yl}carbonyl)imidazolidin-4-on

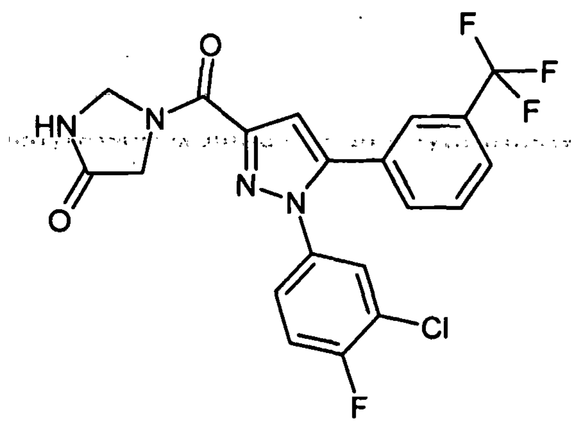

Die Herstellung der Titelverbindung erfolgt ausgehend von der Verbindung aus Beispiel 95A analog zur Synthese der Verbindung aus Beispiel 23. Man reinigt zusätzlich per präparativer Dünnschichtchromatographie (Kieselgel; Laufmittel: Essigsäureethylester) und erhält 17 mg (29% d. Th.) der Titelverbindung.
$^1$H-NMR (400 MHz, DMSO-$d_6$): $\delta$ = 8.73 (s, 0.6H), 8.63 (s, 0.4H), 7.83-7.75 (m, 2H), 7.69-7.50 (m, 4H), 7.41-7.34 (m, 1H), 7.30-7.27 (d, 1H), 5.34 (s, 0.8H), 4.91 (s, 1.2H), 4.45 (s, 1.2H), 3.99 (s, 0.8H).
LC-MS (Methode 7): $R_t$ = 1.95 min; MS (ESIpos): m/z = 453 $[M+H]^+$.

__Beispiel 28__

1-({1-(3-Chlor-4-fluorphenyl)-5-[3-fluor-5-(trifluormethyl)phenyl]-1*H*-pyrazol-3-yl}carbonyl)imidazolidin-4-on

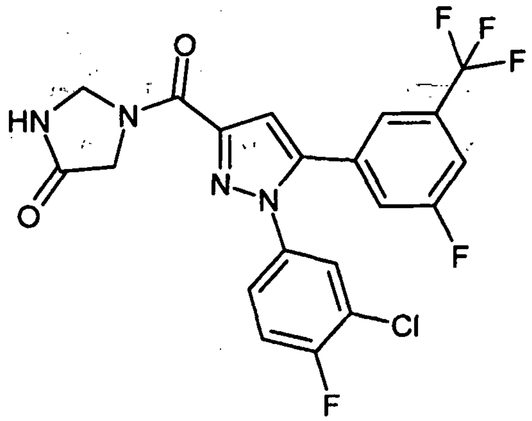

Die Herstellung der Titelverbindung erfolgt ausgehend von der Verbindung aus Beispiel 96A analog zur Synthese der Verbindung aus Beispiel 23. Man erhält 17 mg (27% d. Th.) der Titelverbindung.
$^1$H-NMR (400 MHz, DMSO-$d_6$): $\delta$ = 8.73 (s, 0.6H), 8.63 (s, 0.4H), 7.84 (dd, 1H), 7.77 (d, 1H), 7.60 (d, 1H), 7.55 (dt, 1H), 7.46-7.37 (m, 2H), 7.37-7.35 (m, 1H), 5.33 (s, 0.8H), 4.91 (s, 1.2H), 4.44 (s, 1.2H), 3.99 (s, 0.8H).
LC-MS (Methode 7): $R_t$ = 2.01 min; MS (ESIpos): m/z = 471 $[M+H]^+$.

__Beispiel 29__

1-{[1-(3-Chlorphenyl)-5-{3-[3-(dimethylamino)propoxy]phenyl}-1*H*-pyrazol-3-yl]carbonyl}imidazolidin-4-on-formiat

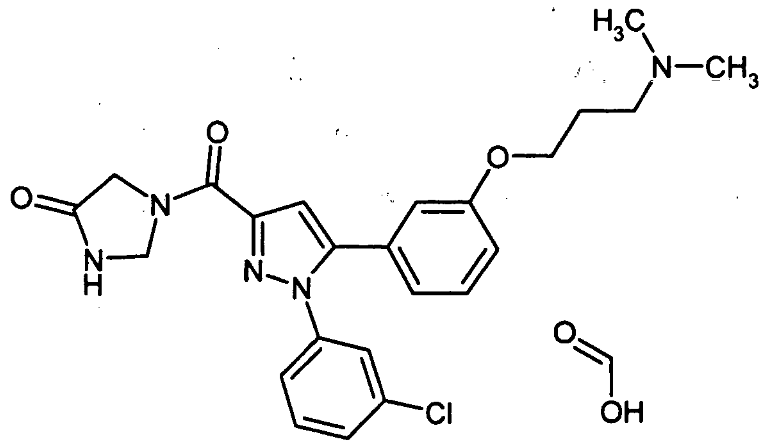

49.6 mg (0.096 mmol) der Verbindung aus Beispiel 120A und 0.48 ml (0.96 mmol) einer 2M Lösung von Dimethylamin in Tetrahydrofuran werden über Nacht bei 80 °C in 3 ml Ethanol gerührt. Man reinigt anschließend die Reaktionsmischung über die präparative HPLC (RP18-Säule; Laufmittel: Acetonitril/Wasser-Gradient unter Zusatz von 0.1% Ameisensäure). Man erhält 6.2 mg (23% d. Th.) der Titelverbindung.
$^1$H-NMR (400 MHz, DMSO-$d_6$): $\delta$ = 8.72 (s, 0.6H), 8.62 (s, 0.4H), 8.18 (s, 1H), 7.57-7.43 (m, 3H), 7.31-7.24 (m, 2H), 7.13-7.11 (m, 1H), 6.98-6.93 (m, 1H), 6.88-6.86 (m, 1H), 6.80 (d, 1H), 5.34 (s, 0.8H), 4.90 (s, 1.2H), 4.46 (s, 1.2H), 3.98 (s, 0.8H), 3.92 (t, 2H), 2.33 (t, 2H), 2.15 (s, 6H), 1.82-1.74 (m, 2H).
LC-MS (Methode 5): $R_t$ = 0.83 min; MS (ESIpos): m/z = 468 $[M+H]^+$.

__Beispiel 30__

1-({1-(3-Chlorphenyl)-5-[3-fluor-5-(trifluormethyl)phenyl]-1*H*-pyrazol-3-yl}carbonyl)imidazolidin-4-on

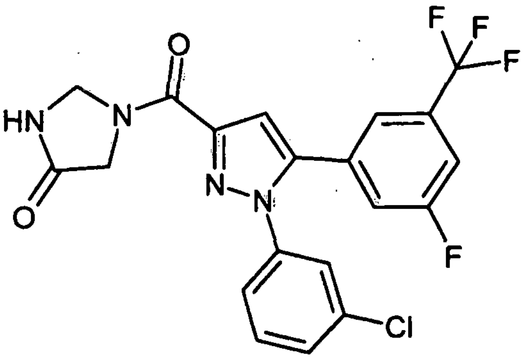

Die Herstellung der Titelverbindung erfolgt ausgehend von der Verbindung aus Beispiel 97A analog zur Synthese der Verbindung aus Beispiel 23 unter Zusatz von 0.1% Ameisensäure bei der präparativen HPLC. Man erhält 60 mg (51% d. Th.) der Titelverbindung.
$^1$H-NMR (400 MHz, DMSO-$d_6$): $\delta$ = 8.73 (s, 0.6H), 8.63 (s, 0.4H), 7.77 (d, 1H), 7.64-7.55 (m, 3H), 7.53-7.46 (m, 1H), 7.44-7.40 (m, 1H), 7.39-7.29 (m, 2H), 5.33 (s, 0.8H), 4.91 (s, 1.2H), 4.45 (s, 1.2H), 3.99 (s, 0.8H).
LC-MS (Methode 1): $R_t$ = 2.38 min; MS (ESIpos): m/z = 453 [M+H]$^+$.

**Beispiel 31**

1-({1-(3-Chlorphenyl)-5-[3-(3-pyrrolidin-1-ylpropoxy)phenyl]-1H-pyrazol-3-yl}carbonyl)imidazolidin-4-on-formiat

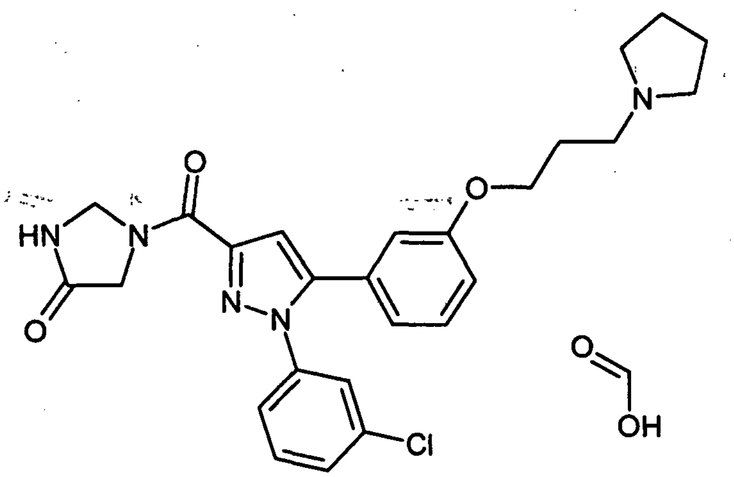

Die Herstellung der Titelverbindung erfolgt ausgehend von der Verbindung aus Beispiel 118A analog zur Synthese der Verbindung aus Beispiel 23. Man erhält 3.2 mg (33% d. Th.) der Titelverbindung.
$^1$H-NMR (400 MHz, DMSO-$d_6$): $\delta$ = 8.71 (s, 0.6H), 8.61 (s, 0.4H), 8.17 (s, 1H), 7.56-7.44 (m, 3H), 7.32-7.24 (m, 2H), 7.13-7.11 (m, 1H), 6.98-6.94 (m, 1H), 6.89-6.86 (m, 1H), 6.82-6.78 (m, 1H), 5.34 (s, 0.8H), 4.90 (s, 1.2H), 4.45 (s, 1.2H), 3.98 (s, 0.8H), 3.94 (t, 2H), 2.49-2.43 (m, 6H), 1.86-1.78 (m, 2H), 1.71-1.66 (m, 4H).
LC-MS (Methode 5): $R_t$ = 0.86 min; MS (ESIpos): m/z = 494 [M+H]$^+$.

**Beispiel 32**

1-{[1-(3-Bromphenyl)-5-(3-chlorphenyl)-1H-pyrazol-3-yl]carbonyl}imidazolidin-4-on

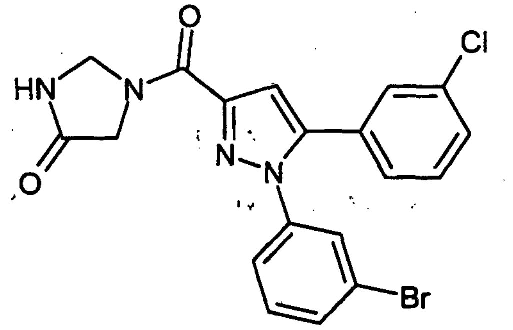

Die Herstellung der Titelverbindung erfolgt ausgehend von der Verbindung aus Beispiel 98A analog zur Synthese der Verbindung aus Beispiel 23. Man erhält 122 mg (52% d. Th.) der Titelverbindung.

$^1$H-NMR (400 MHz, DMSO-$d_6$): $\delta$ = 8.72 (s, 0.6H), 8.62 (s, 0.4H), 7.71-7.65 (m, 2H), 7.51-7.38 (m, 4H), 7.36-7.29 (m, 1H), 7.22-7.17 (m, 2H), 5.34 (s, 0.8H), 4.90 (s, 1.2H), 4.45 (s, 1.2H), 3.98 (s, 0.8H).

LC-MS (Methode 5): $R_t$ = 1.19 min; MS (ESIpos): m/z = 445 $[M+H]^+$.

**Beispiel 33**

1-{[1-(3-Bromphenyl)-5-(3-chlor-4-fluorphenyl)-1*H*-pyrazol-3-yl]carbonyl}imidazolidin-4-on

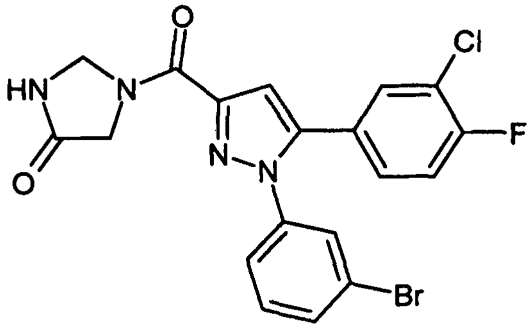

Die Herstellung der Titelverbindung erfolgt ausgehend von der Verbindung aus Beispiel 99A analog zur Synthese der Verbindung aus Beispiel 23. Man erhält 186 mg (79% d. Th.) der Titelverbindung.

$^1$H-NMR (400 MHz, DMSO-$d_6$): $\delta$ = 8.72 (s, 0.6H), 8.62 (s, 0.4H), 7.73 (t, 1H), 7.69 (d, 1H), 7.52 (dt, 1H), 7.47-7.40 (m, 1H), 7.37-7.31 (m, 1H), 7.28-7.24 (m, 2H), 7.21-7.16 (m, 1H), 5.33 (s, 0.8H), 4.90 (s, 1.2H), 4.44 (s, 1.2H), 3.98 (s, 0.8H).

LC-MS (Methode 7): $R_t$ = 1.93 min; MS (ESIpos): m/z = 463 $[M+H]^+$.

**Beispiel 34**

1-{[1-(3-Chlorphenyl)-5-(3-hydroxyphenyl)-1*H*-pyrazol-3-yl]carbonyl}imidazolidin-4-on

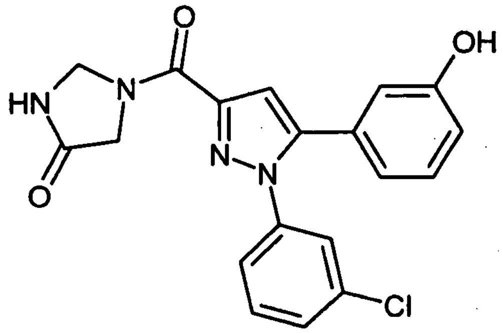

100 mg (0.25 mmol) der Verbindung aus Beispiel 100A, 25.5 mg (0.30 mmol) Imidazolidin-4-on und 141 mg (0.27 mmol) PyBOP werden in 2 ml Tetrahydrofuran vorgelegt und bei Raumtemperatur mit 47 μl (0.27 mmol) N,N-Diisopropylethylamin versetzt. Man rührt bei Raumtemperatur über Nacht und reinigt anschließend die Reaktionsmischung über die präparative HPLC (RP18-Säule; Laufmittel: Acetonitril/Wasser-Gradient). Man erhält 83.5 mg (72% d. Th.) 1-({5-[3-(Benzyloxy)phenyl]-1-(3-chlorphenyl)-1*H*-pyrazol-3-yl}carbonyl)imidazolidin-4-on.

$^1$H-NMR (400 MHz, DMSO-$d_6$): δ = 8.72 (s, 0.6H), 8.62 (s, 0.4H), 7.56-7.43 (m, 3H), 7.41-7.24 (m, 7H), 7.13-7.11 (m, 1H), 7.07-7.03 (m, 1H), 7.01-6.98 (m, 1H), 6.80 (d, 1H), 5.34 (s, 0.8H), 5.06 (s, 2H), 4.90 (s, 1.2H), 4.46 (s, 1.2H), 3.98 (s, 0.8H).

LC-MS (Methode 9): $R_t$ = 2.25 min; MS (ESIpos): m/z = 473 $[M+H]^+$.

317 mg (0.671 mmol) 1-({5-[3-(Benzyloxy)phenyl]-1-(3-chlorphenyl)-1H-pyrazol-3-yl}carbonyl)imidazolidin-4-on werden in 5.6 ml konz. Essigsäure vorgelegt, mit 357 mg (0.168 mmol) Palladium auf Aktivkohle (5%ig) versetzt und bei Raumtemperatur unter Wasserstoffatmosphäre über Nacht gerührt. Anschließend wird das Reaktionsgemisch filtriert, das Filtrat eingeengt und der Rückstand über die präparative HPLC (RP18-Säule; Laufmittel: Acetonitril/Wasser-Gradient) gereinigt. Man erhält 119 mg (46% d. Th.) der Titelverbindung.

$^1$H-NMR (400 MHz, DMSO-$d_6$): δ = 9.67 (s, 1H), 8.71 (s, 0.6H), 8.62 (s, 0.4H), 7.55-7.43 (m, 3H), 7.30-7.16 (m, 2H), 7.03-7.01 (m, 1H), 6.82-6.77 (m, 1H), 6.72-6.65 (m, 2H), 5.34 (s, 0.8H), 4.90 (s, 1.2H), 4.46 (s, 1.2H), 3.98 (s, 0.8H).

LC-MS (Methode 1): $R_t$ = 1.90 min; MS (ESIpos): m/z = 383 $[M+H]^+$.

<u>**Beispiel 35**</u>

1-({1-(3-Chlorphenyl)-5-[3-(2-methoxyethoxy)phenyl]-1*H*-pyrazol-3-yl}carbonyl)imidazolidin-4-on

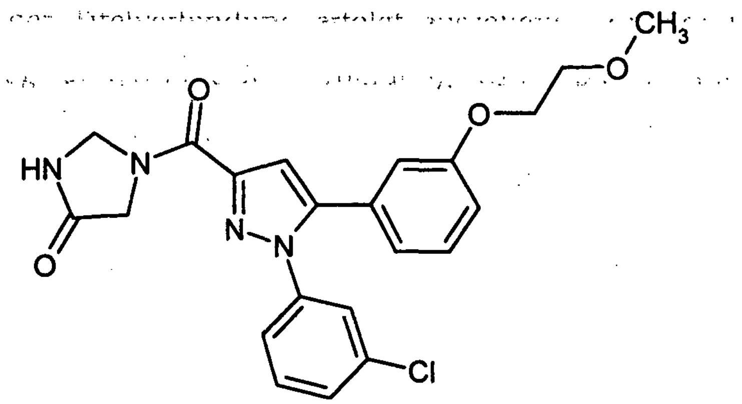

Die Herstellung der Titelverbindung erfolgt ausgehend von der Verbindung aus Beispiel 117A analog zur Synthese der Verbindung aus Beispiel 23. Zur Abtrennung von 1,1',1"-Phosphoryltripyrrolidin wird in wenig Tetrahydrofuran aufgenommen, mit Wasser versetzt bis sich ein Niederschlag bildet, das Tetrahydrofuran im Vakuum entfernt, der Niederschlag abgesaugt und im Hochvakuum getrocknet. Man erhält 10 mg (19% d. Th.) der Titelverbindung.

$^1$H-NMR (400 MHz, DMSO-$d_6$): δ = 8.72 (s, 0.6H), 8.62 (s, 0.4H), 7.56-7.44 (m, 3H), 7.32-7.24 (m, 2H), 7.13-7.11 (m, 1H), 7.00-6.96 (m, 1H), 6.91 (s, 1H), 6.80 (d, 1H), 5.34 (s, 0.8H), 4.90 (s, 1.2H), 4.46 (s, 1.2H), 4.03 (t, 2H), 3.98 (s, 0.8H), 3.60 (t, 2H), 3.28 (s, 3H).

LC-MS (Methode 1): $R_t$ = 2.13 min; MS (ESIpos): m/z = 441 $[M+H]^+$.

**Beispiel 36**

1-({1-(3-Chlor-4-fluorphenyl)-5-[3-(2-hydroxyethoxy)phenyl]-1*H*-pyrazol-3-yl}carbonyl)imidazolidin-4-on

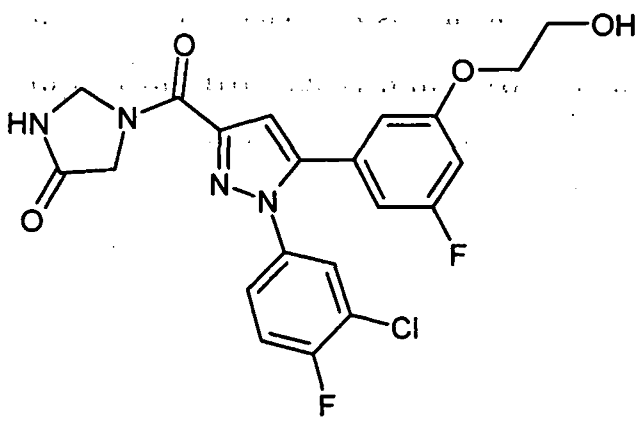

1.66 g (4.73 mmol) der Verbindung aus Beispiel 101A, 1.04 g (5.21 mmol) der Verbindung aus Beispiel 125A und 3.69 g (7.10 mmol) PyBOP werden in 80 ml Tetrahydrofuran vorgelegt und bei Raumtemperatur mit 1.73 ml (9.94 mmol) N,N-Diisopropylethylamin versetzt. Man rührt bei Raumtemperatur über Nacht, versetzt mit Wasser, entfernt das Tetrahydrofuran im Vakuum, extrahiert den Rückstand mit Essigsäurethylester, engt ein und reinigt den Rückstand per Flash-Chromatographie (Laufmittel: Essigsäureethylester/Methanol 200/1). Man erhält 1.24 g (63% d. Th.) 1-{[1-(3-Chlor-4-fluorphenyl)-5-(3-fluor-5-hydroxyphenyl)-1*H*-pyrazol-3-yl]carbonyl}imidazolidin-4-on.

LC-MS (Methode 7): $R_t$ = 1.59 min; MS (ESIpos): m/z = 419 $[M+H]^+$.

100 mg (0.239 mmol) 1-{[1-(3-Chlor-4-fluorphenyl)-5-(3-fluor-5-hydroxyphenyl)-1H-pyrazol-3-yl]carbonyl}imidazolidin-4-on werden in 2.5 ml trockenem Aceton vorgelegt, mit 36.3 mg (0.263 mmol) Kaliumcarbonat und 149 mg (1.19 mmol) 2-Bromethanol versetzt und über Nacht unter Rückfluss erhitzt. Anschließend wird das Reaktionsgemisch über die präparative HPLC (RP18-Säule; Laufmittel: Acetonitril/Wasser-Gradient) gereinigt. Man erhält 30 mg (26% d. Th.) der Titelverbindung.

$^1$H-NMR (400 MHz, DMSO-$d_6$): $\delta$ = 8.72 (s, 0.6H), 8.62 (s, 0.4H), 7.80 (dd, 1H), 7.53 (dt, 1H), 7.40-7.32 (m, 1H), 7.19-7.17 (m, 1H), 6.90 (dt, 1H), 6.75-6.68 (m, 2H), 5.32 (s, 0.8H), 4.92-4.85 (m, 2.2H), 4.44 (s, 1.2H), 4.01-3.92 (m, 2.8H), 3.65 (q, 2H).

LC-MS (Methode 7): $R_t$ = 1.53 min; MS (ESIpos): m/z = 463 $[M+H]^+$.

**Beispiel 37**

1-({1-(3-Chlorphenyl)-5-[3-(3-morpholin-1-ylpropoxy)phenyl]-1*H*-pyrazol-3-yl}carbonyl)imidazolidin-4-on

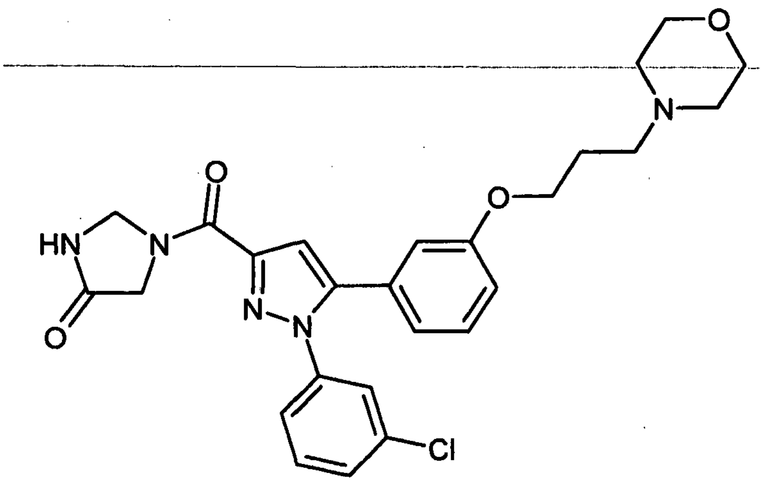

Die Herstellung der Titelverbindung erfolgt ausgehend von der Verbindung aus Beispiel 120A und Morpholin ohne Zusatz von Tetrahydrofuran analog zur Synthese der Verbindung aus Beispiel 29. Man erhält 40 mg (36% d. Th.) der Titelverbindung.

$^1$H-NMR (400 MHz, DMSO-$d_6$): δ = 8.72 (s, 0.6H), 8.62 (s, 0.4H), 7.56-7.44 (m, 3H), 7.32-7.25 (m, 2H), 7.13-7.11 (m, 1H), 6.96 (dd, 1H), 6.88-6.85 (m, 1H), 6.81 (d, 1H), 5.34 (s, 0.8H), 4.90 (s, 1.2H), 4.46 (s, 1.2H), 3.98 (s, 0.8H), 3.93 (t, 2H), 3.56 (t, 4H), 2.39-2.30 (m, 6H), 1.84-1.75 (m, 2H).

LC-MS (Methode 1): $R_t$ = 1.62 min; MS (ESIpos): m/z = 510 $[M+H]^+$.

**Beispiel 38**

1-{[1-(3-Chlor-4-fluorphenyl)-5-(3-methoxyphenyl)-1*H*-pyrazol-3-yl]carbonyl}imidazolidin-4-on

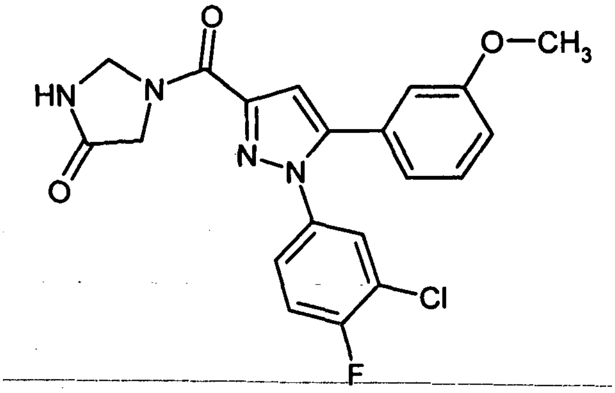

Die Herstellung der Titelverbindung erfolgt ausgehend von der Verbindung aus Beispiel 102A analog zur Synthese der Verbindung aus Beispiel 23. Man erhält 7 mg (12% d. Th.) der Titelverbindung.

$^1$H-NMR (400 MHz, DMSO-$d_6$): δ = 8.72 (s, 0.6H), 8.62 (s, 0.4H), 7.79-7.74 (m, 1H), 7.52 (dt, 1H), 7.38-7.27 (m, 2H), 7.14-7.11 (m, 1H), 7.00-6.95 (m, 1H), 6.93-6.90 (m, 1H), 6.80 (d, 1H), 5.34 (s, 0.8H), 4.90 (s, 1.2H), 4.45 (s, 1.2H), 3.98 (s, 0.8H), 3.71 (s, 3H).

LC-MS (Methode 7): $R_t$ = 1.77 min; MS (ESIpos): m/z = 415 $[M+H]^+$.

**Beispiel 39**

1-({1-(3-Chlorphenyl)-5-[3-(3-(methylamino)propoxy)phenyl]-1*H*-pyrazol-3-yl}carbonyl)imidazolidin-4-on-formiat

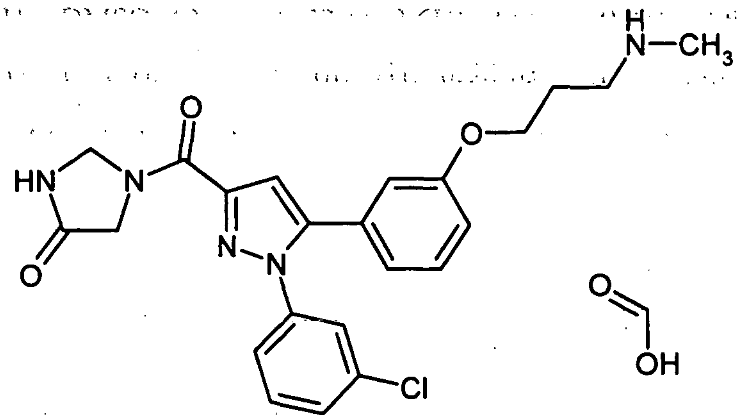

Die Herstellung der Titelverbindung erfolgt ausgehend von der Verbindung aus Beispiel 120A und Methylamin ohne Zusatz von Tetrahydrofuran analog zur Synthese der Verbindung aus Beispiel 29. Man erhält 46 mg (44% d. Th.) der Titelverbindung.

$^1$H-NMR (400 MHz, DMSO-$d_6$): $\delta$ = 8.73 (s, 0.6H), 8.63 (s, 0.4H), 8.28 (s, 1H), 7.56-7.44 (m, 3H), 7.32-7.25 (m, 2H), 7.13-7.11 (m, 1H), 7.00-6.94 (m, 1H), 6.92 (t, 1H), 6.81-6.75 (m, 1H), 5.35 (s, 0.8H), 4.90 (s, 1.2H), 4.46 (s, 1.2H), 4.02-3.96 (m, 2.8H), 2.78 (t, 2H), 2.42 (s, 3H), 1.94-1.85 (m, 2H).

LC-MS (Methode 1): $R_t$ = 1.62 min; MS (ESIpos): m/z = 454 $[M+H]^+$.

**Beispiel 40**

1-({1-(3-Chlorphenyl)-5-[3-(3-(ethylamino)propoxy)phenyl]-1*H*-pyrazol-3-yl}carbonyl)imidazolidin-4-on-formiat

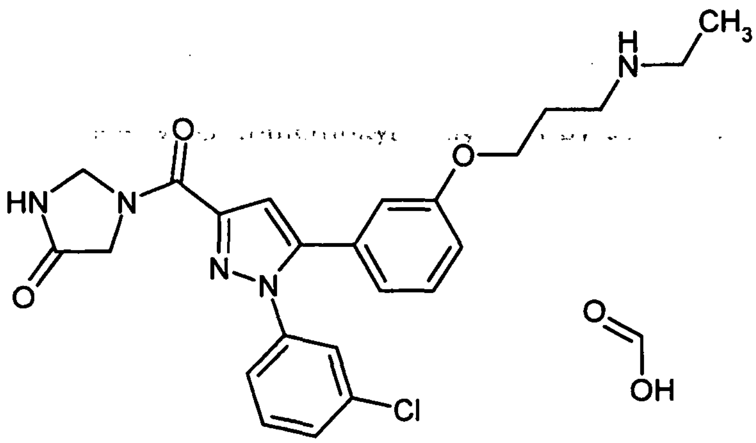

Die Herstellung der Titelverbindung erfolgt ausgehend von der Verbindung aus Beispiel 120A und Ethylamin ohne Zusatz von Tetrahydrofuran analog zur Synthese der Verbindung aus Beispiel 29. Man erhält 23 mg (21% d. Th.) der Titelverbindung.

$^1$H-NMR (400 MHz, DMSO-$d_6$): $\delta$ = 8.72 (s, 0.6H), 8.63 (s, 0.4H), 8.29 (s, 1H), 7.56-7.44 (m, 3H), 7.32-7.24 (m, 2H), 7.13-7.11 (d, 1H), 6.99-6.94 (m, 1H), 6.90 (t, 1H), 6.81-6.76 (m, 1H), 5.35 (s, 0.8H), 4.90 (s, 1.2H), 4.46 (s, 1.2H), 4.01-3.96 (m, 2.8H), 2.78-2.64 (m, 4H), 1.90-1.81 (m, 2H), 1.06 (t, 3H).

LC-MS (Methode 7): $R_t$ = 1.18 min; MS (ESIpos): m/z = 468 $[M+H]^+$.

**Beispiel 41**

1-({5-[3-(3-Azetidin-1-ylpropoxy)phenyl]-1-(3-chlorphenyl)-1*H*-pyrazol-3-yl}carbonyl)imidazolidin-4-on-formiat

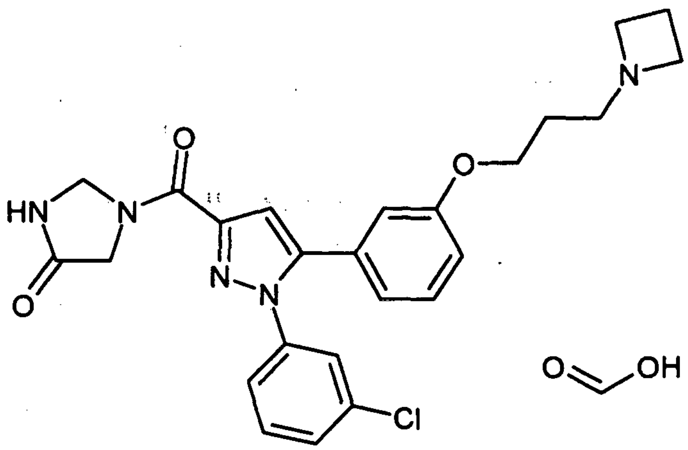

Die Herstellung der Titelverbindung erfolgt ausgehend von der Verbindung aus Beispiel 120A und Azetidin ohne Zusatz von Tetrahydrofuran analog zur Synthese der Verbindung aus Beispiel 29. Man erhält 13 mg (11% d. Th.) der Titelverbindung.

$^1$H-NMR (400 MHz, DMSO-$d_6$): δ = 8.72 (s, 0.6H), 8.62 (s, 0.4H), 8.18 (s, 1H), 7.56-7.44 (m, 3H), 7.31-7.24 (m, 2H), 7.13-7.11 (m, 1H), 6.97-6.92 (m, 1H), 6.88-6.85 (m, 1H), 6.82-6.78 (m, 1H), 5.34 (s, 0.8H), 4.90 (s, 1.2H), 4.46 (s, 1.2H), 3.98 (s, 0.8H), 3.90 (t, 2H), 3.12 (t, 4H), 2.48-2.43 (m, 2H), 2.01-1.92 (m, 2H), 1.67-1.59 (m, 2H).

LC MS (Methode 1): $R_t$ =1.44 min; MS (ESIpos): m/z = 480 [M+H]$^+$.

<u>**Beispiel 42**</u>

1-{[1-(3-Chlorphenyl)-5-{3-[2-(methylamino)ethoxy]phenyl}-1*H*-pyrazol-3-yl]carbonyl}imidazolidin-4-on-formiat

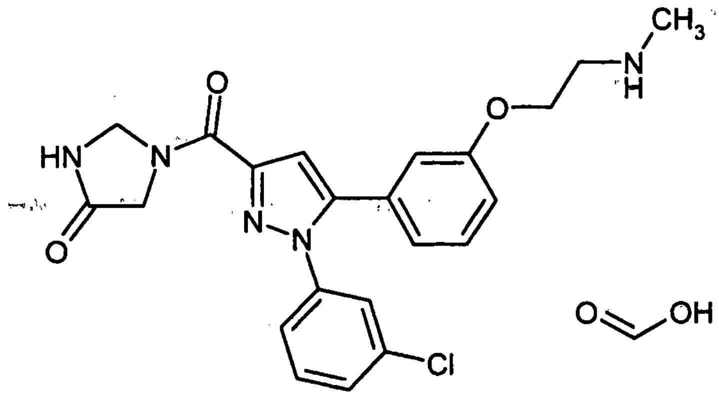

Die Herstellung der Titelverbindung erfolgt ausgehend von der Verbindung aus Beispiel 121A und Methylamin ohne Zusatz von Tetrahydrofuran analog zur Synthese der Verbindung aus Beispiel 29. Man erhält 26 mg (26% d. Th.) der Titelverbindung.

$^1$H-NMR (400 MHz, DMSO-$d_6$): δ = 8.73 (s, 0.6H), 8.63 (s, 0.4H), 8.24 (s, 1H), 7.56-7.43 (m, 3H), 7.33-7.25 (m, 2H), 7.13-7.11 (m, 1H), 7.02-6.97 (m, 1H), 6.94-6.91 (m, 1H), 6.86-6.78 (m, 1H), 5.35 (s, 0.8H), 4.91 (s, 1.2H), 4.46 (s, 1.2H), 4.02 (t, 2H), 3.98 (s, 0.8H), 3.19-3.16 (m, 2H), 2.92 (t, 2H), 2.40 (s, 3H).

LC-MS (Methode 1): $R_t$ = 1.52 min; MS (ESIpos): m/z = 440 [M+H]$^+$.

<u>**Beispiel 43**</u>

1-{[1-(3-Chlorphenyl)-5-{3-[3-(4-methylpiperazin-1-yl)propoxy]phenyl}-1*H*-pyrazol-3-yl]carbonyl}imidazolidin-4-on-formiat

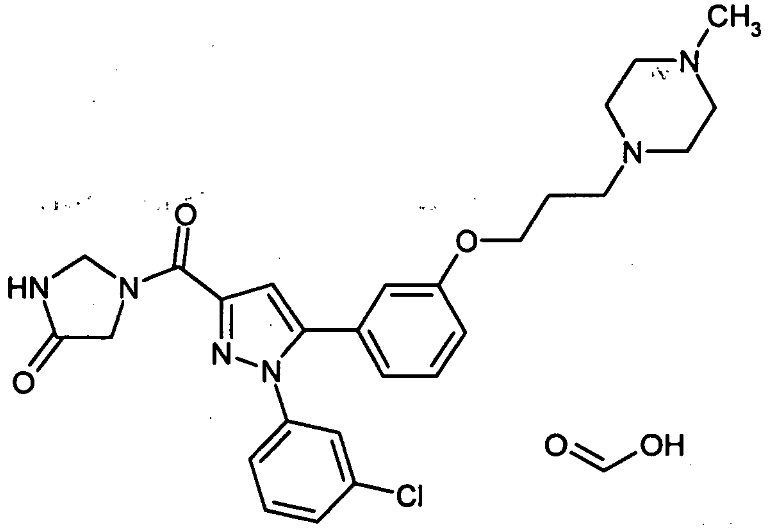

Die Herstellung der Titelverbindung erfolgt ausgehend von der Verbindung aus Beispiel 120A und 1-Methylpiperazin ohne Zusatz von Tetrahydrofuran analog zur Synthese der Verbindung aus Beispiel 29. Man erhält 45 mg (38% d. Th.) der Titelverbindung.

$^1$H-NMR (400 MHz, DMSO-$d_6$): δ = 8.72 (s, 0.6H), 8.62 (s, 0.4H), 8.16 (s, 1H), 7.56-7.43 (m, 3H), 7.31-7.25 (m, 2H), 7.13-7.11 (m, 1H), 6.98-6.93 (m, 1H), 6.88-6.85 (m, 1H), 6.80 (d, 1H), 5.34 (s, 0.8H), 4.90 (s, 1.2H), 4.45 (s, 1.2H), 3.98 (s, 0.8H), 3.92 (t, 2H), 2.39-2.30 (m, 10H), 2.17 (s, 3H), 1.82-1.74 (m, 2H).

LC-MS (Methode 1): $R_t$ = 1.40 min; MS (ESIpos): m/z = 523 $[M+H]^+$.

**Beispiel 44**

1-{[1-(3-Chlorphenyl)-5-{3-[2-(4-methylpiperazin-1-yl)ethoxy]phenyl}-1*H*-pyrazol-3-yl]carbonyl}imidazolidin-4-on-diformiat

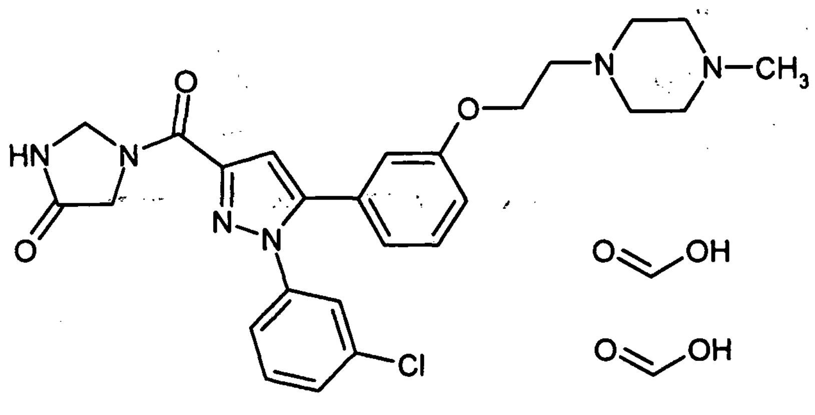

Die Herstellung der Titelverbindung erfolgt ausgehend von der Verbindung aus Beispiel 121A und 1-Methylpiperazin ohne Zusatz von Tetrahydrofuran analog zur Synthese der Verbindung aus Beispiel 29. Man erhält 46 mg (38% d. Th.) der Titelverbindung.

$^1$H-NMR (400 MHz, DMSO-$d_6$): δ = 8.72 (s, 0.6H), 8.62 (s, 0.4H), 8.15 (s, 2H), 7.56-7.44 (m, 3H), 7.31-7.25 (m, 2H), 7.14-7.12 (d, 1H), 6.99-6.95 (m, 1H), 6.90-6.87 (m, 1H), 6.83-6.79 (m, 1H), 5.34 (s, 0.8H), 4.90 (s, 1.2H), 4.46 (s, 1.2H), 4.02-3.97 (m, 2.8H), 2.63-2.58 (m, 2H), 2.48-2.30 (m, 8H), 2.18 (s, 3H).

LC-MS (Methode 1): $R_t$ = 1.39 min; MS (ESIpos): m/z = 509 $[M+H]^+$.

**Beispiel 45**

1-({1-(3-Chlorphenyl)-5-[3-(2-morpholin-4-ylethoxy)phenyl]-1*H*-pyrazol-3-yl}carbonyl)imidazolidin-4-on-formiat

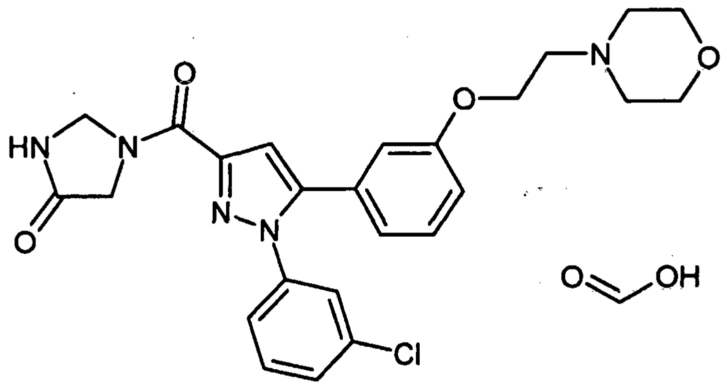

Die Herstellung der Titelverbindung erfolgt ausgehend von der Verbindung aus Beispiel 121A und Morpholin ohne Zusatz von Tetrahydrofuran analog zur Synthese der Verbindung aus Beispiel 29. Man erhält 47 mg (43% d. Th.) der Titelverbindung.

$^1$H-NMR (400 MHz, DMSO-$d_6$): $\delta$ = 8.72 (s, 0.6H), 8.62 (s, 0.4H), 8.14 (s, 1H), 7.56-7.44 (m, 3H), 7.32-7.25 (m, 2H), 7.14-7.12 (m, 1H), 6.99-6.95 (m, 1H), 6.90-6.87 (m, 1H), 6.81 (d, 1H), 5.34 (s, 0.8H), 4.90 (s, 1.2H), 4.46 (s, 1.2H), 4.01 (t, 2H), 3.98 (s, 0.8H), 3.56 (t, 4H), 2.61 (t, 2H), 2.42 (t, 4H).

LC-MS (Methode 1): $R_t$ = 1.58 min; MS (ESIpos): m/z = 496 $[M+H]^+$.

**Beispiel 46**

1-{[1-(3-Chlorphenyl)-5-(3-{2-[(1-methylethyl)amino]ethoxy}phenyl)-1*H*-pyrazol-3-yl]carbonyl}imidazolidin-4-on-formiat

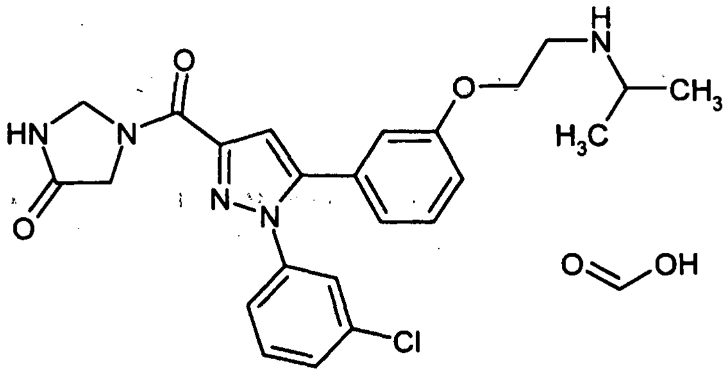

Die Herstellung der Titelverbindung erfolgt ausgehend von der Verbindung aus Beispiel 121A und Isopropylamin ohne Zusatz von Tetrahydrofuran analog zur Synthese der Verbindung aus Beispiel 29. Man erhält 29 mg (28% d. Th.) der Titelverbindung.

$^1$H-NMR (400 MHz, DMSO-$d_6$): $\delta$ = 8.72 (s, 0.6H), 8.63 (s, 0.4H), 8.20 (s, 1H), 7.56-7.44 (m, 3H), 7.33-7.25 (m, 2H), 7.13-7.11 (m, 1H), 7.02-6.97 (m, 1H), 6.94-6.91 (m, 1H), 6.83-6.78 (m, 1H), 5.35 (s, 0.8H), 4.90 (s, 1.2H), 4.46 (s, 1.2H), 4.03-3.97 (m, 2.8H), 2.95-2.85 (m, 3H), 1.03 (d, 6H).

LC-MS (Methode 1): $R_t$ = 1.42 min; MS (ESIpos): m/z = 468 $[M+H]^+$.

**Beispiel 47**

1-({5-[3,5-Bis(trifluormethyl)phenyl]-1-(3-chlorphenyl)-1*H*-pyrazol-3-yl}carbonyl)imidazolidin-4-on

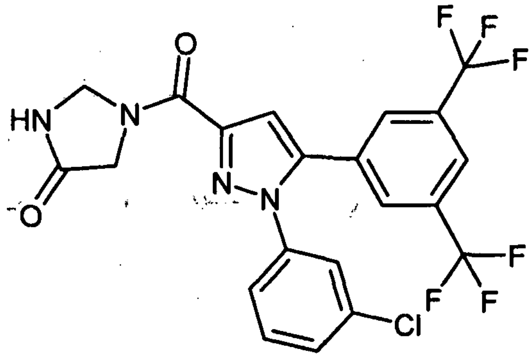

Die Herstellung der Titelverbindung erfolgt ausgehend von der Verbindung aus Beispiel 103A analog zur Synthese der Verbindung aus Beispiel 1. Man erhält 26 mg (72% d. Th.) der Titelverbindung.

$^1$H-NMR (400 MHz, DMSO-$d_6$): $\delta$ = 8.73 (s, 0.6H), 8.63 (s, 0.4H), 8.16 (s, 1H), 7.96 (s, 2H), 7.66-7.63 (m, 1H), 7.60-7.55 (m, 1H), 7.53-7.46 (m, 2H), 7.38-7.31 (m, 1H), 5.34 (s, 0.8H), 4.92 (s, 1.2H), 4.46 (s, 1.2H), 4.00 (s, 0.8H).

LC-MS (Methode 7): $R_t$ = 2.14 min; MS (ESIpos): m/z = 503 $[M+H]^+$.

**Beispiel 48**

1-({1-(4-Chlorphenyl)-5-[3-(trifluormethyl)phenyl]-1*H*-pyrazol-3-yl}carbonyl)imidazolidin-4-on

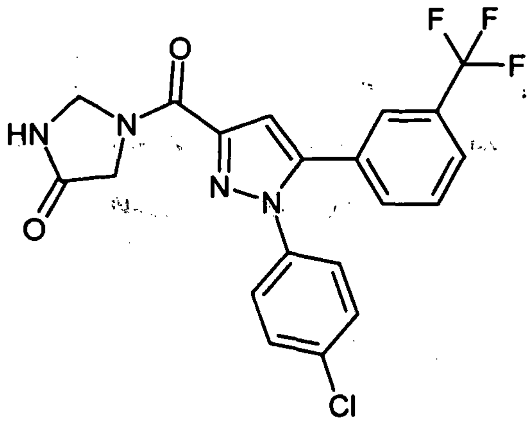

Die Herstellung der Titelverbindung erfolgt ausgehend von der Verbindung aus Beispiel 104A analog zur Synthese der Verbindung aus Beispiel 1. Man erhält 34 mg (92% d. Th.) der Titelverbindung.

$^1$H-NMR (400 MHz, DMSO-$d_6$): $\delta$ = 8.72 (s, 0.6H), 8.65 (s, 0.4H), 7.77 (d, 1H), 7.67-7.60 (m, 2H), 7.59-7.53 (m, 3H), 7.45-7.39 (m, 2H), 7.29-7.27 (m, 1H), 5.33 (s, 0.8 H), 4.91 (s, 1.2H), 4.44 (s, 1.2H), 3.99 (s, 0.8H).

LC-MS (Methode 7): $R_t$ = 1.95 min; MS (ESIpos): m/z = 435 $[M+H]^+$.

Beispiel 49

1-{[1-(4-Chlorphenyl)-5-(3-fluorphenyl)-1*H*-pyrazol-3-yl]carbonyl}imidazolidin-4-on

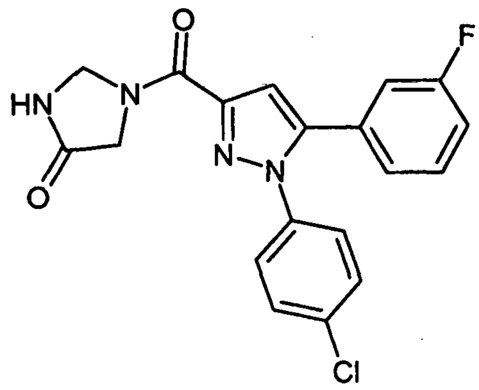

Die Herstellung der Titelverbindung erfolgt ausgehend von der Verbindung aus Beispiel 105A analog zur Synthese der Verbindung aus Beispiel 1, Man erhält 15 mg (39% d. Th.) der Titelverbindung.
$^1$H-NMR (400 MHz, DMSO-$d_6$): δ = 8.71 (s, 0.6H), 8.64 (s, 0.4H), 7.58-7.53 (m, 2H), 7.47-7.38 (m, 3H), 7.29-7.16 (m, 3H), 7.07 (d, 1H), 5.33 (s, 0.8H), 4.90 (s, 1.2H), 4.44 (s, 1.2H), 3.98 (s, 0.8H).
LC-MS (Methode 5): $R_t$ = 1.14 min; MS (ESIpos): m/z = 385 [M+H]$^+$.

**Beispiel 50**

1-{[1-(3-Chlor-4-fluorphenyl)-5-(3-fluor-5-methylphenyl)-1*H*-pyrazol-3-yl]carbonyl}imidazolidin-4-on

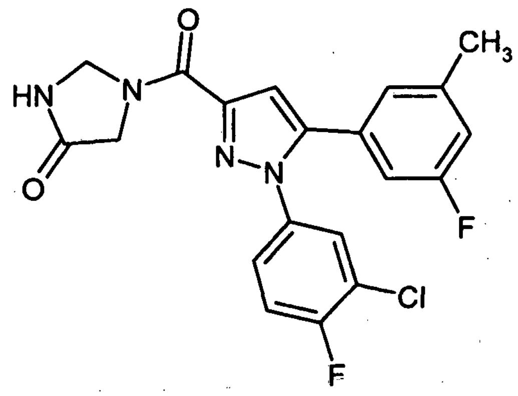

Die Herstellung der Titelverbindung erfolgt ausgehend von der Verbindung aus Beispiel 106A unter Verwendung von 3.2 Äquivalenten N,N-Diisopropylethylamin und unter Zusatz von 0.1% Ameisensäure bei der präparativen HPLC analog zur Synthese der Verbindung aus Beispiel 23. Man erhält 5 mg (6% d. Th.) der Titelverbindung.
$^1$H-NMR (400 MHz, DMSO-$d_6$): δ = 8.72 (s, 0.6H), 8.62 (s; 0.4H), 7.81-7.77 (m, 1H), 7.56-7.49 (m, 1H), 7.38-7.31 (m, 1H), 7.16-7.08 (m, 2H), 7.03-7.00 (m, 1H), 6.95-6.90 (m, 1H), 5.33 (s, 0.8H), 4.90 (s, 1.2H), 4.44 (s, 1.2H), 3.98 (s, 0.8H), 2.28 (s, 3H).
LC-MS (Methode 1): $R_t$ = 2.36 min; MS (ESIpos): m/z = 417 [M+H]$^+$.

**Beispiel 51**

1-{[1-(3-Chlorphenyl)-5-(3-fluor-5-methylphenyl)-1*H*-pyrazol-3-yl]carbonyl}imidazolidin-4-on

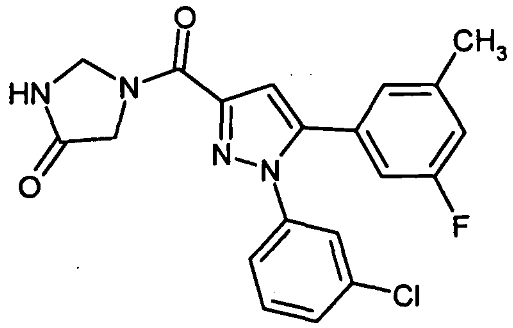

Die Herstellung der Titelverbindung erfolgt ausgehend von der Verbindung aus Beispiel 107A unter Verwendung von 3.2 Äquivalenten N,N-Diisopropylethylamin und unter Zusatz von 0.1% Ameisensäure bei der präparativen HPLC analog zur Synthese der Verbindung aus Beispiel 23. Man erhält 24 mg (19% d. Th.) der Titelverbindung.
$^1$H-NMR (400 MHz, DMSO-$d_6$): $\delta$ = 8.72 (s, 0.6H), 8.62 (s, 0.4H), 7.59-7.45 (m, 3H), 7.30-7.24 (m, 1H), 7.16-7.08 (m, 2H), 7.02 (s, 1H), 6.93-6.87 (m, 1H), 5.34 (s, 0.8H), 4.90 (s, 1.2H), 4.45 (s, 1.2H), 3.98 (s, 0.8H), 2.28 (s, 3H).
LC-MS (Methode 7): $R_t$ = 1.87 min; MS (ESIpos): m/z = 399 [M+H]$^+$.

**Beispiel 52**

1-{[5-(3-Chlor-4-fluorphenyl)-1-(3-cyanophenyl)-1*H*-pyrazol-3-yl]carbonyl}imidazolidin-4-on

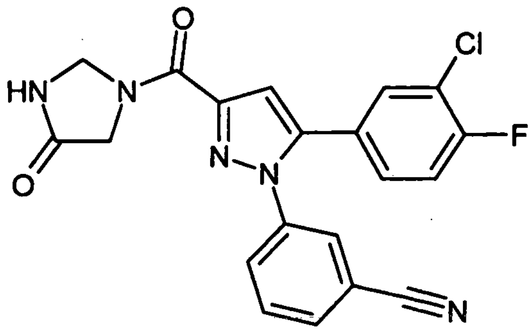

Die Herstellung der Titelverbindung erfolgt ausgehend von der Verbindung aus Beispiel 108A unter Zusatz von 0.1% Ameisensäure bei der präparativen HPLC analog zur Synthese der Verbindung aus Beispiel 23. Man erhält 8 mg (35% d. Th.) der Titelverbindung.
$^1$H-NMR (400 MHz, DMSO-$d_6$): $\delta$ = 8.72 (s, 0.6H), 8.64 (s, 0.4H), 8.08-8.05 (m, 1H), 7.98-7.94 (m, 1H), 7.70-7.65 (m, 2H), 7.53 (dt, 1H), 7.30-7.25 (m, 2H), 7.21-7.16 (m, 1H), 5.33 (s, 0.8H), 4.91 (s, 1.2H), 4.46 (s, 1.2H), 3.99 (s, 0.8H).
LC-MS (Methode 1): $R_t$ = 2.14 min; MS (ESIpos): m/z = 410 [M+H]$^+$.

**Beispiel 53**

1-{[5-(3-Chlorphenyl)-1-(3-cyano-4-fluorphenyl)-1*H*-pyrazol-3-yl]carbonyl}imidazolidin-4-on

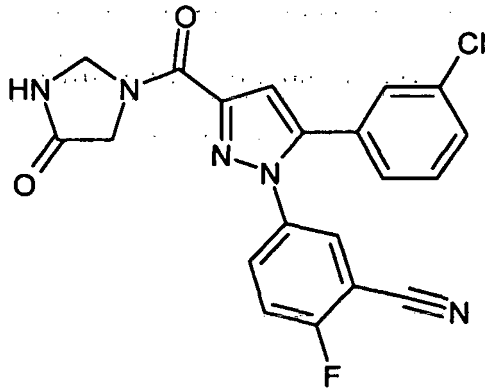

Die Herstellung der Titelverbindung erfolgt ausgehend von der Verbindung aus Beispiel 109A unter Verwendung von 3.2 Äquivalenten N,N-Diisopropylethylamin und unter Zusatz von 0.1% Ameisensäure bei der präparativen HPLC analog zur Synthese der Verbindung aus Beispiel 23. Man erhält 45 mg (77% d. Th.) der Titelverbindung.

$^1$H-NMR (400 MHz, DMSO-$d_6$): $\delta$ = 8.72 (s, 0.6H), 8.65 (s, 0.4H), 8.22-8.17 (m, 1H), 7.77-7.70 (m, 1H), 7.63 (t, 1H), 7.53-7.47 (m, 2H), 7.40 (t, 1H), 7.23-7.21 (m, 1H), 7.16 (d, 1H), 5.34 (s, 0.8H), 4.91 (s, 1.2H), 4.46 (s, 1.2H), 3.99 (s, 0.8H).

LC-MS (Methode 1): $R_t$ = 2.16 min; MS (ESIpos): m/z = 410 $[M+H]^+$.

**Beispiel 54**

1-({1-(3-Chlor-4-fluorphenyl)-5-[3-fluor-5-(2-pyrrolidin-1-ylethoxy)phenyl]-1*H*-pyrazol-3-yl}carbonyl)imidazolidin-4-on

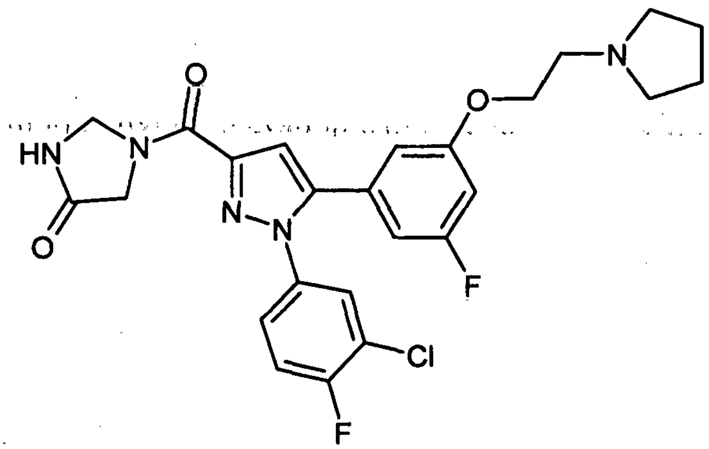

Die Herstellung der Titelverbindung erfolgt ausgehend von der Verbindung aus Beispiel 119A und Pyrrolidin ohne Zusatz von 0.1% Ameisensäure bei der präparativen HPLC analog zur Synthese der Verbindung aus Beispiel 29. Man erhält 2.5 mg (5% d. Th.) der Titelverbindung.

$^1$H-NMR (500 MHz, DMSO-$d_6$): $\delta$ = 8.74 (s, 0.6H), 8.65 (s, 0.4H), 7.84-7.78 (m, 1H), 7.59-7.51 (m, 1H), 7.41-7.33 (m, 1H), 7.20 (s, 1H), 6.90 (d, 1H), 6.77 (d, 1H), 6.67 (s, 1H), 5.32 (s, 0.8H), 4.90 (s, 1.2H), 4.44 (s, 1.2H), 4.05-3.95 (m, 2.8H), 2.71-2.63 (m, 2H), 2.44 (s, 4H), 1.66 (s, 4H).

LC-MS (Methode 1): $R_t$ = 1.52 min; MS (ESIpos): m/z = 516 $[M+H]^+$.

**Beispiel 55**

1-({5-[3-(3-Aminopropoxy)phenyl]-1-(3-chlorphenyl)-1*H*-pyrazol-3-yl}carbonyl)imidazolidin-4-on-formiat

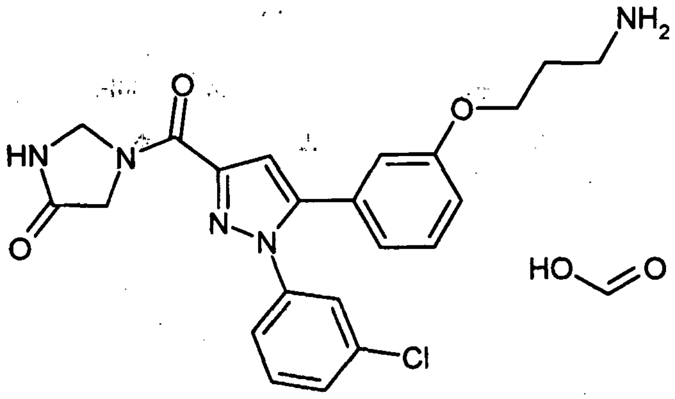

Die Herstellung der Titelverbindung erfolgt ausgehend von der Verbindung aus Beispiel 120A und einer 7N Lösung von Ammoniak in Methanol unter Zusatz von 0.1% Ameisensäure bei der präparativen HPLC analog zur Synthese der Verbindung aus Beispieil 29. Man erhält 10 mg (10% d. Th.) der Titelverbindung.

$^1$H-NMR (400 MHz, DMSO-$d_6$): $\delta$ = 8.72 (s, 0.6H), 8.63 (s, 0.4H), 8.36 (s, 1H), 7.56-7.44 (m, 3H), 7.32-7.24 (m, 2H), 7.12-7.10 (m, 1H), 7.00-6.92 (m, 2H), 6.80-6.75 (m, 1H), 5.35 (s, 0.8H), 4.90 (s, 1.2H), 4.46 (s, 1.2H), 4.04-3.97 (m, 2.8H), 2.81 (t, 2H), 1.92-1.83 (m, 2H).

LC-MS (Methode 1): $R_t$ = 1.41 min; MS (ESIpos): m/z = 440 $[M+H]^+$.

**Beispiel 56**

3-{5-(3-Chlorphenyl)-3-[(4-oxoimidazolidin-1-yl)carbonyl]-1*H*-pyrazol-1-yl}benzolcarbonitril

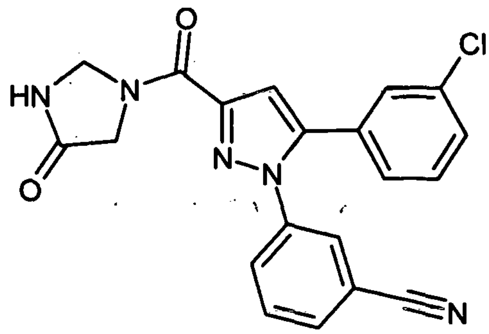

Die Herstellung der Titelverbindung erfolgt ausgehend von der Verbindung aus Beispiel 110A unter Verwendung von 3.2 Äquivalenten N,N-Diisopropylethylamin und unter Zusatz von 0.1% Ameisensäure bei der präparativen HPLC analog zur Synthese der Verbindung aus Beispiel 23. Zur Abtrennung von 1,1',1"-Phosphoryltripyrrolidin wird in wenig Tetrahydrofuran aufgenommen, mit Wasser versetzt bis sich ein Niederschlag bildet, das Tetrahydrofuran im Vakuum entfernt, der Niederschlag abgesaugt und im Hochvakuum getrocknet. Man erhält 42 mg (65% d. Th.) der Titelverbindung.

$^1$H-NMR (400 MHz, DMSO-$d_6$): $\delta$ = 8.72 (s, 0.6H), 8.64 (s, 0.4H), 8.05-8.02 (m, 1H), 7.96-7.92 (m, 1H), 7.68-7.63 (m, 2H), 7.52-7.45 (m, 2H), 7.41 (t, 1H), 7.23-7.21 (m, 1H), 7.18 (dt, 1H), 5.35 (s, 0.8H), 4.91 (s, 1.2H), 4.47 (s, 1.2H), 3.99 (s, 0.8H).

LC-MS (Methode 1): $R_t$ = 2.10 min; MS (ESIpos): m/z = 392 $[M+H]^+$.

**Beispiel 57**

3,3'-{3-[(4-Oxoimidazolidin-1-yl)carbonyl]-1*H*-pyrazol-1,5-diyl}dibenzolcarbonitril

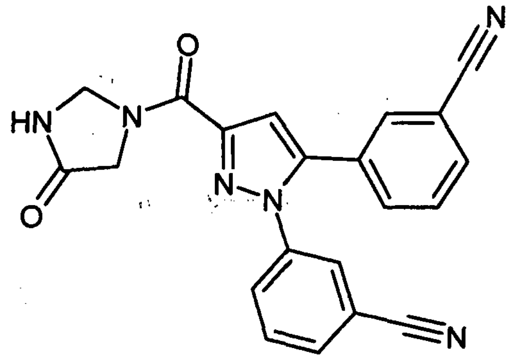

Die Herstellung der Titelverbindung erfolgt ausgehend von der Verbindung aus Beispiel 111A unter Verwendung von 3.2 Äquivalenten N,N-Diisopropylethylamin und unter Zusatz von 0.1% Ameisensäure bei der präparativen HPLC analog zur Synthese der Verbindung aus Beispiel 23. Man erhält 16 mg (29% d. Th.) der Titelverbindung.

$^1$H-NMR (400 MHz, DMSO-$d_6$): $\delta$ = 8.73 (s, 0.6H), 8.65 (s, 0.4H), 8.06-8.03 (m, 1H), 7.97-7.87 (m, 3H), 7.67-7.63 (m, 2H), 7.58 (t, 1H), 7.52 (dt, 1H), 7.29-7.27 (m, 1H), 5.36 (s, 0.8H), 4.91 (s, 1.2H), 4.47 (s, 1.2H), 3.99 (s, 0.8H).

LC-MS (Methode 5): $R_t$ = 0.91 min; MS (ESIpos): m/z = 383 [M+H]$^+$.

**Beispiel 58**

5-{5-(3-Cyanphenyl)-3-[(4-oxoimidazolidin-1-yl)carbonyl]-1*H*-pyrazol-1-yl}-2-fluorbenzolcarbonitril

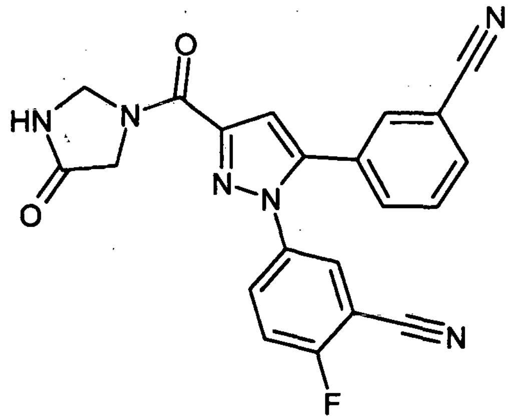

Die Herstellung der Titelverbindung erfolgt ausgehend von der Verbindung aus Beispiel 112A unter Verwendung von 3.2 Äquivalenten N,N-Diisopropylethylamin und unter Zusatz von 0.1% Ameisensäure bei der präparativen HPLC analog zur Synthese der Verbindung aus Beispiel 23. Zur Abtrennung von 1,1',1"-Phosphoryltripyrrolidin wird in wenig Tetrahydrofuran aufgenommen, mit Wasser versetzt bis sich ein Niederschlag bildet, das Tetrahydrofuran im Vakuum entfernt, der Niederschlag abgesaugt und im Hochvakuum getrocknet. Man erhält 25 mg (48% d. Th.) der Titelverbindung.

$^1$H-NMR (400 MHz, DMSO-$d_6$): $\delta$ = 8.73 (s, 0.6H), 8.66 (s, 0.4H), 8.23-8.18 (m, 1H), 7.97-7.94 (m, 1H), 7.89 (dt, 1H), 7.76-7.70 (m, 1H), 7.66-7.50 (m, 3H), 7.29-7.27 (m, 1H), 5.34 (s, 0.8H), 4.91 (s, 1.2H), 4.46 (s, 1.2H), 3.99 (s, 0.8H).

LC-MS (Methode 1): $R_t$ = 1.93 min; MS (ESIpos): m/z = 401 [M+H]$^+$.

**Beispiel 59**

3-{5-(3-Chlor-5-fluorphenyl)-3-[(4-oxoimidazolidin-1-yl)carbonyl]-1*H*-pyrazol-1-yl}benzolcarbonitril

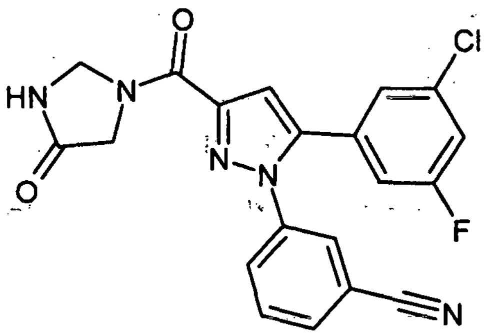

Die Herstellung der Titelverbindung erfolgt ausgehend von der Verbindung aus Beispiel 113A unter Verwendung von 3.2 Äquivalenten N,N-Diisopropylethylamin und unter Zusatz von 0.1% Ameisensäure bei der präparativen HPLC analog zur Synthese der Verbindung aus Beispiel 23. Man erhält 58 mg (77% d. Th.) der Titelverbindung.

$^1$H-NMR (400 MHz, DMSO-$d_6$): $\delta$ = 8.72 (s, 0.6H), 8.64 (s, 0.4H), 8.08-8.04 (m, 1H), 7.98-7.94 (m, 1H), 7.70-7.65 (m, 2H), 7.54 (dt, 1H), 7.30-7.28 (m, 1H), 7.28-7.25 (m, 1H), 7.21-7.16 (m, 1H), 5.34 (s, 0.8H), 4.91 (s, 1.2H), 4.46 (s, 1.2H), 3.99 (s, 0.8H).

LC-MS (Methode 5): $R_t$ = 1.10 min; MS (ESIpos): m/z = 410 [M+H]$^+$.

**Beispiel 60**

5-{5-(3-Chlor-5-fluorphenyl)-3-[(4-oxoimidazolidin-1-yl)carbonyl]-1*H*-pyrazol-1-yl}-2-fluorbenzolcarbonitril

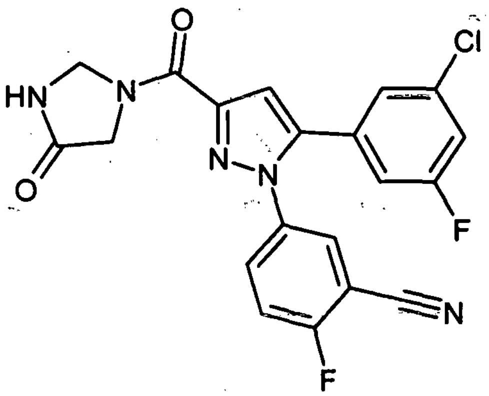

Die Herstellung der Titelverbindung erfolgt ausgehend von der Verbindung aus Beispiel 114A unter Verwendung von 3.2 Äquivalenten N,N-Diisopropylethylamin und unter Zusatz von 0.1% Ameisensäure bei der präparativen HPLC analog zur Synthese der Verbindung aus Beispiel 23. Man erhält 44 mg (48% d. Th.) der Titelverbindung.

$^1$H-NMR (400 MHz, DMSO-$d_6$): $\delta$ = 8.73 (s, 0.6H), 8.65 (s, 0.4H), 8.24-8.19 (m, 1H), 7.79-7.72 (m, 1H), 7.65 (t, 1H), 7.54 (dt, 1H), 7.32-7.29 (m, 1H), 7.29-7.27 (m, 1H), 7.22-7.17 (m, 1H), 5.33 (s, 0.8H), 4.91 (s, 1.2H), 4.45 (s, 1.2H), 3.99 (s, 0.8H).

LC-MS (Methode 5): $R_t$ = 1.14 min; MS (ESIpos): m/z = 428 [M+H]$^+$.

**Beispiel 61**

3-{1-(3-Chlorphenyl)-3-[(4-oxoimidazolidin-1-yl)carbonyl]-1*H*-pyrazol-5-yl}-5-fluorbenzolcarbonitril

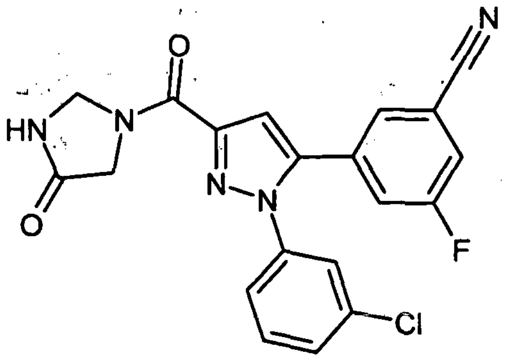

80.0 mg (0.190 mmol) mit 81%iger Reinheit der Verbindung aus Beispiel 131A, 49.5 mg (0.247 mmol) der Verbindung aus Beispiel 125A und 175 mg (0.337 mmol) PyBOP werden in 3.0 ml Tetrahydrofuran vorgelegt und bei Raumtemperatur mit 125 µl (0.179 mmol) N,N-Diisopropylethylamin versetzt. Man rührt bei Raumtemperatur über Nacht, verdünnt die Reaktionslösung mit Dichlormethan und wäscht anschließend mit 1 N wässriger Hydrogenchlorid-Lösung. Nach Trocknung der organischen Phase über Magnesiumsulfat, Filtration und Einengen i. Vak. wird das Rohprodukt über präparative HPLC (RP18-Säule; Laufmittel: Acetonitril/Wasser-Gradient) gereinigt. Man erhält 56.0 mg (72% d. Th.) der Titelverbindung.

$^1$H-NMR (400 MHz, DMSO-$d_6$): δ = 8.73 (br. s., 0.6H), 8.62 (s, 0.4H), 7.94 (d, 1H), 7.71 (br. s., 1H), 7.66-7.44 (m, 4H), 7.37-7.23 (m, 2H), 5.33 (s, 0.8H), 4.91 (s, 1.2H), 4.45 (s, 1.2H), 3.99 (s, 0.8H).

LC-MS (Methode 5): $R_t$ = 1.09 min; MS (ESIpos): m/z = 410 $[M+H]^+$.

**Beispiel 62**

3-{1-(3-Chlor-4-fluorphenyl)-3-[(4-oxoimidazolidin-1-yl)carbonyl]-1*H*-pyrazol-5-yl}-5-fluorbenzolcarbonitril

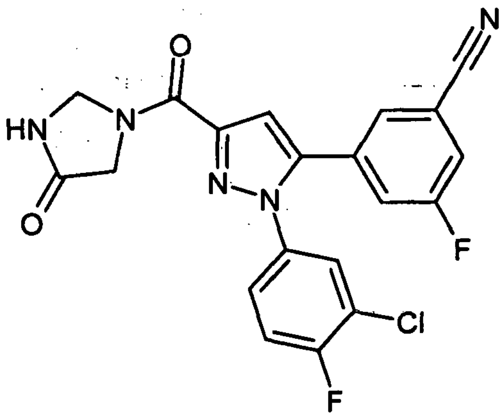

100 mg (0.263 mmol) der Verbindung aus Beispiel 129A, 57.8 mg (0.289 mmol) der Verbindung aus Beispiel 125A und 205 mg (0.394 mmol) PyBOP werden in 5.0 ml Tetrahydrofuran vorgelegt und bei Raumtemperatur mit 146 µl (0.841 mmol) *N,N*-Diisopropylethylamin versetzt. Man rührt bei Raumtemperatur über Nacht, verdünnt die Reaktionslösung mit Dichlormethan und wäscht anschließend mit 1 N wässriger Hydrogenchlorid-Lösung. Nach Trocknung der organischen Phase über Magnesiumsulfat, Filtration und Einengen i. Vak. wird das Rohprodukt über präparative HPLC (RP18-Säule; Laufmittel: Acetonitril/Wasser-Gradient) gereinigt. Man erhält 78.0 mg (66% d. Th.) der Titelverbindung.

$^1$H-NMR (400 MHz, DMSO-$d_6$): δ = 8.73 (s, 0.6H), 8.63 (s, 0.4H), 7.94 (d, 1H), 7.84 (d, 1H), 7.73 (d, 1H), 7.61-7.48 (m, 2H), 7.43-7.25 (m, 2H), 5.33 (s, 0.8H), 4.91 (s, 1.2H), 4.44 (s, 1.2H), 3.99 (s, 0.8H).

LC-MS (Methode 1): $R_t$ = 2.09 min; MS (ESIpos): m/z = 428 $[M+H]^+$.

**Beispiel 63**

5-{5-(3-Cyan-5-fluorphenyl)-3-[(4-oxoimidazolidin-1-yl)carbonyl]-1*H*-pyrazol-1-yl}-2-fluorbenzolcarbonitril

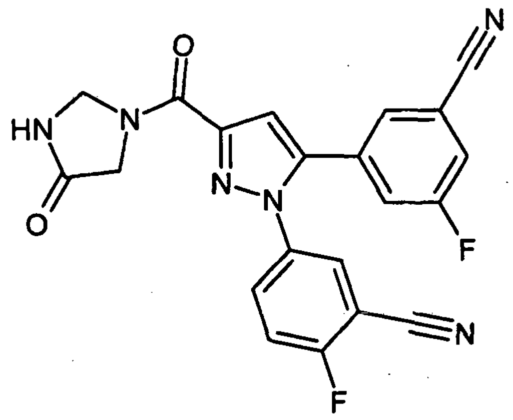

80.0 mg (0.228 mmol) der Verbindung aus Beispiel 133A, 50.3 mg (0.251 mmol) der Verbindung aus Beispiel 125A und 178 mg (0.394 mmol) PyBOP werden in 5.0 ml Tetrahydrofuran vorgelegt und bei Raumtemperatur mit 127 µl (0.731 mmol) N,N-Diisopropylethylamin versetzt. Man rührt bei Raumtemperatur über Nacht, verdünnt die Reaktionslösung mit Dichlormethan und wäscht anschließend mit 1 N wässriger Hydrogenchlorid-Lösung. Nach Trocknung der organischen Phase über Magnesiumsulfat, Filtration und Einengen i. Vak. wird das Rohprodukt über präparative HPLC (RP18-Säule; Laufmittel: Acetonitril/Wasser-Gradient) gereinigt. Der erhaltene Feststoff wird in Acetonitril/Diethylether suspendiert, 30 min gerührt und anschließend filtriert. Man erhält 20.0 mg (21% d. Th.) der Titelverbindung.

$^1$H-NMR (400 MHz, DMSO-$d_6$): $\delta$ = 8.73 (s, 0.6H), 8.65 (s, 0.4H), 8.27-8.13 (m, 1H), 7.95 (d, 1H), 7.74 (d, 2H), 7.68-7.52 (m, 2H), 7.33 (s, 1H), 5.34 (s, 0.8H), 4.91 (s, 1.2H), 4.46 (s, 1.2H), 3.99 (s, 0.8H).

LC-MS (Methode 5): $R_t$ = 1.00 min; MS (ESIpos): m/z = 419 [M+H]$^+$.

**Beispiel 64**

3-{1-(3-Cyanphenyl)-3-[(4-oxoimidazolidin-1-yl)carbonyl]-1*H*-pyrazol-5-yl}-5-fluorbenzolcarbonitril

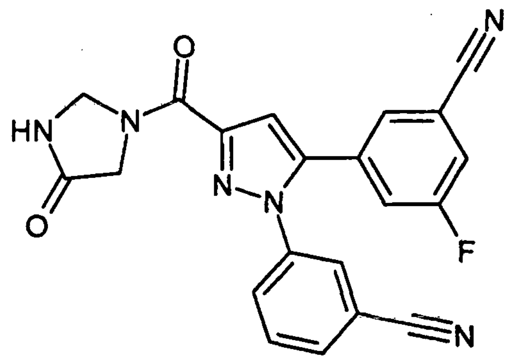

100 mg (0.301 mmol) der Verbindung aus Beispiel 135A, 66.2 mg (0.331 mmol) der Verbindung aus Beispiel 125A und 235 mg (0.451 mmol) PyBOP werden in 5.0 ml Tetrahydrofuran vorgelegt und bei Raumtemperatur mit 168 µl (0.936 mmol) *N,N*-Diisopropylethylamin versetzt. Man rührt bei Raumtemperatur über Nacht, verdünnt die Reaktionslösung mit Dichlormethan und wäscht anschließend mit 1 N wässriger Hydrogenchlorid-Lösung. Nach Trocknung der organischen Phase über Magnesiumsulfat, Filtration und Einengen i. Vak. wird das Rohprodukt über präparative HPLC (RP18-Säule; Laufmittel: Acetonitril/Wasser-Gradient) gereinigt. Der erhaltene Feststoff wird in Acetonitril/Diethylether suspendiert, 30 min gerührt und anschließend filtriert. Man erhält 18.0 mg (15% d. Th.) der Titelverbindung.

$^1$H-NMR (400 MHz, DMSO-$d_6$): $\delta$ = 8.73 (s, 0.6H), 8.64, (s, 0.4H), 8.07 (d, 1H), 8.00-7.90 (m, 2H), 7.76-7.70 (m, 1H), 7.69-7.62 (m, 2H), 7.56 (dd, 1H), 7.37-7.31 (m, 1H), 5.35 (s, 0.8 H), 4.91 (s, 1.2H), 4.46 (s, 1.2H), 3.99 (s, 0.8H).
LC-MS (Methode 5): $R_t$ = 0.97 min; MS (ESIpos): m/z = 401 $[M+H]^+$.

B) Bewertung der physiologischen Wirksamkeit

**Abkürzungen:**

| | |
|---|---|
| DMSO | Dimethylsulfoxid |
| FKS | Fötales Kälber Serum (Biochrom AG, Berlin, Deutschland) |
| PBS | Phosphate buffered saline |
| MTP | Mikrotiterplatte |
| ELISA | Enzyme-linked immunosorbent assay |

Die Eignung der erfindungsgemäßen Verbindungen zur Behandlung von durch Retroviren hervorgerufenen Erkrankungen kann in folgenden Assay-Systemen gezeigt werden:

**In vitro Assays**

**Biochemischer Reverse Transkriptase Assay**

Es wird der "Reverse Transcriptase Assay, colorimetric" (Roche Diagnostics GmbH, Mannheim, Deutschland) entsprechend den Herstellerangaben verwendet. Die Prüfsubstanzen werden in DMSO gelöst und in 5er Schritten verdünnt in dem Test eingesetzt (DMSO Endkonzentration 1%). Die resultierenden Werte der photometrischen Auswertung (405/492nm) sind bei der Negativkontrolle (Ansatz ohne Reverse Transkriptase) kleiner 0,1 und liegen bei der Positivkontrolle (Ansatz ohne Prüfsubstanz) im Bereich von 1,5. Die $IC_{50}$-Werte der Prüfsubstanzen werden als die Konzentration der Prüfsubstanzverdünnung ermittelt, bei der die gemessene optische Dichte 50% der Positivkontrolle beträgt.
Es wird gefunden, dass die erfindungsgemäßen Verbindungen die Reverse Transkriptase Aktivität hemmen. Experimentelle Daten sind in Tabelle A zusammengefasst.

**Light Assay mit wildtyp und Inhibitor-resistenten HI-Reporterviren**

Für diesen Assay werden HIV-$1_{NL4-3}$ Reporterviren verwendet, die anstelle des *nef* Gens das *lu164* Gen (Luziferase 164) tragen. Die Viren werden durch Transfektion von 293T-Zellen mit dem entsprechenden proviralen pNL4-3 Plasmid generiert (Lipofectamine Reagent, Invitrogen, Karlsruhe, Deutschland). Ausgehend von der proviralen Plasmid-DNA werden mit dem "QuikChange II XL Site-Directed Mutagenesis Kit" (Stratagene, Cedar Creek, Texas, USA) Viren mit definierten Resistenzmutationen im Reverse Transkriptase Gen hergestellt. Es werden u.a. folgende Mutationen generiert: A98G, A98G-K103N-V108I, A98S, F227C, F227L, G190A, G190S, K101E, K101Q-K103N, K103N, K103N-F227L, K103N-G190A, K103N-G190S, K103N-M230L, K103N-N348I, K103N-P225H, K103N-V108I, K103N-V108I-P225H, K103N-V179F-Y181C, K103N-Y181C, K103N-Y181C-G190A, L100I, L100I-K103N, L100I-K103N-V179I-Y181C, L100I-K103N-Y181C, L234I, N348I, P225H, P236L, V106A, V106A-E138K, V106A-F227C, V106A-F227L, V106I, V106I-Y188L, V106M, V108I, V179F-Y181C, V179I, V179I-Y181C, Y181C, Y181C-G190A, Y181C-M230L, Y181I, Y188L. Mit diesen Reporterviren infizierte MT4 7F2 Zellen sekretieren Luziferase ins Medium, was die luminometrische Quantifizierung der Virusreplikation ermöglicht.
Für den Ansatz einer 96-well MTP werden 3 Millionen MT4 7F2 Zellen pelletiert, in 1 ml RPMI 1640 Medium ohne Phenolrot (Invitrogen, Karlsruhe, Deutschland) / 10% FKS / 10% AIM-V (Invitrogen, Karlsruhe, Deutschland) suspendiert und zusammen mit einer geeigneten Menge des entsprechenden HIV-$1_{NL4-3}$ Reportervirus für 2 Stunden bei 37°C inkubiert (Pelletinfektion). Nicht adsorbierte Viren werden anschließend mit PBS ausgewaschen, die infizierten Zellen wieder pelletiert und in 8 ml RPMI 1640 Medium ohne Phenolrot / 2% oder 10% FKS / 10% AIM-V suspendiert. Davon werden 80$\mu$l pro Well in eine weiße 96-well MTP zu 20$\mu$l Prüfsubstanz in geeigneter Verdünnung pipettiert. Um Randeffekte zu vermeiden werden die Randwells der MTP nicht für Substanzverdünnungen verwendet. Die zweite vertikale Reihe der MTP enthält nur infizierte Zellen (Viruskontrolle) und die elfte vertikale Reihe nur nicht infizierte Zellen (Zellkontrolle) jeweils in RPMI 1640 Medium ohne Phenolrot / 2% oder 10% FKS / 10% AIM-V. Die übrigen Wells der MTP enthalten die erfindungsgemäßen Verbindungen in unterschiedlichen Konzentrationen ausgehend von der dritten

vertikalen Reihe, von der aus die Prüfsubstanzen in 3er Schritten bis zur zehnten vertikalen Reihe $3^7$-fach verdünnt werden. Die Prüfsubstanzen sind in DMSO gelöst, wobei die DMSO Endkonzentration im Testansatz schließlich 1% beträgt. Die Testansätze werden 5 Tage bei 37°C / 5% $CO_2$ inkubiert und nach Zugabe von 15µl Lu164-Substrat (5mg/ml Coelenterazin gelöst in 30µM Glutathion/DMSO, 100mM NaCl, 1M MES, 100mM Glutathion) luminometrisch ausgewertet. Die resultierenden Werte liegen bei der Viruskontrolle im Bereich von 1.000.000 RLUs (relative Lichteinheiten) und bei der Zellkontrolle bei 300 bis 400 RLUs. Die $EC_{50}$-Werte der Prüfsubstanzen werden als die Konzentration ermittelt, bei der die in RLUs gemessene Virusreplikation 50% der unbehandelten infizierten Zellen beträgt.

Es wird gefunden, dass die erfindungsgemäßen Verbindungen die HIV-Replikation hemmen. Experimentelle Daten sind in Tabelle A zusammengefasst.

**PBL- und H9-Assay mit wildtyp HIV-1**

Primäre menschliche Blutlymphozyten (PBLs) werden über Ficoll-Paque Leucosep Röhrchen (Greiner Bio-One, Frickenhausen, Deutschland) aus Blut isoliert und in RPMI 1640 Medium (Invitrogen, Karlsruhe, Deutschland) / 10% FKS mit Phytohaemagglutinin (90µg/ml) und Interleukin-2 (40U/ml) 3 Tage stimuliert.

Für den Ansatz einer 96-well MTP werden 3 Millionen PBLs pelletiert, in 1ml RPMI 1640 Medium / 10% FKS suspendiert und zusammen mit einer geeigneten Menge HIV-$1_{LAI}$ (NIH AIDS Research & Reference Reagent Program, Germantown, USA) für 2 Stunden bei 37°C inkubiert (Pelletinfektion). Nicht adsorbierte Viren werden anschließend mit PBS ausgewaschen, die infizierten Zellen wieder pelletiert und in 18ml RPMI 1640 Medium / 10% FKS / Interleukin-2 (40U/ml) suspendiert. Davon werden 180µl pro well in eine weiße 96-well MTP zu 20µl Prüfsubstanz in geeigneter Verdünnung pipettiert. Alternativ wird das HIV nach Zubereitung der Substanzverdünnungen in der MTP zusammen mit den Zellen zupipettiert und wird nicht mehr ausgewaschen (Überstandsinfektion). Um Randeffekte zu vermeiden werden die Randwells der MTP nicht für Substanzverdünnungen verwendet. Die zweite vertikale Reihe der MTP,enthält nur infizierte Zellen (Viruskontrolle) und die elfte vertikale Reihe nur nicht infizierte Zellen (Zellkontrolle) jeweils in RPMI 1640 Medium / 10% FKS / Interleukin-2 (40U/ml). Die übrigen Wells der MTP enthalten die erfindungsgemäßen Verbindungen in unterschiedlichen Konzentrationen ausgehend von der dritten vertikalen Reihe, von der aus die Prüfsubstanzen in 3er Schritten bis zur zehnten vertikalen Reihe $3^7$-fach verdünnt werden. Die Prüfsubstanzen sind in DMSO gelöst, wobei die DMSO Endkonzentration im Testansatz schließlich 1% beträgt. Die Testansätze werden bei 37°C / 5% $CO_2$ inkubiert. Nach 5 und 7 Tagen erfolgt die Abnahme von jeweils 50µl zellfreiem Überstand aus jedem Well zur Bestimmung der enthaltenen p24 Menge mittels p24 ELISA (HIV-1 $p24^{CA}$ Antigen Capture Assay Kit, NCI-Frederick Cancer Research and Development Center, Frederick, USA). Aus den resultierenden Werten der photometrischen Auswertung (450/620nm) werden die $EC_{50}$-Werte der Prüfsubstanzen als die Konzentration ermittelt, bei der die p24 Menge 50% der unbehandelten infizierten Zellen beträgt.

Alternativ werden H9-Zellen (ATCC, Wesel, Deutschland) anstelle von PBLs zur Testung der Prüfsubstanzen eingesetzt. H9-Zellen werden nach oben beschriebenem Muster in RPMI 1640 Medium mit 2% oder 10% FKS als HIV-$1_{LAI}$ Überstandsinfektion 5 Tage bei 37°C / 5% $CO_2$ inkubiert (20µl Substanzverdünnung und 80µl Zellen/Virus pro Well). Anschließend wird je Well 10µl AlamarBlue (Invitrogen, Karlsruhe, Deutschland) zugegeben und die MTPs für 3 Stunden bei 37°C inkubiert, bevor die fluorimetrische Auswertung erfolgt (544/590nm). Die resultierenden Werte liegen bei den unbehandelten nicht infizierten Zellen bei etwa 40.000 und bei den unbehandelten infizierten Zellen bei etwa 7.000. Im niedrigen Konzentrationsbereich werden die $EC_{50}$-Werte der Prüfsubstanzen als die Konzentration ermittelt, bei der die Fluoreszenz 50% der unbehandelten nicht infizierten Zellen beträgt (jeweils abzüglich der Werte der unbehandelten infizierten Zellen). Außerdem werden im hohen Konzentrationsbereich die $CC_{50}$-Werte der Prüfsubstanzen als die Konzentration ermittelt, bei der die Fluoreszenz 50% der unbehandelten nicht infizierten Zellen beträgt (jeweils abzüglich der Werte der unbehandelten infizierten Zellen).

Es wird gefunden, dass die erfindungsgemäßen Verbindungen die HIV-Replikation hemmen. Experimentelle Daten sind in Tabelle A zusammengefasst.

**Assay zur Bestimmung der zytotoxischen Wirkung der Prüfsubstanzen**

Zur Bestimmung der zytotoxischen Wirkung der Prüfsubstanzen in nicht infizierten Zellen werden die Substanzen in entsprechenden Konzentrationen auf durchsichtige 96-well MTPs pipettiert und mit nicht infizierten Zellen (z.B. H9, PBLs, THP-1, MT4 7F2, CEM, Jurkat) inkubiert (analog zu den oben beschriebenen Assays). Nach 5 Tagen wird zu den Testansätzen je Well 1/10 Volumen AlamarBlue zugegeben und die MTPs für 3 Stunden bei 37°C inkubiert. Anschließend erfolgt die fluorimetrische Auswertung (544/590nm). Die resultierenden Werte liegen bei nicht behandelten Zellen je nach Zellart zwischen 20.000 und 40.000. Die $CC_{50}$-Werte der Prüfsubstanzen werden als die Konzentration ermittelt, bei der die Fluoreszenz 50% der unbehandelten Zellen beträgt. Prüfsubstanzen, die im Konzentrationsbereich der Wirkung zytotoxische Befunde zeigen, werden nicht in ihrer antiviralen Wirksamkeit bewertet.

**Tabelle A:**

| Beispiel-Nr. | $IC_{50}$ (nM) RT-Assay | $EC_{50}$ (nM) H9 Zellen HIV-$1_{LAI}$ 10% FKS | $EC_{50}$ (nM) MT4 7F2 Zellen HIV-$1_{NL4-3}$ wt 2% FKS | $EC_{50}$ (nM) MT4 7F2 Zellen HIV-$1_{NL4-3}$ K103N-Y181C 2% FKS |
|---|---|---|---|---|
| Beispiel 53 | 46 | 1 | 0,3 | 33 |
| Beispiel 33 | 8 | 0,5 | 0,07 | 3 |
| Beispiel 22 | 131 | 2 | 0,3 | 10 |
| Beispiel 52 | 38 | 2 | 0,1 | 17 |
| Beispiel 2 | 34 | 0,2 | 0,06 | 11 |
| Beispiel 1 | 38 | 2 | 0,2 | 3 |
| Beispiel 21 | 59 | 3 | 1 | 38 |
| Beispiel 25 | 99 | 2 | 0,6 | 30 |
| Beispiel 26 | 104 | 11 | 2 | 65 |
| Beispiel 32 | 65 | 0,7 | 0,1 | 25 |
| Beispiel 36 | 52 | 3 | 0,6 | 45 |
| Beispiel 30 | 297 | 8 | 3 | 16 |
| Beispiel 38 | 365 | 120 | 9 | 80 |
| Beispiel 3 | 37 | 0,2 | 0,03 | 7 |
| Beispiel 4 | 75 | 11 | 1 | 45 |
| Beispiel 51 | 60 | 1 | 0,4 | 15 |
| Beispiel 37 | 175 | 33 | 18 | 2000 |
| Beispiel 60 | 9 | 0,9 | 0,1 | 4 |
| Beispiel 59 | 8 | 1 | 0,2 | 10 |
| Beispiel 56 | 14 | 0,1 | 0,03 | 12 |
| Beispiel 61 | 35 | 3 | 1 | 80 |
| Beispiel 62 | 49 | 9 | 2 | 63 |
| Beispiel 63 | 102 | 150 | 20 | 237 |
| Beispiel 64 | 25 | 100 | 5 | 250 |

**In vivo Assay**

**Tiermodell:**

NOD Scid Mäuse, in der Regel 5 - 6 Wochen alt, werden von kommerziellen Züchtern (z. B. Taconic oder Jackson Laboratory) bezogen. Die Tiere werden unter sterilen Bedingungen (einschließlich Streu und Futter) in Isolatoren gehalten.

Eine definierte Anzahl von Zellen (z. B. 5 x $10^6$ T-Zellen (z. B. C8166)) wird mit einer geeigneten m.o.i. (z.B. 0.01 $TCID^{50}$) mit HIV infiziert. Die infizierten Zellen werden in Kollagenschwämme eingebracht. Die so vorbehandelten Schwämme werden den Mäusen unter die Rückenhaut implantiert. Die Mäuse werden einmal oder mehrfach täglich per oral, intraperitoneal, subcutan oder intravenös behandelt, wobei die erste Behandlung vor der Implantation liegen kann. Die Behandlungsgruppen umfassen in der Regel 10 Mäuse. Mindestens eine Gruppe wird mit Placebo behandelt, mindestens eine Gruppe mit einer bekanntermaßen wirksamen Substanz (= Positivkontrolle) und in der Regel mehrere Gruppen mit der erfindungsgemäßen Substanz. Die Tagesdosis der erfindungsgemäßen Substanz liegt zwischen 0.01 mg und 100 mg pro kg Körpergewicht. Die Formulierung der Substanzen erfolgt in 2 % DMSO / 0.5 % Methylcellulose

in PBS oder einer anderen geeigneten Mischung, die die Löslichkeit der Substanzen unterstützt. Die Behandlungsdauer beträgt in der Regel viereinhalb Tage. Nach der letzten Substanzapplikation werden die Tiere getötet und die Schwämme entnommen. Die virusinfizierten Zellen werden durch Kollagenaseverdau aus dem Schwamm gewonnen.

Aus den Zellen wird Gesamt-RNA gewonnen, die in der quantitativen PCR auf den Gehalt an Virus-RNA überprüft wird. Die Menge an Virus-RNA wird anhand der Menge eines house-keeping Gens (z. B. GAPDH) normalisiert. Ermittelt wird die Menge an HIV-RNA nach Substanzbehandlung im Vergleich zur placebobehandelten Kontrollgruppe. Wurde ein HIV verwendet, das eine Luziferase trägt, kann zusätzlich oder ersatzweise eine Luziferase-Messung durchgeführt werden. In diesem Fall wird die HIV-Menge über die Höhe des Luziferase-Signals bestimmt, da es in diesem Fall als Maß für die Virusreplikation dient. Die statistische Auswertung erfolgt mittels geeigneter Computerprogramme, z. B. Graph Pad Prism.

**B) <u>Bewertung der pharmakokinetischen Eigenschaften</u>**

***In vivo* Studien**

Zur Bestimmung der *in vivo* Pharmakokinetik werden die Testsubstanzen Mäusen, Ratten und Hunden intravenös und oral appliziert. Bei intravenösen Studien wird zur Bestimmung der pharmakokinetischen Eigenschaften der Testsubstanzen eine Dosis von 0.5 mg/kg in allen Spezies gewählt. Bei oraler Gabe wird den Nagern 3 mg/kg appliziert, Hunden 1 mg/kg. Die Testsubstanzen werden zur intravenösen Gabe für Nager in 99% Plasma, 1% DMSO formuliert, bei oraler Gabe in PEG 400, Ethanol und Wasser in variierenden Anteilen. Letzteres Vehikel wird bei Hunden für beide Applikationsrouten verwendet.

Männliche Wistar Ratten werden vor Applikation der Testsubstanzen katheterisiert, so dass die Blutproben mit Hilfe des gelegten Katheters oder durch Punktion der Vena cava zu verschiedenen Zeitenpunkten über ein Intervall von 2 min bis zu 26 h entnommen werden können.

Weiblichen BalbC Mäusen werden die Testsubstanzen als bolus Injektion intravenös appliziert, die Probengewinnung erfolgt in diesem Falle ausschließlich durch Punktion der Vena cava über ein Intervall von 2 min bis zu 26 h. Bei weiblichen Beagle Hunden erfolgt die Applikation ausschließlich durch eine 15 minütige intravenöse Infusion. Die Proben werden durch Punktion der Vena brachialis oder der Vena jugularis über ein Intervall von 10 min bis zu 26 h gewonnen.

Die quantitative Bestimmung der Substanzen erfolgt aus dem gewonnenen Tierplasma und Eichproben, die in Plasma eingestellt werden. Die Plasmaproteine werden durch Fällung mit Acetonitril (ACN) entfernt. Anschließend werden die Proben mittels HPLC auf einer Agilent 1100 LC Anlage (Agilent, Santa Clara, Californien, USA) unter Verwendung unterschiedlicher Säulen, z.B. Luna C8, LichroCart Purospher Star RP18e aufgetrennt. Das HPLC System ist über ein Turbo Ion Spray Interface an ein Triple Quadropole Massenspektrometer API 3000 (Applied Biosystems, Darmstadt, Deutschland) gekoppelt. Die Auswertung des Plasmakonzentrations-Zeitverlaufs erfolgt unter Einsatz eines internen Standards und Verwendung eines validierten Kinetikauswerteprogramms.

Neben Studien zur Bestimmung der pharmakokinetischen Parameter der Testsubstanzen *in vivo* werden Bestimmungen der relativen Bioverfügbarkeit aus Suspension (Formulierung: Tylose Suspension) versus Lösung in der Ratte sowie Hochdosisstudien im Vorfeld von Wirkversuchen und Toxikologischen Studien in Mäusen, Ratten und Hunden durchgeführt.

**<u>Plasmastabilität</u>**

Das verwendete Plasma der unterschiedlichen Spezies (BalbC Maus, Wistar Ratte, Beagle Hund und Mensch) wird durch Blutabnahme, in mit Li-Heparin beschichtete Monovetten und anschließender Zentrifugation, frisch gewonnen. Zur Bestimmung der Plasmastabilität der Testsubstanzen wird je 500 ng/ml bei 37 °C, zu je 2 ml in Plasma inkubiert. Zu verschiedenen Zeitpunkten, über ein Intervall bis zu 3 h, werden Proben dem Inkubationsgefäß entnommen. Die gewonnenen Proben werden mit ACN gefällt, um die Umsetzung zu stoppen und die Plasmaproteine abzutrennen. Die Proben werden äquivalent zu den *in vivo* Studien analysiert.

**<u>Mikrosomale und Hepatozyten Inkubationen</u>**

Inkubationen mit Lebermikrosomen verschiedener Spezies (BalbC Maus, Wistar Ratte, Beagle Hund, Mensch) werden in einem Gesamtvolumen von 1.5 ml bei 37 °C auf einem modifizierten Multiprobe II® Robotersystem (Canberra Packard) bzw. Janus® Robotersystem (Perkin Elmer) durchgeführt.

Die Inkubationsmischungen enthalten jeweils 0.5 µg/ml Testsubstanz sowie 0.2 - 0.5 mg/ml mikrosomales Protein. Zusätzlich wird 0.05 M Phosphatpuffer (pH = 7.4), 1mM EDTA, 5 mM Glucose-6-phosphat und 1.5 U/ml Glucose-6-phosphate Dehydroxygenase aus *Leuconostoc Mesenteroides* zugesetzt. Die mikrosomale Inkubationen werden

durch Zugabe von $NADP^+$ (Endkonzentration: 1mM) gestartet.

Zur Bestimmung der metabolischen Stabilität der Testsubstanzen in frisch isolierten und kultivierten Ratten-Hunde-, und Humanhepatozyten werden jeweils 1 Millionen Zellen/ml verwendet. Äquivalent dem mikrosomalen Assay werden den Hepatozyten jeweils 0.5 µg/ml Testsubstanz zugesetzt.

Nach 2, 5, 10, 20, 30, 45 und 60 min, oder nach 2, 10, 20, 30, 50, 70 und 90 min bei stabileren Verbindungen, werden 125 µl aus dem jeweiligen Inkubationsansatz entnommen und mit ACN versetzt, um die enzymatischen Reaktionen zu stoppen. Nach der Zentrifugation werden die Proben mittels LC-MS/MS (API 2000 oder 3000, Applied Biosystems) analysiert. "$CL_{blood}$ well-stirred"- und "$F_{max}$ well-stirred"-Werte werden aus den jeweiligen Halbwertszeiten der Verbindungen in den mikrosomalen Inkubationen errechnet. Der Substratabbau kann mit folgenden Formeln beschrieben werden (Houston JB, Utility of in-vitro drug-metabolism data in predicting in-vivo metabolic-clearance, Bioch. Pharm. 47 (9) 1469-1479 (1994); Obach RS; Baxter JG; Liston TE; Silber BM; Jones BC; MacIntyre F; Rance DJ; Wastall P, The prediction of human pharmacokinetic parameters from preclinical and in vitro metabolism data, J. Pharmacol. Exp. Ther. 283 (1) 46-58 (1997)):

$$CL'_{intrinsic}\ [ml/(min\cdot kg)] = (0.693\ /\ in\ vitro\ t_{1/2}\ [min]) \cdot (Lebergewicht\ [g\ Leber\ /kg\ Körpergewicht]) \cdot (mikrosomales\ Protein\ [mg]\ /\ Lebergewicht\ [g])\ /\ (mikrosomales\ Protein\ [mg]\ /\ Inkubationsvolumen\ [ml]).$$

Die Blut-Clearance "$CL_{blood}$" wird ohne die Berücksichtigung von Proteinbindungen durch das "well-stirred" Modell beschrieben (Pang KS; Rowland M, Hepatic clearance of drugs. I. Theoretical considerations of a "well-stirred" model and a "parallel tube" model. Influence of hepatic blood flow, plasma and blood cell binding, and the hepatocellular enzymatic activity on hepatic drug clearance, J Pharmacokinet Biopharm 5 (6): 625-53 (1977)):

$$CL_{blood}\ well\text{-}stirred\ [L/(h\cdot kg)] = (Q_H\ [L/(h\cdot kg)] \cdot CL'_{intrinsic}\ [L/(h\cdot kg)]\ )\ /\ (Q_H\ [L/(h\cdot kg)] + CL'_{intrinsic}\ [L/(h\cdot kg)]).$$

Für die Ratten beträgt das spezifische Lebergewicht 32 g/kg Körpergewicht und der hepatische Blutfluss 4.2 L/(h·kg). Der spezifische mikrosmale Proteingehalt der Rattenleber wurde mit 40 mg/g Leber veranschlagt. Die spezifischen Hochrechnungsfaktoren weiterer Spezies sind in folgender Tabelle wiedergegeben und beruhen zum Teil auf Literaturdaten, zum Teil auf eigenen Bestimmungen. Für Hepatozyten wird eine Zellzahl von 110 Mio/g Leber bei allen Spezies zur Berechnung herangezogen.

| | Maus m | Maus w | Ratte m | Hund m/w | Mensch m/w |
|---|---|---|---|---|---|
| Mikrosomales Protein/g Leber [mg] | 40 | 40 | 40 | 40 | 40 |
| Leber [g]/kg Körpergewicht | 50 | 43 | 32 | 39 | 21 |
| Leberblutfluss [L/(h·kg)] | 5.4 | 5.4 | 4.2 | 2.1 | 1.32 |

C) <u>Ansführungsbeispiele für pharmazeutische Zusammensetzungen</u>

Die erfindungsgemäßen Verbindungen können folgendermaßen in pharmazeutische Zubereitungen überführt werden:

**<u>Tablette:</u>**

<u>Zusammensetzung:</u>

100 mg der Verbindung von Beispiel 1, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg, Durchmesser 8 mm, Wölbungsradius 12 mm.

Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

**Oral applizierbare Lösung:**

Zusammensetzung:

500 mg der Verbindung von Beispiel 1, 2.5 g Polysorbat und 97 g Polyethylenglycol 400. Einer Einzeldosis von 100 mg der erfindungsgemäßen Verbindung entsprechen 20 g orale Lösung.

Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

**i.v. Lösung:**

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5%, PEG 400 Lösung 30%) gelöst. Die Lösung wird steril filtriert und in sterile und pyrogenfreie Injektionsbehältnisse abgefüllt.

**Patentansprüche**

**1.** Verbindung der Formel

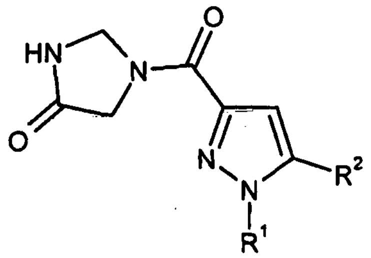

(I),

in welcher

$R^1$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, ($C_1$-$C_4$) Alkyl und ($C_1$-$C_4$)-Alkoxy,
worin
($C_1$-$C_4$)-Alkyl und ($C_1$-$C_4$)-Alkoxy ihrerseits ein- bis dreifach, gleich oder verschieden, mit Resten ausgewählt aus der Reihe Halogen, Cyano, Hydroxy, ($C_1$-$C_4$)-Alkoxy, Amino, Mono-($C_1$-$C_4$)-alkylamino, Di-($C_1$-$C_4$)-alkylamino, ($C_3$-$C_7$)-Cycloalkyl und 4-bis 7-gliedriges Heterocyclyl substituiert sein können,
wobei die letztgenannten Cycloalkyl- und Heterocyclyl-Reste ih-rerseits jeweils bis zu dreifach, gleich oder verschieden, mit Halogen, Cyano, ($C_1$-$C_4$)-Alkyl, Trifluormethyl, Hydroxy, ($C_1$-$C_4$)-Alkoxy, Trifluormethoxy, Oxo, Amino, Mono-($C_1$-$C_4$)-alkylamino und Di-($C_1$-$C_4$)-alkylamino substituiert sein können,
$R^2$ für Phenyl steht,
wobei Phenyl substituiert ist mit 1 bis 3 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, ($C_1$-$C_4$)-Alkyl und ($C_1$-$C_4$)-Alkoxy,

worin
$(C_1-C_4)$-Alkyl und $(C_1-C_4)$-Alkoxy ihrerseits ein- bis dreifach, gleich oder verschieden, mit Resten ausgewählt aus der Reihe Halogen, Cyano, Hydroxy, $(C_1-C_4)$-Alkoxy, Amino, Mono-$(C_1-C_4)$-alkylamino, Di-$(C_1-C_4)$-alkylamino, $(C_3-C_7)$-Cycloalkyl und 4-bis 7-gliedriges Heterocyclyl substituiert sein können,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkylamino und Di-$(C_1-C_4)$-alkylamino substituiert sein können,

oder eines ihrer Salze, ihrer Solvate oder der Solvate ihrer Salze.

**2.** Verbindung nach Anspruch 1, **dadurch gekennzeichnet, dass**

$R^1$ für Phenyl steht,
wobei Phenyl substituiert ist mit 1-bis 2 Substituenten, wobei die Substituenten unabhängig voneinander ausgewählt werden aus der Gruppe bestehend aus Halogen, Hydroxy, Cyano, Nitro, Trifluormethyl, Trifluormethoxy, Trifluormethylthio, $(C_1-C_4)$-Alkyl und$(C_1-C_4)$-Alkoxy,
$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Hydroxy, Cyano, Nitro, Trifluormethoxy, Trifluormethylthio, $(C_1-C_4)$-Alkyl und $(C_1-C_4)$-Alkoxy,
worin
$(C_1-C_4)$-Alkoxy seinerseits ein- bis dreifach, gleich oder verschieden, mit Resten ausgewählt aus der Reihe Halogen, Cyano, Hydroxy, $(C_1-C_4)$-Alkoxy, Amino, Mono-$(C_1-C_4)$-alkylamino, Di$(C_1-C_4)$-alkylamino, $(C_3-C_7)$-Cycloalkyl und 4- bis 7-gliedriges Heterocyclyl substituiert sein kann,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C1-C_4)$-Alkoxy, Trifluormethoxy, Oxo, Amino, Mono-$(C_1-C_4)$-alkylamino und Di-$(C_1-C_4)$-alkylamino substituiert sein können,

oder eines ihrer Salze, ihrer Solvate oder der Solvate ihrer Salze.

**3.** Verbindung nach Anspruch 1 oder 2, **dadurch gekennzeichnet, dass**

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl und Methoxy,
$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethoxy, Methyl und $(C_1-C_3)$-Alkoxy,
worin
$(C_1-C_3)$-Alkoxy seinerseits ein- bis dreifach, gleich oder verschieden, mit Resten ausgewählt aus der Reihe Hydroxy, $(C_1-C_4)$-Alkoxy, Amino, Mono-$(C_1-C_4)$-alkylamino, Di-$(C_1-C_4)$-alkylamino und 4- bis 7-gliedriges Heterocyclyl substituiert sein kann,
wobei die letztgenannten Heterocyclyl-Reste ihrerseits jeweils mit $(C_1-C_4)$-Alkyl substituiert sein können,

oder eines ihrer Salze, ihrer Solvate oder der Solvate ihrer Salze.

**4.** Verbindung nach einem der Ansprüche 1 bis 3, **dadurch gekennzeichnet, dass**

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethyl und Methoxy,
$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen, Cyano, Trifluormethoxy, Methyl und Methoxy,

oder eines ihrer Salze, ihrer Solvate oder der Solvate ihrer Salze.

**5.** Verbindung nach einem der Ansprüche 1 bis 4, **dadurch gekennzeichnet, dass**

$R^1$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano,
$R^2$ für Phenyl steht,
wobei Phenyl substituiert ist mit 1 bis 2 Substituenten, wobei die Substituenten unabhängig voneinander ausgewählt werden aus der Gruppe bestehend aus Halogen und Cyano

oder eines ihrer Salze, ihrer Solvate oder der Solvate ihrer Salze.

6. Verbindung nach Anspruch 1, **dadurch gekennzeichnet, dass** sie der Formel

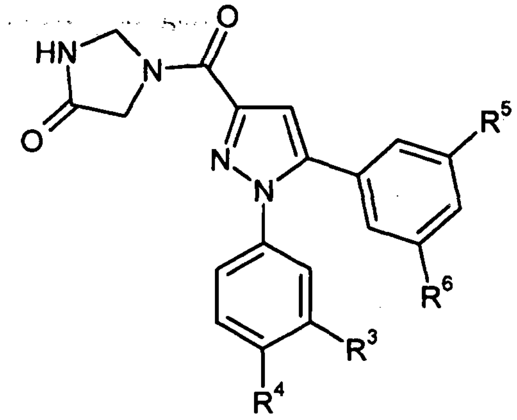

(Ia),

entspricht, in welcher

$R^3$ für Wasserstoff, Halogen, Hydroxy, Cyano, Nitro, Trifluormethyl, Trifluormethoxy, Trifluormethylthio, $(C_1-C_4)$-Alkyl oder $(C_1-C_4)$-Alkoxy steht,
$R^4$ für Wasserstoff oder Halogen steht,
$R^5$ für Halogen, Hydroxy, Cyano, Nitro, Trifluormethyl, Trifluormethoxy, Trifluormethylthio, $(C_1-C_4)$-Alkyl oder $(C_1-C_4)$-Alkoxy steht,
worin
$(C_1-C_4)$-Alkoxy seinerseits ein- bis dreifach, gleich oder verschieden, mit Resten ausgewählt aus der Reihe Halogen, Cyano, Hydroxy, $(C_1-C_4)$-Alkoxy, Amino, Mono-$(C_1-C_4)$-alkylamino, Di$(C_1-C_4)$-alkylamino, $(C_3-C_7)$-Cycloalkyl und 4- bis 7-gliedriges Heterocyclyl substituiert sein kann,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo, Amino, Mono-$(C_1-C_4)$-alkyl-amino und Di-$(C_1-C_4)$-alkylamino substituiert sein können,
$R^6$ für Wasserstoff oder Halogen steht,

oder eines ihrer Salze, ihrer Solvate oder der Solvate ihrer Salze.

7. Verbindung nach Anspruch 6, **dadurch gekennzeichnet, dass**

$R^3$ für Halogen, Hydroxy, Cyano, Nitro, Trifluormethyl, Trifluormethoxy, Trifluormethylthio, $(C_1-C_4)$-Alkyl oder $(C_1-C_4)$-Alkoxy steht,
$R^4$ für Wasserstoff oder Halogen steht,
$R^5$ für Halogen, Hydroxy, Cyano, Nitro, Trifluormethoxy, Trifluormethylthio, $(C_1-C_4)$-Alkyl oder $(C_1-C_4)$-Alkoxy steht,
worin
$(C_1-C_4)$-Alkoxy seinerseits ein- bis dreifach, gleich oder verschieden, mit Resten ausgewählt aus der Reihe Halogen, Cyano, Hydroxy, $(C_1-C_4)$-Alkoxy, Amino, Mono-$(C_1-C_4)$-alkylamino, Di$(C_1-C_4)$-alkylamino, $(C_3-C_7)$-Cycloalkyl und 4- bis 7-gliedriges Heterocyclyl substituiert sein kann,
wobei die letztgenannten Cycloalkyl- und Heterocyclyl-Reste ihrerseits jeweils bis zu dreifach, gleich oder verschieden, mit Halogen, Cyano, $(C_1-C_4)$-Alkyl, Trifluormethyl, Hydroxy, $(C_1-C_4)$-Alkoxy, Trifluormethoxy, Oxo,

Amino, Mono-($C_1$-$C_4$)-alkylami-no und Di-($C_1$-$C_4$)-alkylamino substituiert sein können,
$R^6$ für Wasserstoff oder Halogen steht,

oder eines ihrer Salze, ihrer Solvate oder der Solvate ihrer Salze.

**8.** Verbindung nach Anspruch 6 oder 7, **dadurch gekennzeichnet, dass**

$R^3$ für Halogen, Cyano, Trifluormethyl oder Methoxy steht,
$R^4$ für Wasserstoff oder Halogen steht,
$R^5$ für Halogen, Cyano, Trifluormethoxy, Methyl oder ($C_1$-$C_3$)-Alkoxy steht,
worin
($C_1$-$C_3$)-Alkoxy seinerseits ein- bis dreifach, gleich oder verschieden, mit Resten ausgewählt aus der Reihe Hydroxy, ($C_1$-$C_4$)-Alkoxy, Amino, Mono-($C_1$-$C_4$)-alkylamino, Di-($C_1$-$C_4$)-alkylamino und 4- bis 7-gliedriges Heterocyclyl substituiert sein kann,
wobei die letztgenannten Heterocyclyl-Reste ihrerseits jeweils mit ($C_1$-$C_4$)-Alkyl substituiert sein können,
$R^6$ für Wasserstoff oder Halogen steht,

oder eines ihrer Salze, ihrer Solvate oder der Solvate ihrer Salze.

**9.** Verbindung nach einem der Ansprüche 6 bis 8, **dadurch gekennzeichnet; dass**

$R^3$ für Halogen, Cyano, Trifluormethyl oder Methoxy steht,
$R^4$ für Wasserstoff, Chlor oder Fluor steht,
$R^5$ für Halogen, Cyano, Trifluormethoxy, Methyl oder Methoxy steht,
$R^6$ für Wasserstoff, Chlor oder Fluor steht,

oder eines ihrer Salze, ihrer Solvate oder der Solvate ihrer Salze.

**10.** Verbindung nach einem der Ansprüche 6 bis 9, **dadurch gekennzeichnet, dass**

$R^3$ für Halogen oder Cyano steht,
$R^4$ für Wasserstoff oder Fluor steht,
$R^5$ für Halogen oder Cyano steht,
$R^6$ für Wasserstoff oder Fluor steht,

oder eines ihrer Salze, ihrer Solvate oder der Solvate ihrer Salze.

**11.** Verbindung nach einem der Ansprüche 6 bis 10, **dadurch gekennzeichnet, dass**

$R^3$ für Chlor oder Cyano steht,
$R^4$ für Fluor steht,
$R^5$ für Chlor oder Cyano steht,
$R^6$ für Fluor steht,

oder eines ihrer Salze, ihrer Solvate oder der Solvate ihrer Salze.

**12.** Verbindung nach einem der Ansprüche 6 bis 10, **dadurch gekennzeichnet, dass**

$R^3$ für Chlor oder Cyano steht,
$R^4$ für Fluor steht,
$R^5$ für Chlor oder Cyano steht,
$R^6$ für Wasserstoff steht,

oder eines ihrer Salze, ihrer Solvate oder der Solvate ihrer Salze.

**13.** Verbindung nach einem der Ansprüche 6 bis 10, **dadurch gekennzeichnet, dass**

$R^3$ für Chlor oder Cyano steht,

$R^4$ für Wasserstoff steht,
$R^5$ für Chlor oder Cyano steht,
$R^6$ für Wasserstoff steht,

oder eines ihrer Salze, ihrer Solvate oder der Solvate ihrer Salze.

**14.** Verbindung nach einem der Ansprüche 6 bis 10, **dadurch gekennzeichnet, dass**

$R^3$ für Chlor oder Cyano steht,
$R^4$ für Wasserstoff steht,
$R^5$ für Chlor oder Cyano steht,
$R^6$ für Fluor steht,

oder eines ihrer Salze, ihrer Solvate oder der Solvate ihrer Salze.

**15.** Verbindung nach Anspruch 6, **dadurch gekennzeichnet, dass**

$R^3$ für Halogen, Cyano, Trifluormethyl oder Methoxy steht,
$R^4$ für Wasserstoff oder Halogen steht,
$R^5$ für Trifluormethyl steht,
$R^6$ für Fluor steht,

oder eines ihrer Salze, ihrer Solvate oder der Solvate ihrer Salze.

**16.** Verbindung nach Anspruch 6, **dadurch gekennzeichnet, dass**

$R^3$ für Wasserstoff steht,
$R^4$ für Fluor oder Chlor steht,
$R^5$ für Halogen, Cyano, Trifluormethoxy, Methyl oder Methoxy steht,
$R^6$ für Wasserstoff oder Halogen steht,

oder eines ihrer Salze, ihrer Solvate oder der Solvate ihrer Salze.

**17.** Verfahren zur Herstellung einer Verbindung der Formel (I) nach Anspruch 1, wobei eine Verbindung der Formel

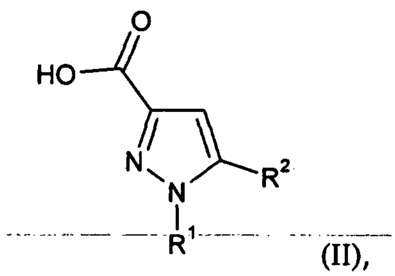

(II),

in welcher
$R^1$ und $R^2$ die in Anspruch 1 angegebene Bedeutung aufweisen,
mit Imidazolidin-4-on oder einem Salz von Imidazolidin-4-on umgesetzt wird.

**18.** Verbindung nach einem der Ansprüche 1 bis 16 zur Behandlung und/oder Prophylaxe von Krankheiten.

**19.** Verwendung einer Verbindung nach einem der Ansprüche 1 bis 16 zur Herstellung eines Arzneimittels zur Behandlung und/oder Prophylaxe von Krankheiten.

**20.** Verwendung einer Verbindung nach einem der Ansprüche 1 bis 16 zur Herstellung eines Arzneimittels zur Behandlung und/oder Prophylaxe von retroviralen Erkrankungen.

**21.** Verwendung nach Anspruch 20, **dadurch gekennzeichnet, dass** die retrovirale Erkrankung eine Infektion mit dem

HI-Virus ist.

**22.** Arzneimittel enthaltend mindestens eine Verbindung nach einem der Ansprüche 1 bis 16 in Kombination mit mindestens einem weiteren Wirkstoff.

**23.** Arzneimittel enthaltend mindestens eine Verbindung nach einem der Ansprüche 1 bis 16 in Kombination mit mindestens einem inerten, nichttoxischen, pharmazeutisch geeigneten Hilfsstoff.

**24.** Arzneimittel nach Anspruch 22 oder 23 zur Behandlung und/oder Prophylaxe von retroviralen Erkrankungen.

**25.** Arzneimittel nach Anspruch 24, **dadurch gekennzeichnet, dass** die retrovirale Erkrankung eine Infektion mit dem HI-Virus ist.

**26.** Antiviral wirksame Menge mindestens einer Verbindung nach einem der Ansprüche 1 bis 16 oder eines Arzneimittels nach einem der Ansprüche 22 bis 25 zur Verwendung in einem Verfahren zur Bekämpfung von viralen Erkrankungen in Menschen und Tieren.

## Claims

**1.** Compound of the formula

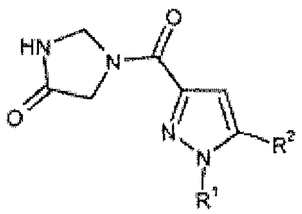

(I),

in which

$R^1$ stands for phenyl,
where phenyl is substituted with 1 to 3 substitutes, the substitutes being selected independently of one another from the group consisting of halogen, hydroxy, cyano, nitro, trifluoromethyl, trifluoromethoxy, trifluoromethylthio, $(C_1-C_4)$-alkyl and $(C_1-C_4)$-alkoxy,
in which
$(C_1-C_4)$-alkyl and $(C_1-C_4)$-alkoxy can themselves be substituted one to three times, the same way or differently, with residues selected from the group consisting of halogen, cyano, hydroxy, $(C_1-C_4)$-alkoxy, amino, mono-$(C_1-C_4)$-alkylamino, di-$(C_1-C_4)$-alkylamino, $(C_3-C_7)$-cycloalkyl and 4- to 7-membered heterocyclyl,
in which said cycloalkyl and heterocyclyl residues can themselves be substituted one to three times, the same way or differently, with halogen, cyano, $(C_1-C_4)$-alkyl, trifluoromethyl, hydroxy, $(C_1-C_4)$- alkoxy, trifluoromethoxy, oxo, amino, mono-$(C_1-C_4)$-alkylamino and di-$(C_1-C_4)$-alkylamino,
$R^2$ stands for phenyl,
where phenyl is substituted with 1 to 3 substitutes, the substitutes being selected independently of one another from the group consisting of halogen, hydroxy, cyano, nitro, trifluoromethyl, trifluoromethoxy, trifluoromethylthio, $(C_1-C_4)$-alkyl and $(C_1-C_4)$-alkoxy,
in which
$(C_1-C_4)$-alkyl and $(C_1-C_4)$-alkoxy can themselves be substituted one to three times, the same way or differently, with residues selected from the group consisting of halogen, cyano, hydroxy, $(C_1-C_4)$-alkoxy, amino, mono-$(C_1-C_4)$-alkylamino, di-$(C_1-C_4)$-alkylamino, $(C_3-C_7)$-cycloalkyl and 4- to 7-membered heterocyclyl, in which said cycloalkyl and heterocyclyl residues can themselves be substituted once to three times, the same way or differently, with halogen, cyano, $(C_1-C_4)$-alkyl, trifluoromethyl, hydroxy, $(C_1-C_4)$- alkoxy, trifluoromethoxy, oxo, amino, mono-$(C_1-C_4)$-alkylamino and di-$(C_1-C_4)$-alkylamino,

or one of their salts, their solvates or the solvates of their salts.

2. Compound according to Claim 1, **characterized in that**

$R^1$ stands for phenyl,
where phenyl is substituted with 1 to 2 substitutes, the substitutes being selected independently of one another from the group consisting of halogen, hydroxy, cyano, nitro, trifluoromethyl, trifluoromethoxy, trifluoromethylthio, ($C_1$-$C_4$)-alkyl and ($C_1$-$C_4$)-alkoxy,
$R^2$ stands for phenyl,
where phenyl is substituted with 1 to 2 substitutes, the substitutes being selected independently of one another from the group consisting of halogen, hydroxy, cyano, nitro, trifluoromethoxy, trifluoromethylthio, ($C_1$-$C_4$)-alkyl and ($C_1$-$C_4$)-alkoxy,
in which
($C_1$-$C_4$)-alkoxy can itself be substituted one to three times, the same way or differently, with residues selected from the group consisting of halogen, cyano, hydroxy, ($C_1$-$C_4$)-alkoxy, amino, mono-($C_1$-$C_4$)-alkylamino, di-($C_1$-$C_4$)-alkylamino, ($C_3$-$C_7$)-cycloalkyl and 4- to 7-membered heterocyclyl,
in which said cycloalkyl and heterocyclyl residues can themselves be substituted one to three times, the same way or differently, with halogen, cyano, ($C_1$-$C_4$)-alkyl, trifluoromethyl, hydroxy, ($C_1$-$C_4$)- alkoxy, trifluoromethoxy, oxo, amino, mono-($C_1$-$C_4$)-alkylamino and di-($C_1$-$C_4$)-alkylamino,

or one of their salts, their solvates or the solvates of their salts.

3. Compound according to Claim 1 or 2, **characterized in that**

$R^1$ stands for phenyl,
where phenyl is substituted with 1 to 2 substitutes, the substitutes being selected independently of one another from the group consisting of halogen, cyano, trifluoromethyl, and methoxy,
$R^2$ stands for phenyl,
where phenyl is substituted with 1 to 2 substitutes, the substitutes being selected independently of one another from the group consisting of halogen, cyano, trifluoromethoxy, methyl and ($C_1$-$C_3$)-alkoxy,
in which
($C_1$-$C_3$)-alkoxy can itself be substituted one to three times, the same way or differently, with residues selected from the group consisting of hydroxy, ($C_1$-$C_4$)-alkoxy, amino, mono-($C_1$-$C_4$)-alkylamino, di-($C_1$-$C_4$)-alkylamino and 4- to 7-membered heterocyclyl,
where said heterocyclyl residues can themselves be substituted in each case with ($C_1$-$C_4$)-alkyl,

or one of their salts, their solvates or the solvates of their salts.

4. Compound according any of Claims 1 to 3, **characterized in that**

$R^1$ stands for phenyl,
where phenyl is substituted with 1 to 2 substitutes, the substitutes being selected independently of one another from the group consisting of halogen, cyano, trifluoromethyl, and methoxy,
$R^2$ stands for phenyl,
where phenyl is substituted with 1 to 2 substitutes, the substitutes being selected independently of one another from the group consisting of halogen, cyano, trifluoromethoxy, methyl and methoxy,

or one of their salts, their solvates or the solvates of their salts.

5. Compound according any of Claims 1 to 4, **characterized in that**

$R^1$ stands for phenyl,
where phenyl is substituted with 1 to 2 substitutes, the substitutes being selected independently of one another from the group consisting of halogen and cyano,
$R^2$ stands for phenyl,
where phenyl is substituted with 1 to 2 substitutes, the substitutes being selected independently of one another from the group consisting of halogen and cyano

or one of their salts, their solvates or the solvates of their salts.

**6.** Compound according to Claim 1, **characterized in that** it corresponds to the formula

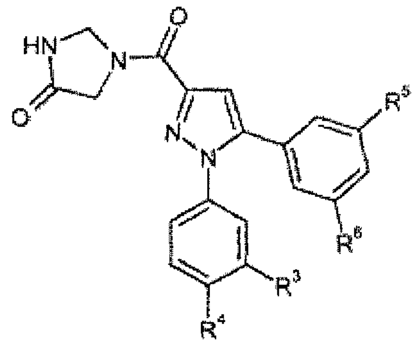

(Ia),

in which

$R^3$ stands for hydrogen, halogen, hydroxy, cyano, nitro, trifluoromethyl, trifluoromethoxy, trifluoromethylthio, $(C_1-C_4)$-alkyl or $(C_3-C_4)$-alkoxy,
$R^4$ stands for hydrogen or halogen,
$R^5$ stands for halogen, hydroxy, cyano, nitro, trifluoromethyl, trifluoromethoxy, trifluoromethylthio, $(C_1-C_4)$-alkyl or $(C_1-C_4)$-alkoxy,
in which
$(C_1-C_4)$-alkoxy can itself be substituted one to three times, the same way or differently, with residues selected from the group consisting of halogen, cyano, hydroxy, $(C_1-C_4)$-alkoxy, amino, mono-$(C_1-C_4)$-alkylamino, di-$(C_1-C_4)$-alkylamino, $(C_3-C_7)$-cycloalkyl and 4- to 7-membered heterocyclyl,
in which said cycloalkyl and heterocyclyl residues can themselves be substituted one to three times, the same way or differently, with halogen, cyano, $(C_1-C_4)$-alkyl, trifluoromethyl, hydroxy, $(C_1-C_4)$- alkoxy, trifluoromethoxy, oxo, amino, mono-$(C_1-C_4)$-alkylamino and di-$(C_1-C_4)$-alkylamino,
$R^6$ stands for hydrogen or halogen,

or one of their salts, their solvates or the solvates of their salts.

**7.** Compound according to Claim 6, **characterized in that**

$R^3$ stands for halogen, hydroxy, cyano, nitro, trifluoromethyl, trifluoromethoxy, trifluoromethylthio, $(C_1-C_4)$-alkyl or $(C_1-C_4)$-alkoxy,
$R^4$ stands for hydrogen or halogen,
$R^5$ stands for halogen, hydroxy, cyano, nitro, trifluoromethoxy, trifluoromethylthio, $(C_1-C_4)$-alkyl or $(C_1-C_4)$-alkoxy,
in which
$(C_1-C_4)$-alkoxy can itself be substituted one to three times, the same way or differently, with residues selected from the group consisting of halogen, cyano, hydroxy, $(C_1-C_4)$-alkoxy, amino, mono-$(C_1-C_4)$-alkylamino, di-$(C_1-C_4)$-alkylamino, $(C_3-C_7)$-cycloalkyl and 4- to 7-membered heterocyclyl,
in which said cycloalkyl and heterocyclyl residues can themselves be substituted one to three times, the same way or differently, with halogen, cyano, $(C_1-C_4)$-alkyl, trifluoromethyl, hydroxy, $(C_1-C_4)$- alkoxy, trifluoromethoxy, oxo, amino, mono-$(C_1-C_4)$-alkylamino and di-$(C_1-C_4)$-alkylamino,
$R^6$ stands for hydrogen or halogen,

or one of their salts, their solvates or the solvates of their salts,

**8.** Compound according to Claim 6 or 7, **characterized in that**

$R^3$ stands for halogen, cyano, trifluoromethyl or methoxy,
$R^4$ stands for hydrogen or halogen,
$R^5$ stands for halogen, cyano, trifluoromethoxy, methyl or $(C_1-C_3)$-alkoxy, in which
$(C_1-C_3)$-alkoxy can itself be substituted one to three times, the same way or differently, with residues selected from the group consisting of hydroxy, $(C_1-C_4)$-alkoxy, amino, mono-$(C_1-C_4)$-alkylamino, di-$(C_1-C_4)$-alkylamino

and 4- to 7-membered heterocyclyl,
where said heterocyclyl residues can themselves be substituted in each case with ($C_1$-$C_4$)-alkyl,
$R^6$ stands for hydrogen or halogen,

or one of their salts, their solvates or the solvates of their salts.

**9.** Compound according any of Claims 6 to 8, **characterized in that**

$R^3$ stands for halogen, cyano, trifluoromethyl or methoxy,
$R^4$ stands for hydrogen, chlorine or fluorine,
$R^5$ stands for halogen, cyano, trifluoromethoxy, methyl or methoxy,
$R^6$ stands for hydrogen, chlorine or fluorine,

or one of their salts, their solvates or the solvates of their salts.

**10.** Compound according any of Claims 6 to 9, **characterized in that**

$R^3$ stands for halogen or cyano,
$R^4$ stands for hydrogen or fluorine,
$R^5$ stands for halogen or cyano,
$R^6$ stands for hydrogen or fluorine,

or one of their salts, their solvates or the solvates of their salts.

**11.** Compound according any of Claims 6 to 10, **characterized in that**

$R^3$ stands for chlorine or cyano,
$R^4$ for fluorine,
$R^5$ stands for chlorine or cyano,
$R^6$ for fluorine,

or one of their salts, their solvates or the solvates of their salts.

**12.** Compound according any of Claims 6 to 10, **characterized in that**

$R^3$ stands for chlorine or cyano,
$R^4$ for fluorine,
$R^5$ stands for chlorine or cyano,
$R^6$ stands for hydrogen,

or one of their salts, their solvates or the solvates of their salts.

**13.** Compound according any of Claims 6 to 10, **characterized in that**

$R^3$ stands for chlorine or cyano,
$R^4$ stands for hydrogen,
$R^5$ stands for chlorine or cyano,
$R^6$ stands for hydrogen,

or one of their salts, their solvates or the solvates of their salts.

**14.** Compound according any of Claims 6 to 10, **characterized in that**

$R^3$ stands for chlorine or cyano,
$R^4$ stands for hydrogen,
$R^5$ stands for chlorine or cyano,
$R^6$ for fluorine,

or one of their salts, their solvates or the solvates of their salts.

**15.** Compound according to Claim 6, **characterized in that**

$R^3$ stands for halogen, cyano, trifluoromethyl or methoxy,
$R^4$ stands for hydrogen or halogen,
$R^5$ for trifluoromethyl,
$R^6$ stands for fluorine,

or one of their salts, their solvates or the solvates of their salts.

**16.** Compound according to Claim 6, **characterized in that**

$R^3$ stands for hydrogen,
$R^4$ stands for fluorine or chlorine,
$R^5$ stands for halogen, cyano, trifluoromethoxy, methyl or methoxy,
$R^6$ stands for hydrogen or halogen,

or one of their salts, their solvates or the solvates of their salts.

**17.** Method to produce a compound of Formula (I) according to Claim 1, in which a compound of the following formula,

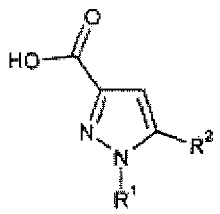

(II),

in which
$R^1$ and $R^2$ have the meaning specified in Claim 1,
is realized with imidazolidin-4 or a salt of imidazolidin-4.

**18.** Compound according to any of Claims 1 to 16 for the treatment and/or prevention of diseases.

**19.** Use of a compound according to any of Claims 1 to 16 to produce a medication for the treatment and/or prevention of diseases.

**20.** Use of a compound according to any of Claims 1 to 16 to produce a medication for the treatment and/or prevention of retroviral diseases.

**21.** Use according to Claim 20, **characterized in that** the retroviral disease is an HIV infection.

**22.** Medication containing at least one compound according to any of Claims 1 to 16 in combination with at least one additional active ingredient.

**23.** Medication containing at least one compound according to any of Claims 1 to 16 in combination with at least one inactive, non-toxic, pharmaceutically appropriate excipient.

**24.** Medication according to Claim 22 or 23 for the treatment and/or prevention of retroviral diseases.

**25.** Medication according to Claim 24, **characterized in that** the retroviral disease is an HIV infection.

**26.** Quantity having an antiviral effect, of at least one compound according to any of Claims 1 to 16 or a medication according to any of Claims 22 to 25 for use in a method to combat viral diseases in humans and animals.

## Revendications

**1.** Composé de la formule

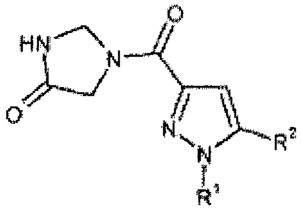

(I),

dans laquelle

$R^1$ représente phényle,
où phényle est substitué par 1 ou 3 substituants, les substituants étant sélectionnés indépendamment l'un de l'autre parmi le groupe constitué par halogène, hydroxy, cyano, nitro, trifluorométhyle, trifluorométhoxy, trifluorométhylthio, ($C_1$-$C_4$)-alkyl et ($C_1$-$C_4$)-alkoxy,
dans laquelle
($C_1$-$C_4$)-alkyle et ($C_1$-$C_4$)-alkoxy peuvent à leur tour être substitués une à trois fois, de façon égale ou différente, par des résidus sélectionnés parmi le groupe constitué par halogène, cyano, hydroxy, ($C_1$-$C_4$)-alkoxy, amino, mono-($C_1$-$C_4$)-alkylamino, di-($C_1$-$C_4$)-alkylamino, ($C_3$-$C_7$)-cycloalkyle et hétérocycle ayant 4 à 7 membres,
dans laquelle lesdits résidus de cycloalkyle et d'hétérocycle peuvent à leur tour être substitués une à trois fois, de façon égale ou différente, par halogène, cyano, ($C_1$-$C_4$)-alkyle, trifluorométhyl, hydroxy, ($C_1$-$C_4$)-alkoxy, trifluorométhoxy, oxo, amino, mono-($C_1$-$C_4$)-alkylamino et di-($C_1$-$C_4$)-alkylamino,
$R^2$ représente phényle,
où phényle est substitué par 1 ou 3 substituants, les substituants étant sélectionnés indépendamment l'un de l'autre parmi le groupe constitué par halogène, hydroxy, cyano, nitro, trifluorométhyle, trifluorométhoxy, trifluorométhylthio, ($C_1$-$C_4$)-alkyl et ($C_1$-$C_4$)-alkoxy,
dans laquelle
($C_1$-$C_4$)-alkyle et ($C_1$-$C_4$)-alkoxy peuvent à leur tour être substitués une à trois fois, de façon égale ou différente, par des résidus sélectionnés parmi le groupe constitué par halogène, cyano, hydroxy, ($C_1$-$C_4$)-alkoxy, amino, mono-($C_1$-$C_4$)-alkylamino, di-($C_1$-$C_4$)-alkylamino, ($C_3$-$C_7$)-cycloalkyle et hétérocycle ayant 4 à 7 membres,
dans laquelle lesdits résidus de cycloalkyle et d'hétérocycle peuvent à leur tour être substitués une à trois fois, de façon égale ou différente, par halogène, cyano, ($C_1$-$C_4$)-alkyle, trifluorométhyle, hydroxy, ($C_1$-$C_4$)-alkoxy, trifluorométhoxy, oxo, amino, mono-($C_1$-$C_4$)-alkylamino et di-($C_1$-$C_4$)-alkylamino,

ou un de leurs sels, solvats ou les solvats de leurs sels.

**2.** Composé selon la revendication 1, **caractérisé en ce que**

$R^1$ représente phényle,
où phényle est substitué par 1 ou 2 substituants, les substituants étant sélectionnés indépendamment l'un de l'autre parmi le groupe constitué par halogène, hydroxy, cyano, nitro, trifluorométhyle, trifluorométhoxy, trifluorométhylthio, ($C_1$-$C_4$)-alkyl et ($C_1$-$C_4$)-alkoxy,
$R^2$ représente phényle,
où phényle est substitué par 1 ou 2 substituants, les substituants étant sélectionnés indépendamment l'un de l'autre parmi le groupe constitué par halogène, hydroxy, cyano, nitro, trifluorométhyle, trifluorométhoxy, trifluorométhylthio, ($C_1$-$C_4$)-alkyl et ($C_1$-$C_4$)-alkoxy,
dans laquelle
($C_1$-$C_4$)-alkoxy peut à son tour être substitué une à trois fois, de façon égale ou différente, par des résidus sélectionnés parmi le groupe constitué par halogène, cyano, hydroxy, ($C_1$-$C_4$)-alkoxy, amino, mono-($C_1$-$C_4$)-alkylamino, di-($C_1$-$C_4$)-alkylamino, ($C_3$-$C_7$)-cycloalkyle et hétérocycle ayant 4 à 7 membres,
dans laquelle lesdits résidus de cycloalkyle et d'hétérocycle peuvent à leur tour être substitués une à trois fois, de façon égale ou différente, par halogène, cyano, ($C_1$-$C_4$)-alkyle, trifluorométhyl, hydroxy, ($C_1$-$C_4$)-alkoxy, trifluorométhoxy, oxo, amino, mono -($C_1$-$C_4$)-alkylamino et di-($C_1$-$C_4$)-alkylamino,

ou un de leurs sels, solvats ou les solvats de leurs sels.

**3.** Composé selon la revendication 1 ou 2, **caractérisé en ce que**

$R^1$ représente phényle,
où phényle est substitué par 1 ou 2 substituants, les substituants étant sélectionnés indépendamment l'un de l'autre parmi le groupe constitué par halogène, cyano, trifluorométhyle et méthoxy,
$R^2$ représente phényle,
où phényle est substitué par 1 ou 2 substituants, les substituants étant sélectionnés indépendamment l'un de l'autre parmi le groupe constitué par halogène, cyano, trifluorométhoxy, méthyle et $(C_1\text{-}C_4)$-alkoxy,
dans laquelle
$(C_1\text{-}C_3)$-alkoxy peut lui-même être substitué une à trois fois, de façon égale ou différente, par des résidus sélectionnés parmi le groupe constitué par hydroxy, $(C_1\text{-}C_4)$-alkoxy, amino, mono-$(C_1\text{-}C_4)$-alkylamino, di-$(C_1\text{-}C_4)$-alkylamino et hétérocycle ayant 4 à 7 membres,
où lesdits résidus d'hétérocycle peuvent à leur tour être substitués dans chaque cas par (C1-C4)-alkyl,

ou un de leurs sels, solvats ou les solvats de leurs sels.

**4.** Composé selon l'une quelconque des revendications 1 à 3, **caractérisé en ce que**

$R^1$ représente phényle,
où phényle est substitué par 1 ou 2 substituants, les substituants étant sélectionnés indépendamment l'un de l'autre parmi le groupe constitué par halogène, cyano, trifluorométhyle et méthoxy,
$R^2$ représente phényle,
où phényle est substitué par 1 ou 2 substituants, les substituants étant sélectionnés indépendamment l'un de l'autre parmi le groupe constitué par halogène, cyano, trifluorométhoxy, méthyle et méthoxy,

ou un de leurs sels, solvats ou les solvats de leurs sels.

**5.** Composé selon l'une quelconque des revendications 1 à 4, **caractérisé en ce que**

$R^1$ représente phényle,
où phényle est substitué par 1 ou 2 substituants, les substituants étant sélectionnés indépendamment l'un de l'autre parmi le groupe constitué par halogène et cyano,
$R^2$ représente phényle,
où phényle est substitué par 1 ou 2 substituants, les substituants étant sélectionnés indépendamment l'un de l'autre parmi le groupe constitué par halogène et cyano,

ou un de leurs sels, solvats ou les solvats de leurs sels.

**6.** Composé selon la revendication 1, **caractérisé en ce qu'**il correspond à la formule

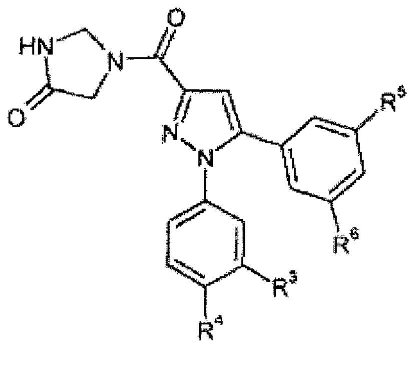

(Ia),

dans laquelle

$R^3$ représente hydrogène, halogène, hydroxy, cyano, nitro, trifluorométhyle, trifluorométhoxy, trifluorométhylthio,

($C_1$-$C_4$)-alkyle ou ($C_1$-$C_4$)-alkoxy,
$R^4$ représente hydrogène ou halogène,
$R^5$ représente halogène, hydroxy, cyano, nitro, trifluorométhyle, trifluorométhoxy, trifluorométhylthio, ($C_1$-$C_4$)-alkyle ou ($C_1$-$C_4$)-alkoxy,
dans laquelle
($C_1$-$C_4$)-alkoxy peut à son tour être substitué une à trois fois, de façon égale ou différente, par des résidus sélectionnés parmi le groupe constitué par halogène, cyano, hydroxy, ($C_1$-$C_4$)-alkoxy, amino, mono-($C_1$-$C_4$)-alkylamino, di-($C_1$-$C_4$)-alkylamino, ($C_3$-$C_7$)-cycloalkyle et hétérocycle ayant 4 à 7 membres,
dans laquelle lesdits résidus de cycloalkyle et d'hétérocycle peuvent à leur tour être substitués une à trois fois, de façon égale ou différente, par halogène, cyano, ($C_1$-$C_4$)-alkyle, trifluorométhyl, hydroxy, ($C_1$-$C_4$)-alkoxy, trifluorométhoxy, oxo, amino, mono-($C_1$-$C_4$)-alkylamino et di-($C_1$-$C_4$)-alkylamino,
$R^6$ représente hydrogène ou halogène,

ou un de leurs sels, solvats ou les solvats de leurs sels.

**7.** Composé selon la revendication 6, **caractérisé en ce que**

$R^3$ représente halogène, hydroxy, cyano, nitro, trifluorométhyle, trifluorométhoxy, trifluorométhylthio, ($C_1$-$C_4$)-alkyle ou ($C_1$-$C_4$)-alkoxy,
$R^4$ représente hydrogène ou halogène,
$R^5$ représente halogène, hydroxy, cyano, nitro, trifluorométhoxy, trifluorométhylthio, ($C_1$-$C_4$)-alkyle ou ($C_1$-$C_4$)-atkoxy,
dans laquelle
($C_1$-$C_4$)-alkoxy peut lui-même être substitué une à trois fois, de façon égale ou différente, par des résidus sélectionnés parmi le groupe constitué par halogène, cyano, hydroxy, ($C_1$-$C_4$)-alkoxy, amino, mono-($C_1$-$C_4$)-alkytamino, di-($C_1$-$C_4$)-alkylamino, ($C_3$-$C_7$)-cycloalkyle et hétérocycle ayant 4 à 7 membres,
dans laquelle lesdits résidus de cycloalkyle et d'hétérocycle peuvent à leur tour être substitués une à trois fois, de façon égale ou différente, par halogène, cyano, ($C_1$-$C_4$)-alkyle, trifluorométhyl, hydroxy, ($C_1$-$C_4$)-alkoxy, trifluorométhoxy, oxo, amino, mono-($C_1$-$C_4$)-alkylamino et di-($C_1$-$C_4$)-alkylamino,

$R^6$ représente hydrogène ou halogène,

ou un de leurs sels, solvats ou les solvats de leurs sels.

**8.** Composé selon la revendication 1 ou 2, **caractérisé en ce que**

$R^3$ représente halogène, cyano, trifluorométhyle ou méthoxy,
$R^4$ représente hydrogène ou halogène,
$R^5$ représente halogène, cyano, trifluorométhoxy, méthyl ou ($C_1$-$C_3$)-alkoxy,
dans laquelle
($C_1$-$C_3$)-alkoxy peut lui-même être substitué une à trois fois, de façon égale ou différente, par des résidus sélectionnés parmi le groupe constitué par hydroxy, ($C_1$-$C_4$)-alkoxy, amino, mono-($C_1$-$C_4$)-alkylamino, di-($C_1$-$C_4$)-alkylamino et hétérocycle ayant 4 à 7 membres,
où lesdits résidus d'hétérocycle peuvent à leur tour être substitués dans chaque cas par ($C_1$-$C_4$)-alkyl,

$R^6$ représente hydrogène ou halogène,

ou un de leurs sels, solvats ou les solvats de leurs sels.

**9.** Composé selon l'une quelconque des revendications 6 à 8, **caractérisé en ce que**

$R^3$ représente halogène, cyano, trifluorométhyle ou méthoxy,
$R^4$ représente hydrogène, chlore ou fluor,
$R^5$ représente halogène, cyano, trifluorométhoxy, méthyl ou méthoxy,
$R^6$ représente hydrogène, chlore ou fluor,

ou un de leurs sels, solvats ou les solvats de leurs sels.

**10.** Composé selon l'une quelconque des revendications 6 à 9, **caractérisé en ce que**

$R^3$ représente hydrogène ou cyano,
$R^4$ représente hydrogène ou fluor,
$R^5$ représente halogène ou cyano,
$R^6$ représente hydrogène ou fluor,

ou un de leurs sels, solvats ou les solvats de leurs sels.

**11.** Composé selon l'une quelconque des revendications 6 à 10, **caractérisé en ce que**

$R^3$ représente chlore ou cyano,
$R^4$ représente fluor,
$R^5$ représente chlore ou cyano,
$R^6$ représente fluor,

ou un de leurs sels, solvats ou les solvats de leurs sels.

**12.** Composé selon l'une quelconque des revendications 6 à 10, **caractérisé en ce que**

$R^3$ représente chlore ou cyano,
$R^4$ représente fluor,
$R^5$ représente chlore ou cyano,
$R^6$ représente hydrogène,

ou un de leurs sels, solvats ou les solvats de leurs sels.

**13.** Composé selon l'une quelconque des revendications 6 à 10, **caractérisé en ce que**

$R^3$ représente chlore ou cyano,
$R^4$ représente hydrogène,
$R^5$ représente chlore ou cyano,
$R^6$ représente hydrogène,

ou un de leurs sels, solvats ou les solvats de leurs sels.

**14.** Composé selon l'une quelconque des revendications 6 à 10, **caractérisé en ce que**

$R^3$ représente chlore ou cyano,
$R^4$ représente hydrogène,
$R^5$ représente chlore ou cyano,
$R^6$ représente fluor,

ou un de leurs sels, solvats ou les solvats de leurs sels.

**15.** Composé selon la revendication 6, **caractérisé en ce que**

$R^3$ représente halogène, cyano, trifluorométhyle ou méthoxy,
$R^4$ représente hydrogène ou halogène,
$R^6$ représente trifluorométhyle,
$R^6$ représente fluor,

ou un de leurs sels, solvats ou les solvats de leurs sels.

**16.** Composé selon la revendication 6, **caractérisé en ce que**

$R^3$ représente hydrogène,
$R^4$ représente fluor ou chlore,

$R^5$ représente halogène, cyano, trifluorométhoxy, méthyl ou méthoxy,
$R^6$ représente hydrogène ou halogène,

ou un de leurs sels, solvats ou les solvats de leurs sels.

**17.** Procédé de production d'un composé de la formule (I) selon la revendication 1, selon lequel un composé de la formule suivante,

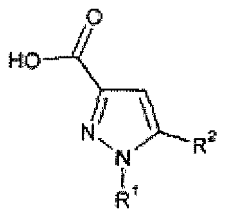

(II),

dans laquelle
$R^1$ et $R^2$ ont la signification spécifiée dans la revendication 1,
est réalisée à l'aide de l'imidazolidine-4 ou d'un sel de l'imidazolidine-4.

**18.** Composé selon l'une quelconque des revendications 1 à 16 destiné au traitement et/ou à la prévention de maladies.

**19.** Utilisation d'un composé selon l'une quelconque des revendications 1 à 16 pour produire un médicament destiné au traitement et/ou la prévention de maladies.

**20.** Utilisation d'un composé selon l'une quelconque des revendications 1 à 16 pour produire un médicament destiné au traitement et/ou à la prévention de maladies rétrovirales.

**21.** Utilisation d'un composé selon la revendication 20, **caractérisée en ce que** la maladie rétrovirale est une infection au VIH.

**22.** Médicament contenant au moins un composé selon l'une quelconque des revendications 1 à 16 en combinaison avec au moins une substance active supplémentaire.

**23.** Médicament contenant au moins un composé selon l'une quelconque des revendications 1 à 16 en combinaison avec au moins un excipient inerte, non toxique, pharmaceutiquement acceptable.

**24.** Médicament selon la revendication 22 ou 23 destiné au traitement et/ou à la prévention de maladies rétrovirales.

**25.** Médicament selon la revendication 24, **caractérisé en ce que** la maladie rétrovirale est une infection au VIH.

**26.** Quantité ayant un effet antiviral, d'au moins un composé selon l'une quelconque des revendications 1 à 16 ou un médicament selon l'une quelconque des revendications 22 à 25 destinée à être utilisée dans une méthode de lutter contre les maladies virales chez les humains et les animaux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5624941 A
- EP 576357 A
- EP 418845 A
- EP 554829 A
- WO 04050632 A
- WO 03037274 A
- WO 06015860 A
- EP 1762568 A
- EP 1591443 A
- WO 07002559 A
- WO 07020388 A
- WO 05080343 A
- WO 07009701 A
- EP 1743637 A
- WO 2005002576 A
- DE 102004054666
- US 2005054707 A1
- WO 2004024147 A
- WO 02100853 A
- WO 2004031178 A
- GB 453061 A
- US 2003229096 A1


### In der Beschreibung aufgeführte Nicht-Patentliteratur


- **PALELLA et al.** *N. Engl. J. Med.,* 1998, vol. 238, 853-860
- Übersicht in Flexner. *Nature Reviews Drug Discovery,* 2007, vol. 6, 959-966
- **KLASSEN ; CARPENTER et al.** highly active antiretroviral therapy = HAART. *J. Am. Med. Assoc.,* 2000, vol. 283, 381-390
- **FINZI et al.** *Nature Med.,* 1999, vol. 5, 512-517
- **RAMRATNAM et al.** *Nature Med.,* 2000, vol. 6, 82-85
- **KAVLICK ; MITSUYA.** Antiretroviral Chemotherapy. ASM Press, 2001, 279-312
- **HOUSTON JB.** Utility of in-vitro drug-metabolism data in predicting in-vivo metabolic-clearance. *Bioch. Pharm.,* 1994, vol. 47 (9), 1469-1479
- **OBACH RS ; BAXTER JG ; LISTON TE ; SILBER BM ; JONES BC ; MACINTYRE F ; RANCE DJ ; WASTALL P.** The prediction of human pharmacokinetic parameters from preclinical and in vitro metabolism data. *J. Pharmacol. Exp. Ther.,* 1997, vol. 283 (1), 46-58
- **PANG KS ; ROWLAND M.** Hepatic clearance of drugs. I. Theoretical considerations of a ''well-stirred'' model and a ''parallel tube'' model. Influence of hepatic blood flow, plasma and blood cell binding, and the hepatocellular enzymatic activity on hepatic drug clearance. *J Pharmacokinet Biopharm,* 1977, vol. 5 (6), 625-53